(19) Europäisches Patentamt
European Patent Office
Office européen des brevets

(11) EP 0 405 537 B1

(12) **EUROPEAN PATENT SPECIFICATION**

(45) Date of publication and mention of the grant of the patent:
**08.09.2004 Bulletin 2004/37**

(51) Int Cl.7: **C07D 209/20**, C07D 403/12, A61K 31/405

(21) Application number: **90112333.1**

(22) Date of filing: **28.06.1990**

(54) **N-substituted cycloalkyl and polycycloalkyl alpha-substituted Trp-Phe- and phenethylamine derivatives**

N-substituierte Cycloalkyl- und Polycycloalkyl-alpha-substituierte Trp-Phe- und Phenethylaminderivate

Dérivés N-substitués cycloalkyl et polycycloalkyl de Trp-phe- et phénéthylamine alpha-substituées

(84) Designated Contracting States:
**AT BE CH DE DK ES FR GB GR IT LI LU NL SE**

(30) Priority: **29.06.1989 US 374327**
**16.10.1989 US 422486**
**05.06.1990 US 530811**

(43) Date of publication of application:
**02.01.1991 Bulletin 1991/01**

(73) Proprietor: **Warner-Lambert Company LLC**
**Morris Plains, New Jersey 07950 (US)**

(72) Inventors:
- **Horwell, David Christopher**
  **Foxton, Cambridge (GB)**
- **Pritchard, Martyn Clive**
  **Swavesey, Cambridge, CB4 5RJ (GB)**
- **Richardson, Reginald Stewart**
  **Haverhill, Suffolk CB9 0LV (GB)**
- **Roberts, Edward**
  **Wood Ditton, Newmarket CB8 9SQ (GB)**
- **Aranda, Julian**
  **D-7801 Vörstetten (DE)**

(74) Representative: **Mansmann, Ivo**
**Gödecke AG**
**Patentwesen**
**Mooswaldallee 1-9**
**79090 Freiburg (DE)**

(56) References cited:
**EP-A- 0 106 281**
**EP-A- 0 224 151**
**EP-A- 0 230 151**

Remarks:
Consolidated with 90911185.8/0479910 (European application No./publication No.) by decision dated 03.07.92.

EP 0 405 537 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

BACKGROUND OF THE INVENTION

Agents acting at central cholecystokinin (CCK) receptors induce satiety (Schick, Yaksh and Go, Regulatory Peptides 14:277-291, 1986). They are also expected to act as analgesics (Hill, Hughes and Pittaway, Neuropharmacology 26:289-300, 1987), and as anticonvulsants (MacVicar, Kerrin and Davison, Brain Research, 406:130-135, 1987).

Reduced levels of CCK-peptides have been found in the brains of schizophrenic patients compared with controls (Roberts, Ferrier, Lee, Crow, Johnstone, Owens, Bacarese-Hamilton, McGregor, O'Shaughnessey, Polak and Bloom. Brain Research 288, 199-211, 1983). It has been proposed that changes in the activity of CCK neurones projecting to the nucleus accumbens may play a role in schizophrenic processes by influencing dopaminergic function (Totterdell and Smith, Neuroscience 19, 181-192, 1986). This is consistent with numerous reports that CCK peptides modulate dopaminergic function in the basal ganglia and particularly the nucleus accumbens (Weiss, Tanzer, and Ettenberg, Pharmacology, Biochemistry and Behaviour 30, 309-317, 1988; Schneider, Allpert and Iversen, Peptides 4, 749-753, 1983). It may therefore be expected that agents modifying CCK receptor activity may have therapeutic value in conditions associated with disturbed function of central dopaminergic function such as schizophrenia and Parkinson's disease.

CCK and gastrin peptides share a common carboxy terminal pentapeptide sequence and CCK peptides can bind to the gastrin receptor of the stomach mucosa and elicit acid secretion in many species including human (Konturek, Gastrointestinal Hormones, Ch. 23, pp 529-564, 1980, ed. G. B. J. Glass, Raven Press, NY). Antagonists of the CCK-B receptor would also be expected to be antagonists at the stomach gastrin receptor and this would also be of value for conditions involving excessive acid secretion.

CCK and gastrin peptides have trophic effects on the pancreas and various tissues of the gastrointestinal tract (Johnson, ibid., pp 507-527), actions which are associated with increased DNA and RNA synthesis. Moreover, gastrin secreting cells are associated with certain gastrointestinal tumors as in the Zollinger-Ellison syndrome (Stadil, ibid., pp 279-739), and some colorectal tumors may also be gastrin/CCK dependent (Singh, Walker, Townsend and Thompson, Cancer Research, 46, 1612 (1986), and Smith, J.P., Gastroenterology, 95 1541 (1988)). Antagonists of CCK/gastrin receptors could therefore be of therapeutic value as antitumor agents.

The CCK peptides are widely distributed in various organs of the body including the gastrointestinal tract, endocrine glands, and the nerves of the peripheral and central nervous systems. Various biologically active forms have been identified including a 33-amino acid hormone and various carboxyl-terminus fragments of this peptide (e.g., the octapeptide CCK26-33 and the tetrapeptide CCK30-33). (G. J. Dockray, Br. Med. Bull., 38 (No. 3):253-258, 1982).

The various CCK peptides are thought to be involved in the control of smooth muscle contractility, exocrine and endocrine gland secretion, sensory nerve transmission, and numerous brain functions. Administration of the native peptides cause gall bladder contraction, amylase secretion, excitation of central neurons, inhibition of feeding, anticonvulsive actions and other behavioral effects. ("Cholecystokinin: Isolation, Structure and Functions," G. B. J. Glass, Ed., Raven Press, New York, 1980, pp 169-221; J. E. Morley, Life Sciences 27:355-368, 1980; "Cholecystokinin in the Nervous System," J. de Belleroche and G. J. Dockray, Ed., Ellis Horwood, Chichester, England, 1984, pp 110-127.)

The high concentrations of CCK peptides in many brain areas also indicate major brain functions for these peptides (G. J. Dockray, Br. Med. Bull., 38 (No. 3):253-258, 1982). The most abundant form of brain CCK found is CCK26-33, although small quantities of CCK30-33 exist (Rehfeld and Gotterman, J. Neurochem., 32:1339-1341, 1979). The role of central nervous system CCK is not known with certainty, but it has been implicated in the control of feeding (Della-Fera and Baile, Science 206:471-473, 1979).

Currently available appetite suppressant drugs either act peripherally, by increasing energy expenditure (such as thyroxine), or in some other manner (such as the biguanides), or act by exerting a central effect on appetite or satiety.

Centrally acting appetite suppressants either potentiate central catecholamine pathways and tend to be stimulants (for example, amphetamine), or influence serotonergic pathways (for example, fenfluramine). Other forms of drug therapy include bulking agents which act by filling the stomach, thereby inducing a "feeling" of satiety.

CCK is known to be present in some cortical interneurones which also contain gamma-aminobutyric acid (GABA) (H. Demeulemeester et al, J Neuroscience 8, 988-1000, 1988). Agents that modify GABA action may have utility as anxiolytic or hypnotic agents (S. C. Harvey, The Pharmacological Basis of Therapeutics (7th ed.) 1985, pp 339-371, MacMillan). Thus, agents which modify CCK action may have parallel anxiolytic or hypnotic activities.

SUMMARY OF THE INVENTION

The invention relates to novel compounds of the formula

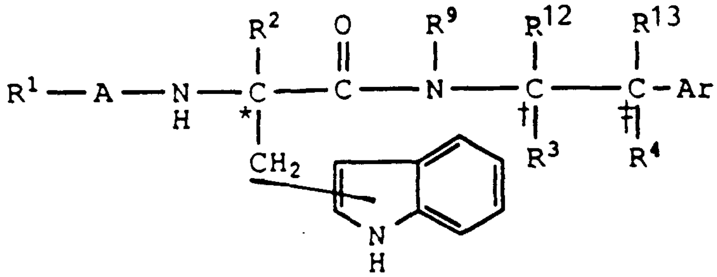

I

and the pharmaceutically acceptable salts thereof wherein $R^1$, $R^2$, $R^3$, $R^4$, $R^9$, $R^{12}$, $R^{13}$, A and Ar are as defined hereinbelow.

The invention also relates to a pharmaceutical composition containing an effective amount of a compound according to formula I in combination with a pharmaceutically acceptable carrier in unit dosage form effective for appetite suppression.

The compounds are also useful as anxiolytics, antipsychotics, especially for treating schizophrenic behavior, as agents in treating disorders of the extrapyramidal motor system, as agents for blocking the trophic and growth stimulating actions of CCK and gastrin, and as agents for treating gastrointestinal motility.

Compounds of the invention are also useful as analgesics and potentiate the effect of morphine. They can be used as an adjunct to morphine and other opioids in the treatment of severe pain such as cancer pain and reduce the dose of morphine in treatment of pain where morphine is contraindicated.

An additional use for compounds such as the iodinated compound of Example 26 is that the suitable radiolabelled iodine-127 isotope gives an agent suitable for treatment of gastrin dependent tumors such as those found in colonic cancers. I-125 radiolabelled compound of Example 26 can also be used as a diagnostic agent by localization of gastrin and CCK-B receptors in both peripheral and central tissue.

The invention further relates to the use of the compounds of formula I for the preparation of a pharmaceutical composition useful in appetite suppression in mammals.

The invention also relates to a pharmaceutical composition for reducing gastric acid secretion containing an effective amount of a compound of formula I in combination with a pharmaceutically acceptable carrier in unit dosage form effective for reducing gastric acid secretion.

The invention further relates to the use of the compounds of formula I for the preparation of a pharmaceutical composition useful in reducing gastric acid secretion.

The invention also relates to a pharmaceutical composition containing an effective amount of a compound of formula I in combination with a pharmaceutically acceptable carrier in unit dosage form effective for reducing anxiety.

The invention further relates to the use of the compounds of formula I for the preparation of a pharmaceutical composition useful in reducing anxiety in mammals.

The invention also relates to a pharmaceutical composition containing an effective amount of a compound of formula I in combination with a pharmaceutically acceptable carrier in unit dosage form effective for treating gastrointestinal ulcers.

The invention further relates to the use of the compounds of formula I for the preparation of a pharmaceutical composition useful in treating gastrointestinal ulcers in mammals.

The invention also relates to a pharmaceutical composition containing an effective amount of a compound of formula I in combination with a pharmaceutically acceptable carrier in unit dosage form effective for treating psychosis, i.e., schizophrenia.

The invention further relates to the use of the compounds of formula I for the preparation of a pharmaceutical composition useful in treating psychosis in mammals.

The invention also relates to pharmaceutical compositions effective for stimulating or blocking CCK or gastrin receptors, for altering the activity of brain neurons, for schizophrenia, for treating disorders of the extrapyramidal motor system, for blocking the trophic and growth stimulating actions of CCK and gastrin, and for treating gastrointestinal motility.

The invention also relates to a pharmaceutical composition for preventing the withdrawal response produced by chronic treatment or abuse of drugs or alcohol.

The invention further relates to the use of the compounds of formula I for the preparation of a pharmaceutical composition useful in treating the withdrawal response produced by withdrawal from chronic treatment or withdrawal from abuse of drugs or alcohol. Such drugs include benzodiazepines, especially diazepam, cocaine, alcohol, and

nicotine. Withdrawal symptoms are treated by administration of an effective withdrawal treating amount of a compound of the instant invention; especially useful are compounds (20) and (20A).

The invention further relates to the use of the compounds of formula I to prepare pharmaceutical and diagnostic compositions for the treatment and diagnosis of the conditions described above.

The invention further provides processes for the preparation of compounds of formula I.

The invention further provides novel intermediates useful in the preparation of compounds of formula I and also provides processes for the preparation of the intermediates.

BRIEF DESCRIPTION OF THE DRAWINGS

Fig. 1 shows inhibition of pentagastrin stimulated gastric acid secretion on the Ghosh and Schild by compound 20.
Fig. 2 shows anxiolytic activity of compound 20 dosed orally in the light/dark exploration test in the mouse.
Fig. 3 shows antipsychotic activity of compound 20A by antagonism of intra-accumbens-dosed amphetamine.
Fig. 4 shows antipsychotic activity of compound 20 by antagonism of intra-accumbens-dosed amphetamine.
Fig. 5 shows the effect of long-term treatment and withdrawal from nicotine; intervention with compound 20.
Fig. 6 shows the effect of long-term treatment and withdrawal from nicotine; intervention with compound 20A.
Fig. 7 shows the effect of long-term treatment and withdrawal from diazepam; intervention with compound 20.
Fig. 8 shows the effect of long-term treatment and withdrawal from diazepam; intervention with compound 20A.
Fig. 9 shows the effect of long-term treatment and withdrawal from alcohol; intervention with compound 20.
Fig. 10 shows the effect of long-term treatment and withdrawal from alcohol; intervention with compound 20A.
Fig. 11 shows the effect of long-term treatment and withdrawal from cocaine; intervention with compound 20.
Fig. 12 shows the effect of long-term treatment and withdrawal from cocaine; intervention with compound 20A.
Fig. 13 shows the effect of compound 20 in the Rat Social Interaction Test for antianxiety agents.
Fig. 14 shows the effect of compound 20 in the Rat Elevated X-Maze (+ Maze) Test for antianxiety agents.
Fig. 15 shows the effects of five compounds of the instant invention as compared to the vehicle and to compound 20 in the Rat Elevated X-Maze Test for antianxiety agents.
Fig. 16 shows that compound 20 depresses the flexor response in a stimulated spinalized decerebrated rat preparation similar to morphine. The effect (lower diagram) of giving compound 20 with morphine greatly potentiates the effect which lasts for 3 hours.

DETAILED DESCRIPTION

The compounds of the present invention are formed by the condensation of two modified amino acids and are therefore not peptides. Rather they are "dipeptoids", synthetic peptide-related compounds differing from natural dipeptides in that the substituent group $R^2$ is not hydrogen.

The compounds of the present invention are represented by the formula

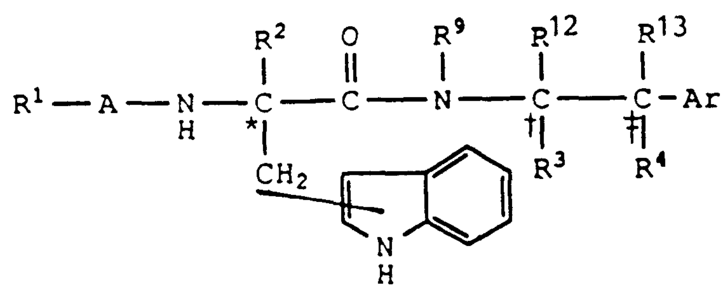

I

or a pharmaceutically acceptable salt thereof wherein:

$R^1$ is a cyclo- or polycycloalkyl hydrocarbon of from three to twelve carbon atoms with from zero to four substituents, each independently selected from the group consisting of: a straight or branched alkyl of from one to six carbon atoms, halogen, CN, OR*, SR*, $CO_2R$*, $CF_3$, $NR^5R^6$, and $-(CH_2)_nOR^5$, wherein R* is hydrogen, straight or branched alkyl of from one to six carbon atoms, $R^5$ and $R^6$ are each independently hydrogen or alkyl of from one to six

carbon atoms; and n is an integer from zero to six;

A is $-(CH_2)_nCO-$,$-SO_2-$, -SO-, -NHCO-,

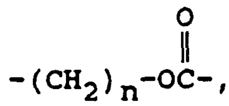

-SCO- -O-(CH$_2$>$_n$CO- or -HC=CHCO- wherein n is an integer from zero to six;

$R^2$ is a straight or branched alkyl of from one to six carbon atoms, $-HC=CH_2$, $-C\equiv CH$, $-CH_2-CH=CH_2$, $-CH_2C\equiv CH$, $-CH_2Ar$, $-CH_2OR^*$, $-CH_2OAr$, $-(CH_2)_nCO_2R^*$, $-(CH_2)_nNR^5R^6$ wherein n, $R^*$, $R^5$ and $R^6$ are as defined above and Ar is as defined below;

$R^3$ and $R^4$ are each independently selected from hydrogen, $R^2$, and $-(CH_2)_{n'}-B-D$, wherein

n' is an integer of from zero to three;

B is a bond

$-OCO(CH_2)_n-$

$-O(CH_2)_n-$

$-NHCO(CH_2)_n-$

$-CONH(CH_2)_n-$

-NHCOCH=CH-

$-COO(CH_2)_n-$

$-CO(CH_2)_n-$

$-S-(CH_2)_n-$

$-SO(CH_2)_n-$

$-SO_2(CH_2)_n-$

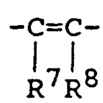

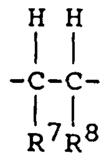

wherein $R^7$ and $R^8$ are independently selected from hydrogen and $R^2$ or together form a ring $(CH_2)_m$ wherein m is an integer of from 1 to 5 and n is as defined above;
D is

-COOR$^*$,

-CONR$^5$R$^6$,

-CN,

-NR$^5$R$^6$,

-OH,

-H, and acid replacements selected from

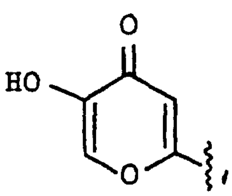,

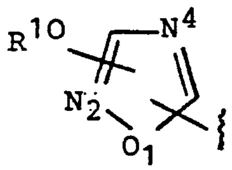

1,2,4oxadiazole

$R^{10}$ is OH, $NH_2$, $CH_3$ or Cl

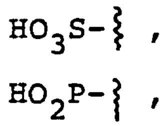

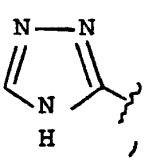, 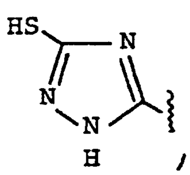, 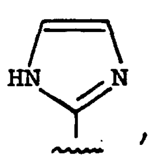, 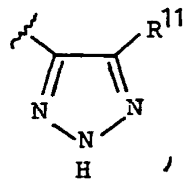,

$R^{11}$ is CN, $CO_2H$, $CF_3$,

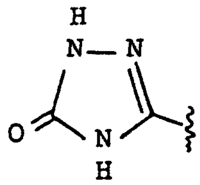,

$PhSO_2NHCO-$,

$CF_3CONHCO-$,

$CF_3SO_2NHCO-$,

$H_2NSO-$,

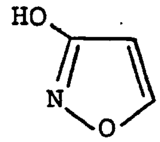,

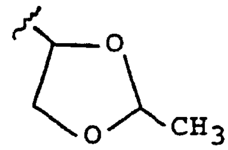,

$-CH_2OR^*$,

$-CHR^2OR^*$,

$-CH_2SR^*$,

$-CHR^2SR^*$,

wherein R*, $R^2$, $R^5$, and $R^6$ are as defined above; $R^9$ is H, or a straight or branched alkyl of from one to six carbon atoms, $-(CH_2)_nCO_2R^*$, $(CH_2)_nOAr'$, $(CH_2)_nAr'$ or $(CH_2)_nNR^5R^6$ , wherein n, R*, $R^5$, and $R^6$ are as defined above or taken from $R^3$ and Ar' is taken from Ar as defined below;
$R^{12}$ and $R^{13}$ can each be independently hydrogen (in which case the carbon atom to which it is attached is a chiral center) or can each be taken with $R^3$ and $R^4$ respectively to form a moiety doubly bonded to the carbon atom (in which case the carbon atom is not chiral); and
Ar is 2 or 3-thienyl, 2 or 3-furanyl, 2, 3 or 4-pyridinyl or an unsubstituted or substituted phenyl

whose substituents if any are each independently hydrogen, fluorine, chlorine, bromine, iodine, methyl, methoxy, trifluoromethyl or nitro.

Preferred cycloalkyl or polycycloalkyl substituents have from six to ten carbon atoms.

Preferred compounds of the instant invention are those wherein cycloalkyl is a substituted or unsubstituted

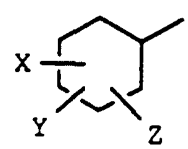

and wherein polycycloalkyl is selected from

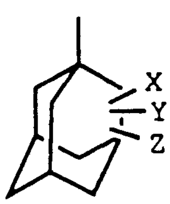,

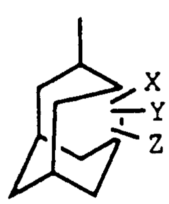,

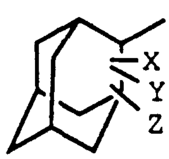,

and

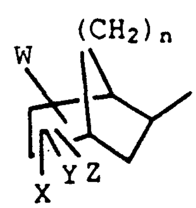

wherein W, X, Y, and Z are each independently hydrogen, a straight or branched alkyl of from one to six carbon atoms, $CF_3$, $NR^5R^6$, $-(CH_2)_nCO_2R^*$, or CN, F, Cl, Br, OR*, SR*, wherein R* is hydrogen or a straight or branched alkyl of from one to six carbon atoms and $R^5$ and $R^6$ are as defined above and n is an integer of from 1 to 3.

Other preferred compounds of the instant invention are those wherein

$R^1$ is 2-adamantyl or 1-(S)-2-endobornyl;

A is -NHCO-, -OCO-, $-SO_2-$, -S(=O)- or $-CH_2CO-$;

$R^2$ is $-CH_3$, $-CH_2CO_2CH_3$ or $-CH_2C{\equiv}CH$;

$R^3$ is $-CH_2$-B-D or H;

$R^4$ is $-(CH_2)_{n'}$-B-D or H; and

$R^9$ is hydrogen or methyl.

More preferred compounds of the instant invention are those wherein

$R^1$ is 2-adamantyl or 1-(S)-2-endobornyl,

A is

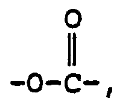

$R^2$ is $-CH_3$;

$R^3$ is H, $-CH_2OH$, $-CH_2OCOCH_2CH_2CO_2H$, $-CH_2OCOCH{=}CHCO_2H$ or $-CH_2NHCOCH_2CH_2Co_2H$, or $-CH_2NHCOCH{=}CHCO_2H$

and $R^4$ is H, $-NHCOCH_2CH_2CO_2H$ ([D] configuration or $-NHCOCH{=}CHCO_2H$ ([D] configuration).

The D and the L configurations are possible at the chiral centers and are included in the scope of the invention:

1. Preferred is when $R^2$ is $-CH_3$[D] configuration;
2. Preferred is when $R^3$ is $-CH_2OCOCH_2CH_2CO_2H$ or $-CH_2NHCOCH_2CH\ _2CO_2H$ with the [D] configuration at the Trp α-carbon atom and the [L] configuration at the Phe-α-carbon atom; and
3. Preferred is when $R^4$ is $-NHCOCH_2CH_2CO_2H$[D] configuration or $NHCOCH{=}CHCO_2H$[D] configuration with the [D] configuration at the Trp α-carbon atom

Most preferred compounds of the instant invention are:

C1. [1S-[1α,2β[S*[S*(E)]],4α]]-4-[[2-[[3-(1H-indol-3-yl)-2-methyl-1-oxo-2-[[[(1,7,7-trimethylbicyclo[2.2.1]hept-2-yl)oxy]carbonyl]amino]propyl]amino]-1-phenylethyl]amino]-4-oxo-2-butenoic acid,

C2. [1S-[1α,2β[S*(S*)],4α]]-4-[[2-[[3-(1H-indol-3-yl)-2-methyl-1-oxo-2-[[[(1,7,7-trimethylbicyclo[2.2.1]hept-2-yl)oxy]carbonyl]amino]propyl]methylamino]-1-phenylethyl]amino]-4-oxobutanoic acid,

C 3. [1S-[1α,2β[S*(S*)],4α]]-4-[[2-[[3-(1H-indol-3-yl)-2-methyl-1-oxo-2-[[[(1,7,7-trimethylbicyclo[2.2.1]hept-2-yl)amino]carbonyl]amino]propyl]amino]-1-phenylethyl]amino]-4-oxobutanoic acid,

C 4. [R-(R*,R*)]-4-[[2-[[3-(1H-indol-3yl)-2-methyl-1-oxo-2--[(tricyclo[3.3.1.$1^{3,7}$]dec-2-ylsulfonyl)amino]propyl]amino]-1-phenylethyl]amino]-4-oxobutanoic acid,

C 5. [R-(R*,S*)]-4-[[2-[[3-(1H-indol-3-yl)-2-methyl-1-oxo-2-[(tricyclo[3.3.1.$1^{3,7}$]dec-2-ylsulfonyl)amino]propyl]amino]-3-phenylpropyl]amino]-4-oxobutanoic acid,

C 6. [1R-[1α[R*(S*)],2β]] and [1S-[1α[S*(R*)],2β]]-4-[[2-[[2-[[[(2-fluorocyclohexyl)oxy]carbonyl]amino]-3-(1H-indol-3-yl)-2-methyl-1-oxopropyl]amino]-3-phenylpropyl]amino]-4-oxobutanoic acid,

C 7. [1R-[1α[R*(S*)],2β]] and [1S-[1α[S*(R*)],2β]]-4-[[2-[[2-[[[(2-fluorocyclohexyl)oxy]carbonyl]amino]-3-(1H-indol-3-yl)-2-methyl-1-oxopropyl]methylamino]-3-phenylpropyl]amino]-4-oxobutanoic acid,

C 8. [1R-[1α[R*(S*)],2β]] and [1S-[1α[S*(R*)],2β]-4-[[2-[[3-(1H-indol-3-yl)-2-methyl-1-oxo-2-[[[[2-(trifluoromethyl)-cyclohexyl]oxy]carbonyl]amino]propyl]amino]-3-phenylpropyl]-amino]-4-oxobutanoic acid,

C9. [1R-[1α[R*(S*)],2β]] and [1S-[1α[S*(R*)],2β]]-4-[[2-[[3-(1H-indol-3-yl)-2-methyl-1-oxo-2-[[[[2-(trifluoromethyl)-cyclohexyl]oxy]carbonyl]amino]propyl]methylamino]-3-phenylpropyl]amino]-4-oxobutanoic acid,

C 10. (R-(R*,S*)]-4-([2-((3-(1H-indol-3-yl)-2-methyl-1-oxo-2-[[(tricyclo[3.3.1.$1^{3,7}$]dec-2-yloxy)carbonyl]-amino]propyl]methylamino]-3-phenylpropyl]amino]-4-oxobutanoic acid,

C 11. [1S-(1α,2β(S *(R*)],4α])-(1-(1H-indol-3-ylmethyl)-1-methyl-2-oxo-2-[[2-[[1-oxo-3-(1H-tetrazol-5-yl)propyl]amino]-1-(phenylmethyl)ethyl]amino]ethyl]carbamic acid, 1,7,7-trimethylbicyclo[2.2.1]hept-2-yl ester,

C 12. [1S-(1α,2β[S*(R*)],4α]]-[1-(1H-indol-3-ylmethyl)-1-methyl-2-oxo-2-[[2-[[1-oxo-3-(1H-tetrazol-5-yl)propyl]amino]-2-phenylethyl]amino]ethyl]carbamic acid, 1,7,7-trimethyl-bicyclo[2.2.1]hept-2-yl ester,

C 13. N-[2-methyl-N-[(tricyclo[3.3.1.$1^{3,7}$]dec-2-yloxy)-carbonyl]-D-tryptophyl]-L-phenylalanylglycine,

C 14. N-[2-methyl-N-[(tricyclo[3.3.1.$1^{3,7}$]dec-2-yloxy)-carbonyl]-D-tryptophyl]-L-phenylalanyl-β-alanine and

C 15. (R)-tricyclo[3.3.1.$1^{3,7}$]dec-2-yl [1-(1H-indol-3-yl-methyl)-1-methyl-2-[methyl(2-phenylethyl)amino]-2-oxo-ethyl]carbamate.

In addition most especially preferred compounds of the instant invention are:

C 16. (±)-Trans-2-chlorocyclohexyl [1-(1H-indol-3-ylmethyl) -1-methyl-2-oxo-2-[(2-phenylethyl)amino]ethyl]carbamate,
C 17. 2-chlorocyclohexyl [2-[[1-(hydroxymethyl)-2-phenylethyl]amino]-1-(1H-indol-3-ylmethyl)-1-methyl-2-oxoethyl]carbamate,
C 18. 2-[[2-[[[(2-chlorocyclohexyl)oxy]carbonyl]amino]-3-(1H-indol-3-yl)-2-methyl-1-oxopropyl]amino]-3-phenylpropyl butanedioate,
C 19. 2-[[2-[[[(2-methylcyclohexyl)oxy]carbonyl]amino]-3-(1H-indol-3-yl)-2-methyl-1-oxopropyl]amino]-3-phenylpropyl butanedioate,
C 20. (±)-tricyclo[3.3.1.1$^{3,7}$]dec-2-yl [1-(1H-indol-3-ylmethyl)-1-methyl-2-oxo-2-[(2-phenylethyl)amino]-ethylcarbamate,
C 21. tricyclo[3.3.1.1$^{3,7}$]dec-2-yl [2-[[1-(hydroxymethyl)-2-phenylethyl]amino]-1-(1H-indol-3-ylmethyl)-1-methyl-2-oxoethyl] carbamate,
C 22. 2-[[3-(1H-indol-3-yl)-2-methyl-1-oxo-2-[[(tricyclo-[3.3.1.1$^{3,7}$]dec-2-yloxy)carbonyl]amino]propyl]amino]-3-phenylpropyl butanedioate,
C23. 2-[[3-(1H-indol-3-yl)-2-methyl-1-oxo-2-[[(tricyclo-[ 3.3.1.1$^{3,7}$]dec-2-yloxy) carbonyl]amino]propyl]amino]-1-phenylethyl butanedioate,
C 24. [R-(R*,R*)]-4-[[2-[[3-(1H-indol-3-yl)-2-methyl-1-oxo-2-[[(tricyclo[3.3.1.1$^{3,7}$]dec-2-yloxy)carbonyl]amino]-propyl]amino]-1-phenylethyl]amino]-4-oxobutanoic acid,
C 25. [1S-[1α,2β[S*(S*)],4α]]-4-[[2-[[3-(1H-indol-3-yl) -2-methyl-1-oxo-2-[[[(1,7,7-trimethylbicyclo-2.2.1]hept-2-yl) oxy]carbonyl]amino]propyl]amino]-1-phenylethyl]amino]-4-oxobutanoic acid,
C 26. [R-[R*,S*-(E)]]-4-[[2-[[3-(1H-indol-3-yl)-2-methyl-1-oxo-2-[[tricyclo[3.3.1.1$^{3,7}$]dec-2-yloxy)carbonyl]amino]-propyl]amino]-3-phenylpropyl]amino]-4-oxo-2-butenoic acid,
C 27. [R-(R*,S*)]-4-[[2-[[3-(1H-indol-3-yl)-2-methyl-1-oxo-2-[ [(tricyclo[3.3.1.1$^{3,7}$]dec-2-yloxy) carbonyl]amino]propyl]-amino]-3-phenylpropyl]amino]-4-oxobutanoic acid,
C 28. (R)-tricyclo[3.3.1.1$^{3,7}$]dec-2-yl [1-(1H-indol-3-ylmethyl)-1-methyl-2-[methyl(2-phenylethyl)amino]-2-oxoethylcarbamate,
C 29. [R-(R*,S*) ]-[[2-[[3-(1H-indol-3-yl) -2-methyl-1-oxo-2-[[(tricyclo[3.3.1.1$^{3,7}$]dec-2-yloxy)]carbonyl]amino]propyl]-amino]-3-phenylpropyl]sulfinyl]acetic acid, ethyl ester,
C 30. [R-(R*,S*)]-[[2-[[3-(1H-indol-3-yl)-2-methyl-1-oxo-2-[[(tricyclo(3.3.1.1$^{3,7}$]dec-2-yloxy)]carbonyl]amino]propyl]-amino]-3-phenylpropyl]sulfonyl]acetic acid, ethyl ester,
C 31. [R-(R*,S*)]-[[2-[[3-(1H-indol-3-yl)-2-methyl-1-oxo-2-[[(tricyclo[3.3.1.1$^{3,7}$]dec-2-yloxy)]carbonyl]amino]propyl]-amino]-3-phenylpropyl]sulfinyl]acetic acid,
C 32. [R-[R*,R*-(E)]]-4-[[2-[[3-(1H-indol-3-yl)-2-methyl-1-oxo-2-[[(tricyclo[3.3.1.1$^{3,7}$]dec-2-yloxy)]carbonyl]amino] propyl]-amino]-1-phenylethyl]amino]-4-oxo-2-butenoic acid,
C 33. [R-(R*,S*)]-[[2-[[2-[3-(1H-indol-3-yl) -2-methyl-1-oxo-2-[[(tricyclo[3.3.1.1$^{3,7}$]dec-2-yloxy)]carbonyl]amino] propyl]-amino]-3-phenylpropyl]thio]acetic acid,
C 34. [1S-[1α,2β[S*[S*(E)]],4α]]-4-[[2-[[3-(1H-indol-3-yl)-2-methyl-1-oxo-2-[[[(1,7,7-trimethylbicyclo-[2.2.1]hept-2-yl)oxy]carbonyl]amino]propyl]amino]-1-phenylethyl]amino]-4-oxo-2-butenoic acid, methyl ester, (Bicyclo system is 1S-endo),
C 35. [1S-[1α,2β[S*[S*(E)]],4α]]-4-[[2-[[3-(1H-indol-3-yl)-2-methyl-1-oxo-2-[[[(1,7,7-trimethylbicyclo-[2.2.1]hept-2-yl)oxy]carbonyl]amino]propyl]amino]-1-phenylethyl]amino]-4-oxo-2-butenoic acid, (Bicyclo system is 1S-endo),
C 36. [R-(R*,R*)]-3-[[2-[[3-(1H-indol-3-yl)-2-methyl-1-oxo-2-[[(tricyclo[3.3.1.1$^{3,7}$]dec-2-yloxy)]carbonyl]amino]propyl)-amino]-1-phenylethyl]amino]-3-oxo-propanoic acid,
C 37. [R-(R*,S*)]-3-(1H-indol-3-ylmethyl)-3-methyl-4,10-dioxo-6-(phenylmethyl)-11-oxo-8-thia-2,5-diazatridecanoic acid, tricyclo(3.3.1.1$^{3,7}$]dec-2-yl or ester,
C 38. [R-(R*,S*)]-β-[[3-(1H-indol-3-yl)-2-methyl-1-oxo-2-[[(tricyclo[3.3.1.1$^{3,7}$]dec-2-yloxy)]carbonyl]amino]propyl]-amino]benzenebutanoic acid,
C 39. [R-(R*,S*)]-N-[3-[[3-(1H-indol-3-yl)-2-methyl-1-oxo-2-[[(tricyclo[3.3.1.1$^{3,7}$]dec-2-yloxy)]carbonyl]amino]propyl]-amino]-4-phenylbutyl]glycine,
C 40. [R-[R*,S*-(E)]]-4-[[2-[[3-(1H-indol-3-yl)-2-methyl-2-[[(bicyclo[3.3.1]non-9-yloxy)carbonyl]amino]-1-oxopropyl]-amino]-3-phenylpropyl]amino]-4-oxo-2-butenoic acid,
C41. mono [R-(R*,R*)]-2-[[3-(1H-indol-3-yl)-2-methyl-1-oxo-2-[[(tricyclo[3.3.1.1$^{3,7}$]dec-2-yloxy)carbonyl]amino]-1-phenylethyl butanedioate,
C42. 3-[[3-[[3-(1H-indol-3-yl)-2-methyl-1-oxo-2-[[(tricyclo-[3.3.1.1$^{3,7}$]dec-2-yloxy)]carbonyl]amino]propyl]amino]-1-oxo-2-phenylpropyl]amino]propanoic acid (TRP is R, other center is RS),
C43. [1R-[1α[R*(S*)],2β]]-4-[[2-[[3-(1H-indol-3-yl)-2-methyl-2-[[[(2-methyl-1-cyclohexyl)oxy]carbonyl]amino]-1-oxopropyl]-amino]-3-phenylpropyl]amino]-4-oxo-2-butenoic acid, (-)-Isomer,
C44. [1R-[1α[R*(S*)],2β]]-4-[[2-[[3-(1H-indol-3-yl)-2-methyl-2-[[[(2-methyl-1-cyclohexyl)oxy]carbonyl]amino]-1-oxopropyl]-amino]-3-phenylpropyl]amino]-4-oxobutanoic acid, (-)-Isomer,
C45. [1R-[1α[R*(S*)],2β]]-4-[[2-[[3-(1H-indol-3-yl)-2-methyl-2-[[[(2-methyl-1-cyclohexyl)oxy]carbonyl]amino]-1-oxopropyl]-amino]-1-phenylethyl]amino]-4-oxo-2-butenoic acid, (-)-Isomer,
C46. [1R-[1α[R*(S*)],2β]]-4-[[2-[[3-(1H-indol-3-yl)-2-methyl-2-[[[(2-methyl-1-cyclohexyl)oxy]carbonyl]amino]-1-oxopropyl]-amino]-1-phenylethyl]amino]-4-oxobutanoic acid, (-)-Isomer,
C47. 2-methylcyclohexyl-[1R-[1α[R*(S*)]],2S]-[2-[[1-(hydroxymethyl)-2-phenylethyl]amino]-1-(1H-indol-3-ylmethyl)-1-methyl-2-oxoethyl]carbamate,
C48. [R-[R*,S*-(E,E)]]-6-[[3-(1H-indol-3-yl)-2-methyl-1-oxo-2-[[(tricyclo-[3.3.1.1$^{3,7}$]dec-2-yloxy)carbonyl]amino] propyl]-amino]-7-phenyl-2,4-heptadienoic acid,
C49. [R-(R*,R*)]-[2-[[2-[[1,4-dioxo-4-(1H-tetrazol-5-ylamino)-butyl]amino]-2-phenylethyl]amino]-1-(1H-indol-3-yl-methyl)-1-methyl-2-oxoethyl]carbamic acid,
C50. tricycl0-[3.3.1.1$^{3,7}$]dec-2-yl-(S-[R*,S*-(E)]]-12-(1H-indol-3-ylmethyl)-12-methyl-3,11-dioxo-9-(phenylmethyl)-2-oxa-7,10,13-triazatetradec-4-en-14-oate,
C51. [R-(R*,S*)]-3-[[2-[[3-(1H-indol-3-yl)-2-methyl-1-oxo-2-[[(tricyclo-[3.3.1.1$^{3,7}$]dec-2-yloxy)carbonyl]amino]propyl]-amino]-3-phenylpropyl]amino]-3-oxopropanoic acid,
C52. ethyl [R-(R*,S*)]-[[2-[[2-[3-(1H-indol-3-yl)-2-methyl-1-oxo-2-[[(tricyclo[3.3.1.1$^{3,7}$]dec-2-yloxy)carbonyl]amino]-propyl]amino]-3-phenylpropyl]thio]acetate,
C53. [R-(R*,S*)]-β-[3-(1H-indol-3-yl)-2-methyl-1-oxo-2-[[tricyclo[3.3.1.1$^{3,7}$]dec-2-yloxy)carbonyl]amino]-propyl] amino]-4-iodo-benzenebutanoic acid,
C54. [R-(R*,R*)]-(2-[[3-(1H-indol-3-yl)-2-methyl-1-oxo-2-[[(1(tricyclo[[(3.3.1.1$^{3,7}$]dec-2-yloxy)carbonyl]amino]-propyl)amino]-1-phenylethoxy]acetic acid,
C55. [[3-[[3-(1H-indol-3-yl)-2-methyl-1-oxo-2-[[tricyclo(3.3.1.1$^{3,7}$]dec-2-yloxy)carbonyl]amino]-propyl]amino]-1-oxo-2-phenylpropyl)amino]acetic acid (TRP center is R, other center is RS),
C56. (R)-[[[2-[[3-(1H-indol-3-yl)-1-oxo-2-methyl-2-[[(tricyclo[3.3.1.1$^{3,7}$]dec-2-yloxy)carbonyl]amino]-propyl]amino]-1-phenylethylidene]amino]oxy]acetic acid,
C57. [R-(R*,S*)]-β-[[3-(1H-indol-3-yl)-2-methyl-1-oxo-2-[[(tricyclo[3.3.1.1$^{3,7}$]dec-2-yloxy)carbonyl]amino]propyl]-amino]benzenebutanoic acid,
C58. [R-(R*,S*)]-N-[3-[[3-(1H-indol-3-yl)-2-methyl-1-oxo-2-[[(tricyclo-[3.3.1.1$^{3,7}$]dec-2-yloxy)carbonyl]propyl]amino]-4-phenylbutyl]glycine,
C 59. 2-[[[2-[[3-(1H-indol-3-yl)-2-methyl-1-oxo-2-[[(tricyclo-[3.3.1.1$^{3,7}$]dec-2-yloxy)carbonyl]amino]propyl]amino]-1-phenylethyl]amino]carbonyl]cyclopropanecarboxylic acid (cyclopropane ring is trans-(±) other centres are R),
C 60. carbamic acid, [1-(1H-indol-3-ylmethyl)-1-methyl-2-oxo-2-[[2-[[1-oxo-3-(1H-tetrazol-5-yl)propyl]amino]-2-phenylethyl]-amino]ethyl)-,tricyclo[3.3.1.1$^{3,7}$]dec-2-yl ester,[R,(R*,S*]-,
C61. benzeneheptanoic acid, α-[[3-(1H-indol-3-yl)-2-methyl-1-oxo-2-[[(tricyclo[3.3.1.1$^{3,7}$]dec-2-yloxy)carbonyl) amino]-propyl]amino]-,[R-(R*,S*)]-,
C 62. methyl-(±)-β-[[(2-phenylethyl)amino]carbonyl]-1β-[[(tricyclo[3.3.1.1$^{3,7}$]dec-2-yloxy)carbonyl]amino]-1H-indole-3-butanoate,
C 63. [R-(R*,S*)]-4-[[2-(1H-indol-3-yl)-2-methyl-1-oxo-2-[ [tricyclo[3.3. 1. 1 $^{3,7}$]dec-2-yloxylcarbonyl]amino]propyl]-amino]-3-phenylpropyl]amino]-4-oxo-2-butenoic acid,
C 64. bicyclo[2.2.1]heptane-2-acetic acid, 3-[[[[2-[[1-(hydroxymethyl)-2-phenylethyl]amino]-1-(1H-indol-3-ylmethyl)-1-methyl-2-oxoethyl]amino]carbonyl]oxy]-4,7,7-trimethyl-,[1R-[1α,2β,3α[R*(S*)],4α]]-,
C 65. butanoic acid, 4-[[2-[[3-(1H-indol-3-yl)-2-methyl-2-[[[(2-methyl-1-cyclohexyl)oxy]carbonyl]amino]-1-oxopropyl]-amino]-1-phenylethyl]amino]-4-oxo-[1R-[1α[R*(R*)]2β]]-((-)-isomer),
C 66. 2-butenoic acid, 4-[[2-[[3-(1H-indol-3-yl)-2-methyl-2-[[[(2-methyl-1-cyclohexyl)oxy]carbonyl]amino]-1-oxopropyl]-amino]-1-phenylethyl]amino]-4-oxo-,[1R-[1α[R*(R*)],2β]]-((-)-isomer),
C67. butanoic acid, 4-[[2-[[3-(1H-indol-3-yl)-2-methyl-2-[[[(2-methyl-1-cyclohexyl)oxy]carbonyl]amino]-1-oxopropyl]-amino]-3-phenylpropyl]amino]-4-oxo-[1R-[1α[R*(S*)],2β]]-((-)-isomer), and
C68. 2-butenoic acid, 4-[[2-[[3-)1H-indol-3-yl)-2-methyl-2-[[[(2-methyl-1-cyclohexyl)oxy]carbonyl]amino]-1-oxopropyl]-amino]-3-phenylpropyl]amino]-4-oxo-[IR[1α[R*(S*)],2β]]-((-)-isomer).

Additionally preferred are the compounds:

C 69. [[3-[[3-(1H-indol-3-yl)-2-methyl-1-oxo-2-[[tric -yclo(3-3.1-1$^{3,7}$]dec-2-yloxy)carbonyl]amino)propyl)amino]-1 -oxo-2-phenylpropyl]amino]acetic acid
(TRP center is R, other center is RS)

C 70. [R-(R*,R*}] 2-([3-{1H-indol-3-yl)-2-methyl-1-oxo-2-([(tric -yclo[3.3.1.1$^{3,7}$]dec-2-yloxy)carbonyl]amIno]propyl]amIno]-1 -phenylethoxy]acetic acid

C 71. [1R-[1α,2β[R*(R*)]]]-2-[[[2-[[3-(1H-Indol-3-yl).2-methyl-1-oxo-2-[[(tricyclo[3.3.1.1$^{3,7}$]dec-2-yloxy)carbonyl]amino]propyl]amino]-1-phenylethyl]amino]carbonyl]cyclopropane carboxylic acid

C 72. [1S-[1α,2β(S*(S*)]]]-2-[[[2-[[3-(1H-indol-3-yl)-2-me thyl-1-oxo-2-[[(tricyclo(3.3.1.1$^{3,7}$]dec-2-yloxy)carbonyl]amino]propyl]amino]-1-phenylethyl]amino]carbonyl]cyclopropane carboxylic acid

C 73. [R-R*,R*)]-3-[2-[[3-(1H-indol-3-yl)-2-methyl-1-oxo-2-[[ (tricyclo[3.3.1.1 $^{3,7}$]dec-2-yloxy)carbonyl]amino)propyl]ami no]-1-phenylethoxy]propanoic acid

C 74. [R-(R*,R*)]-mono 2-[[3-(1H-indol-3-yl)-2-methyl-1-oxo 2-[[(tricyclo(3.3.1.1$^{3,7}$)dec-2-yloxy)carbonyl)amino)-propyl] amino] -1-phenylethyl butanedioic acid

C75. 3-[[3-[[3-(1H-indol-3-yl)-2-mathyl-1-oxo-2-[ [(tricyclo(3.3.1.1 $^{3,7}$]dec-2-yloxy)carbonyl]amino]propyl]am ino]-1-oxo-2-phenylpropyl]amino]propanoic acid
(TRP is R, other center is RS)

C 76 [R-(R*,S*)]-β[[3-(1H-indol-3-yl)-2-methyl-1-oxo -2-[[(tricyclo[3.3.1.1$^{3,7}$]dec-2-yloxy)carbonyl]amino]propyl]amino]-4-iodobenzenebutanoic acid,

C 77. [1R-[1α[R*(S*)],2β]]-4-[[2-[[3-(1H-indol-3-yl)-2-methyl-2-[[[(2-methyl-1-cyclohexyl)oxy]carbonyl]amino]-1-oxopropyl]amino]-3 -phenylpropyl]amino]-4-oxo-2-butenoic acid,
((-)-isomer)

C78 [1R-[1α[R*(S*)],2β]]-4-[[2-[[3-(1H-indol-3-yl)-2-methyl-2-[[[(2-methyl-1-cyclohexyl)oxy]carbonyl]amino]-1-oxopropyl] amino]-3-phenylpropyl]amino]-4-oxobutanoic acid,
((-)-isomer)

C 79. [1R-(1α[R*(R*)],2β]]-4-[[2-[[3-(1H-indol-3-yl)-2-methyl-2-[[[(2-methyl-1-cyclohexyl)oxy]carbonyl]amino]-1-oxopropyl]amino]-1 -phenylethyl]amino]-4-oxo-2-butenoic acid
((-)-isomer)

C 80. 1R-[1α[R*(R*)],2β]]-4-([2-[[3-(1H-indol-3-yl)-2-methyl-2-[[[(2-me thyl-1-cyclohexyl)oxy]carbonyl]amino]-1-oxopropyl]amino]-1-phenylethyl]amino]-4-oxobutanoic acid
((-)-isomer)

C 81. (R-(R*,S*)]-!g/-[[3-(1H-indol-3-yl)-2-methyl-1-ox o-2-[[(tricyclo[3.3.1.1$^{3,7}$]dec-2-yloxy)carbonyl]amino]prop yl]amino]benzeneheptanoic acid

C 82. 2-[[[2-[[3-(1H-indol-3-yl)-2-methy1-1-oxo-2-[((tricyclo(3.3.1.1$^{3,7}$]dec-2-yloxy)carbonyl]amino]propyl]amino]-1-phenylethyl]amino]carbonyl]cyclopropanecarboxylic acid
(cyclopropyl ring is trans-(±), other centers are R)

C 83. 2-methylcyclohexyl [1R-(1α(R*(S*)]],2β]-[2-[(1-(hydroxymethyl)-2-phenylethyl]amino]-1-(1H-1ndol-3-ylmethyl)-1-methyl-2-oxoethyl]-carbamate
((-)-isomer)

C 84. [R-[R*,S*-(E, E)]]-6-[[3-(1H-indol-3-yl)-2-methyl-1-oxo-2-[[(tricyclo[3.3.1.1$^{3,7}$]dec-2-yloxy)carbonyl]amino]propyl ]amino]-7-phenyl-2,4-heptidienoic acid

C 85 tricyclo[3.3.1.1$^{3,7}$]dec-2-yl (2-[[1-(hydroxymethyl)-2-hydroxy -2-phenylethyl]amino]-1-(1H-indol-3-ylmethyl)-1-methyl-2-oxo-ethyl] carbamate

C 86. tricyclo[3.3.1.1$^{3,7}$]dec-2-yl [R-(R*,R*)]-[1-(1H-indol-3-ylmethyl)-1-methyl-2-oxo-2-[[2 -[[1-oxo-3-(1H-tetrazol-5-yl)propyl]amino]-2-phenylethyl]ami no]ethyl]carbamate

C 87. [R-(R*,S*)]-[[2-[[3-(1H-indol-3-yl)-2-methyl-1-oxo-2-[[(t ricyclo[3.3.1 .1 $^{3,7}$]dec-2-yloxy)carbonyl]amino]propyl]amino ]-3-phenylpropyl]sulfinyl]acetic acid

C 88. [R-(R*, S*)]-[[-2-[(3-(1H-indol-3-yl)-2-methyl-1-oxo-2-[[(tri cyclo[3.3.1.1$^{3,7}$-]dec-2-yloxy) carbonyl]amino]propyl]amino] -3-phenylpropyl]sulfonyl]acetic acid

C 89. Ethyl [R-(R*,S*)]-[[2-[[3-(1H-indol-3-yl)-2-methyl-1-oxo-2-[[(tri cyclo[3.3.1.1$^{3,7}$]dec-2-yloxy)]carbonyl]amino] propyl]amino] -3-phenylpropyl]sulfonyl]acetate

C 90. 2-chlorocyclohexyl [2-[[1-(hydroxymethyl)-2-phenylethyl]amino]-1 -(1H-indol-3-ylmethyl)-1-methyl-2-oxoethyl]carbamate
Isomer II
Ring centers are trans, trp center is D, other center is S) ((-) or (+) form)

C 91. [R-[R*,R*(E)]]-4-[[2-[[3-(1H-indol-3-yl)-2-methyl-1-oxo-2-[[(tricyclo[3.3.1.1$^{3,7}$]dec-2-ylamino)carbonyl]amino]propyl ]amino]-1-phenylethyl]amino]-4-oxo-2-butenoic acid

C 92. [R-(R*,R*)]-4-[[2-[[3-(1H-indol-3-yl)-2-methyl-1-oxo-2-[[ (tricyclo[3.3.1.1$^{3,7}$]dec-2-yloxy)carbonyl]amino]propyl]ami no]-1-phenylethyl]amino]-4-oxobutanoic acid

C 93. [R-(R*.S*)]-mono[2-[[3-(1H-indol-3-yl)-2-methyl-1-oxo-2-[[(tricyclo[3.3.1.1$^{3,7}$]dec-2-yloxy)carbonyl]amino] propyl ]amino]-3-phenylpropyl] butanedioate

C 94. tricyclo[3.3.1.1$^{3,7}$]dec-2-y] [R-(R*,S*)-[2-[[1-(hydroxymethy1)-2-phenylethyl]amino]-1 -(1H-indol-3-ylmethyl)-1-methyl-2-oxoethyl]-carbamate

C 95. [1S-[1α,2β[S*[S*(E)]],4α]]-4-[[2-[[3-(1H-indol-3-yl)-2-methyl-1-oxo-2-[[[(1,7,7-trimethylbicyclo[2.2.1]hept-2-yl) oxy]carbonyl]amino]propyl]amino]-1-phenylethyl]amino]-4-oxo -2-Butenoic acid
(Bicyclo system is 1S-endo)

C 96. [1S-[1α,2β[S*(S*)],4α]]-4-[[2-[[3-(1H-indol-3-yl)-2-methyl -1-oxo-2-[[[(1,7,7-trimethylbicyclo[2.2.1]hept-2-yl) oxy] carbonyl]amino]propyl]amino]-1-phenylethyl]amino]-4-oxo-2-butenoic acid
(Bicyclo system is 1S-endo)

C 97. [R-[R*,S*-(E)]]-4-[[2-[[3-(1H-indol-3-yl)-2-methyl-1-oxo-2-[[(tricyclo[3.3.1.1$^{3,7}$]dec-2-yloxy)carbonyl]amino] propyl]a mino]-3-phenylpropyl]amino]-4-oxo-2-butenoic acid

C 98. N-[2-methyl-N-[(tricyclo[3.3.1.1$^{3,7}$]dec-2-yloxy)c arbonyl]-D-tryptophyl]-L-phenylalanylglycine

C 99. (R-(R*,S*)]-4-[[2-[[3-(1H-indol-3-yl)-2-methyl-1-oxo-2-[[ (tricyclo[3.3.1.1$^{3,7}$ ]dec-2-yloxy)carbonyl]amino]propyl]amino]-3-phenylpropyl]amino]-4-oxobutanoic acid

C 100. tricyclo[3.3.1.1$^{3,7}$]dec-2-yl (R-( R*, R*)]-[2-[[2-[[1,4-dioxo-4-(1H-tetrazol-5-ylamino)butyl]amino]-2-phenylethyl]amino]-1-(1H-indol-3-ylmethyl)-1-methyl-2-oxoethyl]carbamate

C 101. [R-(R*,R*)]-3-[[2-[[3-(1H-indol-3-yl)-2-methyl-1-oxo-2-[ [(tricyclo[3.3.1.1$^{3,7}$]dec-2-yloxy)carbonyl]amino] propyl]am ino]-1-phenylethyl]amino]-3-oxopropanoic acid

C 102. [R-(R*,S*)]-3-[[2-[[3-(1H-indol-3-yl)-2-methyl-1-oxo-2-[ [(tricyclo[3.3.1.1 $^{3,7}$]dec-2-yloxy)carbonyl]amino] propyl]am ino]-3-phonylpropyl]amino]-3-oxopropanoic acid

C 103. (R-[R*,S*-(E)]]-4-[[2-[[3-(1H-indol-3-yl)-2-methyl-2-[[(bic yclo[3.3.1]non-9-yloxy)carbonyl]amino]-1-oxopropyl]amino]-3-phenylpropyl]amino]-4-oxo-2-Butenoic acid

C 104. [R-(R*,S*)]-5-[[2-[[3-(1H-indol-3-yl)-2-methyl-1-oxo-2-[ [(tricyclo[3.3.1.1 $^{3,7}$]dec-2-yloxy)carbonyl]amino] propyl]am ino]-3-phenylpropyl]amino]-5-oxopentanoic acid

C 105. Ethyl [R-(R*,S*)]-[[2-[[3-(1H-indol-3-yl)-2-methyl-1-oxo-2[[(tri cyclo[3.3.1.1$^{3,7}$]dec-2-yloxy)carbonyl]aminol-propyl]amino]-3-phenylpropyl]sulfinyl]-acetate

C 106. [R-[R*,R*-(E)]]-4-[[2-[[3-(1H-indol-3-yl)-2-methyl-1-oxo-2-[[(tricyclo[3.3.1.1$^{3,7}$]dec-2-yloxy) carbonyl]amino] propyl]a mino]-1-phenylethyl]amino]-4-oxo-2-butenoic acid

C 107. [R-(R*,S*)]-N-[3-[[3-(1H-indol-3-yl)-2-methyl-1-oxo-2-[[(tr icyclo[3.3.1.1$^{3,7}$]dec-2-yloxy)carbonyl]amino]propyl]amino] -1-oxo-4-phenylbutyl]-8-alanine

C 108. N-[N-[α-methyl-N-((tricyclo[3.3.1.1$^{3,7}$]dec-2-yloxy)carbonyl]-D-tryptophyl]-L-phenylalanyl]-β-Alanine

C 109. [R-R*,S*)]-3-[[2-[[3-(1H-indol-3-yl)-2-methyl-1-oxo-2-[ [(tricyclo[3.3.1.1$^{3,7}$]dec-2-yloxy)carbonyl]amino]propyl]am ino]-3-phenylpropyl]thio]-propanoic acid

C 110. [R-(R*,S*)]-[[2-[[3-(1H-indol-3-yl)-2-methyl-1-oxo-2-[[( tricyclo[3.3.1.1$^{3,7}$]dec-2-yloxy)carbonyl]amino]propyl]amin o]-3-phenylpropyl]thio]acetic acid

C 111. [R-(R*,S*)]-β-[[3-(1H-indol-3-yl)-2-methyl-1-oxo -2-[[(tricyclo[3.3.1.1$^{3,7}$]dec-2-yloxy)carbonyl]amino]propy 1]amino]benzenebutanoic acid

C 112. tricyclo[3.3.1.1$^{3,7}$]dec-2-yl [R-(R*,S*)]-3-(1H-Indol-3-ylmethyl)-3-methyl-4,10-dioxo-6-(phenylmethyl)-11-Oxa-8-thia-2,5-diazatridecanoate

Tables I and II illustrate representative compounds of the invention. The C numbers on the left hand column correspond to the C-compound numbers given above. In Table I and on top of the formula in Table II C-compound numbers do not correspond to Example or Claim numbers. Stereochemistry is not shown in the Table I.

In addition to the compounds of the above tables the compounds of the present invention include compounds of formula I wherein the indole moiety is a 2-indolyl.

The compounds include solvates and hydrates and pharmaceutically acceptable salts of the compounds of formula I.

The compounds of the present invention can have multiple chiral centers including those designated in the above formula I by an *, †, ‡ depending on their structures. For example, when $R^3$ taken with $R^{12}$ and $R^4$ taken with $R^{13}$ form double bonds to these carbon atoms they are no longer chiral. In addition, centers of asymmetry may exist on substituents $R^1$, $R^9$, $R^3$, $R^4$ and/or Ar. In particular the compounds of the present invention may exist as diastereomers, mixtures of diastereomers, or as the mixed or the individual optical enantiomers. The present invention contemplates all such forms of the compounds. The mixtures of diastereomers are typically obtained as a result of the reactions described more fully below. Individual diastereomers may be separated from mixtures of the diastereomers by conventional techniques such as column chromatography or repetitive recrystallizations. Individual enantiomers may be separated by convention method well known in the art such as conversion to a salt with an optically active compound, followed by separation by chromatography or recrystallization and reconversion to the nonsalt form.

The preferred stereochemistry of the compounds of the invention is that exhibited by the compound of Example 20.

The compounds of the present invention can be formed by coupling individual substituted α-amino acids by methods well known in the art. (See, for example, standard synthetic methods discussed in the multi-volume treatise "The Peptides, Analysis, Synthesis, Biology," by Gross and Meienhofer, Academic Press, New York.) The individual substituted alpha amino acid starting materials are generally known or, if not known, may be synthesized and, if desired, resolved by methods within the skill of the art. (Synthesis of racemic [DL]-α-methyl tryptophan methyl ester - see Braña, M. F., et al, J. Heterocyclic Chem., 1980, 17:829.)

A key intermediate in the preparation of compounds of formula I is a compound of formula

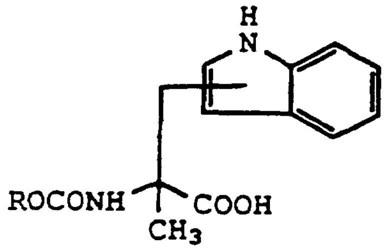

II

wherein R is selected from $R^1$, 9-fluorenylmethyl, Bz and other suitable N-blocking groups. These are useful as intermediates in the preparation of compounds of formula I. The compounds wherein R is 1-adamantyl, 2-adamantyl, 4-protoadamantyl, 9-fluorenylmethyl, exo-bornyl, endo-bornyl, exo-norbornyl, endonorbornyl, 2-methylcyclohexyl, 2-chlorocyclohexyl, or camphoryl are novel and are preferred.

The disclosure of U.S. 4,757,151 is hereby incorporated by reference. It describes the 9-fluorenylmethyl blocking group.

Compounds of formula II are prepared by reacting

ROH III

wherein R is as defined above, with phosgene or a phosgene substitute to produce a corresponding compound of formula

ROCOCl IV

and then reacting a compound of formula IV with α-methyltryptophan to produce the desired compound of formula II above.

Alternatively, a compound of formula IV can be reacted with an α-methyltryptophan methyl ester to produce

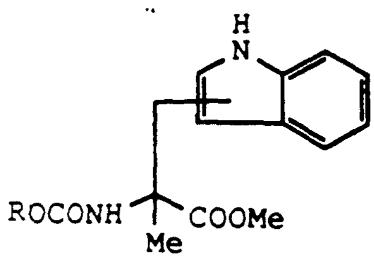

V

which can be converted to a compound of formula II by known means such as hydrolysis with aqueous lithium hydroxide.

Scheme I below illustrates procedures for preparing intermediates useful in producing final products of formula I.

Key intermediate (2) is prepared from the alcohol form of a radical selected from 1-adamantyl, 2-adamantyl, 4-protoadamantyl, 9-fluorenylmethyl, exo-bornyl, endo-bornyl, exo-norbornyl, endo-norbornyl, 2-methylcyclohexyl, 2-chlorocyclohexyl, and camphoryl. The alcohol is dissolved in a solvent such as methylene chloride. It is then converted to the corresponding chloroformate by reaction with bis(trichloromethyl) carbonate in pyridine at about 0°C. The product is formed by condensation with an amine such as α-methyl-D-tryptophan methyl ester. The reaction is carried out in a solvent such as THF to produce, for example, N-[(2-adamantyloxy)carbonyl]-α-methyl-D-tryptophan methyl ester. This is then treated with lithium hydroxide and stirred at room temperature overnight to produce the corresponding carboxylic acid. This novel key intermediate (2) is useful in the production of compounds of formula I as described hereinafter in Schemes II and III.

Alternatively a chloroformate can be converted to (2) by reaction with an alkaline solution of α-methyl-DL-tryptophan.

In another process, (sequence 3,4,5,6,)tert-butyloxycarbonyl-L-phenylalaninol in pyridine is treated with p-toluene sulphonyl chloride to give the corresponding tosylate. The tosylate is treated with sodium azide in N,N-dimethylformamide to produce the corresponding azide. This is converted to the free aminoazide (6) by reaction with p-toluene sulphonic acid in dichloromethane solution at room temperature. This is then reacted with the desired compound of formula 2 to produce a compound of the instant invention as, for example in schemes I, II and II.

Similarly (sequence 7-12) tert-butyloxycarbonyl-D-2-phenyl glycinol can be converted to the corresponding amine-substituted azide (10) using the above procedure. A solution of benzyl hydrogen succinate is reacted with an equimolar mixture of N,N-dicyclohexyl-carbodiimide and 1-hydroxybenzotriazole. The reaction is carried out in ethyl acetate for about an hour. Subsequent addition of the free amine (10) to the reaction mixture yields an amide (11). The azide portion of (11) is hydrogenated over a Lindlar catalyst to form the amine (12).

A solution of 2-adamantyloxycarbonyl-α-methyl-D-tryptophan in ethyl acetate reacts with an equimolar solution of N,N-dicyclohexyl-carbodiimide and 1-hydroxybenzotriazole. The reaction mixture is left to stir at room temperature for about an hour. Subsequently the amine (12) in scheme I, in ethyl acetate is allowed to react for 18 hours at room temperature to form the dipeptoid benzyl ester (scheme II). Finally, the benzyl ester is hydrogenolysed for four hours using a palladium on carbon catalyst. After filtering and washing, the filtrate yields the desired product of formula I.

SCHEME I

INTERMEDIATES

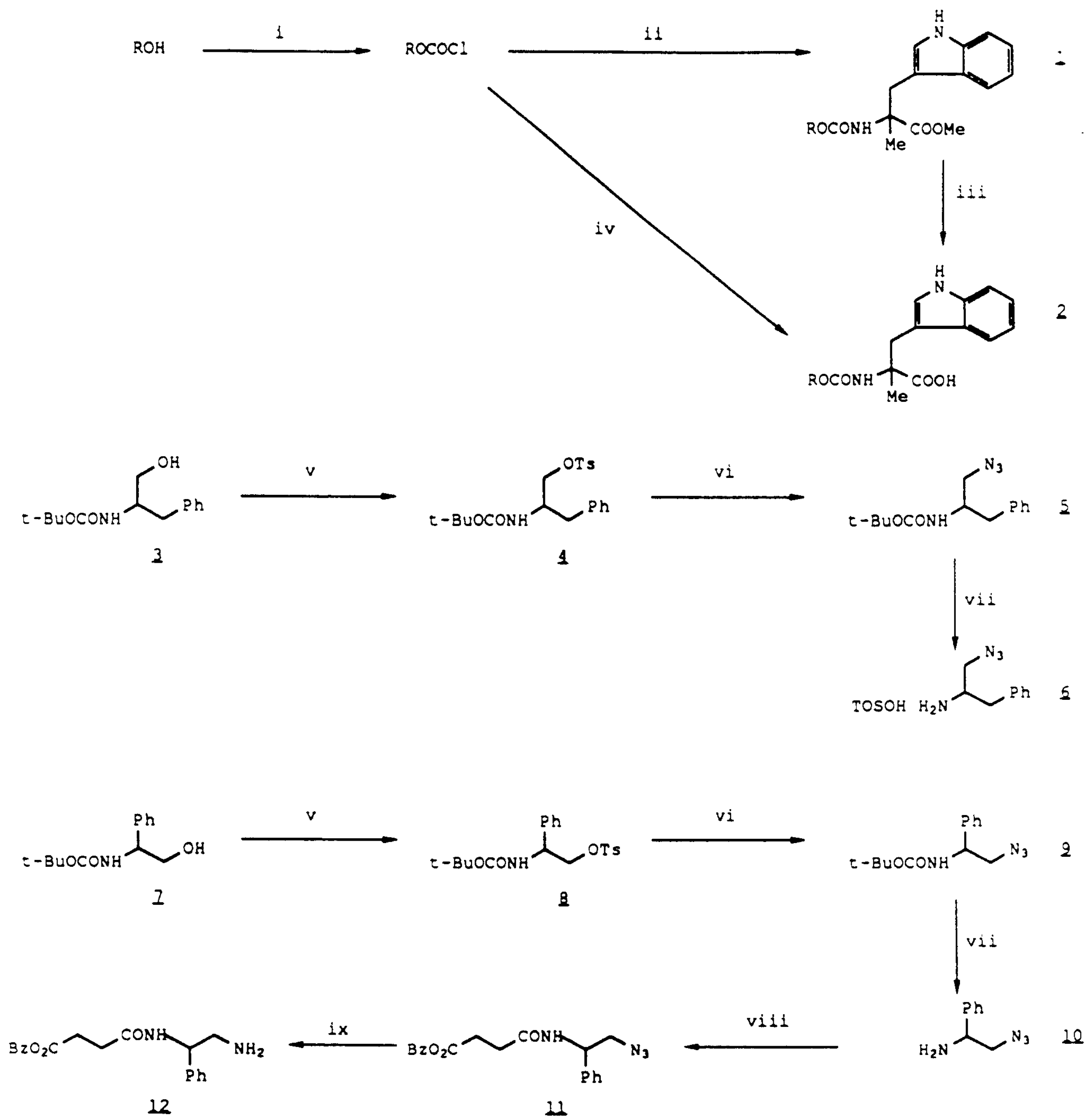


1 2 3 4 5 6 7 8 9 10 11 12

KEY (i) $COCl_2$, disphosgene or triphosgene, pyridine

(ii) α-methyl tryptophan methylester

(iii) LiOH, aq.1,4 dioxan

(iv) α-methyl tryptophan

(v) TsCl, pyridine or $NEt_3$

(vi) $NaN_3$, DMF

(vii) TSOH, $CH_2Cl_2$

(viii) Benzyl hydrogen succinate, DCCI, HOBT

(ix) Lindlar, EtOH

Whenever R in intermediate of formula II is other than $R^1$, it may be removed at an appropriate point in the synthesis by methods known in the art for each respective group and the desired $R^1$ substituted therefore.

Scheme II below illustrates processes for the preparation of compounds of formula I using key intermediate, compound (2) from the Scheme I.

One process, as illustrated by sequence 2, 13, 14, involves reacting 2-adamantyloxycarbonyl-α-methyl-D-tryptophan with dicyclohexylcarbodiimide (DCCI) and 1-hydroxybenzotriazole (HOBT) in ethyl acetate solution.

Subsequent addition of 2-amino-1-phenyl ethanol produces an alcohol as in compound (13) of the scheme. This alcohol is then reacted with succinic anhydride to yield compound (14), a compound of the instant invention.

Another process of the invention is illustrated by sequence 2, 16, 15 of Scheme II. In this process intermediate (2) is reacted with DCCI and pentafluorophenol in ethyl acetate. After stirring for an hour at room temperature the mixture is reacted with L-phenylalaninol to yield a compound (16). This is then refluxed with succinic anhydride and DMAP for 24 hours. The reaction mixture is washed and the organic phase dried over $MgSO_4$. Evaporation of the solvent yields a compound as illustrated by (15).

In the sequence 2, 21, 22 intermediate (2) (R is 9-fluorenylmethyl) in solution with pentafluorophenol is treated with a solution of DCCI in ethyl acetate. This solution is stirred for one hour at 0°C and then for four hours at room temperature. After filtering and washing the precipitated DCU, the combined filtrates react with 2-phenylethylamine to produce compound (21). This compound is converted to the free amine (22) by reaction with a 20% piperidine in DMF solution. This can be treated with a substituted chloroformate to produce the desired amide (21).

In another process, sequence 2, 16, 17, and then 18, or 19 or 20, compound (12) is converted to compound, (16) (R is 9-fluorenylmethyl) as discussed above. The amide (16) is converted to a free amine (17) by reaction with 20% pyridine in DMF.

A solution of the amine (17) is reacted with a substituted acetylchloride to form the corresponding substituted acylamide (18).

Alternatively, a solution of free amine (17) is reacted with a substituted sulphonylchloride to form the corresponding sulphonamide (19). The reaction takes place in THF and dimethylaminopyridine (DMAP) solution at room temperature for about four hours.

Additionally a solution of free amine (17) may be reacted with a substituted isocyanate to produce a desired compound (20). This may be converted, if desired, to a pharmaceutically acceptable salt.

SCHEME II

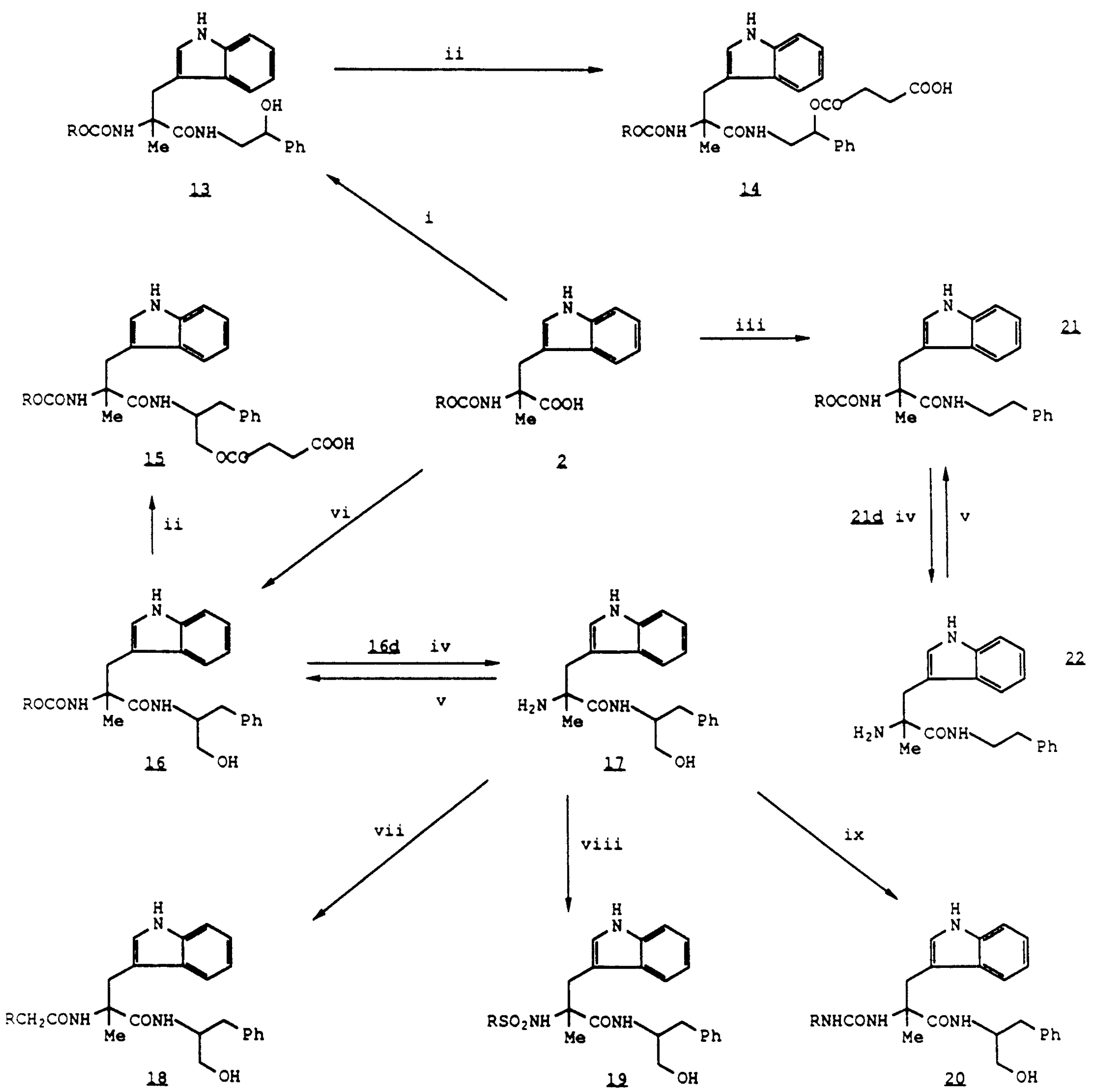


KEY
(i) DCCI, HOBT, (+) or (-) 2-amino-1-phenyl ethanol
(ii) Succinic anhydride, DMAP
(iii) DCCI, PFP, 2-phenethylamine
(iv) 20% piperidine in DMF
(v) ROCOCl
(vi) DCCI, PFP, L-phenylalaninol
(vii) R-acetylchloride
(viii) R-sulphonylchloride
(ix) R-isocyanate

Scheme III below illustrates processes for preparing compounds of formula I.

One process is indicated by the sequence 2, 23, 24 of the scheme. The 2-adamantyloxycarbonyl-α-methyl-D-tryptophan intermediate in ethyl acetate is treated sequentially with DCCI and HOBT and later reacted with an amine (12 in Scheme I) to produce a desired benzyl ester (23). This is reduced to the free carboxylic acid (24) using hydrogen and a 10% palladium on carbon catalyst for about four hours. The reaction mixture is filtered, washed and concentrated in vacuo to yield (24).

Another process is illustrated by sequence 2, 25, 26 and 27 or 28. In this process compound (2) is reacted with DCCI and PFP in ethyl acetate. After stirring for an hour at room temperature the mixture is reacted with the amino-azide (6 in Scheme I) to yield a compound (25). This is then dissolved in five percent acetic acid; ninety-five percent ethanol and converted to a crude amine acetate (26) by hydrogenation in the presence of a catalyst such as ten percent palladium in carbon.

Compound (26) may then be reacted with succinic anhydride to form the free carboxylic acid (28).

Also compound (26) is reacted with fumaryl dichloride to produce compound (27).

Compound (27) or (28) may be converted, if desired, to a pharmaceutically acceptable salt thereof.

SCHEME III

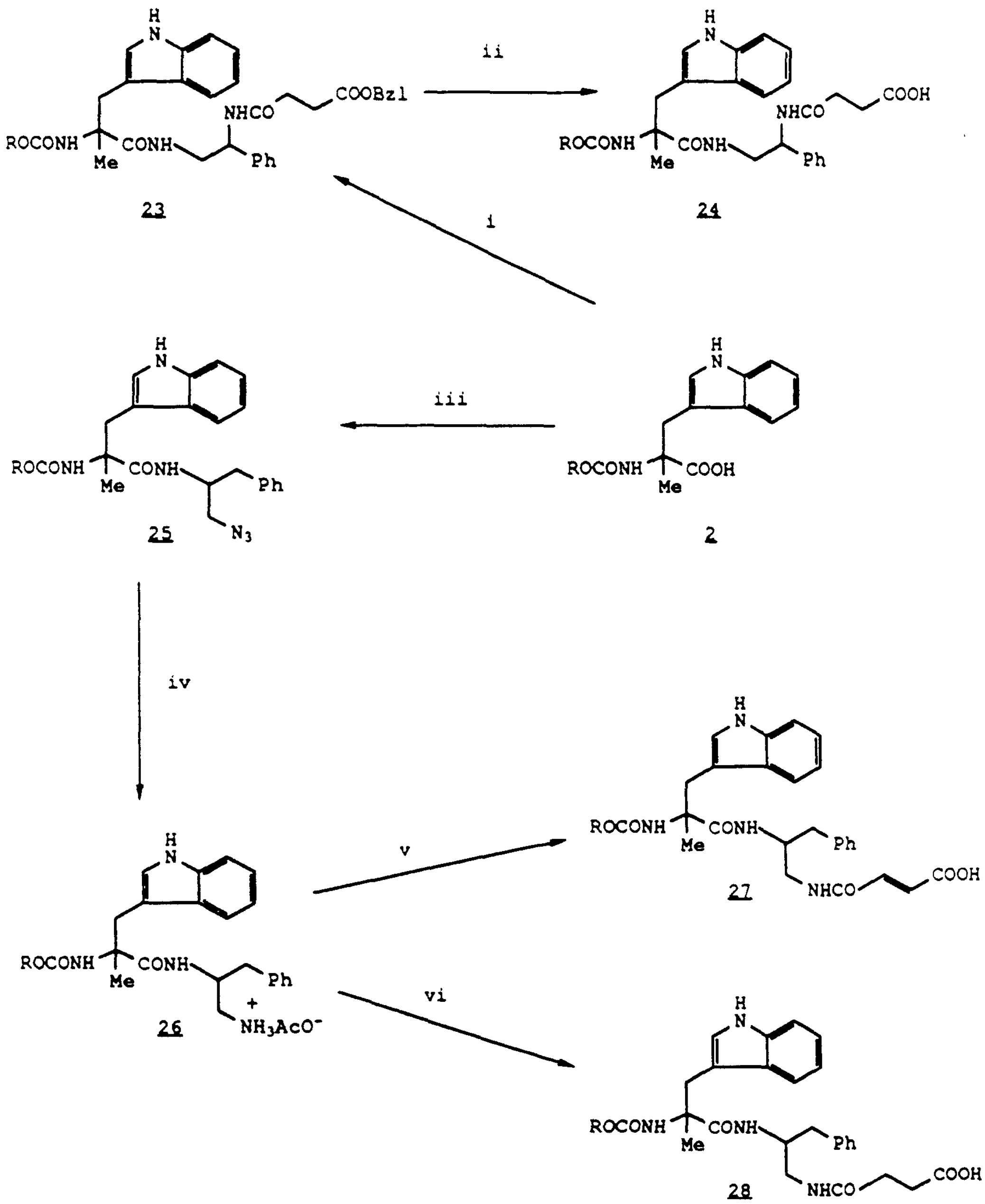


23 24 25 2 26 27 28

KEY (i) 12, DCCI, HOBT
(ii) 10% Pd/C, EtOH
(iii) 6, DCCI, PFP
(iv) 10% Pd/C, 1% AcOH in EtOH
(v) i. Fumaryl dichloride; ii. $OH^-$
(vi) Succinic anhydride, DMAP

Scheme IV below describes the synthesis of the 2-substituted indole analogs of formula I.

The indole ethyl 2-carboxylate is protected on the indole nitrogen by tosylation to give (6) which is reduces by Red-Al to the corresponding 2-hydroxymethyl compound (7). The alcohol (7) is converted into the corresponding bromide (8) using bromine and triphenylphosphene. The bromide (8) is used to alkylate the anion of the Schiff's base (8A) derived from the methyl ester of alanine to give the Schiff's base (9) as a racemate. The hydrolysis of the Schiff's base gives the free amine (10) which is condensed with 2-adamantylchloroformate to give the methyl ester (11) which is hydrolyzed with potassium hydroxide in ethanol followed by further acid work up to give the free carboxylic acid (12).

This acid, which is the 2-indole analog of the intermediate (2) is also condensed with amines such as previously illustrated in Schemes I and V to produce final products, for example, condensation of (12) with phenylethylamine gives compound (13A) and with (S)-(-)-2-amino-3-phenyl-1-propanol to give the (13B) as a mixture of diastereoisomers. These are separated by chromatography to give diastereoisomer 1 and diastereoisomer 2 foam with Rf 0.70 and 0.65 in MeOH/$CH_2Cl_2$ in ratio 1:9

## SCHEME IV

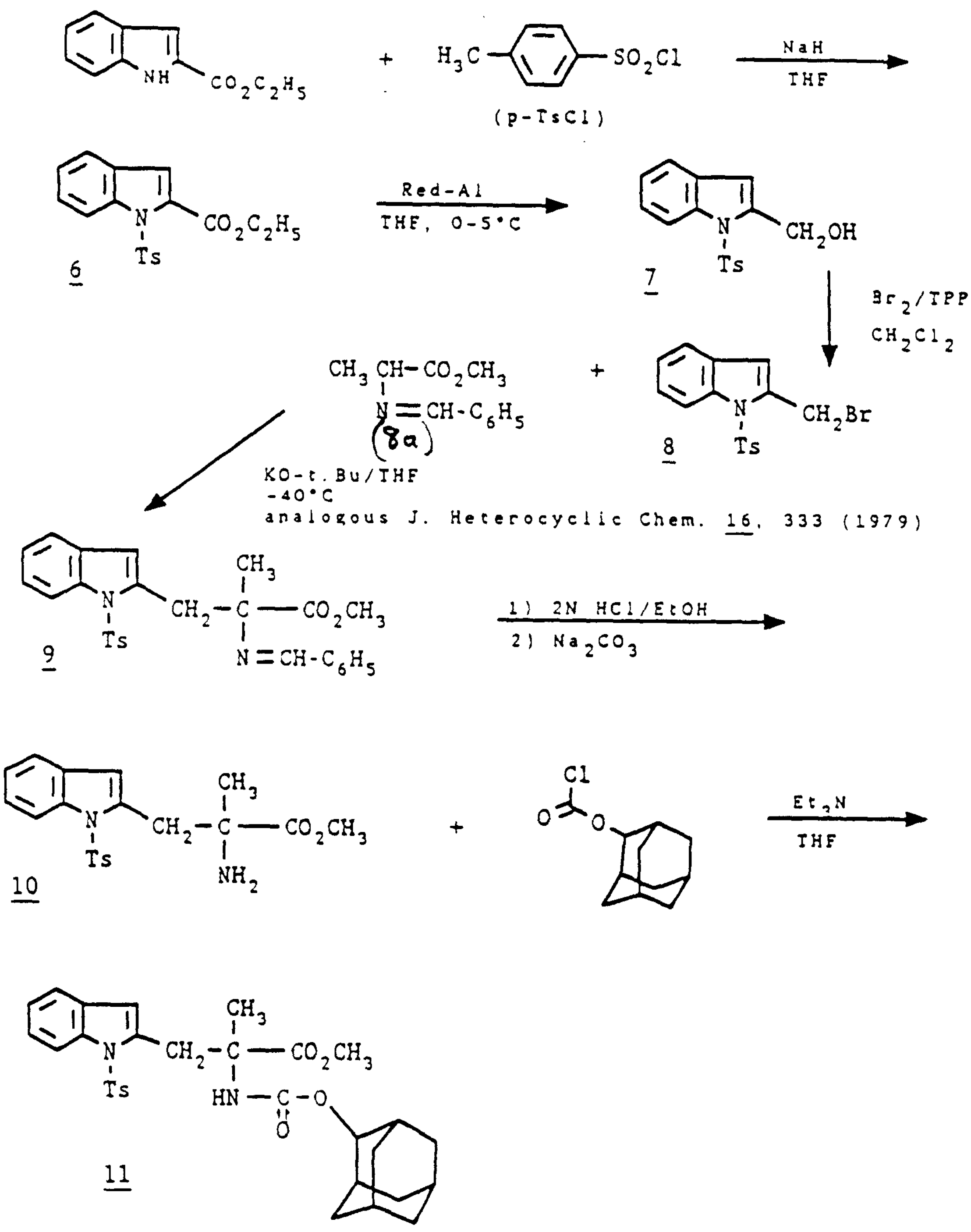


6

7

8

9

10

11

SCHEME IV CONTINUED

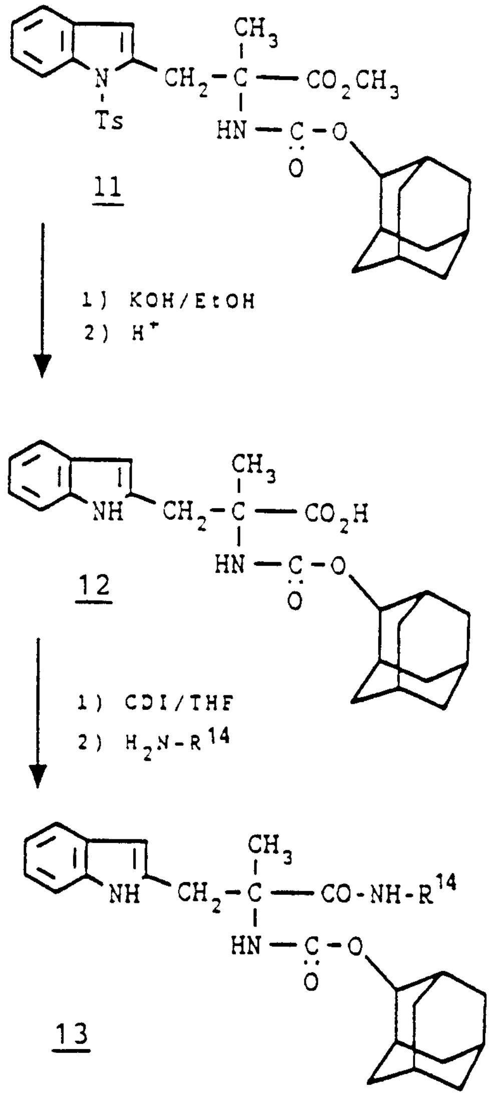


13A $R^{14}$ = $-CH_2-CH_2-C_6H_5$

13B $R^{14}$ = 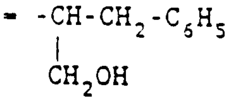

Scheme V below illustrates synthesis of preferred C-terminal side chains $R^3$ and $R^4$ used to prepare the final products illustrated in Scheme VI.

Thus the conversion of (35) to (37) is accomplished by condensing the isobutylformyl ester of (35) with 2-(trimethylsilyl)ethanol to give intermediate (36) followed by cleavage of the TMS group with TFA to give (37).

The oxime ester intermediate (40) is prepared by acylation of aminoacetophenone hydrochloric acid (38) with 2-(trimethylsilyl)ethylchloroformate in THF following by condensation with hydroxylamine hydrochloride and sodium

acetate to give an oxime. Compound (39) was then prepared by adding methyl bromoacetate in the presence of 10% NaOH and TBAB in toluene. The trimethylsilylethyl group is then selectively removed with tetrabutylammonium fluoride.

Intermediate (42) is prepared from the alcohol (41) in the steps involving tosylation of the alcohol, displacement of the tosylate by sodium azide in DMF followed by catalytic reduction.

The tetrazole carboxylic acid intermediate (44) is prepared from the nitrile (43) in three steps by addition of azide to form a tetrazole which is protected by benzylation followed by hydrolysis of the methyl ester to the free carboxylic acid using an aqueous THF solution of lithium hydroxide.

The diene ester (47) is prepared from the BOC-protected phenylalanine (45) through aldehyde (46) using the Wittig reagent $Ph_3P=CHCH=CHCO_2CH_3$.

The intermediate ether (50) is prepared from the chlorohydroxy compound (48) involving displacement of the chloride with sodium azide followed by alkylation of the anion of the hydroxyl group with methyl iodoacetate to give the azido ether (49) which is then reduced under catalytic conditions.

The ethyl ester (52) is prepared by catalytic hydrogenation of nitrile (51).

SCHEME V

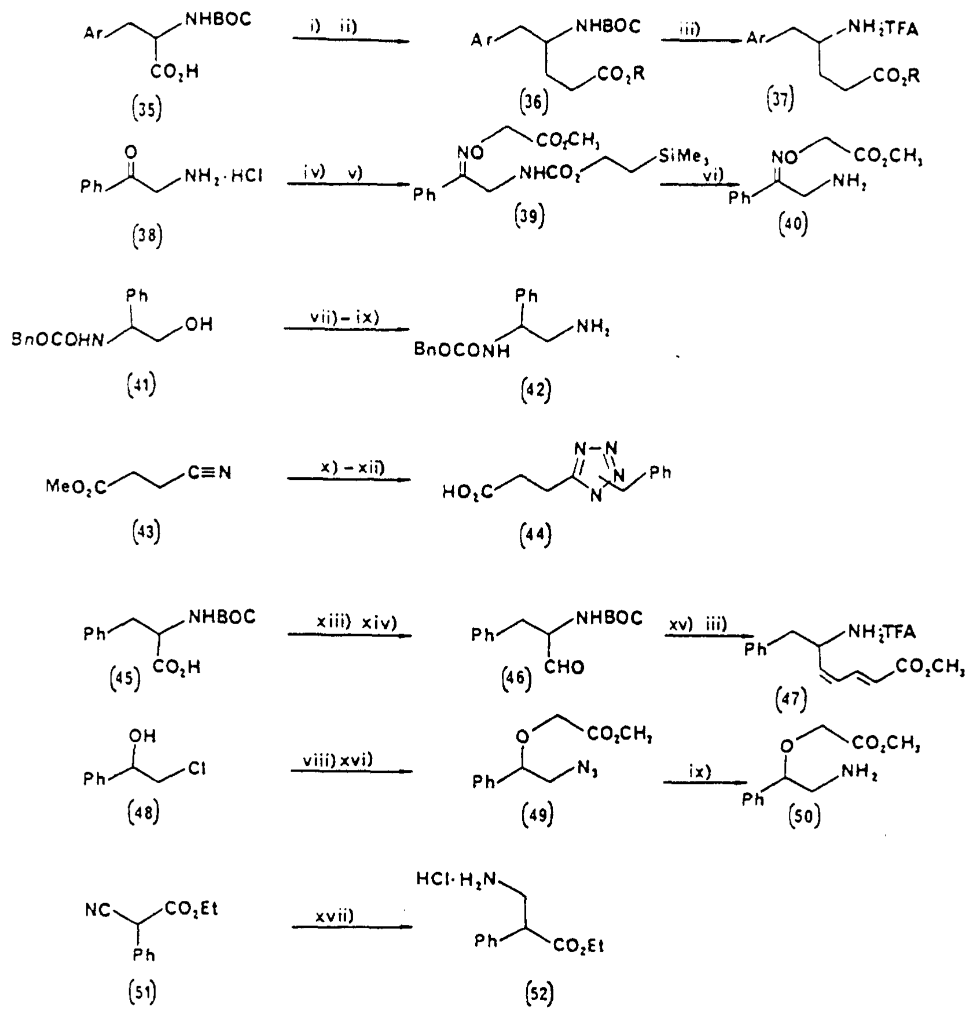


R is Methyl, when Ar is phenyl,

R is 2-(Trimethylsilyl)ethyl, when Ar is p-iodo phenyl

Key : (i) N-methyl morpholine, isobutylchloroformate, THF; (ii) Silver benzoate, $NEt_3$, MeOH <u>or</u> 2-(trimethylsilyl)ethanol; (iii) TFA, $CH_2Cl_2$; (iv) 2-(Trimethylsilyl)ethylchloroformate, $NEt_3$, THF; (v) $NH_2OH.HCl$, $CH_3CO_2Na$, EtOH/$H_2O$; then $[CH_3(CH_2)_3]_4NBr$, $BrCH_2CO_2Me$, 10% NaOH, toluene; (vi) TBAF; pTsCl, $NEt_3$, $CH_2Cl_2$; (viii) $NaN_3$, DMF, Δ; (ix) $H_2$, Lindlar catalyst, EtOAc; (x) $NaN_3$, $NH_4Cl$, DMF, Δ; (xi) BzBr, $Cs_2CO_3$, DMF; (xii) LiOH, aq THF; (xiii) $CH_3NHOCH_3.HCl$, isobutylchloroformate, N-methyl morpholine, THF; (xiv) LAH, THF; (xv) $Ph_3P=CH.CH=CHCO_2CH_3$, THF; (xvi) NaH, $ICH_2CO_2CH_3$, TMEDA, THF; (xvii) 10% Pd/C, $H_2$, HCl/EtOH.

Scheme VI below shows the synthesis of compounds further illustrating preferred examples of $R^3$ and $R^4$ of formula I.

Key intermediate (2) is converted into the O-ether-linked side chain carboxylic acid (54) by condensation with the amine (50 of Scheme V) as described above, with subsequent hydrolysis.

Compound (65) with an α-pentanoic acid side chain is prepared by hydrogenation followed by hydrolysis of the unsaturated ester (64) which is prepared by condensation of flexible acid (2) with amine (47 of Scheme V).

The glycyl derivative (56) is prepared by condensation of the benzyl ester of glycine with the acid (55) followed by catalytic hydrogenation to remove the benzyl group. The acid (55) in turn is prepared from the flexible acid (2) by condensation with the amine (52 of Scheme V).

The oxime ether carboxylic acid (57) is also prepared from the flexible acid intermediate (2) by condensation with intermediate (40) (Scheme V) followed by hydrolysis of the ethyl ester with aqueous lithium hydroxide in THF.

The tetrazole (62) is prepared by condensation of the amine (60) with the benzylated tetrazole carboxylic acid (44 of Scheme V) followed by removal of the benzyl group by catalytic hydrogenation.

The intermediate amine (60) is prepared from the flexible acid (2) by condensation of the amine (42) of Scheme V followed by removal of the benzyloxycarbonyl group by catalytic hydrogenation.

The α-glycinate derivative (59) is prepared by condensation of the α-acetic acid derivative (58) with ethylglycinate followed by hydrolysis of the ethyl ester with 1M NaOH in ethanol.

The acid (58) is prepared from the key intermediate (2) by condensation with (37) of Scheme V (wherein R is methyl and Ar is phenyl) followed by hydrolysis of the methyl ester with aqueous lithium hydroxide in THF.

The α-acetic acid (53) is prepared from the key acid (2) by condensation with (39) of Scheme V (wherein R is 2-(trimethylsilyl)ethyl and Ar is p-iodophenyl) followed by removal of the 2-(trimethylsilyl)ethyl protecting group with tetrabutyl ammonium fluoride in THF.

## SCHEME VI

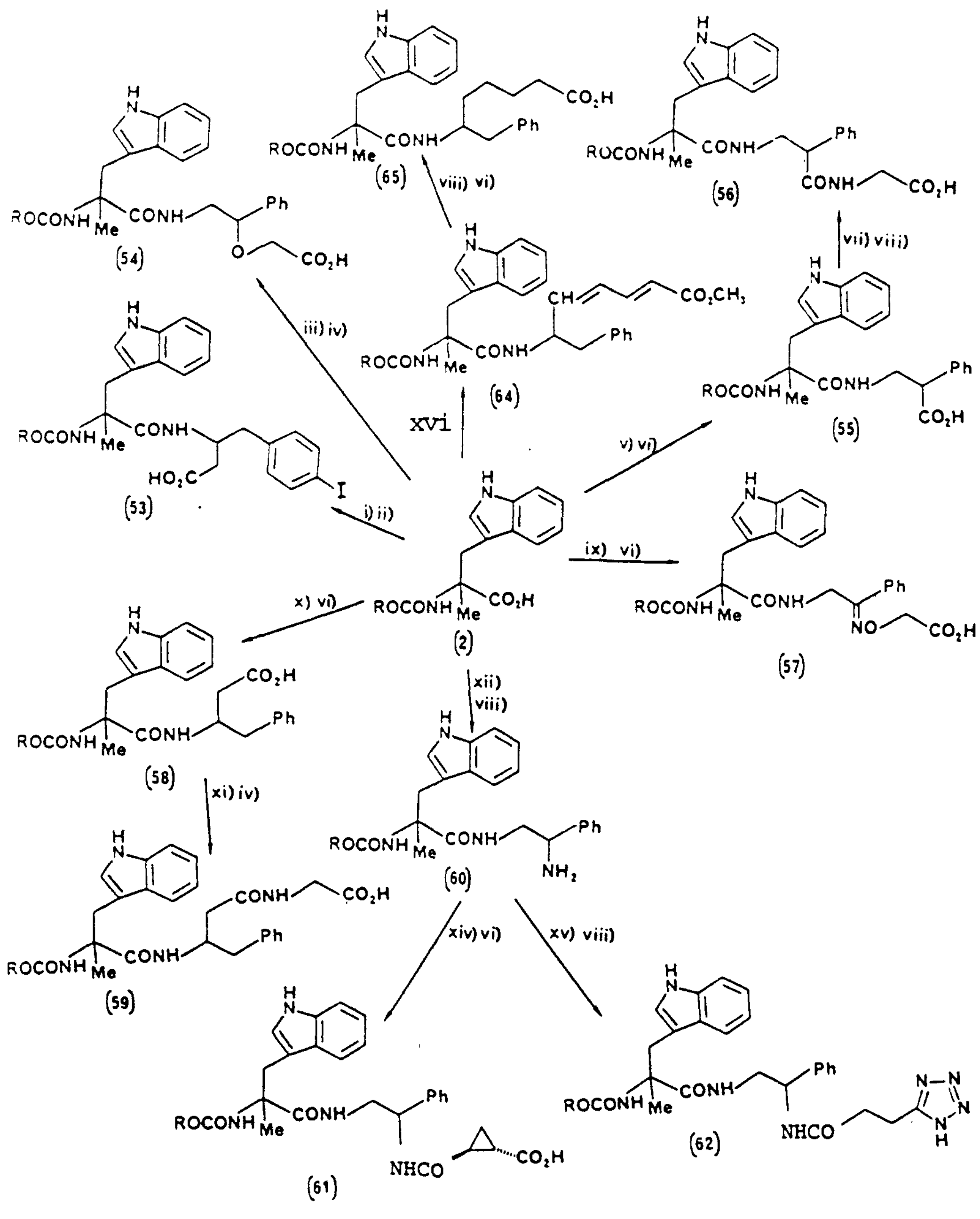


R = 2 Adamantyl

Key : (i) DCC, HOBt, 37, EtOAc; (ii) TBAF, THF; (iii) DCC, HOBt, 50, $NEt_3$, EtOAc; (iv) 1M NaOH, EtOH; (v) DCC, HOBt, 52, $NEt_3$, EtOAc; (vi) LiOH, aq THF; (vii) DCC, HOBt, $HCl.NH_2CH_2CO_2Bn$, $NEt_3$, EtOAc; (viii) 20% $Pd(OH)_2/C$, $H_2$ EtOH; (ix) DCC, HOBt, 40, EtOAc; (x) DCC, HOBt, 37, $NEt_3$, EtOAc; (xi) DCC, HOBt, $HCl.H_2NCH_2CO_2Et$, $NEt_3$, EtOAc; (xii) DCC, HOBt, 42, EtOAc;
(xiv) DCC, HOBt, mono methyl cyclopropanedicarboxylate, EtOAc; (xv) DCC, PFP, 44, EtOAc; (xvi) DCC, HOBt, 47, $NEt_3$, EtOAc.

Scheme VII below shows the synthesis of compound 71 which illustrates an example of formula I wherein $R^2$ is the functional group $CH_2CO_2Me$.

The starting formyl tryptophan (66) is protected on the indole nitrogen by BOC and protected on the carboxylic acid as benzyl ester (67). The N-formyl group is then dehydrated with triphosgene to form the corresponding isonitrile of which the anion of which is formed on treatment with LDA and then alkylated with methyl bromoacetate to give (68).

The isonitrile (68) is hydrolyzed using ethanolic HCl to the corresponding amine which is directly converted to (69) by acylationwith 2-adamantylchloroformate. The benzyl ester group of (69) is then selectively removed by hydrogenation using 10% palladium on carbon and the resulting free carboxylic acid (70) is then condensed with phenylethylamine to generate the final product (71).

SCHEME VII

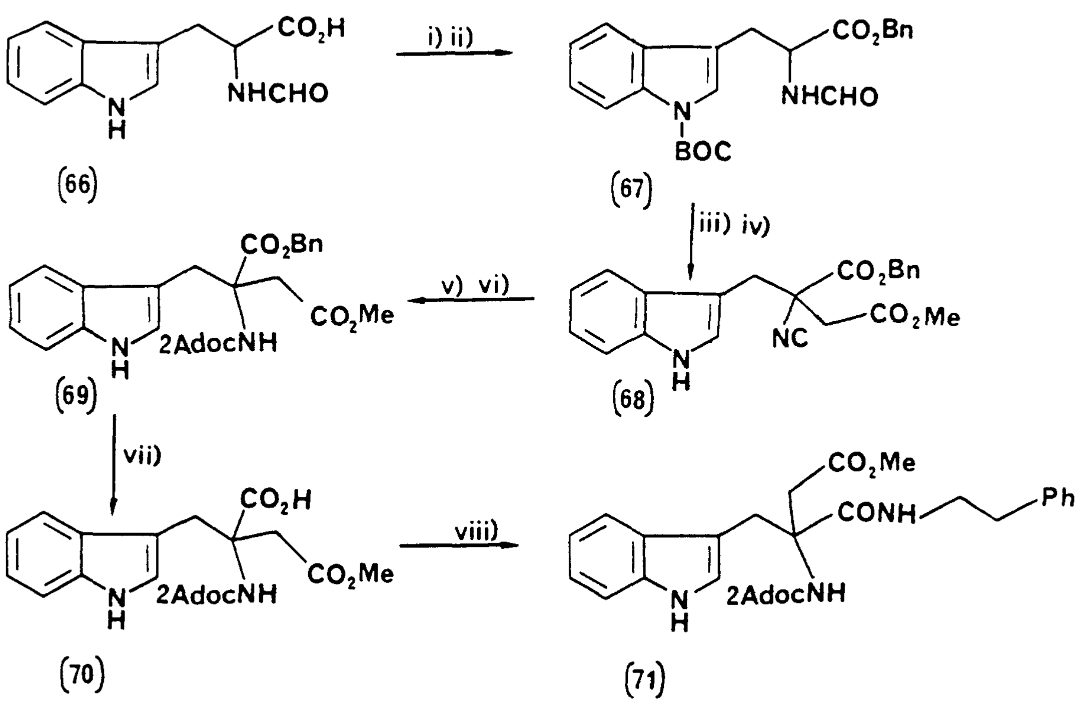


(66) (67) (68) (69) (70) (71)

Key : (i) $Cs_2CO_3$, BnBr, DMF; (ii) $(Boc)_2O$, DMAP, DMF; (iii) Triphosgene, $NEt_3$, $CH_2Cl_2$; (iv) $BrCH_2CO_2CH_3$, LDA, HMPA, THF; (v) Ethanolic HCl; (vi) 2-Adamantyl chloroformate, $NEt_3$, EtOAc; (vii) $H_2$, 10% Pd/C, ethanol; (viii) DCC, PFP, phenethylamine, EtOAc.

Scheme VIII below illustrates the synthesis of a difunctionalized derivative of formula I when $R^3$ is hydroxymethylene and $R^4$ is hydroxyl. Intermediate (2) is condensed with L-(+)-threo-2-amino-1-phenyl-1,3-propandiol using the PFP ester of (2).

SCHEME VIII

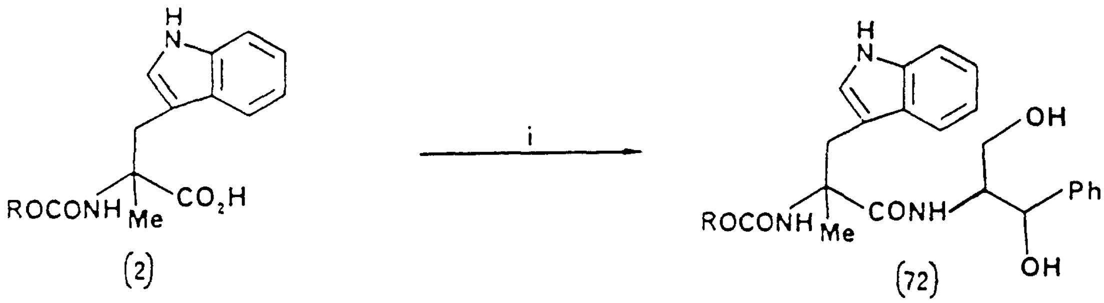


(2)

(72)

Reagents : (i) PFP, DCC, L-(+)threo-2-amino-1-phenyl-1,3-propanediol, EtOAc;

Scheme IX below illustrates a preferred mild procedure to prepare compound (82) when the TMS ester (81) is cleaved to the carboxylic acid (82) under mild conditions using tetrabutylammonium fluoride in THF. The scheme also illustrates the preparation of compound (80) by acetylation of amine (60K) with succinic anhydride in ethylacetate.

SCHEME IX

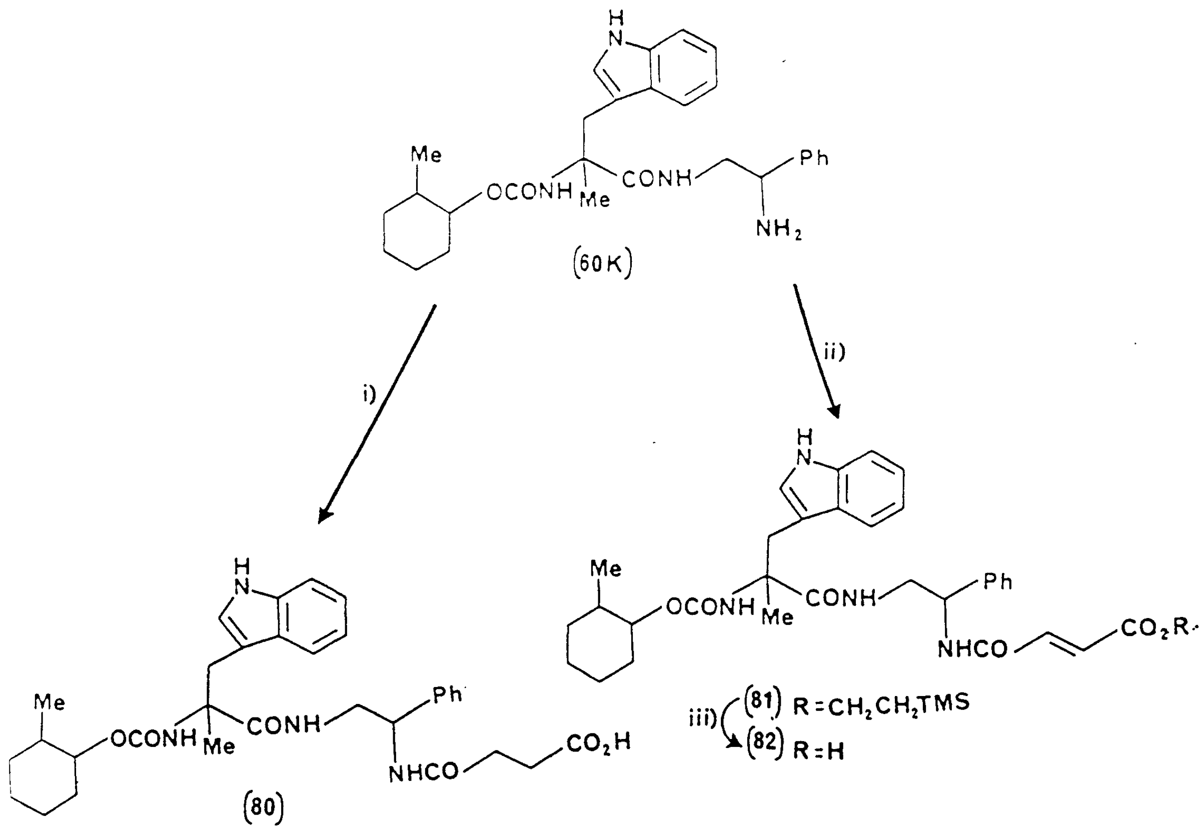


(60K)

(80)

(81) R = $CH_2CH_2TMS$

(82) R = H

Reagents : (i) Succinic anhydride, EtOAc; (ii) PFP, DCC, trans. $Me_3SiCH_2CH_2OCOCH{=}CHCO_2H$, EtOAc; (iii) $(n\text{-}Bu)_4N^+F^-$, THF.

The biological activity of compounds of the present invention was evaluated employing an initial screening test which rapidly and accurately measured the binding of the tested compound to known CCK receptor sites. Specific CCK receptors have been shown to exist in the central nervous system. (See Hays et al, Neuropeptides 1:53-62, 1980; and Satuer et al, Science, 208:1155-1156, 1980.

In this screening test, the cerebral cortices taken from male CFLP mice weighing between 30-40 g were dissected on ice, weighed, and homogenized in 10 volumes of 50 mM Tris-HCl buffer (pH 7.4 at 0-4°C). The resulting suspension was centrifuged, the supernate was discarded, and the pellet was washed by resuspension in Tris-HCl buffer followed by recentrifugation. The final pellet was resuspended in 20 volumes of 10 nM Hepes buffer (pH 7.2 at 23°C) containing 130 mM NaCl, 4.7 nM KCl, 5 nM $MgCl_2$, 1 nM EDTA, 5 mg/ml bovine albumin, and bacitracin (0.25 mg/ml).

In saturation studies, cerebral cortical membranes were incubated at 23°C for 120 minutes in a final volume of 500 µliter of Hepes incubation buffer (pH 7.2) together with 0.2-20 nM tritiated-pentagastrin (Amersham International, England).

In the displacement experiments, membranes were incubated with a single concentration (2 nM) of ligand, together with increasing concentrations ($10^{-11}$ to $10^{-14}$M) of competitive test compound. In each case, the nonspecific binding was defined as that persisting in the presence of the unlabeled octapeptide $CCK_{26\text{-}33}$ ($10^{-6}$M).

Following incubation, radioactivity bound to membranes was separated from that free in solution by rapid filtration through Whatman GF/B filters and washed three times with 4 ml of ice cold Tris-HCl buffer. Filters from samples

incubated with tritiated-pentagastrin were placed in polyethylene vials with 4 ml of scintillation cocktail, and the radioactivity was estimated by liquid scintillation spectrometry (efficiency 47-52%).

The specific binding to CCK receptor sites was defined as the total bound tritiated-pentagastrin minus the amount of tritiated-pentagastrin bound in the presence of $10^{-6}$ octapeptide, $CCK_{26-33}$.

Saturation curves for specific tritiated-pentagastrin binding to mouse cortical membranes were analyzed by the methods of Scatchard (Ann. New York Acad. Sci. 51:660-672, 1949, and Hill (J. Physiol. 40:IV-VIII, 1910, to provide estimates for the maximum number of binding sites ($B_{max}$) and the equilibrium dissociation constant ($K_a$).

In displacement experiments, inhibition curves were analyzed by either logit-log plots or the iterative curve fitting computer program ALLFIT (DeLean, Munson and Redbard, 1978) to provide estimates of the $IC_{50}$ and nH (apparent Hill coefficient) values). ($IC_{50}$ values were defined as the concentration of test compound required to produce 50% inhibition of specific binding.)

The inhibition constant ($K_i$) of the test compound was then calculated according to the Cheng-Prusoff equation:

$$K_i = \frac{IC_{50}}{1 + [L]/K_a}$$

where [L] is the concentration of radiolabel and $K_a$ is the equilibrium dissociation constant.

The $K_i$/M values for several representative compounds of the present invention are present in Table III.

Compounds of the present invention are useful as appetite suppressants as based on the tests described hereinbelow.

In the Palatable Diet Feeding assay, adult male Hooded Lister rats weighing between 200-400 g were housed individually and trained to eat a palatable diet. This diet consisted of Nestlés sweetened condensed milk, powdered rat food and rat water which when blended together set to a firm consistency. Each rat was presented with 20-30 g of the palatable diet for 30 minutes per day during the light phase of the light-dark cycle over a training period of five days. The intake of palatable diet was measured by weighing the food container before and after the 30-minute access period (limits of accuracy 0.1 g). Care was taken to collect and correct for any spillage of the diet. Rats had free access to pellet food and water except during the 30-minute test period.

After the training period, dose-response curves were constructed for CCK8 and several representative compounds of the present invention (n = 8-10 rats per dose level). $MPE_{50}$ values (±95% confidence limits) were obtained for the anorectic effects of these compounds and are shown in Table III.

In therapeutic use as appetite suppression agents, the compounds of the instant invention are administered to the patient at dosage levels of from about 200 to about 2800 mg per day.

Table III below shows the binding and efficacy data.

TABLE III

| Binding and Efficacy Data on Inhibition of Feeding in Rats | | | |
|---|---|---|---|
| | Binding to Central CCK Receptors | | Inhibition of Feeding on Rat Palatable Diet Assay |
| Example No. | $K_i$ (µM) | (n)[a] | I.P. $MPE_{50}$ (mg/kg) |
| 1 | 1.23 | (3) | NT |
| 2 | 3.15 | (3) | 9.6 |
| 3 | 0.26 | (3) | 30.7 |
| 4 | 0.17 | (3) | >20 |
| 5 | 2.23 | (3) | 33.6 |
| 6 | 0.44 | (3) | NT |
| 7 | 0.76 | (3) | NT |
| 8 | 0.84 | (3) | NT |
| 9 | 7.50 | (2) | NT |
| 10 | 8.80 | (2) | NT |
| 11 | 0.054 | (3) | NT |
| 12 | 0.085 | (3) | NT |

NT = Not tested
* $MPE_{50}$ value = the dose of compound producing 50% of the maximum effect possible, which in these experiments would be zero food intake.
(n)[a] = number of assays.

TABLE III (continued)

| Binding and Efficacy Data on Inhibition of Feeding in Rats | | | |
|---|---|---|---|
| | Binding to Central CCK Receptors | | Inhibition of Feeding on Rat Palatable Diet Assay |
| Example No. | $K_i$ (μM) | (n)[a] | I.P. $MPE_{50}$ (mg/kg) |
| 13 | 0.127 | (3) | NT |
| 14 | 10.5 | (1) | 19.5 |
| 15 | 0.026 | (3) | 15.7 |
| 16 | 0.03 | (2) | 10.5 |
| 17 | 0.063 | (2) | 13.1 |
| 18 | 21.02 | (1) | NT |
| 19 | 0.014 | (2) | NT |
| 19A | 0.00008 | (1) | NT |
| 20 | 0.0085 | (2) | 17.4 |
| 20A | 0.003 | (3) | NT |
| 33 | 0.006 | (1) | NT |
| 32 | 0.0051 | (1) | NT |
| 40 | 0.0039 | (1) | NT |
| 41 | 0.00029 | (1) | NT |
| 43 | 0.004 | (1) | NT |

NT = Not tested
* $MPE_{50}$ value = the dose of compound producing 50% of the maximum effect possible, which in these experiments would be zero food intake.
(n)[a] = number of assays.

Male Hooded Lister rats (175-250 g) were housed individually and fasted overnight (free access to water). They were anesthetized with urethane (1.5 g/kg IP) and the trachea cannulated to aid spontaneous respiration. The stomach was perfused continuously using a modification of the original method of Ghosh & Schild in "Continuous recording of acid secretion in the rat", Br. J. Pharmac. 13:54-61, 1956 as described by Parsons in "Quantitative studies of drug-induced gastric acid secretion". (Ph.D. Thesis, University of London, 1969). The cavity of the stomach was perfused at a rate of 3 ml/min with 5.4% w/v glucose solution through both the esophageal and body cannula. The fluid was propelled by a roller pump (Gilson, Minipuls 2), through heating coils to bring its temperature to 37 ± 1°C. The perfusion fluid was collected by the fundic collecting funnel and passed to a pH electrode connected to a Jenway pH meter (PHM6). An output was taken from the pH meter to a Rikadenki chart recorder for the on-line recording of the pH of the gastric perfusate.

Pentagastrin was stored as a frozen aliquot and diluted to the required concentrations with sterile 0.9% w/v NaCl. Novel compounds were dissolved in sterile 0.9% w/v NaCl on the day of the experiment. Drugs were administered IV through a cannulated jugular vein as a bolus in a dose volume of 1 ml/kg washed in with 0.15 ml 0.9% w/v NaCl. Basal pH was allowed to stabilize before administration of compounds was begun. Typically 30 minutes elapsed between surgery and the first compound administration.

Example 20 antagonized the stimulation of gastric acid secretion produced by a standard dose of 1 nmole/kg pentagastrin (Figure 1). Example 16 also attenuated the amount of gastric acid secreted in response to a 1 nmole/kg dose of pentagastrin (initial pentagastrin response 254 μmoles/l $H^+$, after Example 16 (cumulative dose of 1.1 μmole/kg) 128 μmoles/l $H^+$). With both compounds the antagonism was reversible with full recovery of the response to pentagastrin.

The compounds of the instant invention are also useful as antiulcer agents as discussed hereinbelow.

Aspirin-induced gastric damage was assessed in groups 10 rats each.

All animals were fasted for 24 hours before and throughout the experiment. Drug or vehicle was given 10 minutes before an oral dose of 1 ml of a 45-mg/ml suspension of aspirin in 0.5% carboxymethylcellulose (CMC).

The animals were sacrificed five hours after aspirin administration and the stomachs removed and opened for examination.

Gastric damage was scored as follows:

| Score | |
|---|---|
| 1 | Small hemorrhage |

(continued)

| Score | |
|---|---|
| 2 | Large hemorrhage |
| 3 | Small ulcer |
| 4 | Large ulcer |
| 5 | Perforated ulcer |

The mean ulcer score in the saline control group was 12.1 ± 6.85 (±SD). Treatment with ranitidine (15 mg/kg PO) inhibited ulcer formation by 74% giving an ulcer score of 3.2 ± 2.35 (p <0.001 compared with controls). Treatment with [R-(R*,R*)-4-[[2-[[3-(1H-indol-3-yl)-2-methyl-1-oxo-2-[[(tricyclo[3.3.1.1$^{3,7}$]dec-2-yloxy)carbonyl]amino]propyl]-amino]-1-phenylethyl]amino]-4-oxobutanoic acid (10 mg/kg PO) resulted in an ulcer score of 6.3 ± 4.14 (p <0.05 compared with controls), a 48% reduction in ulcer formation.

The specific dosages employed, however, may be varied depending upon the patient, the severity of the condition being treated, and the activity of the compound employed. Determination of optimum dosages is within the skill of the art.

The compounds of the instant invention are also useful as anxiolytic agents as described and discussed below.

Figure 2 illustrates the effectiveness of orally administered Example 20 as regards anxiolytic activity. Anxiolytic activity was assessed in the light/dark exploration test in the mouse (B. J. Jones, et al, Br. J. Pharmacol. 93:985-993, 1988).

In Figure 2 the number of mice was 5 and the pretreatment time was 40 minutes. The compound was given p.o. in 0.1, 1, and 10 mg/kg doses.

The apparatus was an open-topped box, 45 cm long, 27 cm wide, and 27 cm high, divided into a small (2/5) area and a large (3/5) area by a partition that extended 20 cm above the walls. There was a 7.5 x 7.5 cm opening in the partition at floor level. The small compartment was painted black and the large compartment white. The floor of each compartment was marked into 9 cm squares. The white compartment was illuminated by a 100-watt tungsten bulb 17 cm above the box and the black compartment by a similarly placed 60-watt red bulb. The laboratory was illuminated with red light.

All tests were performed between 13 hundred hours, 0 minutes and 18 hundred hours, 0 minutes. Each mouse was tested by placing it in the center of the white area and allowing it to explore the novel environment for five minutes. Its behavior was recorded on videotape and the behavioral analysis was performed subsequently from the recording. Five parameters were measured: the latency to entry into the dark compartment, the time spent in each area, the number of transitions between compartments, the number of lines crossed in each compartment, and the number of rears in each compartment.

In this test an increase in the time spent in the light area is a sensitive measure of, that is directly related to, the anxiolytic effects of several standard anxiolytic drugs. Drugs were dissolved in water or saline and administered either subcutaneously, intraperitoneally, or by mouth (PO) via a stomach needle.

Example 20 and compound [R-(R*,R*)]-4-[[2-[[3-(1H-indol-3-yl)-2-methyl-1-oxo-2-([(tricyclo(3.3.1$^{3,7}$]dec-2-yloxy)carbonyl]amino]propyl]amino]-1-phenylethyl]amino]-4-oxobutenoic acid were active by the subcutaneous route. Control animals showed 3% crossings into the dark area over five-minute measurement periods. Mice treated with 1 mg/kg (SC) of compound (20) showed 85 crossings into the light area and only 24 crossings into the dark area, a significant (p <0.01) difference from the control anxious mice. Diazepam (0.25 mg/kg IP) had an effect identical to compound (20) in the same experiment. In additional experiments compound [R-(R*,R*)]-4-[[2-[[3-(1H-indol-3-yl)-2-methyl-1-oxo-2-[[(tricyclo[3.3.1$^{3,7}$]dec-2-yloxy)carbonyl]amino]propyl]amino]-1-phenylethyl]amino]-4-oxobutenoic acid (a mg/kg SC) and compound (20) (1 mg/kg PO) significantly (p <0.01) increased the time spent in the light area of the test box.

The compounds of the instant invention are useful as antipsychotic agents. Example 20 (which is shown as compound (24) in Scheme III) and Example 20A were tested for their ability to reduce the effects of intra-accumbens amphetamine in the rat as described hereinafter.

Male Sprague Dawley (CD) Bradford strain rats were used. The rats were housed in groups of five at a temperature of 21 ± 2°C on a 12 hour light-dark cycle of lights-on between 07 hours 00 minutes and 20 hours 00 minutes. Rats were fed CRM diet (Labsure) and allowed water ad libitum.

Rats were anesthetized with chloral hydrate (400 mg/kg SC) and placed in a Kopf stereotaxic frame. Chronically indwelling guide cannulae (constructed of stainless steel tubing 0.65 mm diameter held bilaterally in Parspex holders) were implanted using standard stereotaxic techniques to terminate 3.5 mm above the center of the nucleus accumbens (Ant. 9.4, Vert. 0.0, Lat. 1.6) or 5.0 mm above the central nucleus of the amygdala (Ant. 5.8, Vert. -1.8, Lat. ±4.5) (atlas of De Groot, 1959). The guides were kept patent during a 14-day recovery period using stainless steel

stylets, 0.3 mm diameter, which extended 0.5 mm beyond the guide tips.

Rats were manually restrained and the stylets removed. Intracerebral injection cannulae, 0.3 mm diameter, were inserted and drugs delivered in a volume of 0.5 µl over 5 seconds (a further 55 seconds was allowed for deposition) from Hamilton syringes attached via polythene tubing to the injection units. Animals were used on a single occasion only.

Behavioral experiments were conducted between 07 hours 30 minutes and 21 hours 30 minutes in a quiet room maintained at 22 ± 2°C. Rats were taken from the holding room and allowed one hour to adapt to the new environment. Locomotor activity was assessed in individual screened Perspex cages (25 x 15 x 15 cm (high) (banked in groups of 30) each fitted with one photocell unit along the longer axis 3.5 cm from the side; this position has been found to minimize spurious activity counts due to, for example, preening and head movements when the animal is stationary. Interruptions of the light beam were recorded every 5 minutes. At this time animals were also observed for the presence of any nonspecific change in locomotor activity, e.g., sedation, prostration, stereotyped movements, that could interfere with the recording of locomotor activity.

The abilities of the Example 20 and 20A to inhibit the hyperactivity caused by the injection of amphetamine into the nucleus accumbens of the rat was measured.

An increase in locomotor activity followed the bilateral injection of amphetamine (20 µg) into the nucleus accumbens; peak hyperactivity (50 to 60 counts 5 $minutes^{-1}$) occurred 20 to 40 minutes after injection.

Intraperitoneal injection of the rats with Examples 20A (20 mg/kg or 30 mg/kg) or Examples 20 (10 mg/kg) reduced the hyperactivity caused by the intra-accumbens injection of amphetamine (Figures 3 and 4). This test is known to be predictive of antipsychotic activity (Costall, Domeney & Naylor & Tyers, Brit J Pharmac 92:881-894).

Figure 3 shows the antagonism of intra-acccumbens amphetamine (20 µg) by Example 20A . The amphetamine control is shown by -□-, the vehicle by -◆-, the -Δ- shows Example 20 at 1 mg/kg IP and -▲- shows the compound at 10 mg/kg IP. The number tested was five. The *P is <0.05. The time in minutes is shown versus activity (counts/5 minutes).

Figure 4 shows the antagonism of intra-accumbens amphetamine (20 µg) for Example 20. The figure is described as for Figure 3 above.

The compounds of the instant invention prevent and treat the withdrawal response produced when chronic treatment by a drug is stopped or when alcohol abuse is stopped. These compounds are therefore useful as therapeutic agents in the treatment of chronic drug or alcohol abuse as discussed and described below.

The effect of the compounds of the instant invention is illustrated, for example, in the mouse "light/dark box" test in Figures 5-12.

In Figure 5, five animals were given nicotine, 0.1 mg/kg i.p. b.d. for 14 days. After a 24-hour withdrawal period, Example 20 was given at 1.0 mg/kg i.p. b.d. The increased time spent in the light area is a sensitive measure of the effect of Example 20 as an agent to treat withdrawal effects from nicotine.

Figure 6 illustrates the effect of long-term treatment and withdrawal from nicotine using Example 20A . Five mice were given nicotine at 0.1 mg/kg i.p. b.d. for 14 days. After a withdrawal period of 24 hours, Example 20A was given at 10 mg/kg i.p. b.d. The effect of Example 20A can be seen in the increase of time spent in the light area.

Figure 7 illustrates the effect of long-term treatment and withdrawal from diazepam with intervention with Example 20.

Five mice were given diazepam, at 10 mg/kg i.p. b.d. for seven days. Withdrawal was for a 24-hour period; Example 20 was given at 1.0 mg/kg i.p. b.d. The increased time spent in the light section shows the effect of Example 20.

Figure 8 illustrates the effect of Example 20A on the long-term treatment and withdrawal from diazepam. Five mice were given diazepam at 10 mg/kg i.p. b.d. for seven days. After a withdrawal period of 24 hours, Example 20A was given at 10 mg/kg i.p. b.d. The amount of time spent in the light section after Example 20A is administered demonstrates the effectiveness of the compound.

Figure 9 illustrates the effect Examples 20A on the long-term treatment and withdrawal from alcohol. Five mice were given alcohol in drinking water 8% w/v for 14 days. After a withdrawal period of 24 hours, Example 20 was given at 1.0 mg/kg i.p. b.d. The amount of time spent in the light section after the compound was administered demonstrates the effectiveness of the compound.

Figure 10 shows the effect of Examples 20A on long-term treatment and withdrawal from alcohol. Five mice were given alcohol in drinking water, 8% w/v for 14 days. After a withdrawal period of 24 hours, Example 20A was given at 10 mg/kg i.p. b.d. The increased time spent in the light section shows the effect of Example 20A on the mice.

Figure 11 illustrates the effectiveness in the long-term treatment and withdrawal from cocaine. Five mice were given cocaine as 1.0 mg/kg i.p. b.d. for 14 days. The increased time in the light section illustrates the effectiveness of Example 20 in the treatment.

Figure 12 shows the effect of long-term treatment and withdrawal from cocaine with the intervention of Example 20A. Five mice were given cocaine at 1.0 mg/kg i.p. b.d. for 14 days after a withdrawal period of 24 hours, Example 20A was given at 1.0 mg/kg i.p. b.d. The effect of intervention with Example 20A is shown by the increase in time spent in the light section.

Figure 13 shows the anxiolytic effects of Example 20 in the Rat Social Interaction Test on a dose range of 0.001 to 1.0 mg/kg when paired rats are dosed s.c. The anxiolytic effect of Example 20 are indicated by the increase in time spent in social interaction compared with the control value C. (Costall, B., University of Bradford)

Figure 14 shows the anxiolytic effects of Example 20 in the Rat Elevated X-Maze Test on a dose range of 0.01 to 1.0 mg/kg s.c. The anxiolytic effect is indicated by the time spent in the open arm end section compared with control C.

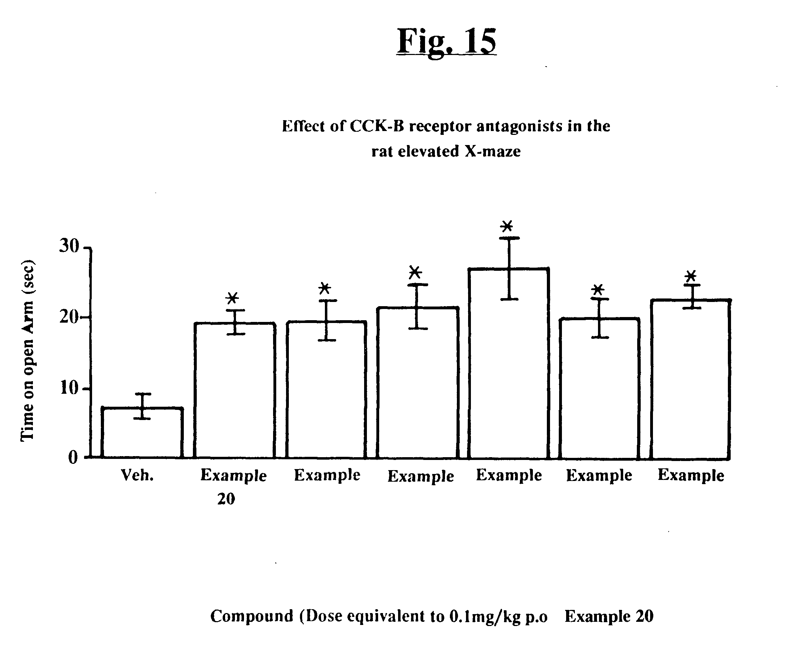

Figure 15 shows the anxiolytic effects of five compounds of the invention as compared to the vehicle alone and to Example 20 in the Rat Elevated X-Maze Test. The dose was equivalent to 0.1 mg/kg p.o. Example 20.

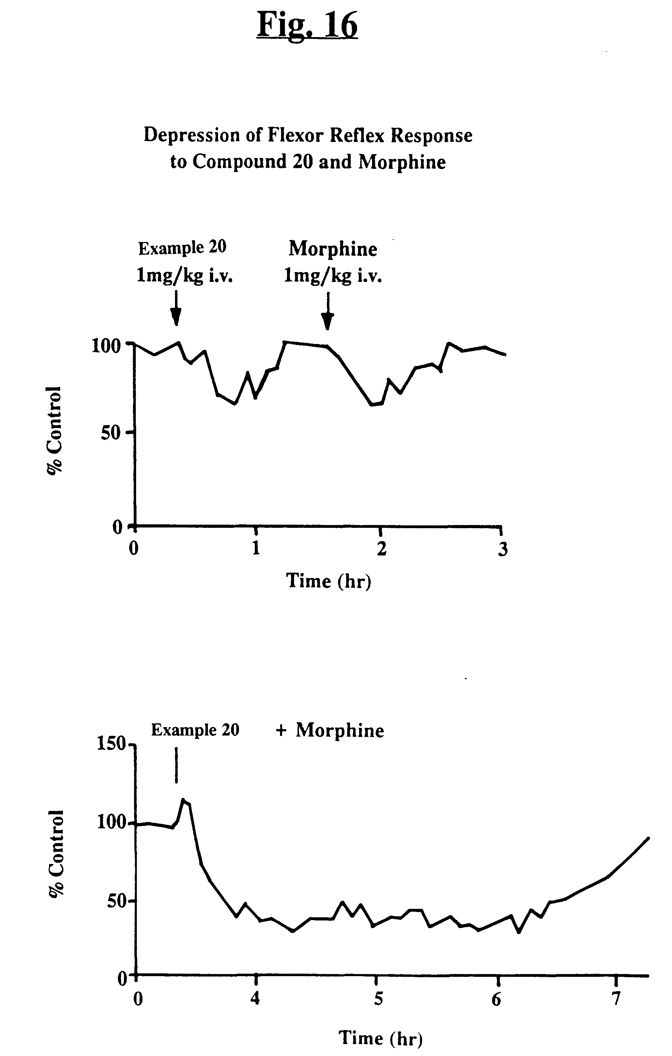

Figure 16 shows that Example 20 depresses the flexor response in a stimulated spinalized decerebrated rat preparation similar to morphine. The effect (lower diagram) of giving Example 20 with morphine greatly potentiates the effect which lasts for 3 hours.

For preparing pharmaceutical compositions from the compounds of this invention, inert, pharmaceutically acceptable carriers can be either solid or liquid. Solid form preparations include powders, tablets, dispersible granules, capsules, cachets, and suppositories.

A solid carrier can be one or more substances which may also act as diluents, flavoring agents, solubilizers, lubricants, suspending agents, binders, or tablet disintegrating agents; it can also be an encapsulating material.

In powders, the carrier is a finely divided solid which is in a mixture with the finely divided active component. In tablets, the active component is mixed with the carrier having the necessary binding properties in suitable proportions and compacted in the shape and size desired.

For preparing suppository preparations, a low-melting wax such as a mixture of fatty acid glycerides and cocoa butter is first melted and the active ingredient is dispersed therein by, for example, stirring. The molten homogeneous mixture is then poured into convenient sized molds and allowed to cool and solidify.

The powders and tablets preferably contain 5 to about 70% of the active component. Suitable carriers are magnesium carbonate, magnesium stearate, talc, lactose, sugar, pectin, dextrin, starch, tragacanth, methyl cellulose, sodium carboxymethyl cellulose, a low-melting wax, cocoa butter, and the like.

A preferred pharmaceutically acceptable salt is the N-methyl glucamine salt.

The term "preparation" is intended to include the formulation of the active component with encapsulating material as a carrier providing a capsule in which the active component (with or without other carriers) is surrounded by a carrier which is thus in association with it. Similarly, cachets are included.

Tablets, powders, cachets, and capsules can be used as solid dosage forms suitable for oral administration.

Liquid form preparations include solutions, suspensions, and emulsions. Sterile water or water-propylene glycol solutions of the active compounds may be mentioned as an example of liquid preparations suitable for parenteral administration. Liquid preparations can also be formulated in solution in aqueous polyethylene glycol solution.

Aqueous solutions for oral administration can be prepared by dissolving the active component in water and adding suitable colorants, flavoring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known to the pharmaceutical formulation art.

Preferably the pharmaceutical preparation is in unit dosage form. In such form, the preparation is divided into unit doses containing appropriate quantities of the active component. The unit dosage form can be a packaged preparation, the package containing discrete quantities of the preparation, for example, packeted tablets, capsules, and powders in vials or ampoules. The unit dosage form can also be a capsule, cachet, or tablet itself, or it can be the appropriate number of any of these packaged forms.

Examples A-I are illustrative of methods of preparing the precursors or intermediates of the final products which are illustrated in Examples 1-45 (corresponding to compounds 1-45 described in the figures and experimental) but not as numbers corresponding to the numbers given in the schemes.

Intermediate Example A

<u>N-[(1-Adamantyloxyl carbonyl]-α-methyl-DL-tryptophan.</u>

To a solution of α-methyl-<u>DL</u>-tryptophan (2.18 g, 10 mmol) in 1<u>M</u> NaOH solution (10 ml) at 0°C was added $NaHCO_3$ (0.92 g, 11 mmol) followed by a solution of 1-adamantylfluoroformate (2.18 g, 11 mmol) in 1,4 dioxan (10 ml). The mixture was stirred at 0°C for one hour and then 24 hours at room temperature.

The dioxan was removed <u>in vacuo</u> and the aqueous phase extracted with three portions of ether (30 ml). The aqueous phase was cooled in ice and covered with ethyl acetate (30 ml) before acidifying to pH 2-3 with sodium hydrogen sulphate solution. Following a further two organic or ethyl acetate extractions, the organic layers were combined, washed with water (30 ml), and dried over $MgSO_4$. Ethyl acetate was removed <u>in vacuo</u> to give 1-adamanty-

loxycarbonyl-α-methyl-DL-tryptophan (1.154 g, 29%) as a white solid, recrystallized from ethyl acetate, mp 206-218°C (EtOAc); IR (film) 1681 $cm^{-1}$; NMR ($CD_3OD$) δ 1.43 (3H, s), 1.68 (6H, br.s), 2.13 (9H, br.s), 3.35 (2H, ABq J 14Hz), 6.95-7.56 (5H, m).

Intermediate Example B

2-Adamantylchloroformate.

To a stirred solution of 2-adamantanol (0.912 g, 6 mmol) in dry $CH_2Cl_2$ (15 ml) was added bis(trichloromethyl) carbonate (0.653 g), pyridine in dry $CH_2Cl_2$ (10 ml) at 0°C. The reaction mixture was warmed to room temperature and stirred for two hours. The solvent was removed in vacuo at 30°C, taken up in ethyl acetate (30 ml) and stirred for 10 minutes. The pyridinium hydrochloride precipitate was filtered off and the solvent removed in vacuo at 30°C, to give an oil which solidified upon standing (1.29 g, 100%). IR (film) 1778 $cm^{-1}$; NMR ($CDCl_3$) δ 1.55-1.65 (2H, m), 1.70-1.80 (4H, m), 1.85-1.95 (4H, m), 2.00-2.10 (2H, m), 2.15-2.20 (2H, m), 5.02 (1H, 6, J 3.3Hz CHOCOCl).

Intermediate Example C

N-[(2-Adamantyloxy)carbonyl]-α-methyl-D-tryptophan methyl ester.

To a stirred solution of 2-adamantylchloroformate (0.965 g, 4.5 mmol) in dry THF (10 ml) was added a solution of α-methyl-D-tryptophan methyl ester (0.928 g, 4 mmol) in dry THF (20 ml) followed by a solution of triethylamine (0.808 g, 8 mmol) in dry THF (20 ml) dropwise. After 15 minutes, the reaction mixture was filtered, the solvent removed in vacuo and column chromatographed using 2% MeOH:98% $CH_2Cl_2$ as eluant to yield the title compound (1.42 g, 89%) as a syrup. IR (film) 1740-1695 b.r $cm^{-1}$; NMR ($CDCl_3$) δ 1.50-1.60 (2H, m), 1.67 (3H, s), 1.70-2.10 (12H, m), 3.38 (1H, d, J = 14.5Hz), 3.50-3.60 (1H, br.s), 3.68 (3H, s), 4.86 (1H, br.s), 5.28 (1H, br.s), 6.93 (1H, d, J 2.4Hz); 7.04-7.10 (2H, m), 7.33 (1H, d, J 8.2Hz) 7.54 (1H, d, J 7.8Hz), 8.18 (1H, br.s)

Intermediate Example D

N-[(2-Adamantyloxyl)carbonyl]-α-methyl-D-tryptophan.

To a stirred solution of N-[(2-adamantyloxy)carbonyl]-α-methyl-D-tryptophan methyl ester (1.36 g, 3.3 mmol) in aqueous 1,4-dioxan (1:2) (20 ml) was added an excess of LiOH (0.210 g, 5 mmol) and stirred at room temperature overnight. After removing the solvent in vacuo the residue was chromatographed using 5% MeOH:95% CH2Cl2 then 10% MeOH:90% $CH_2Cl_2$ as eluants to yield the acid (0.953 mg, 90%) as a white solid, crystallized from n-hexane, mp 210-215°C (EtOAc/n-hexane); IR (film) 1689 $cm^{-1}$; NMR ($CDCl_3$-$D_2O$), δ 1.3-2.2 (14H, m), 1.70 (3H, s), 3.26 (1H, d, J 13.5Hz), 3.63 (1H, d, 13.5Hz); 4.77 (1H, br.s), 6.85-7.60 (5H, m).

Intermediate Example E

(±)-9H-Fluoren-9-ylmethyl [1-(1H-indol-3-ylmethyl)-1-methyl-2-oxo-2-[(2-phenylethyl)aminolethyl)carbamate.

To a solution of N-[(9H-fluoren-9-ylmethyloxy)-carbonyl]-α-methyl-DL-tryptophan (8.80 g, 20 mmol) in dry ethyl acetate (350 ml) was added pentafluorophenol (3.68 g, 20 mmol) and stirred for 10 minutes. The reaction mixture was cooled to 0°C and a solution of dicyclohexylcarbodiimide (20 mmol) in ethyl acetate (25 ml) was added dropwise. This solution was stirred for one hour at 0°C then at room temperature for four hours before leaving it at 4°C overnight. The mixture was filtered and the precipitate washed with cold ethyl acetate (30 ml) and a solution of 2-phenethylamine (2.66 g, 22 mmol) in ethyl acetate (30 ml) was added dropwise to the combined filtrates. The mixture was left to stir for 48 hours at room temperature. The reaction mixture was filtered and the residue washed with cold ethyl acetate (2 x 30 ml) to give the title compound (3.73 g, 75%). The filtrates were combined and the solvent removed in vacuo and taken up again in ethyl acetate (5 ml) to give a second crop of 1.67 g (15%), a total of 90% yield as a white solid, mp 179-181°C (EtOAc); IR (film) 1708, 1652 $cm^{-1}$; NMR (DMSO $d_6$) δ 1.30 (3H, s), 2.64 (2H, t, J 7.2Hz), 3.2-3.3 (4H, m), 4.19 (1H, t, J 6.7Hz), 4.25-4.40 (2H, m), 6.9-7.9 (20 H, m), 10.8 (1H, s).

Intermediate Example F

(±)-α-amino-α-methyl-N-(2-phenylethyl)-1H-indole-3-propanamide.

(±)-9H-Fluoren-9-ylmethyl [1-(1H-indol-3-ylmethyl)-1-methyl-2-oxo-2-[(2-phenylethyl)amino]ethyl]carbamate (10 g, 18.4 mmol) was dissolved in a 20% piperidine in DMF solution (50 ml) and stirred for 12 hours at room temperature. The solvent was removed in vacuo and chromatographed over silica gel using $CH_2Cl_2$ then 5% MeOH:95% $CH_2Cl_2$ as eluants. The title compound was crystallized from ethyl acetate (4.73 g, 80%), mp 106-110°C (EtOAc); IR (film) 1646 $cm^{-1}$; NMR ($CDCl_3$) δ 1.39 (3H, s), 2.56-2.74 (2H, m), 2.82 (1H, d, J 14Hz), 3.28-3.40 (1H, m), 3.48 (1H, d, J 14Hz), 3.44-3.53 (1H, m), 7.1-7.7 (11H, m), 8.3 (1H, s); Anal. ($C_{20}H_{23}N_3O$) C, H, N.

Intermediate Example G

9H-Fluoren-9-ylmethyl-[2-[[1-(hydroxymethyl)-2-phenylethyl]amino]-1-(1H-indo1-3-ylmethyl)-1-methyl-2-oxoethyl] carbamate, mixture of [S-(R*,R*)] and (R-(R*,S*)] isomers.

A solution of N-[(9H-fluoren-9-ylmethoxy)carbonyl]-α-methyl-DL-tryptophan (10 g, 22.7 mmol) and pentafluorophenol (4.18 g, 22.7 mmol) in dry ethyl acetate (200 ml) was treated dropwise at 0°C with a solution of dicyclohexylcarbodiimide (4.9 g, 24 mmol) in ethyl acetate (20 ml). This was allowed to warm to room temperature and stirred for a further hour. This mixture was then treated with a solution of L-phenylalaninol (3.775 g, 25 mmol) in ethyl acetate (15 ml) dropwise and the resultant mixture left stirring for 15 hours. This mixture was filtered and the filtrate washed sequentially with 2M citric acid solution, 1M NaOH solution, saturated $NaHCO_3$ solution then water before being dried over $MgSO_4$ and concentrated to an oil in vacuo. This oil was subjected to silica gel chromatography using 4% MeOH: 96% $CH_2Cl_2$ as eluant to give the title compound (11.7 g, 90%), as a white solid and a mixture of two diastereoisomers. These two diastereoisomeric forms were separated by further chromatographic purification using 1% i PrOH, 99% $CHCl_3$ as the eluant to give equal amounts of the pure diastereoisomers as white amorphous solids.

Isomer I
[R-(R*,S*)]-9H-Fluoren-9-ylmethyl [2-[[1-(hydroxymethyl)-2-phenylethyl]amino]-1-(1H-indol-3-ylmethyl]-1-methyl-2-oxoethyl]carbamate.
mp 89-93°C ($CHCl_3$); IR (KBr) 1696, 1651 $cm^{-1}$; NMR ($CDCl_3$) δ 1.35 (3H, s) 2.74 (2H, m), 3.30 (2H, Abq, J, 14.5 Hz), 3.45 (1H, dd, J 11 and 6Hz), 3.70 (1H, m), 4.14 (2H, m), 4.46 (2H, dq, J 10.5 and 6Hz), 5.09 (1H, s), 6.10 (1H, d, J 8Hz), 6.65 (1H, d, J 2Hz), 7.07-7.80 (17H, m) 7.98 (1H, s); Anal. ($C_{36}H_{35}N_3O_4$), C, H, N.

Isomer II
[S-(R*,R*)]-9H-Fluoren-9-ylmethyl [2-[[1-(hydroxymethyl)-2-phenylethyl]amino]-1-(1H-indol-3-ylmethyl)-1-methyl-2-oxoethyl]carbamate, mp 89-93°C ($CHCl_3$); IR (KBr) 1703 and 1646 $cm^{-1}$; NMR ($CDCl_3$) δ 1.50 (3H, s), 2.70 (2H, dq, J 14 and 8Hz), 3.20 (2H, Abq J 14.5Hz) 3.41 (1H, dd, J 11.5 and 5Hz), 3.60 (1H, dd, J 11.5 and 3.5Hz), 4.12 (2H, m), 4.35 (2H, m), 5.37 (1H, s), 6.06 (1H, d, J 8Hz), 6.75 (1H, d, J 2Hz), 7.08-7.77 (17H, m), 8.07 (1H, s); Anal. ($C_{36}H_{35}N_3O_4 \cdot 0.25H_2O$) C, H, N.

Intermediate Example H

(R)-Tricyclor[3.3.1$^{3,7}$]dec-2-yl [2-[[2-hydroxy-2-phenylethyl]amino,]-1-(1H-indol-3-ylmethyl)-1-methyl-2-oxoethyl] carbamate.

A solution of 2-adamantyloxycarbonyl-α-methyl-D-tryptophan (0.060 g, 0.15 mmol) in ethyl acetate (7 ml) was treated with dicyclohexylcarbodiimide (0.034 g, 0.165 mmol) and 1-hydroxybenzotriazole (0.022 g, 0.163 mmol). After stirring for two hours at room temperature, 2-amino-1-phenyl ethanol (0.021 g, 0.153 mmol) in ethyl acetate (2 ml) was added and the reaction mixture stirred for a further two hours. The suspension was then filtered and the filtrate concentrated in vacuo to leave a colorless gum (0.175 g). The crude product was chromatographed over alumina using 80% EtOAc:20% n-hexane as eluant, to give the title compound as a slightly impure white solid (0.058 g, 74%); IR (film) 3338, 2927, 2855, 1690 and 1622 $cm^{-1}$; NMR (inter alia) ($CDCl_3$) δ 1.50-2.05 (17H, m), 3.15-3.55 (4H, m), 3.75 (1H, m), 4.85 (1H, m), 5.10 and 5.20 (each 0.5H, s), 6.55 (1H, m), 7.00-7.40 (9H, m), 7.60 (1H, d, J 9Hz) 8.15 (1H, 2s).

Intermediate Example I

(4-Nitrophenyl) methyl[1R-(1α,2α, 3β)]-2-[(chlorocarbonyl)-oxy]-1, 7, 7-trimethylbicyclo[2.2.1]heptane-3-acetate.

Method as for Intermediate Example B except using [IR-(2-endo, 3-exo]-3-hydroxy-4,7,7-trimethyl bicyclo [2.2.1]heptane-2-acetic acid, para nitro benzyl ester ; IR (film) 1773 and 1741 $cm^{-1}$; NMR ($CDCl_3$) δ 0.88 (3H, s), 0.89 (3H, s), 1.05 (3H, s), 1.06-1.15 (1H, m), 1.25-1.40 (1H, m), 1.50-1.80 (3H, m), 2.45 (1H, dd, J 7 and 15Hz), 2.55-2.85 (2H, m), 4.41 (1H, d, J 4Hz), 5.20 (2H, s), 7.50 (2H, d, 8Hz), 8.22 (2H, d, J 8Hz).

Example 1

(±)-Tricyclo[3.3.1$^{3,7}$]dec-1-yl [1-(1H-indol-3-ylmethyl)-1-methyl-2-oxo-2-[(2-phenylethyl)amino]ethyl]carbamate.

To a solution of N-[(tricyclo[3.3.1.13,7]dec-1-yloxy)carbonyl]-α-methyl-DL-tryptophan (1.0 g, 2.5 mmol) in 1,4 dioxan (40 ml) was added a solution of pentafluorophenol (0.465 g, 2.5 mmol) in 1,4 dioxan (5 ml) and stirred at room temperature for 15 minutes, cooled to 0°C and a solution of dicyclohexylcarbodiimide (0.547 g, 2.65 mmol) in 1,4 dioxan (10 ml) was added dropwise. This was allowed to stir at room temperature for two hours before phenethylamine (0.333 g, 2.75 mmol) was added in one portion. The mixture was left stirring for 24 hours.

The reaction mixture was filtered before removing the solvent in vacuo, and the residue taken up in ethyl acetate (30 ml) and washed with 1M citric acid solution (2 x 10 ml), saturated $NaHCO_3$ solution (3 x 10 ml), 1M NaOH solution (2 x 10 ml), brine (2 x 10 ml), and water (2 x 20 ml). The organic phase was dried over $MgSO_4$ and the solvent evaporated in vacuo to yield a white solid (0.617 g, 49%), mp 84-86°C (EtOAc); IR (film) 1700, 1660 $cm^{-1}$; NMR ($CDCl_3$), δ 1.50 (3H, s), 1.63 (6H, br.s), 2.00-2.05 (6H, m), 2.14 (3H, br.s), 2.66 (1H, t, J 7.2Hz), 2.67 (1H, t, J 6.9Hz), 3.19 (1H, d, J 14.5Hz), 3.4-3.50 (3H, m), 4.93 (1H, br.s), 6.30 (1H, br.s), 6.98-7.60 (10H, m), 8.24 (1H, br.s).

Example 2

(±)-Trans-2-chlorocyclohexyl [1-(1H-indol-3-ylmethyl)-1-methyl-2-oxo-2-[(2-phenylethyl)amino]ethyl]carbamate.

To a stirred solution of trans(±)-2-chlorocyclohexyl chloroformate (0.16 g, 0.75 mmol) in anhydrous THF (5 ml) at room temperature was added dropwise a solution of α-methyl-DL-tryptophylphenethylamide (0.23 g, 0.7 mmol) in THF (5 ml), followed by a solution of triethylamine (0.07 g, 0.7 mmol) in THF (5 ml). The reaction was complete after 30 minutes by thin layer chromatographic analysis. The solvent was removed in vacuo and the residue taken up in ethyl acetate (30 ml) and washed successively with 1M aqueous citric acid (2 x 20 ml), saturated $NaHCO_3$ solution (2 x 20 ml), 1M NaOH solution (20 ml) and water (4 x 20 ml). The organic phase was dried over $MgSO_4$ and filtered. Removal of the solvent by vacuum distillation gave the title compound (0.273 g, 81%), a white solid crystallized from ether-hexane, mp 69-78°C (ether-hexane); IR (film) 1709 and 1656 $cm^{-1}$; NMR ($CDCl_3$) δ 1.2-1.4 (3H, m), 1.54 (3H, s), 1.6-1.8 (3H, m), 2.03-2.23 (2H, m), 2.63-2.69 (2H, m), 3.2-3.5 (4H, m), 3.72-3.79 (1H, m), 4.67-4.73 (1H, m), 5.23 (1H, br.s), 6.1-6.2 (1H, m), 7.0-7.6 (10H, m), 8.08 (1H, br.s); Anal. ($C_{27}H_{32}N_3O_3Cl$), C, H, Cl, N.

Example 3

(±)-Trans-2-chlorocyclohexyl [2-[[1-(hydroxymethyl)-2-phenylethyl]amino]-1-(1H-indol-3-ylmethyl)-1-methyl-2-oxoethyl]carbamate. (D-tryptophan residue; L-phenylalanine residue).

To a stirred solution of (±)-trans-2-chlorocyclohexyl chloroformate (1.94 g, 9.1 mmol) in anhydrous THF (10 ml) at room temperature was added dropwise a solution of α-methyl-D-tryptophan-L-phenylalaninol (2.9 g, 8.3 mmol) in THF (20 ml), followed by a solution of triethylamine (9.92 g, 9.1 mmol) in THF (10 ml). The reaction was complete after 30 minutes as assayed by thin layer chromatography. The reaction mixture was filtered and the solvent removed in vacuo. The residue was purified by chromatography over silica using $CH_2Cl_2$ then 4% MeOH:93% $CH_2Cl_2$ as eluants. Recrystallization from ethyl acetate gave the product (3.1 g, 73%) as white needles, mp 117-127°C (EtOAc); IR (film) 1699 and 1600 $cm^{-1}$; NMR ($CDCl_3$) δ 1.20-1.45 (3H, m), 1.32 (3H, s), 1.40 (3H, s), 1.70-1.80 (3H, m), 2.09-2.25 (2H, m), 2.67-2.83 (2H, m); 3.28-3.52 (3H, m); 3.68-3.83 (2H, m), 4.10-4.30 (1H, m), 4.68-4.80 (1H, m), 5.97 (1H, s), 6.08 (1H, s), 6.09 1H, d, J 7.9 Hz), 6.19 (1H, d, J 7.6 Hz), 6.91-7.60 (10H, m), 8.08 (1H, m); Anal. ($C_{28}H_{34}N_3O_4Cl\cdot0.25$ $H_2O$), C, H, N, Cl.

Example 4

2-[[2-[[[2-chlorocyclohexyl)oxy]-carbonyl]amino]-3-(1H-indol-3-yl)-2-methyl-1-oxo-propyl]amino]-3-phenylpropyl butanedioate.

A solution of 2-chlorocyclohexyl [2-[[1-(hydroxymethyl)-2-phenylethyl]amino]1-(1H-indol-3-ylmethyl)-1-methyl-2-oxoethyl]carbamate (1.3 g, 2.54 mmol), succinic anhydride (0.254 g, 2.54 mmol) and 4-N,N-dimethylaminopyridine (0.62 g, 5.08 mmol) in dry ethyl acetate (50 ml) was refluxed for 18 hours. The reaction mixture was then washed with 1M citric acid solution, then water and dried over $MgSO_4$. Concentration in vacuo yielded an oil which was subjected to silica gel chromatography using 10% MeOH:90% $CH_2Cl_2$ as eluant to give the title compound (0.86 g, 55%) as an amorphous solid, mp 75°C (EtOAc-hexane); IR (film) 3370, 1723 and 1659 $cm^{-1}$; NMR ($CDCl_3$) δ 1.30 (3H, m), 1.45 (1.5H, s), 1.58 (1.5H, s), 1.66 (3H, m) 2.16 (2H, m), 2.60 (5H, m), 2.79 (1H, dd J 11 and 6 Hz), 3.28 (2H, Abq $J_{AB}$ 14.5 Hz);3.85 (3H, m), 4.45 (1H, m), 4.70 (1H, m), 5.45 (1H, br.s), 6.5 (1H, m), 6.90-7.70 (10H, M), 8.37 (0.5H, s) and 8.49 (0.5H, s). Anal. ($C_{32}H_{38}N_3O_7Cl$), C, H, N, Cl.

Example 5

[R-(R*,S*)]-N-[1-(hydroxymethyl)-2-phenylethyl]-α-methyl-α-[(tricyclo[3.3.1.1$^{3,7}$]dec-1-ylacetyl)-amino]-1H-indole-3-propanamide.

A solution of α-methyl-D-tryptophyl-L-phenylalaninol (1 g, 2.85 mmol) and 4-N,N-dimethylaminopyridine (.35 g, 2.87 mmol) in dry THF (50 ml) at 0°C was treated dropwise, with stirring, with a solution of 1-adamantylacetyl chloride (0.605 g, 2.85 mmol). A precipitate formed immediately. The reaction mixture was left until all starting materials were consumed as assayed by TLC and IR spectroscopy. The final TLC showed three spots (10% MeOH:90% $CH_2Cl_2$). The reaction mixture was washed with 1M citric acid solution and extracted into ethyl acetate. The organic phase was then washed with water and dried over $MgSO_4$. Concentration in vacuo gave a syrup (1.7 g) which was chromatographed over silica using 2% MeOH:98% $CH_2Cl_2$ as eluant to yield the title compound (1.35 g, 90%) as a white solid crystallized from ethyl acetate-hexane, mp 91-94°C (EtOAc-hexane); IR (KBr) 3304 and 1652 $cm^{-1}$; NMR ($CDCl_3$) δ 1.48 (9H, m), 1.59 (6H, m), 1.76 (2H, q, J 13 Hz), 1.9 (3H, m), 2.74 (2H, d, J 7Hz), 3.21 (1H, half ABq J 14.5Hz), 3.30 (1H, 6, J 6Hz), 3.40 91H, half Abq J 14.5Hz), 3.45 (1H, m), 3.70 (1H, m), 4.16 (1H, m), 5.91 (1H, s), 6.38 (1H, d, J 8Hz), 6.92 (1H, d, J 3Hz), 7.07-7.27 (7 H, m), 7.35 (1H, d, J 8Hz), 7.56 (1H, d, J 8Hz) and 8.54 (1H, s); Anal. ($C_{33}H_{41}N_3O_3 \cdot 0.25\ H_2O$), C, H, N.

Example 6

(±)-Tricyclo[3.3.1.1$^{3,7}$]dec-2-yl [1-(1H-indol-3-ylmethyl)-1-methyl-2-oxo-2[(2-phenylethyl) amino]-ethyl]carbamate.

Method was as described for Example 2 but using 2-adamantyl chloroformate. The product was obtained as a solid from $CCl_4$-hexane (0.385 g, 77%), mp (noncrystalline) 79-85°C; IR (film) 1701 and 1656 $cm^{-1}$, NMR ($CDCl_3$) δ 1.5-1.6 (2H, m), 1.54 (3H, s), 1.7-2.0 (12H, m), 2.6 (2H, t, J 7Hz), 3.26 (1H, d, J 14.5Hz), 3.40-3.50 (3H, m), 4.79 (1H, br.s), 5.15 (1H, br.s), 6.20 (1H, t), 6.95-7.11 (10H, m), 8.08 (1H, s); Anal. ($C_{31}H_{37}N_3O_3$), C, H, N.

Example 7

(±)-Endo-1,7,7-trimethylbicyclo[2.2.1]hept-2-yl-[1-(1H-indol-3-ylmethyl)-1-methyl-2-oxo-2-[(2-phenylethyl)amino] ethyl]-carbamate.

Method was as described for Example 2, but using 1-(S)-2-endobornyl chloroformate. The crude residue was chromatographed over silica using $CHCl_3$ as eluant to obtain the product (0.443 g, 88%) as a colorless foam, mp (noncrystalline) 65-69°C; IR (film) 3327, 1702 and 1658 $cm^{-1}$; NMR ($CDCl_3$) δ 0.81 (3H, s), 0.85 (3H, s), 0.89 (3H, s), 0.96-1.02 (1H, m), 1.11-1.30 (3H, m), 1.54 (1.5H, s), 1.54 (1.5H, s), 1.65-1.82 (2H, m), 2.32 (1H, m), 2.65 (2H, t, J 7Hz), 3.25 (1H, half ABq, J 14.5Hz), 3.39-3.49 (3H, m), 4.84 (1H, m), 5.21 (1H, br.s), 6.14 (1H, br.s), 6.95 (1H, d, J 2Hz), 7.03-7.26 (7H, m), 7.35 (1H, d, J 8Hz), 7.58 (1H, d, J 8Hz) and 8.18 (1H, s).

Example 8

(±)-Exo-1,7,7-trimethylbicyclo[2.2.1]hept-2-yl-[1-(1 H-indol-3-ylmethyl)-1-methyl-2-oxo-2-[(2-phenyl-ethyl)amino] ethyl]-carbamate.

Method was as described for Example 2, but using (±)-exo-bornyl chloroformate. The crude residue was chromatographed over silica using $CHCl_3$ as eluant to give the title product as a pale yellow foam (0.294 g, 59%), mp (noncrystalline) 61-65°C; IR (film) 1705 and 1658 $cm^{-1}$; NMR ($CDCl_3$) δ 0.75-1.30 (13H, m), 1.45-1.82 (6H, m), 2.63 (2H, m), 3.23 (1H, half ABq J 14.5Hz), 3.35-3.52 (3H, m), 4.56 (1H, m), 5.18 (0.5H, s), 5.25 (0.5H, s), 6.16 (1H, m), 6.95 (1H, d, J 2Hz), 6.99-7.25 (7H, m), 7.34 (1H, d, J 8Hz), 7.57 (1H, d, J 8Hz), and 8.19 (1H, s).

Example 9

(±)-Exo-bicyclo[2.2.1]hept-2-yl [1-(1H-indol-3-ylmethyl)-1-methyl-2-oxo-2-[(2-phenylethyl)amino]ethyl]carbamate.

Method was as described for Example 2 but using (±) exo-norbornyl chloroformate. The crude residue was chromatographed over silica using $CH_2Cl_2$ then 2% MeOH:98% $CH_2Cl_2$ as eluants to yield the title compound (0.346 g, 75%) as a colorless foam, mp (noncrystalline) 74-78°C; IR (film) 3341, 1703 and 1656 $cm^{-1}$; NMR ($CDCl_3$) δ 1.06-1.16 (3H, m), 1.33-1.51 (3H, m), 1.53 (1.5H, s), 1.54 (1.5H, s), 1.65-1.70 (2H, m), 2.24 (2H, br.s), 2.65 (2H, m), 3.21 (1H, half ABq J 14.5Hz), 3.39-3.47 (3H, m), 4.51 (1H, d, J 6.5Hz), 5.09 (1H, s), 6.15 (1H, br.s), 6.95 (1H, d, J 2Hz), 7.03-7.25 (7H, m), 7.35 (1H, d, J 8Hz), 7.57 (1H, d, J 8Hz), 8.24 (1H, s); Anal. ($C_{28}H_{33}N_3O_3 \cdot 0.25$ $H_2O$), C, H, N.

Example 10

(±)-Endo-bicyclo[2.2.1]hept-2-yl [1-(1H-indol-3-ylmethyl)-1-methyl-2-oxo-2-[(2-phenylethyl)amino]ethyl]carbamate.

Method was as described for Example 2, but using (±)-endo-norbornyl chloroformate. The crude residue was chromatographed over silica using 50% EtOAc:50% n-hexane as eluant to obtain the title compound (0.318 g, 69%) as a colorless foam, mp (noncrystalline) 62-68°C; IR (film) 3325, 1703, and 1654 $cm^{-1}$; NMR ($CDCl_3$) δ 0.94 (1H, m), 1.19-1.40 (4H, m), 1.48-1.72 (5H, m), 1.95 (1H, m), 2.19 (1H, br.s), 2.43 (1H, br.s), 2.65 (2H, t, J 7Hz), 3.23 (1H, half ABq J 14.5Hz), 3.39-3.48 (3H, m), 4.88 (1H, m), 5.17 (0.5H, s), 5.21 (0.5H,s), 6.16 (1H, m), 6.94 (1H, d, J 2Hz), 7.04-7.25 (7H, m), 7.35 (1H, d, J 8Hz), 7.57 (1H, d, J 8Hz), 8.16 (1H, s); Anal. ($C_{28}H_{33}N_3O_3 \cdot 0.75$ $H_2O$), C, H, N.

Example 11

2.5-Methano-1H-inden-7-yl [2-[[1-[hydroxymethyl)-2-phenylethyl]amino]-1-(1H-indol-3-ylmethyl)-1-methyl-2-oxoethyl] carbamate.

Synthetic method was as described for Example 3 but using 4-protoadamantylchloroformate. The product was chromatographed over silica using 4% MeOH:96% $CH_2Cl_2$ as eluant to give the title compound (80%) as a white amorphous solid and as a mixture of two diastereoisomers (about the protoadamantane; D-tryptophan residue). mp 90-92°C (EtOAc-hexane), (IR) film) 3318, 1691 and 1662 $cm^{-1}$; NMR ($CDCl_3$) δ 1.34 (1.5H, s), 1.36 (1.5H, s), 1.3-2.5 (14H, m), 2.74-2.78 (2H, m), 3.13 (1H, br.s), 3.43 (1H, m), 3.6T (1H, m), 4.17 (1H, br.s), 4.95 (1H, dt, J 3 and 8Hz), 5.03 (0.5H, s), 5.06 (0.5H, s), 6.22 (1H, d, J 8Hz), 6.89 (1H, s), 7.05-7.26 (7Y, m), 7.33 (1H, d, J 8Hz), 7.54 (1H, d, J 8Hz) and 8.51 (1H, br.s); Anal. ($C_{32}H_{39}N_3O_4$), C, H, N.

Example 12

2-[3-1H-indol-3-yl)-2-methyl-2-[[[(octahydro-2,5-methano-1H-inden-7-yl)oxy]carbonyl]amino]-1-oxopropyl]-3-phenylpropyl butanedioate.

Synthetic method as described for Example 4 except using the alcohol from Example 11. Product chromatographed over silica using 2% MeOH, 98% $CHCl_3$ as eluant to give a white amorphous solid (80%) and a mixture of two diastereoisomers (about protoadamantane), mp 56-57°C (EtOAc-hexane); IR (film) 1724 and 1659 $cm^{-1}$; NMR ($CDCl_3$) δ 1.25-2.50 (17H, m), 2.59 (6H, m), 3.25 (2H, 2x ABq, J 14.5Hz), 3.91 (2H, m), 5.51 (1H, br), 6.62 (1H, m), 6.92-7.57 (10H, m), 8.65 (1H, br.s), and 9.04 (1H, br); Anal. ($C_{36}H_{43}N_3O_7 \cdot 1.25H_2O$), C, H, N.

Example 13

(R)-Tricyclo[3.3.1.1$^{3,7}$]dec-1-yl-[1-(1 H-indol-3-ylmethyl)-1-methyl-2-oxo-2-[(2-phenylethyl)amino]ethyl]carbamate.

Synthetic method was as described for Example 1 but using 2-adamantyloxycarbonyl-α-methyl-D-tryptophan. The product was chromatographed over silica using 4% MeOH:96% $CH_2Cl_2$ as eluant to give the title compound (0.13 g, 26%) as a white solid, mp 82-88°C ($CHCl_3$-hexane); IR (film) 1699 and 1659 $cm^{-1}$; NMR ($CDCl_3$) δ 1.5-1.6 (17H, m), 2.67 (2H, 6, J 7Hz), 3.26 (1H, d, J 14.5Hz), 3.4-3.5 (3H, m), 4.80 (1H, br.s), 5.15 (1H, br.s), 6.17 (1H, br.s), 6.95-7.60 (10H, m) and 8.05 (1H, br.s); Anal. ($C_{31}H_{37}N_3O_3 \cdot 0.25H_2O$), C, H, N.

Example 14

(±)-trans-2-Chlorocyclohexyl-[2-[[1-(hydroxymethyl)-2-phenylethyl]amino]-1-(1H-indol-3ylmethyl]-1-methyl-2-oxoethyl]-carbamate.

Synthetic method was as described for Example 3, except α-methyl-L-tryptophan-L-phenylalaninol was used. The product was chromatographed over silica using 4% MeOH:96% $CH_2Cl_2$ as eluant to give the title compound (60%) as a colorless foam; mp (noncrystalline) 82-86°C; IR (film) 3402, 1703 and 1657 $cm^{-1}$; NMR ($CDCl_3$) δ 1.32 (3H, m), 1.54 (1.5H, s), 1.57 (1.5H, s), 1.58-1.75 (4H, m), 2.04 (1H, m), 2.20 (1H, m), 2.66 (2H, m), 3.15 (1H, half ABq, J 14.5Hz), 3.26 (1H, half ABq, J 14.5Hz), 3.45 (1H, dd, J 6 and 11Hz), 3.60 (0.5H, m), 3.75 (1.5H, m), 4.05 (0.5H, m), 4.17 (0.5H, m), 4.70 (1H, m), 5.27 (0.5H, s), 5.29 (0.5H, s), 6.12 (1H, m), 6.88 (0.5H, d, J 2Hz), 6.92 (0.5H, d, J 2Hz), 7.08-7.28 (7H, m), 7.30 (1H, d, J 8Hz), 7.57 (1H, d, J 8Hz), and 8.13 (1H, br.s); Anal. ($C_{28}H_{34}N_3O_4Cl$), C, H, N, Cl.

Example 15

[R-(R*,S*)]-Tricyclo[3.3.1.1$^{3,7}$]dec-2-yl-[2-[[1-(hydroxymethyl)-2-phenylethyl]amino]-1-(1H-indol-3-ylmethyl)-1-methyl-2-oxoethyl]carbamate.

**Step 1**

Following the procedure from Example G, Fmoc-α-methyl-D-tryptophyl-L-phenylalaninol (7 g, 12.2 mmol) was dissolved in a 20% solution of piperidine in DMF (50 ml) and left stirring 12 hours at room temperature. The solvent was then evaporated and the residue chromatographed on silica using $CH_2Cl_2$ then 4% MeOH:96% $CH_2Cl_2$ as eluants to yield the product (4 g, 95%) as a colorless foam. IR (film 3305 and 1646 $cm^{-1}$; NMR ($CDCl_3$) δ 1.28 (3H, s), 2.71 (2H, ABx, J 8 and 13.5Hz), 2.78 (1H, half ABq, J 14Hz), 2.91 (3H, br.s), 3.43 (1H, half ABq, J 14Hz), 3.45 (2H, ABx, J 6 and 11Hz), 4.03 (1H, m), 6.96 (1H, d, J 2Hz), 7.03-7.23 (7H, m), 7.29 (1H, d, J 8Hz), 7.67 (1H, d, J 7.5Hz) and 8.64 (1H, s).

Step 2

A solution of the α-methyl-D-tryptophyl-L-phenylalaninol (0.5 g, 1.42 mmol) and 4-N,N-dimethylaminopyridine (0.2 g, 1.64 mmol), in anhydrous THF (20 ml) was treated dropwise with a solution of 2-adamantylchloroformate (1.4 mmol) in anhydrous THF (20 ml) at room temperature. The reaction was monitored by IR spectroscopy. Once complete, the reaction mixture was diluted with ethyl acetate and washed with 1M citric acid solution, then water. The dried ($MgSO_4$) organic phase was evaporated to dryness and chromatographed over silica using 2% MeOH:98% $CH_2Cl_2$ as eluant. This gave the required compound (65% along with 20% carbonate impurity. NOTE: Some of the more acid labile urethanes required chromatography on neutral stationary phases. mp 96-100°C (EtOAc-hexane); IR (KBr) 3316, 1695 and 1658 $cm^{-1}$; NMR ($CD_3OD$) δ 1.28 (3H, s), 1.55 (2H, m), 1.68-2.06 (12H, m), 2.76 (2H, ABx, J 13.5 and 17Hz), 3.31 (2H, Abq, J 14.5Hz), 3.45 (2H, m), 4.12 (1H, m), 4.78 (1H, br.s) and 6.8-7.5 (10H, m); Anal. ($C_{32}H_{39}N_3O_4$), C, H, N.

Example 16

[R-(R*.S*)]-2-[[3-(1H-indol-3-yl)-2-methyl-1-oxo-2-[[(tricyclo[3.3.1.1$^{3,7}$]dec-2-yloxy)carbonyl]aminol-propyl]amino]-3-phenylpropyl butanedioate.

Following the procedure as described for conversion of Example 4, this compound was prepared from the product of Example 15. The product was isolated as a single diastereoisomer, chromatographed over a reverse phase silica stationary phase using 50% MeOH:50% $H_2O$, then 75% MeOH:25% $H_2O$ eluants to give a white amorphous

solid (98% yield), mp 66-69°C (MeOH-$H_2O$); IR (film) 1718 and 1660 cmHH-1; NMR ($CDCl_3$) δ 1.54 (5H, m), 1.70-2.00 (12H, m), 2.62 (4H, s), 2.76 (2H, ABx, J 13 and 13.5Hz), 3.33 (2H, ABq, J 14.5Hz), 3.90 (2H, m), 4.35 (1H, m), 4.88 (1H, br.s), 6.8 (1H, s), 7.1-7.3 (7H, m), 7.34 (1H, d, J 8Hz), 7.59 (1H, d, J 8Hz) and 8.25 (1H, s); Anal. ($C_{36}H_{23}N_3O_7$), C, H, N.

Example 17

2-[[3-(1H-indol-3-yl)-2-methyl-1-oxo-2-[[(tricyclo-[3.3.1.1$^{3,7}$]dec-2-yloxy)carbonyl]amino]propyl]aminol-1-phenylethyl butanedioate.

A solution of the alcohol from Example H (0.058 g, 0.113 mmol) in ethyl acetate (10 ml) was refluxed with succinic anhydride (0.013 g, 0.13 mmol) and 4-N,N-dimethylaminopyridine (0.027 g, 0.22 mmol), for 24 hours. The reaction mixture was then washed with 1M citric acid solution and the organic phase dried over $MgSO_4$. Evaporation of the solvent in vacuo yielded colorless gum (0.13 g) which was subjected to chromatography over silica using 10% MeOH:90% $CH_2Cl_2$ then 20% MeOH:80% $CH_2Cl_2$ as eluants, to yield the title compound as a noncrystalline white solid (0.021 g, 30%) and a mixture of two diastereoisomers, mp 94-100°C (MeOH-$CH_2Cl_2$);IR (film) 3352, 2911, 2855, 1722 and 1665 $cm^{-1}$; NMR ($CDCl_3$) δ 1.45-2.10 (17H, m), 2.60 (4H, br.s), 3.15-3.50 (4H, m), 3.85 (1H, br.m), 4.90 (1H, 2 br.s), 5.60 (0.5 H, s), 5.00 (0.5H, s), 6.95-7.60 (10H, m); Anal. ($C_{35}H_{41}N_3O_7 \cdot 1.25H_2O$), C, H, N.

Example 18

α-[[[(7,7-dimethyl-2-oxobicyclo[2.2.1]-hept-1-yl)-methyl]sulfonyl]amino]-N-[1-(hydroxymethyl)-2-phenyl-ethyl]-α-methyl-1H-indole-3-propanamide-(Trp center R, phenylalanyl center S).

A solution of the free base from Example 15, Step 1 (0.322 g, 0.92 mmol) and 4-N,N-dimethylaminopyridine (0.25 g, 2 mmol) in anhydrous THF (20 ml) was treated dropwise with a solution of 10-(+)-camphorsulphonylchloride (0.23 g, 0.92 mmol) in THF (15 ml). The reaction mixture was left stirring at room temperature for four hours before being quenched with water. The reaction mixture was diluted with ethyl acetate and washed with saturated $NaHCO_3$ solution then water, then 1M citric acid solution, then water. The dried ($MgSO_4$) organic phase was evaporated in vacuo and the residue chromatographed over silica using 2% MeOH:98% $CH_2Cl_2$ then 4% MeOH: 96% $CH_2Cl_2$ as eluants to give the title compound as a foam. An amorphous solid was obtained from EtOAc-hexane (0.4 g, 70%); mp 81-85°C (EtOAc-hexane); IR (KBr) 3259, 1742, 1672, 1359, and 1170 $cm^{-1}$; NMR ($CDCl_3$) δ 0.75 (3H, s), 1.01 (3H, s), 1.28 (1H, m), 1.48 (1H, m), 1.64-1.99 (7H, m), 2.24 (1H, br.s), 2.29 (1H, br.s), 2.57 (1H, m), 2.76 and 3.33 (2H, ABq, J 14.5Hz), 3.40 (2H, m), 3.39 (1H, m), 4.10 (2H, m), 5.80 (3H, br.), 6.78 (2H, d, J 7Hz), 7.07-7.25 (5H, m), 7.40 (1H, d, J 8Hz), 7.51 (1H, d, J 8Hz), 7.57 91H, s) and 9.60 (1H, s).

Example 19

[R-(R*.S*)]-4-[[2-[[3-(1H-indol-3-yl]-2-methyl-1-oxo-2-[[(tricyclor[3.3.1.1$^{3,7}$]dec-2-yloxy)carbonyl]amino]-propyl] amino]-3-phenylpropyl] amino]-4-oxobutanoic acid.

Step 1

A cooled (ice-water bath) solution of tert-butyloxycarbonyl-L-phenylalaninol (2.043 g, 8.14 mmol) in anhydrous pyridine (9 ml) was treated with p-toluene sulphonyl chloride (1.6 g, 8.14 mmol) with stirring. This mixture was left overnight at 4°C before being poured into ice water (600 ml). The solid formed was filtered, washed with ice-cold water then n-hexane, and dried in vacuo to give the required tosylate (3 g, 95%) pure enough to be used in Step 2 without further purification, mp 96-98°C (EtOAc-hexane); IR (KBr), 3320, 3029, 2978 and 1713 $cm^{-1}$; NMR ($CDCl_3$) δ 1.38 (9H, s); 2.45 (3H, s), 2.8 (2H, m), 3.9 (3H, m), 4.71 (1H, br.), 7.05-7.79 (9H, m).

Step 2

A solution of the tosylate from Step 1 (3 g, 7.4 mmol) in anhydrous, N,N-dimethylformamide (20 ml) was treated with sodium azide (0.52 g, 8 mmol) and the resulting mixture heated to 120°C for 1.5 hours. This was allowed to cool and then concentrated in vacuo. The syrup was diluted with ethyl acetate and washed with water (X3). The organic phase was dried over $MgSO_4$ and evaporated to give the azide (1.31 g) as a slightly impure waxy solid, and used as such in Step 3, mp 44-45°C; IR (film) (inter alia) 3341, 2978, 2101 and 1698 $cm^{-1}$.

Step 3

A solution of the impure urethane (1.17 g) as prepared in Step 2, was dissolved in dichloromethane (25 ml) and stirred with p-toluene sulphonic acid (1 g, 5.3 mmol) at room temperature for 18 hours. The solvent was evaporated in vacuo and the residue redissolved in ethyl acetate. This solution was washed with water, saturated $NaHCO_3$ solution then water and the organic phase dried over $MgSO_4$. The solvent was removed in vacuo to give a crude syrup (0.6 g) which was fractionated over silica using 5% MeOH:95% $CH_2Cl_2$ as eluant to give the pure free amine (0.4 g, 54%) as a syrup. (IR) film, 2100 $cm^{-1}$, NMR ($CDCl_3$) δ 1.28 (2H, s), 2.54 (1H, half ABx, J 18 and 12Hz), 2.76 (1H, half ABx, J 18 and 12Hz), 3.10-3.34 (3H, m) 7.14-7.31 (5H, m).

Step 4

A solution of 2-adamantyloxycarbonyl-α-methyl-D-tryptophan (0.9 g, 2.27 mmol) and pentafluorophenol (0.418 g, 2.27 mmol) in anhydrous ethyl acetate (35 ml) at 0°C was treated with a solution of dicyclohexylcarbodiimide (0.468 g, 2.27 mmol) in ethyl acetate (6 ml). This mixture was allowed to warm to room temperature and stirred a further two hours before the amine (0.4 g, 2.27 mmol) as prepared in Step 3, was added. This mixture was left 48 hours, filtered, and the filtrate washed with saturated $NaHCO_3$ solution, then water, then 1M citric acid solution and water again. The organic phase was dried over $MgSO_4$ and the solvent evaporated in vacuo to give a syrup which was chromatographed over reverse phase silica using 20% H2O:80% MeOH as eluant. This gave [R-(R*,S*)]-tricyclo [3.3.1.1$^{3,7}$]dec-2-yl [2-[[1-(azidomethyl)-2-phenylethyl]amino]-1-(1H-indol-3-ylmethyl)-1-methyl-2-oxoethyl]carbamate (0.6 g, 48%), which was crystallized from EtOAc-n-hexane, mp 77-78°C (EtOAC-n-hexane); IR (film) 3339, 2909, 2102, 1699 and 1668 $cm^{-1}$; NMR ($CDCl_3$) δ 1.45-2.1 (17H, m), 2.73 (2H, m), 3.10 (2H, m), 3.40 (2H ABq, J 14Hz), 4.25 (1H, m), 4.84 (1H, s), 5.17 (1H, s), 6.45 (1H, d, J 8Hz), 6.95 (1H, d, J 2Hz), 7.00-7.60 (9H, m), and 8.61 (1H, s); Anal. ($C_{32}H_{38}N_6O_3$).

Step 5

A solution of (R-(R*,S *)]-tricyclo[3.3.1.1$^{3,7}$]dec-2-yl-[2-[[1-(azidomethyl)-2-phenylethyl]amino]-1-(1H-indol-3-ylmethyl)-1-methyl-2-oxoethyl]carbamate (0.2 g, 0.36 mmol) in 5% acetic acid:95% ethanol (100 ml) was treated with 10% palladium on carbon (0.02 g, 10% w/w) and put under an atmosphere of hydrogen at a pressure of 51 psi at 30°C with agitation. After no more hydrogen was seen to be taken up, the mixture was filtered over celite and concentrated in vacuo to a foam (0.25 g) which was used immediately in Step 6. IR (film 1676 br $cm^{-1}$.

Step 6

The crude amine acetate (0.25 g) as prepared in Step 5, was dissolved in anhydrous ethyl acetate (30 ml) and treated with succinic anhydride (0.15 g, 1.5 mmol) and DMAP (0.15 g, 1.23 mmol) and heated under reflux for 18 hours. The solution was then washed with 1M citric acid solution then water. The organic phase was dried over $MgSO_4$ and evaporated in vacuo. The resultant residue was chromatographed over reverse phase silica using 20% H2O:80% MeOH as eluant to give the title compound (0.1 g, 44% from Step 5) as a white solid crystallized from ethyl acetate-hexane, mp 110-114°C (EtOAc-hexane); IR (film) 3306, 2906, 2854, 1695 and 1651 $cm^{-1}$; NMR ($CDCl_3$) δ 1.34-1.97 (17H, m), 2.38 (2H, m), 2.55 (2H, m) 2.62 (2H, m), 2.98 (1H, m), 3.27 (2H, m), 3.45 (1H, m), 4.20 (1H, m), 4.77 (1H, s), 5.43 (1H, br.s), 6.05 (1H, br.s), 6.43 (1H, br.s), 6.85-7.55 (10H, m) and 8.91 (1H, s); Anal. ($C_{36}H_{44}N_4O_6$), C, H, N.

Example 19A

[R-(R*,S*)]-4-[[2-[[3-(1H-Indol-3-yl)-2-methyl-1-oxo-2-[[tricyclo[3.3.1.1$^{3,7}$]dec-2-yloxy]carbonyl]amino]propyl]-amino]-3-phenylpropyl]amino]-4-oxo-2-butenoic acid.

Step 1

A suspension of mono methyl fumarate (200 mg, 1.54 mmol) in EtOAc (20 ml) was treated with pentafluorophenol (340 mg, 1.85 mmol), and dicyclohexylcarbodiimide (349 mg, 1.69 mmol) and allowed to stirr for 3 h. After this time the suspension was filtered and the filtrate treated with the amine from Example 19 Step 5 (816 mg, 1.54 mmol) and left stirring for 18 h at room temp. The reaction mixture was then filtered, the filtrate evaporated in vacuo and the residue chromatographed over reverse phase silica gel using 75% MeOH in $H_2O$ as eluant to give the product as an amorphous white solid (867 mg, 88%); mp 161-166°C (MeOH/$H_2O$) ; $[\alpha]^{20}_D$ + 13.3° (c = 1.04, MeOH) ; IR (film) 1728, 1700 and 1666 $cm^{-1}$ ; NMR ($CDCl_3$) δ 1.34 (3H, s), 1.50-1.60 (2H, m), 1.70-2.10 (12H, m), 2.73 (2H, d, J 7Hz),

3.10-3.25 (1H, m), 3.28 (1H, d, J 15Hz), 3.38, (1H, d, J 15Hz), 3.70-3.80 (1H, m) 3.75 (3H, s), 4.25-4.35 (1H, m), 4.80 (1H, s), 5.00 (1H, s), 6.12 (1H, d, J 8Hz), 6.80 (1H, d, J 16Hz), 6.92 (1H, d, J 16Hz), 6.93 (1H, d, J 2Hz), 7.05-7.30 (8H, m), 7.35 (1H, d, J 8Hz), 7.57 (1H, d, J 8Hz), 8.21 (1H, s) ; Anal. $C_{37}$ $H_{44}$ $N_4$ $0_3.H_2O$; C, H, N.

**Step 2**

The methyl ester from step 1 (867 mg, 1.35 mmol) as a solution in THF (35 ml) at 0°C was treated dropwise with aqueous LiOH solution (13.5 ml of a 0.1M soln, 1.35 mmol). The resultant mixture was stirred at 0°C for 4.5 h and allowed to warm to room temperature and acidifed with 1M citric acid soln. The mixture was concentrated to one third of its original volume and the residue extracted with EtOAc (75 ml) and washed with $H_2O$ (75 ml). The organic phase was dried over $MgSO_4$, filtered and evaporated in vacuo. The residue then was purified by chromatography over reverse phase silica gel using 75% MeOH in $H_2O$ as eluant to give the product as an amorphous white solid (611 mg, 72%); mp 166-170°C (MeOH/$H_2O$); $[\alpha]^{20}_D$ + 105.2° (c = 1.07, MeOH) ; IR (film) 3341, 1706 and 1665cm$^{-1}$ ; NMR ($CDCl_3$) δ 1.38 (3H, s), 1.45-1.55 (2H, m), 1.70-2.10 (12H, m), 2.00 ($CO_2H$ and $H_2O$), 2.60-2.80 (2H, m), 3.10-3.20 (1H, br m), 3.22 (1H, d, J 12Hz), 3.34 (1H, d, J 14Hz), 3.50-3.60 (1H, br m), 4.20-4.30 (1H, br m), 4.78 (1H, s), 5.23 (1H, s), 6.35-6.45 (1H, br m), 6.75 (1H, d, J 15.5Hz), 6.89 (1H, d, J 15.5Hz), 6.90 (1H, d, J 2Hz), 7.00-7.30 (8H, m), 7.31 (1H, d, J 8Hz), 7.54 (1H, d, J 8Hz), 8.54 (1H, s); Anal. $C_{36}H_{42}N_4O_6$; C, H, N.

Example 20 (Compound (24),Scheme III)

[R-(R*,R*)-4-[[2-[[3-(1H-indol-3-yl)-2-methyl-1-oxo-2-[[(tricyclo[3.3.1.1.$^{3,7}$]dec-2-yloxy)carbonyl]amino]-propyl] amino]-1-phenylethyl]amino]-4-oxobutanoic acid.

Step 1

To a solution of tert-butyloxycarbonyl-D-2-phenylglycinol (5.85 g), 24.7 mmol) in anhydrous dichloromethane (60 ml) at 0°C was added triethylamine (5.08 g, 50.3 mmol) followed by ptoluene sulphonylchloride (6.8 g, 35.7 mmol) as a solution in dichloromethane (10 ml). The reaction mixture was allowed to warm to room temperature and left 18 hours. The mixture was then diluted with dichloromethane (100 ml) and washed with 1M citric acid solution. The organic phase was dried over $MgSO_4$ and evaporated in vacuo to leave a solid which was recrystallized from ethyl acetate-hexane (6.8 g, 70%), mp 114-118°C (EtOAC-hexane); IR (film) 3388, 2978, 1713, 1365 and 1176 cm$^{-1}$; NMR ($CDCl_3$) δ 1.40 (9H, s), 2.43 (3H, s), 4.20 (2H, m), 4.89 (1H, br.s), 5.10 (1H, br.s), 7.27 (2H, m), 7.31 (5H, m), 7.65 (2H, d, J 8Hz); Anal. ($C_{20}H_{25}NO_5S$), C, H, N.

Step 2

Method was as described for Example 19, Step 2, but using the tosylate prepared in Example 20, Step 1 (2.37 g, 70%), not purified, mp 76-78°C; IR (film), 3380, 2095, 1682 and 1515 cm$^{-1}$; NMR ($CDCl_3$) δ 1.44 (9H, s), 3.763 (2H, m), 4.87 (1H, br.s), 5.03 (1H, br.s), 7.30-7.40 (5H, m).

Step 3

Method was as described for Example 19, Step 3, but using the urethane prepared in Example 20, Step 2 (3.43 g, >100%) used without further purification in Step 4; IR (film) 3030 and 2104 cm$^{-1}$; NMR ($CDCl_3$) δ 3.37 (1H, dd, J 8 and 12Hz), 3.52 (1H, dd, J 5 and 12Hz), 4.13 (1H, dd, J 5 and 8 Hz), 7.20-7.40 (5H, m).

Step 4

To a solution of benzyl-hemisuccinate (3.14 g, 15.1 mmol) in ethyl acetate (60 ml) was added N,N-dicyclohexylcarbodiimide (3.42 g, 16.6 mmol) and 1-hydroxybenzotriazole (2.24 g, 16.6 mmol). The reaction mixture was left one hour before the amine (2.23 g) as prepared in Step 3 was added as a solution in ethyl acetate (5 ml). This final mixture was left stirring for a further three hours before being filtered and the filtrate evaporated in vacuo to yield a gum (10 g) which was chromatographed over silica using 25% EtOAc:75% n-hexane then 50% EtOAc:50% n-hexane as eluants to yield the required amidoazide (3.96 g, 70%) as a white solid, mp 51-54°C (EtOAC-hexane); IR (film) 3295, 3065, 2103, 1736 and 1651 cm$^{-1}$; NMR ($CDCl_3$) δ 2.55 (2H, t, J 7Hz), 2.72 (2H, t, J 6Hz), 3.63 (2H, d, J 7Hz), 5.12 (2H, s), 5.16 (1H, m), 6.25 (1H, br.d), 7.30-7.40 (10H, m); Anal. ($C_{19}H_{20}N_{12}O_2$), C, H, N.

Step 5

To a solution of the amidoazide (1.659 g, 4.7 mmol) as prepared in Step 4, in absolute ethanol (45 ml) was added Lindlar catalyst (0.664 g, 40% w/w). The reaction was then put under an atmosphere of hydrogen for three hours. The reaction mixture was then filtered over celite and washed with ethanol. The solvent was evaporated in vacuo and the residue used immediately without further purification in Step 6 (1.07 g, ca. 70%). IR (film) 3325, 1733, 1703 and 1651 $cm^{-1}$; NMR ($(CD_3)_2SO$) δ 2.65 (2H, m), 2.70 (2H, m), 4.74 (1H, br.q), 5.08 (2H, s), 7.20-7.40 (10H, m), 8.25 (1H, d).

Step 6

2-Adamantyloxycarbonyl-α-methyl-D-tryptophan (1.36 g, 3.4 mmol), as a solution in ethyl acetate (30 ml) was treated sequentially with N,N-dicyclohexylcarbodiimide (0.778 g, 3.8 mmol) and 1-hydroxybenzotriazole (0.51 g, 3.8 mmol) and left stirring for one hour before the amine (1.07 g) as prepared in Step 5 was added as a solution in ethyl acetate (5 ml). The resulting reaction mixture was left stirring at room temperature for 18 hours before being filtered. The filtrate was concentrated in vacuo to give a gum (3.4 g) which was chromatographed over reverse phase silica using 30% $H_2O$:70% MeOH then 20% $H_2O$:80% MeOH as eluants to yield the required product (1.403 g, 41% from Step 5) as a noncrystalline solid. IR (film) 3305, 2856, 1729, 1695 and 1651 $cm^{-1}$; NMR ($CDCl_3$) δ 1.47 (3H, s), 1.50-2.05 (14H, m), 2.57 (2H, m), 2.70 (2H, q, J 5Hz), 3.35 (1H, m), 3.40 (2H, dd, J 15Hz), 3.95 (1H, m), 4.86 (1H, br.s), 5.11 (3H, s), 6.40 (1H, br.s), 7.00 (1H, d), 7.05-7.35 (9H, m), 7.57 (1H, d, J 7Hz), 3.27 (1H, s).

Step 7

A solution of the benzyl ester, as prepared in Step 6 (1.403 g, 2.0 mmol), in absolute ethanol (50 ml) was treated with 10% palladium on carbon (0.14 g, 10% w/w) and placed under an atmosphere of hydrogen for four hours. The reaction mixture was then filtered over celite and washed with ethanol, then acetone. The filtrate was concentrated in vacuo to yield the title compound (0.967 g, 79%) which was recrystallized from methanol, mp 142-146°C (MeOH); IR (film 3306, 2908, 1713 and 1670 $cm^{-1}$; NMR ($(CD_3)_2SO$) δ 1.20 (3H, s), 1.49 (2H, br.s), 1.65-1.85 (8H, m), 1.95 (4H, m), 2.39 (4H, br.s), 3.40 (4H, br.m), 4.69 (1H, br.s), 4.96 (1H, br.d J 6Hz), 6.70 (1H, s), 6.90 (2H, s), 7.01 (1H, 5, J 7Hz), 7.22 (1H, m), 7.31 (5H, br.s), 7.44 (1H, d, J 7Hz), 7.78 (1H, br.s), 8.30 (1H, s) and 10.85 (1H, s); Anal. ($C_{35}H_{42}N_4O_6 \cdot 0.5H_2O$), C, H, N.

Example 20A

In an analogous manner but using 1-(S)-2-endobornyloxycarbonyl-[D]-α-methyltrypthophan, [1S-[1α,2β[S*(S*)],4β)]-4-[[2-[(3-(1H-indol-3-yl)-2-methyl-1-oxo-2-(([(1,7,7-trimethylbicyclo-[2.2.1]hept-2-yl)oxy]carbonyl]amino]-propyl]amino]-1-phenethyl]amino]-4-oxobutanoic acid was prepared.

Example 21

(R)-tricyclo[3.3.1.1$^{3,7}$]dec-2-yl [1-(1H-indol-3-ylmethyl)-1-methyl-2[methyl(2-phenylethyl)amino]-2-oxoethyl] carbamate

The method is as described in Example 19, Step 4, except N-Methyl-phenethylamine was used. 50 mg was obtained (61% yield) as an amorphous white solid, mp 90-95°C (MeOH-$H_2O$); IR (film), 3295, 2855, 1698 and 1625 $cm^{-1}$; NMR ($CDCl_3$) δ 1.5-2.0 (17H, m) 2.84 (2H, br.t, J 7Hz), 3.07 (3H, br.s), 3.4-3.8 (4H, m) 4.86 (1H, br.s), 5.28 (1H, br.s), 6.95-7.30 (8H, m); 7.35 (1H, d, J 8Hz), 7.56 (1H, d, J 8Hz), 8.2 (1H, br.s); Anal. ($C_{32}H_{39}N_3O_3$), C, H, N.

Example 22

[R-[R*,R*-(E)]]-4-[[2-[[3-(1H-indol-3-yl)-2-methyl-1-oxo-2-[[(tricyclo[3.3.1.1$^{3,7}$]dec-2-yloxy)carbonyl]amino]propyl]-amino]-1-pbenylethyl]aminol-4-oxo-2-butenoic acid

Step 1

To a solution of tert butyloxycarbonyl-D-phenyl-glycinol (5.85 g, 24.7 mmol) in anhydrous dichloro-methane (60 ml) at 0°C was added triethylamine (5.08 g, 50.3 mmol) followed by p-toluene sulphonyl chloride (6.8 g, 35.7 mmol) as a solution in dichloro-methane (10 ml). The reaction mixture was allowed to warm to room temperature and left 18

hours. The mixture was then diluted with dichloromethane (100 ml) and washed with 1M citric acid solution (100 ml). The organic phase was dried over anhydrous $MgSO_4$ and evaporated in vacuo to leave a solid which was recrystallized from ethyl acetate/n-hexane; (6.8 g, 70%). mp 114-118°C (EtOAc/n-hexane); IR (film) 3388, 2978, 1713, 1365, and 1176 $cm^{-1}$; NMR ($CDCl_3$) δ 1.40 (9H, s), 2.43 (3H, s), 4.20 (2H, m), 4.89 (1H, br.s), 7.27 (2H, m), 7.31 (5H, m), 7.65 (2H, d, J 8Hz); Anal. ($C_{20}H_{25}NO_5S$), C, B, N.

Step 2

A solution of the tosylate (4.67 g, 11.9 mmol) in anhydrous DMF (60 ml) was treated with sodium azide (868 mg, 13.4 mmol). The mixture was heated to 120°C for 1.5 hours. After cooling, the solution was poured into water (250 ml), and the aqueous layer extracted with an equal volume of ether. The ethereal phase was washed with water, dried over $MgSO_4$ and the solvent removed in vacuo to yield the desired azide as a white crystalline solid, used without further purification (2.37 g, 70%), mp 76-78°C; IR (film) 3380, 2095, 1682, and 1515 $cm^{-1}$; NMR ($CDCl_3$) δ 1.44 (9H, s), 3.76 (2H, m), 4.87 (1H, br.s), 5.03 (1H, br.s), 7.30-7.40 (5H, m).

Step 3

A solution of the azide (6.44 g, 24.6 mmol) in anhydrous ethyl acetate (100 ml) was subjected to an atmosphere of hydrogen at a pressure of 45 psi over Lindlar catalyst (2.58 g, 40% w/w) for 6 hours at room temperature. After this time the reaction mixture was filtered through filter aid and washed through with more ethyl acetate. The crude product, in solution, was used immediately in the next step of the reaction sequence. IR (film) 3350, 3000, and 1696 $cm^{-1}$; NMR ($CDCl_3$) δ 1.43 (9H, s), 2.10 (2H, br.s), 3.10 (2H, br.s), 4.70 (1H, m), 5.45 (1H, br.s), 7.25-7.40 (5H, m).

Step 4

To a solution of Fmoc-α-Me-D-Trp-OH (1.800 mg, 4.091 mmol) in ethyl acetate (35 ml) was added N,N'-dicyclohexylcarbodiimide (927 mg, 4.50 mmol) and 1-hydroxybenzotriazole hydrate (689 mg, 4.50 mmol). After stirring at room temperature of 1 hour, the amine (965 mg, 4.09 mmol), in ethyl acetate (5 ml) was added to the suspension. After stirring for a further 3 hours, the reaction mixture was filtered and the filtrate evaporated in vacuo to yield a gum (2.9 g). The crude product was purified by column chromatography using 25% to 75% EtOAc in n-hexane as eluant, to yield the desired amide as a yellow, noncrystalline solid (1970 mg, 73%), mp 78-82°C; IR (film) 3300, 3100-2900, 1695, and 1660 $cm^{-1}$; NMR ($CDCl_3$) δ 1.40 (9H, br.s), 1.50 (3H, s), 3.30-3.50 (3H, m), 3.65 (1H, m), 4.15 (1H, br.s), 4.41 (2H, br.s), 4.75 (1H, m), 5.35 (1H, s), 5.45 (1H, m), 6.55 (1H, br.s), 6.83 (1H, br.s), 7.10-7.45 (12H, m), 7.50-7.65 (3H, m), 7.75 (2H, m), 8.05 (1H, br.s).

**Step 5**

To a cooled solution (0°C) of the urethane (3.611 g, 5.488 mmol) in anhydrous dichloromethane (40 ml) was added p-toluene sulphonic acid (1.301 g, 6.839 mmol). The reaction mixture was allowed to warm to room temperature and left 18 hours. Dichloromethane (100 ml) was then added and the mixture washed with saturated sodium hydrogen carbonate solution (100 ml). The organic phase was dried ($MgSO_4$) and evaporated to yield the amine as a yellow noncrystalline solid purified by chromatography using 5% MeOH in $CH_2Cl_2$ as eluant (2.915 g, 95%), mp 84-88°C; IR (film) 3300-3400, 1713, and 1658 $cm^{-1}$; NMR ($CDCl_3$) δ 1.50 (3H, s), 1.65 (2H, br.s), 3.15 (1H, m), 3.25 (1H, Ha of ABq, J 15Hz), 3.45-3.55 (2H, m), 3.95 (1H, m), 4.15 (1H, t, J 8Hz), 4.35-4.50 (2H, m), 5.32 (1H, s), 6.43 (1H, br.t), 6.77 (1H, d, J 12Hz), 7.05-7.45 (12H, m), 7.50-7.65 (3H, m), 7.75 (2H, m), 8.05 (1H, s); m/e 559 (M+, base peak); Anal. ($C_{35}H_{34}N_4O_3 \cdot 0.25C_6H_{14}$), C, H, N.

Step 6

Fmoc-α-Me-D-TrpNHCH$_2$CH (NHCOCHCHCO$_2$Me) Ph; [R-[R*,R*-(E)]]-4-[[2-[[2-[[(9H-Fluoren-9-ylmethoxy)-carbonyl] aminol-3-(1H-indol-3-yl)-2-methyl-1-oxo-Propyl]amino]-1-phenylethyl]aminol-4-oxo-2-butenoic acid methyl ester

To a solution of mono-methyl fumarate (330 mg, 2.54 mmol) in ethyl acetate (50 ml) was added 1-hydroxybenzotriazole hydrate (390 mg, 2.55 mmol) followed by N,N'dicyclohexyl-carbodiimide (570 mg, 2.77 mmol). After stirring for 1 hour at room temperature, the amine from step 5 (1.40 g, 2.51 mmol) in ethyl acetate (3 ml) was added and the resulting suspension stirred on 18 hours. The reaction mixture was then filtered, the filtrate evaporated in vacuo and the residue purified by chromatography over silica gel using 50 to 75% EtOAc in nhexane as eluant to yield the product as a white amorphous solid. (1.21 g, 72%), mp 78-82°C; IR (film) 3309, 3064, 2950, 1724, and 1668 $cm^{-1}$;

NMR ($CDCl_3$) δ 1.39 (3H, s) 3.30 (3H, m), 3.69 (3H, s), 4.05 (1H, m) 4.16 (1H, t, J to 8Hz), 4.40 (1H, dd, J 8 and 11Hz), 5.16 (1H, s), 5.21 (1H, m), 6.21 (1H, m) 6.78 (1H, d, J 15Hz), 6.79 (1H, d, J 2Hz), 7.03 (1H, d, J 15Hz), 7.15 to 7.60 (16H, m), 7.77 (2H, t, J 8Hz), 8.17 (1H, s); Anal. ($C_{40}H_{38}N_4O_6{\cdot}5H_2O$), C, H, N.

Step 7

H-α-Me-D-TrpNHCH$_2$CH(NHCOCHCHCO$_2$Me)Ph; [R-[R*,R*-(E)]]-4-[[2-[[2-Amino-3-(1H-indol-3-yl)-2-methyl-1-oxopropyl]amino]-1-phenylethyl]aminol-4-oxo-2-butenoic acid methyl ester

Piperidine (156 mg, 1.84 mmol) was added to a solution of the urethane (1.21 g, 1.81 mmol) in anhydrous DMF (20 ml) at 0°C. The reaction mixture was allowed to warm to room temperature, and after 4 hours was concentrated to a gum. This crude product was chromatographed over silica-gel using 2.5% to 5% MeOH in $CH_2Cl_2$ was eluant to give the amine as a noncrystalline, pale yellow solid (801 mg, 97%). mp 75-77°C; IR (film) 3400-3300, 3100, 2900, 1728, 1660, and 1646 $cm^{-1}$; NMR ($CDCl_3$) δ 1.41 (3H, s), 1.60 (2H, br.s), 2.81 (1H, Ha of ABq, J 15Hz), 3.45-3.60 (3H, m), 5.00 (1H, m), 6.80 (1H, d, J 16Hz), 6.90-7.20 (9H, m), 7.40 (1H, d, J 8Hz), 7.64 (2H, br.d, J 8Hz), 7.90 (1H, t, J 6Hz), 8.31 (1H, br.s); Anal. ($C_{25}H_{28}N_4O_4$), C, H, N.

Step 8

2-Adoc-α-Me-D-TrpDNHCH$_2$CH(NHCOCHCHCO$_2$Me)Ph; [R-[R*.R*-(E)]1-4-[[2-[[3-(1H-Indol-3-yl)-2-methyl-1-oxo-2-[[(tricyclor3.3.1.1$^{3,7}$]dec-2-yloxy)carbonyl]- amino]propyl]amino]-1-phenylethyl]amino]-4-oxo-2-butenoic acid methyl ester

To an ice-cooled solution of the amine (794 mg, 1.77 mmol) in anhydrous THF (10 ml) was added 2-adamantyl chloroformate (380 mg, 1.77 mmol) in THF (3 ml) followed by the triethylamine (215 mg, 2.13 mmol) in TBF (2 ml) dropwise. The reaction mixture was stirred at room temperature for 3 hours and then concentrated in vacuo to give a brown residue (11 g). The crude product was purified by column chromatograpby using 60% ethyl acetate/n-hexane as eluant to give the desired urethane (51c) as an amorphous solid (734 mg, 66%), mp 109-112°C; IR (film) 3440-3300, 2900, 1720, and 1667 $cm^{-1}$; NMR ($CDCl_3$) δ 1.42 (3H, s), 1.54 (2H, m)·, 1.70-2.05 (12H, m), 3.34 (1H, Ha of ABq, J 14Hz), 3.42 (1H, m), 3.50 (1H, Hb of ABq, J 14Hz), 3.79 (3H, s), 4.05 (1H. m), 4.84 (1H, br.s), 5.03 (1H, s), 5.20 (1H, m), 6.35 (1H, m), 6.82 (1H, d, J 15Hz), 6.95-7.35 (10H, m), 7.57 (2H, d, J 8Hz), 8.30 (1H, s); Anal. ($C_{36}H_{42}N_4O_6{\cdot}0.5H_2O$), C, H, N.

Step 9

2-Adoc-α-Me-D-TrpNHCH$_2$CH(NHCOCHCHCO$_2$H)Ph; [R-[R*,R*(E)]]-4-[[2-[[3-(1H-indol-3-yl) -2-methyl-1-oxo-2-[[(tricyclo [3.3.1.1$^{3,7}$]dec-2-yloxy)carbonyl]-amino]propyl]amino]-1-phenylethyl]amino]-4-oxo-2-butenoic acid

Aqueous lithium hydroxide (12.16 ml of a 0.1M solution, 1.22 mmol) was added dropwise to a solution of the methyl ester (726 mg, 1.16 mmol) in THF (73 ml) at 0°C over a 2-hour period. The reaction mixture was then allowed to warm to room temperature and left stirring for 18 hours. After this time hydrochloric acid (1.34 ml of a 1M solution) was added and the mixture concentrated. Ethyl acetate (150 ml) and water were then added and the separated organic phase dried over $MgSO_4$ and evaporated to give a crude solid. This chromatographed over reverse phase silica using 75% MeOH in $H_2O$ as eluant to yield the desired product as an amorphous solid (324 mg, 46%), mp 145-150°C; $[\alpha]^{20}$ +13.70 (c = 0.24, $CHCl_3$); IR (film) 3300, 2910, 1706, and 1667 $cm^{-1}$; NMR (DMSO-d6) δ 1.18 (3H, s), 1.74 (2H, m), 1.65-2.00 (12H, m), 3.30-3.50 (4H + $H_2O$), 4.66 (1H, br.s), 5.06 (1H, m), 6.52 (1H, d, J 15Hz), 6.77 (1H, br.s), 6.90-7.10 (4H, m), 7.20-7.35 (6H, m), 7.44 (1H, d, J 8Hz), 7.82 (1H, t, J 6Hz), 8.78 (1H, br.s), 10.85 (1H, s); Anal. ($C_{35}H_{40}N_4O_6{\cdot}0.5H_2O$), C, H, N.

Example 23

[R-(R*,S*)1-j[2-([3-(1H-indol-3-yl)-2-methyl-1-oxo-2-[[(tricyclo[3.3.1.1$^{3,7}$]dec-2-yloxy)carbonyl]-amino]propyl]amino]-3-phenylpropyl]sulfinyl]acetic acid

Step 1

Sodium periodate (908 mg, 4.24 mmol) in water (10 ml) was added dropwise to sulphide BOCNHCH($CH_2SCH_2CO_2Et$)$CH_2Ph$ (750 mg, 2.12 mmol) in methanol (20 ml) at room temperature. This mixture was left for 2

hours, concentrated to one-third its volume and partitioned between ethyl acetate and a sodium chloride solution. The organic phase was dried over $MgSO_4$, filtered and evaporated in vacuo to a white solid (782 mg, 100%) which was a mixture of two diastereoisomers and used as such without further purification. IR (film) 1739, 1689, and 1046 $cm^{-1}$; NMR ($CDCl_3$) δ 1.27 (3H, t, J 7Hz), 1.41 (4.5H, s), 1.42 (4.5H, s), 2.92-3.20 (4H, m), 3.66-3.84 (2H, m), 4.18-4.29 (3H, m), 4.80 (0.5H, br.), 5.30 (0.5H, br.), 7.19-7.35 (5H, m).

Step 2

$H_2$NCH $CH_2SOCH_2CO_2$Et) $CH_2$Ph; (S)-[(2-Amino-3-phenylpropyl)sulfinyl]acetic acid ethyl ester

The N-BOC-protected sulphoxide (462 mg, 1.25 mmol) was stirred in dichloromethane containing trifluoroacetic acid (5 ml of 1:1 mixture) for 1 hour at room temperature. All volatiles were removed in vacuo to give a syrup which was used without further purification (479 mg).

Step 3

2-Adoc-α-Me-D-TrpNHCH($CH_2SOCH_2CO_2$Et)$CH_2$Ph; [R-R*,S*)]-[[2-[[3-(1H-indol-3-1)-2-methyl-1-oxo-2-[[(tricyclo [3.3.1.1$^{3,7}$]dec-2-yloxy)carbonyl]amino]-propyl]aminol-3-phenylpronyl]sulfinyl]acetic acid ethyl ester

N,N'-Dicyclobexylcarbodiimide (165 mg, 0.801 mmol) was added to a solution of 2-ADOCαMe-D-TrPOH (286 mg, 0.720 mmol) and 1-hydroxybenzotriazole hydrate (122 mg, 0.797 mmol) in ethyl acetate (10 ml). After 1 hour the crude amine salt (63)) (345 mg, 0.9 mmol) and triethylamine (243 mg, 2.40 mmol) in ethyl acetate (10 ml) was added dropwise and the mixture allowed to stir at room temperature for 22 hours. This mixture was filtered and the filtrate washed with 1M citric acid solution (2 x 10 ml), saturated sodium hydrogen carbonate solution (2 x 10 ml) and a sodium chloride solution (10 ml). The organic phase was dried over $MgSO_4$, filtered and evaporated in vacuo and the residue chromatographed over silica gel using 2% MeOH in $CH_2Cl_2$ as eluant to give the product as a white amorphous solid (263 mg, 56%) as a mixture of two diastereoisomers, mp 87-99°C; IR (film) 1719, 1659, and 1072 $cm^{-1}$; NMR (CDCl) δ 1.22-1.28 (3H, m), 1.47-2.00 (17H, m), 2.81-3.14 (4H, m), 3.22-3.49 (2H, m), 3.56-3.79 (2H, m), 4.16-4.23 (2H, m), 4.48 (1H, m), 4.80 (1H, s), 5.21 (1H, s) 6.77-7.62 (11H, m); MS m/e (EI) 648 (72) 130 (100); Anal. ($C_{36}H_{45}N_3O_6S$), C, H, N, S.

Step 4

2-Adoc-α-Me-D-TrpNHCH($CH_2SOCH_2CO_2$H)$CH_2$Ph; [R-(R*,S*)]-[[2-[[3-[1H-indol-3-yl)-2-methyl-1-oxo-2-[[(tricyclo [3.3.1.1$^{3,7}$]dec-2-yloxy)carbonyl]amino]-propyl]amino]-3-phenylpropyl]sulfinyl]acetic acid

Lithium hydroxide (8.3 ml of a 0.1M solution, 0.83 mmol) was added dropwise to a cooled solution of ester (487 mg, 0.752 mmol) in THF (45 ml). The mixture was stirred for 6 hours at room temperature, then hydrochloric acid (9.1 ml of a 0.1M solution, 0.91 mmol) was added and the THF evaporated. The residue was taken up in ethyl acetate and washed with water, the organic phase was dried over $MgSO_4$, filtered, and concentrated to a residue which was chromatographed over reverse phase silica gel using 80% MeOH in $H_2O$ as eluant and yielded the product as an amorphous white solid (304 mg, 65%), mp 125-141°C; IR (film) 1709 and 1664 $cm^{-1}$; NMR ($CDCl_3$) δ 1.50-2.04 (17H, m), 2.68-3.05 (4H, m), 3.16-3.77 (4H, m), 4.39-4.46 (1H, m), 4.80 (1H, br.s), 5.46 (2H, br.), 6.99-7.34 (10H, m), 7.54 (1H, d, J 8Hz), 8.79 (1H, br.); MS m/e (FAB) 620 (100); Anal. ($C_{34}H_{41}N_3O_6 \cdot 1.2H_2O$), C, H, N, S.

Example 24

[R-(R*,S*1]-[[2-([2-[[3-(1H-indol-3-yl) -2-methyl-1-oxo-2-[[(tricyclo [3.3.1.1$^{3,7}$] dec-2-yloxy) carbonyl]amino]-propyl] amino]-3-phenylpropyl]thio]acetic acid

Step 1

BOCNHCH$_2$ CH$_2$OMs) $CH_2$Ph; (S)-[1-[[(Methylsulfonyl)oxylmethyl]-2-phenylethyl]-carbamic acid 1,1-dimethyl ethyl ester

Methane sulphonyl chloride (2.51 g, 21.9 mmol) in anhydrous THF (10 ml) was added dropwise to a solution of N-tert.-BOC-L-phenylalaninol (5.00 g, 19.9 mmol) and triethylamine (2.77 g, 27.4 mmol) in anbydrous THF (20 ml) at 0°C. After 1 hour the reaction mixture was filtered and the filtrate concentrated in vacuo to a solid which was recrystallized from ethyl acetate-n-hexane (6.35 g, 97%), mp 106-108°C (EtOAc/n-hexane); IR (film) 1682, 1356, and 1167 $cm^{-1}$; NMR ($CDCl_3$) δ 1.38 (9H, s), 2.81-2.91 (2H, m), 3.01 (3H, s), 4.09-4.25 (3H, m), 4.72 (1H, br.s), 7.20-7.35 (5H, m).

Step 2

$BocNHCH(CH_2SCH_2CO_2Et)CH_2Ph$; (S)-[[2-[[(1.1-Dimethylethoxy)carbonyl]amino]-3-phenylpropyl] thio] acetic acid ethyl ester

Ethyl-2-mercaptoacetate (1.206 g, 10.4 mmol) in anhydrous THF (10 ml) was added dropwise at room temperature to a suspension of 60% sodium hydride (400 mg, 10.0 mmol) stirred in THF (30 ml). After 1.5 hours, the mesylate (2) (3.0 g, 9.11 mmol) in THF (15 ml) was added dropwise over a 5-minute period. After stirring for 24 hours at room temperature the solvent was removed in vacuo and the residue partitioned between ethyl acetate and sodium chloride solution. The organic phase was dried over $MgSO_4$, filtered and the solvent evaporated in vacuo to give an oil which was chromatographed over silica gel using $CH_2Cl_2$ as eluant to give the product as a syrup (1.58 g, 49%), IR (film) 1733 and 1713 $cm^{-1}$; NMR ($CDCl_3$) δ 1.26 (3H, t, J 7Hz), 1.41 (9H, s), 2.66-2.89 (4H, m), 3.25 (2H, dd, J 4 and 14Hz), 4.03 (1H, m), 4.18 (2H, q, J 7Hz), 4.75 (1H, s), 7.18-7.32 (5H, m).

**Step 3**

$H_2NCH(CH_2SCH_2CO_2Et)CH_2Ph \cdot CF_3CO_2H$; (S)-[(2-Amino-3-phenylpropyl)thio]acetic acid ethyl ester trifluoroacetate (SALT) (1:1)

The N-protected ester (225 mg, 0.637 mmol) was stirred for 30 minutes in neat trifluoroacetic acid (3 ml) at room temperature. Excess trifluoroacetic acid was evaporated in vacuo to give the crude trifluoroacetate salt, which was used immediately without further purification, yield 321 mg.

**Step 4**

2-Adoc-α-Me-D-$TrpNHCH(CH_2SCH_2CO_2Et)CH_2Ph$; [R-(R*,S*)]-[[2-[[3-(1H-Indol-3-yl)-2-methyl-1-oxo-2-[[(tricyclor [$3.3.1.1^{3,7}$]dec-2-yloxy)carbonyl]amino]-propyl]amino]-3-phenylpropylthio]acetic acid ethyl ester

N,N'-dicyclohexylcarbodiimide (145 mg, 0.704 mmol) was added to a stirred solution of 2-Adoc-α-Me-D-TrPOH (254 mg, 0.640 mmol) and 1-hydroxybenzotriazole hydrate (122 mg, 0.797 mmol), in ethyl acetate (10 ml). After 1 hour 4-dimethylaminopyridine (20 mg, 0.16 mmol) was added followed by a solution of the trifluoroacetate salt (59) 235 mg, 0.64 mmol) and triethylamine (152 mg, 1.50 mmol) in ethyl acetate (10 ml). After stirring at room temperature for 24 hours, the reaction mixture was filtered and the filtrate washed with 1M citric acid solution (2 x 20 ml), saturated sodium hydrogen carbonate solution (2 x 20 ml), then sodium chloride solution (20 ml). The organic phase was dried over $MgSO_4$ and filtered. The filtrate was evaporated in vacuo and the residue chromatographed over silica gel using $CH_2Cl_2$ then 2% MeOH in $CH_2Cl_2$ as eluants to give the product as a white foam (293 mg, 73%), mp 63-68°C; IR (film) 1713 and 1658 $cm^{-1}$; NMR ($CDCl_3$) δ 1.25 (3H, t, J 7Hz), 1.52-2.00 (17H, m), 2.64-2.86 (4H, m) 3.21 (2H, dd, J 4 and 15Hz), 3.31 (1H, Ha of ABq, J 15Hz) 3.49 (1H, Hb of $AB_q$, J 15Hz), 4.16 (2H, q, J 7Hz), 4.31 (1H, m), 4.8 (1H, br.), 5.23 (1H, br.), 6.72 (1H, d, J 8Hz) 6.94 (1H, d, J, 2Hz), 7.07-7.26 (7H, m) 7.34 (1H, d, J 8Hz) 7.62 (1H, d, J 8Hz), 8.17 (1H, br.); MS m/e (FAB) 632 (100); Anal. ($C_{36}H_{45}N_3O_5S$); C, H, N, S.

Step 5

2-Adoc-α-Me-D-$TrpNHCH(CH_2SCH_2CO_2H)$ $CH_2Ph$; [R-(R*,S*)-[[2-[[3-(1H-indol-3-yl)-2-methyl-1-oxo-2-[[(tricyclo [$3.3.1.1^{3,7}$]dec-2-yloxy)carbonyl]amino]-propyl]amino]-3-phenylpropyl]thio]acetic acid

To a solution of the ethyl ester (100 mg, 0.16 mmol) in ethanol (2 ml) was added 1M NaOH (0.17 ml) solution. The resulting homogenous reaction mixture was stirred at room temperature for 2 hours. After this time the solution was concentrated in vacuo and the residue partitioned between ethyl acetate and 1M HCl solution. The organic layer was washed with saturated sodium chloride solution, dried ($MgSO_4$) and concentrated to yield an amorphous solid (80 mg). This crude product was then purified by reverse phase column cbromatography using 66% MeOH in $H_2O$ as eluant to yield the desired product (61) as an amorphous solid (61 mg, 63%), mp 112-130.5°C; IR (film) 1709 and 1657 $cm^{-1}$; NMR ($CDCl_3$) δ 1.50-1.99 (16H, m), 2.45-2.85 (4H, m), 3.15-3.25 (3H, m), 3.44 (1H, Ha of ABq, J 15Hz), 4.29 (1H, m), 4.82 (1H, br.s), 5.40 (1H, br.s), 6.79 (1H, br.m), 6.98-7.25 (9H, m), 7.31 (1H, d, J 8Hz), 7.56 (1H, d, J 8Hz) 8.44 (1H, br.s). MS m/e. (FAB) 135 (100) 604 (13) Anal. ($C_{34}H_{41}N_3O_5S \cdot O.1H_2O$), C, H, N, S.

Example 25

[R-(R*,S*)]-[[2-[[3-(1H-indol-3-yl)-2-methyl-1-oxo-2-[[(tricyclor[3.3.1.1$^{3,7}$]dec-2-yloxy)carbonyl]amino]-propyl]amino]-3-phenylpropyl]sulfonyl]acetic acid

Step 1

BoCNHCH($CH_2SO_2CH_2CO_2Et$ $CH_2Ph$: (S)-[[2-[[(1.1-Dimethylethoxy)carbonyl]amino]-3-phenylpropyl]sulfonyl]acetic acid ethyl ester

A solution of potassium permanganate (411 mg, 2.60 mmol) in water (5 ml) was added dropwise over 5 minutes to a solution of the sulphide, BOCNHCH-($CH_2SCH_2CO_2Et$)$CH_2Ph$, (459 mg, 1.3 mmol) in 50% aqueous acetic acid (10 ml). After 1 hour, a 30% solution of hydrogen peroxide was added until the mixture went colorless. This was then diluted with ethyl acetate and washed with saturated sodium hydrogen carbonate solution. The dried ($MgSO_4$) organic phase was filtered and solvent removed in vacuo to yield the sulphone as a white amorphous solid (424 mg, 85%), mp 141-142°C; IR (film), 1741, 1692, 1323, and 1138 cm$^{-1}$; NMR ($CDCl_3$) δ 1.28 (3H, t, J 7Hz), 1.41 (9H, s), 2.99-3.03 (2H, m), 3.43-3.51 (2H, m), 4.00-4.11 (2H, m), 4.23 (2H, q, J 7Hz), 4.40 (1H, m), 4.95 (1H, br.), 7.20-7.34 (5H, m).

Step 2

$H_2NCH(CH_2SO_2CH_2CO_2Et)$ $CH_2Ph{\cdot}CF_3CO_2H$; (S)-[(2-Amino-3-phenylpropyl)sulfonyl]acetic acid ethyl ester trifluoroacetate (salt) (1:1)

Method as for Example 24, Step 3, except using N-protected ester above, (yield - 439 mg from 424 mg).

Step 3

2-Adoc-α-Me-D-TrpNHCH ($CH_2SO_2CH_2CO_2Et$)$CH_2Ph$; [R-(R*,S*)]-[[2-[[3-(1H-Indol-3-yl)-2-methyl-1-oxo-2-[[(tricyclo [3.3.1.1$^{3,7}$]dec-2-yloxy)carbonyl]amino]-propyl]amino]-3-phenylpropyl]sulfonyl]acetic acid ethyl ester

Method as for Example 24, Step 4, except using the above amine, (yield 55%), mp 69-80°C; IR (film) 1739, 1704, and 1665 cm$^{-1}$; NMR ($CDCl_3$) δ 1.25 (3H, t, J 7Hz), 1.46 (3H, s), 1.52-2.04 (14H, m), 2.91 (1H, dd, J 7 and 14Hz), 3.02 (1H, dd, J 7 and 14Hz), 3.18-3.52 (4H, m), 3.85 (1H, Ha of ABq, J 15hZ), 4.01 (1H, Hb of ABq, J 15Hz), 4.13-4.22 (2H, m), 4.64-4.68 (1H, m) 4.79 (1H, s) 5.07 (1H, s), 6.95-7.39 (10H, m), 7.59 (1H, d, J 8Hz) 8.15 (1H, br.); MS m/e 664 (100); Anal. ($C_{36}H_{45}N_3O_7S$), C, H, N, S.

Step 4

2-Adoc-α-Me-D-TrpNHCH($CH_2SO_2CH_2CO_2H$)$CH_2Ph$; [R-(R*S*)]-[[2-[[3-(1H-Indol-3-yl)-2-methyl-1-oxo-2-[[(tricyclo [3.3.1.1$^{3,7}$]dec-2-yloxy)carbonyl]amino]-propyl]amino]-3-phenylpropyl]sulfonyl]acetic acid

Method as for Example 24, Step 5, except using the carbonic ester, (yield 63%) white amorphous solid, mp 121-136°C; IR (film) 1713, 1664, 1317, and 1116 cm$^{-1}$; NMR ($CDCl_3$) δ 1.46-2.01 (17H, m), 2.94 (2H, d, J 6Hz), 3.17-3.44 (4H, m), 3.92 (2H, br.) 4.63 (1H, m), 4.80 (1H, br.s), 5.32 (2H, br.) 6.95-7.25 (9H, m), 7.31 (1H, d, J 8Hz), 7.54 (1H, d, J 8Hz), 8.46 (1H, br.s); MS m/e 658 (FAB) (100); Anal. ($C_{34}H_{41}N_3O_7S{\cdot}0.1H_2O$), C, H, N, S.

Example 26

[R-(R*.S*) ]-β-[[3-(1H-indol-3-yl)-2-methyl-1-oxo-2-[[tricyclo[3.3.1.1$^{3,7}$]dec-2 yloxy)carbonyl]amino]-propyl]amino]-4-iodo-benzenebutanoic acid

Step 1

(S)-2-t-Butyloxycarbonylamino-3-(4-iodophenyl)propionic acid (0.79 g, 2.0 mmol) was dissolved in anhydrous THF (10 ml) under nitrogen and N-methylmorpholine (0.20 g, 2.0 mmol) was added. The mixture was chilled in ice/salt and isobutylchloroformate (0.27 g, 2.0 mmol) was added dropwise. After stirring for 20 min the mixture was filtered and the precipitate washed with THF. A solution of diazomethane (approx 7 mmol) in $Et_2O$ was added in one portion

to the chilled filtrate, and the solution stirred overnight. After evaporation to dryness, the residue was dissolved in EtOAc and washed with water, 10% citric acid soln, saturated $NaHCO_3$ solution and water. After drying over $MgSO_4$, the solvents were evaporated and the residue recrystallized from EtOAc to give the title compound as pale yellow crystals (0.43 g, 52%); mp 119-122°C; IR (film) 2114 $cm^{-1}$; NMR ($CDCl_3$) δ 1.41 (9H, s), 2.85-3.05 (2H, m), 4.30-4.50 (1H, m), 5.00-5.10 (1H, m), 5.20-5.30 (1H, s), 6.93 (2H, d, J 8Hz), 7.62 (2H, d, J 8Hz); Anal ($C_{15}H_{18}IN_3O_3$), C, H, N.

Step 2

The diazoketone obtained in Step 1 (1.07 g, 2.58 mmol) was suspended in 2-(trimethysilyl) ethanol and a solution of silver benzoate (0.10 g) in triethylamine (1 ml) was added dropwise. After nitrogen evolution had ceased, further silver benzoate (0.01 g) in triethylamine (0.10 ml) was added. After stirring for 15 min the mixture was diluted with EtOAc, treated with charcoal and filtered. The solution was washed with 1M $NaHCO_3$ soln, water, 1M hydrochloric acid, water, 1M $NaHCO_3$ solution and water. The organic phase was dried over $MgSO_4$, filtered and evaporated. The residue was purified by flash chromatography eluting with 20% EtOAc/n-hexane, giving a pale yellow oil (0.80 g, 61 %); NMR ($CDCl_3$) δ 0.05 (9H, s), 0.95-1.00 (2H, m), 1.40 (9H, s), 2.40 (1H, dd, J 6, 16Hz), 2.47.(1H, dd, J 6, 16Hz), 2.76 (1H, dd, J 7, 14Hz), 2.80-2.95 (1H, m), 4.05-4.20 (3H, m), 5.00-5.10 (1H, bd), 6.94 (2H, d, J 8Hz), 7.61 (2H, d, J 8Hz); Anal ($C_{20}H_{32}INO_4Si$), C, H, N.

Step 3

To a solution of (S)-trimethylsilylethyl-3-t-butyloxycarbonylamino-4-(4-iodophenyl)butyrate (0.75 g, 1.5 mmol) from Step 2 in $CH_2Cl_2$ (10 ml) was added trifluoroacetic acid (0.6 ml, 7.8 mmol). After stirring at room temperature overnight the solution was washed with saturated $NaHCO_3$ solution and water. After drying over $MgSO_4$ the solution was filtered and evaporated to dryness to give the desired amine as an oil (0.60 g, 99%); NMR ($CDCl_3$) δ 0.04 (9H, s), 0.95-1.00 (2H, m), 2.29 (1H, dd, J 6, 16Hz), 2.45 (1H, dd, J 4, 16Hz), 2.55 (1H, dd, J 8, 13Hz), 2.71 (1H, dd, J 6, 13Hz), 3.45-3.50 (1H, m), 4.15-4.20 (2H, m), 6.96 (2H, d, J 8Hz), 7.63 (2H, d, J 8Hz); Anal ($C_{15}H_{24}INO_2Si$), C, H, N.

Step 4

α-Methyl-N-[(tricyclo[3.3.1.1$^{3,7}$]dec-2-yloxy)carbonyl]-R-tryptophan (0.55 g, 1.4 mmol) was stirred in EtOAc (20 ml) under nitrogen. 1-Hydroxy benzotriazole hydrate (0.21 g, 1.4 mmol) was added followed by N,N'-dicyclohexylcarbodiimide. After stirring for 2 h at room temperature the mixture was filtered and to the filtrate was added a solution of (S)-trimethylsilylethyl 3-amino-4(4-iodophenyl) butyrate (0.60 g, 1.5 mmol) from Step 3 in EtOAc (10 ml). After stirring for 16 h the mixture was concentrated in vacuo and the residue purified by flash chromatography eluting with 30% EtOAc/n-hexane. The product was recrystallized twice from EtOAc/n-hexane to give the desired amide as colourless crystals (0.4 g, 36%); mp 98-103°C; NMR ($CDCl_3$) δ 0.02 (9H, s), 0.90-1.00 (2H, m), 1.45-2.05 (17H, m), 2.32 (2H, d, J 5Hz), 2.62 (1H, dd, J 8, 14Hz), 2.75 (1H, dd, J 7, 14Hz), 3.30 (1H, d, J 15Hz), 3.45 (1H, d, J 15Hz), 4.03-4.16 (2H, m), 4.30-4.45 (1H, m), 4.78 (1H, s), 5.11 (1H, s), 6.87 (2H, d, J 9Hz), 6.90 (1H, d, J 3Hz), 7.07 (1H, d, J 7Hz), 7.09 (1H, t, J 7Hz), 7.15 (1H, t, J 8Hz), 7.32 (1H, d, J 8Hz), 7.54 (2H, d, J 8Hz), 7.58 (1H, d, J 8Hz), 8.06 (1H, s).

Step 5

To an ice-cooled solution of the ester obtained in Step 4 (0.30 g, 0.38 mmol) in THF (25 ml) under nitrogen was added dropwise a solution of tetrabutylammonium fluoride (1.0 M in THF, 1.0 ml, 1.0 mmol). After stirring at room temperature for 1 h the reaction mixture was concentrated in vacuo. The residue was taken up in EtOAc and washed with a 10% citric acid solution followed by brine. The organic solution was dried over $MgSO_4$ and concentrated in vacuo. The residue was taken up in MeOH and water added giving the title compound as a colourless solid (0.12 g, 59%); mp 104-109°C; NMR ($d_6$-DMSO) δ 1.21 (3H, s), 1.45-1.60 (2H, m), 1.70-2.05 (12H, m), 2.30-2.50 (2H, m), 2.65-2.85 (2H, m), 3.14 (1H, d, J 15Hz), 3.37 (1H, d, J 15Hz), 4.20-4.35 (1H, m), 4.69 (1H, s), 6.73 (1H, bs), 6.90-7.20 (5H, m), 7.33 (1H, d, J 8Hz), 7.48 (1H, d, J 8Hz), 7.61 (2H, d, J 8Hz), 7.65 (1H, d, J 9Hz), 10.90 (1H, s), 12.25 (1H, bs); Anal ($C_{33}H_{38}IN_3O_5$), C, H, N.

Example 27

[R-(R*,R*1]-[2-[[3-(1H-Indol-3-yl) -2-methyl-1-oxo-2-[[(1(tricyclo[[(3.3.1.1$^{3,7}$]dec-2-yloxy)carbonyl]amino]-propyl] amino]-1-phenylethoxy]acetic acid ,

Step 1

To stirred solution of (R)-2-chloro-1-phenylethanol (3.56 g, 22.89 mmol) in anhydrous DMF (40 mL) was added sodium azide (1.64 g, 25.18 mmol) in one portion. After 8 h at 100°C the mixture was poured onto ice and extracted with $Et_2O$ (3 x 100 mL). The combined $Et_2O$ extracts were washed with water (3 x 50 mL), dried over $MgSO_4$, filtered and the solvent removed in vacuo. The residue was purified by chromatography over silica gel using $CH_2Cl_2$ as eluant which gave the desired azide (3.10 g, 85%) as a colourless oil; IR (film) 3413 and 2107cm$^{-1}$; NMR ($CDCl_3$) δ 2.86 (1H, d, J 3.0Hz), 3.32-3.44 (2H, m), 4.75-4.80 (1H, m), 7.26-7.37 (5H, m); Anal ($C_8H_9N_3O$), C, H, N.

**Step 2**

To a suspension of 60% NaH (149 mg, 3.71 mmol) in anhydrous THF (3 mL) at 0°C and under an $N_2$ atmosphere was added tetramethylethylene diamine (0.90 mL, 5.94 mmol) followed by a solution of (R)-2-azido-1-phenylethanol from Step 1 (485 mg, 2.97 mmol) in anhydrous THF (3 mL) added over 3 min. The cold solution was stirred for 1.5 h and then a solution of methyliodoacetate (742 mg, 3.71 mmol) in anhydrous THF (3 mL) was added dropwise. After 24 h at room temperature the solution was diluted with $Et_2O$ (25 mL) and washed with 5% citric acid solution (2 x 25 mL) and brine (25 mL). The $Et_2O$ layer was dried ($MgSO_4$), filtered and the solvents removed in vacuo. The residue was purified by chromatography over silica gel using $CH_2Cl_2$ as eluant which gave the desired ether (257 mg, 37%) as a white waxy solid; mp 37-41°C; IR (film) 2105 and 1757cm$^{-1}$: NMR ($CDCl_3$) δ 3.29 (1H, dd, J 3.9, 12.9Hz), 3.60 (1H, dd, J 8.1, 12.9Hz), 3.74 (3H, s,). 3.95 (1H, d, J 16.1Hz), 4.12 (1H, d, J 16.4Hz), 4.67 (1H, dd, J 4.0, 8.1Hz), 7.32-7.42 (5H, m); Anal ($C_{11}H_{13}N_3O_3$), C, H, N.

Step 3

A solution of the azido ester from Step 2 (247 mg, 1.05 mmol) and 10M HCl solution (0.53 mL, 5.3 mmol) in absolute EtOH (50 mL) was reduced over 10% Pd/C (25 mg) at 40°C under an atmosphere of $H_2$ at 45 psi for 5 h. The catalyst was filtered off and the solvent removed in vacuo to give the amine hydrochloride (287 mg) which was without further purification in the next step; IR (film) 1738cm$^{-1}$ .

Step 4

To a stirred solution of α-methyl-N-[(tricyclo-[3.3.1.1$^{3,7}$]dec-2-yloxy)carbonyl]-R-tryptophan (333 mg, 0.84 mmol) and 1-hydroxy benzotriazole hydrate (161 mg, 1.05 mmol) in EtOAc (30 mL) was added N,N'-dicyclohexylcarbodiimide (191 mg, 0.92 mmol). After 1 h at room temperature triethylamine (0.174 mL, 1.25 mmol) was added followed by dropwise addition of a solution of the amine hydrochloride from Step 3 (272 mg, 1.05 mmol) in EtOAc (10 mL). After 24 h the reaction mixture was filtered and the EtOAc solution washed with 5% citric acid solution (2 x 25 mL), saturated $NaHCO_3$ solution (2 x 25 mL), 5% citric acid solution (25 mL) and brine (25 mL). The EtOAc extract was dried over $MgSO_4$, filtered and the solvent removed in vacuo. the residue was purified. by chromatography over silica using 30% EtOAc/n-hexane then 70% EtOAc/n-hexane as eluant to give the desired amide as a white solid (200 mg, 40%); mp 74-81°C; IR (film) 1743, 1705 and 1659cm$^{-1}$: NMR ($CDCl_3$) δ 1.26 (3H, t, J 7.2Hz), 1.48-2.04 (17H, m), 3.17-3.26 (1H, m), 3.48-3.60 (2H, m), 3.61-3.68 (1H, m), 3.81 (1H, d, J 16.8Hz), 4.07 (1H, d, J 17.0Hz), 4.15-4.32 (3H, m), 4.84 (1H, s), 5.60 (1H, br s), 7.03-7.42 (10H, m), 7.68 (1H, d, J 7.8Hz), 8.14 (1H, s); Anal ($C_{35}H_{43}N_3O_6$), C, H, N.

Step 5

To a stirred solution of the ester from Step 4 (178 mg, 0.30 mmol) in EtOH (10 ml) at 0°C was added dropwise 1.0M NaOH solution (0.33 ml, 0.33 mmol). The cooled solution was stirred for 2.5 h and then at room temperature for 21 h. A 1.0M HCl solution (0.36 ml, 0.36 mmol) was added and the solvents removed in vacuo. The residue was dissolved in EtOAc (25 ml) and then washed with brine (25 ml). The EtOAc extract was dried over $MgSO_4$, filtered and the solvent removed in vacuo. The residue was purified by chromatography over reverse phase silica using 67% MeOH : 33% $H_2O$ then 75% MeOH : 25% $H_2O$ as eluant giving the acid as a white solid (67 mg, 39%); mp 198-212°C; IR (film) 1700 and 1649cm$^{-1}$; NMR ($CDCl_3$) δ 1.54-2.01 (17H, m), 3.13-3.17 (1H, m), 3.21-3.55 (3H, m), 3.70-3.75(1H, m), 3.95 (1H, d, J 16.6Hz), 4.12 (1H, m), 4.18 (1H, br s), 7.01-7.63 (10H, m); Anal ($C_{33}H_{39}N_3O_6$. 0.5 $H_2O$), C, H, N.

Example 28

[[3-[[3-(1H-Indol-3-yl)-2-methyl-1-oxo-2-[[tricyclo(3.3.1.1$^{3,7}$]dec-2-yloxy)carbonyl]amino]-propyl]amino]-1-oxo-2-phenylpropyl]amino]acetic acid (TRP center is R, other center is RS).

Step 1

A solution of RS-ethylphenylcyanoacetate (5.0 g, 26.43 mmol) and 10M HCl (13.2 ml, 132 mmol) in EtOH (200 ml) was reduced over 10% Pd/C at 30°C under an atmosphere of $H_2$ at 45 psi for 18 h. The catalyst was filtered off and the solvent removed in vacuo giving a solid residue. Recrystallization from EtOH : $Et_2O$ (1 : 3, 100 ml) gave the amine (4.90 g, 81%) as white prisms; mp 158-160°C (EtOH : $Et_2O$); NMR ($d^4$-MeOH) δ 1.22 (3H, t, J 7.1Hz), 3.22 (1H, dd, J 6.0, 12.9Hz), 3.55 (1H, dd, J 8.9, 12.9Hz), 4.09-4.28 (3H, m), 7.28-7.43 (5H, m); Anal ($C_{11}H_{16}Cl\ N\ O_2$. 0.1 $H_2O$), C, H, N.

Step 2

To a stirred solution of α-methyl-N-[(tricyclo-[3.3.1.1$^{3,7}$]dec-2-yloxy)carbonyl]-R-tryptophan (397 mg, 1.0 mmol) and 1-hydroxybenzotriazole hydrate (191 mg, 1.25 mmol) in EtOAc (40 ml) was added N,N'-dicyclohexylcarbodiimide (227 mg, 1.10 mmol). After 1 h the amino ester hyrdochloride from Step 1 (253 mg, 1.10 mmol) was added followed by dropwise addition of a solution of triethylamine (0.153 ml, 1.10 mmol) in EtOAc (5 ml). After stirring at room temperature for 20 h the mixture was filtered and the EtOAc solution washed with 5% citric acid solution (2 x 25 ml), saturated $NaHCO_3$ solution (2 x 25 ml), 5% citric acid solution (25 ml) and brine (25 ml). The EtOAc extract was then dried over $MgSO_4$, filtered and the solvent removed in vacuo. The residue was purified by chromatography over silica using 1% MeOH : 99% $CH_2Cl_2$ as eluant which gave the desired amide (361 mg, 63%) as a white solid; mp 68-77°C; IR (film) 1719 and 1661cm$^{-1}$ ; NMR ($CDCl_3$) δ 1.17 (3H, t, J 7.1Hz), 1.47-1.99 (17H, m), 3.24-3.44 (2H, m), 3.61-3.90 (3H, m), 4.05-4.14 (2H, m), 4.80 (1H, br s), 5.05-5.20 (1H, m), 6.50-6.70 (1H, m), 6.92-7.59 (10H, m), 8.16-8.18 (1H, m); Anal ($C_{34}H_{41}N_3O_5$. 0.25 $H_2O$), C, H, N.

Step 2

To a stirred solution of α-methyl-N [(tricyclo-[3.3.1.1$^{3,7}$]dec-2-yloxy)carbonyl]-R-tryptophan (397 mg, 1.0 mmol) and 1-hydroxybenzotriazole hydrate (191 mg, 1.25 mmol) in EtOAc (40 ml) was added N,N'-dicyclohexylcarbodiimide (227 mg, 1.10 mmol). After 1 h the amino ester hyrdochloride from Step 1 (253 mg, 1.10 mmol) was added followed by dropwise addition of a solution of triethylamine (0.153 ml, 1.10 mmol) in EtOAc (5 ml). After stirring at room temperature for 20 h the mixture was filtered and the EtOAc solution washed with 5% citric acid solution (2 x 25 ml), saturated $NaHCO_3$ solution (2 x 25 ml), 5% citric acid solution (25 ml) and brine (25 ml). The EtOAc extract was then dried over $MgSO_4$, filtered and the solvent removed in vacuo. The residue was purified by chromatography over silica using 1% MeOH : 99% $CH_2Cl_2$ as eluant which gave the desired amide (361 mg, 63%) as a white solid; mp 68-77°C; IR (film) 1719 and 1661cm$^{-1}$; NMR ($CDCl_3$) δ 1.17 (3H, t, J 7.1Hz), 1.47-1.99 (17H, m), 3.24-3.44 (2H, m), 3.61-3.90 (3H, m), 4.05-4.14 (2H, m), 4.80 (1H, br s), 5.05-5.20 (1H, m), 6.50-6.70 (1H, m), 6.92-7.59 (10H, m), 8.16-8.18 (1H, m); Anal ($C_{34}H_{41}N_3O_5$. 0.25 $H_2O$), C, H, N.

Step 3

To a stirred solution of the ester from Step 2 (1.28 g, 2.23 mmol) in THF (130 ml) at 0°C was added dropwise over 75 min 0.1M LiOH solution (24.6 ml, 2.46 mmol). The cooled solution was stirred for 27 h with gradual warming to room temp. A 1.0M HCL solution (2.7 ml, 2.7 mmol) was added and the THF removed in vacuo. The residue was extracted with EtOAc (2 x 50 ml) and the combined organic extracts washed with brine (1 x 50 ml). The EtOAc layer was dried over $MgSO_4$, filtered and the solvent removed in vacuo. The residue was purified by chromatography over reverse phase silica using 67% MeOH : 33% $H_2O$ as eluant which gave the desired acid as a mixture of 2 diastereoisomers and as a white solid; mp 179-188°C; IR (film) 1700, 1657cm$^{-1}$; NMR ($d^4$-MeOH) δ 1.31 and 1.33 (3H, 2s), 1.54-2.03 (14H, m), 3.18-3.81 (5H, m), 4.75 (1H, br s), 6.94-7.50 (10H, m); Anal ($C_{32}H_{37}N_3O_5$. 1.0 $H_2O$), C, H, N.

Step 4

To a stirred solution of the acid from Step 3 (272 mg, 0.50 mmol) and 1-hydroxybenzotriazole hydrate (96 mg, 0.63 mmol) in EtOAc (30 ml) was added N,N'-dicyclohexylcarbodiimide (124 mg, 0.60 mmol). After 1 h at room temperature glycine benzylester hydrochloride (151 mg, 0.75 mmol) was added followed by triethylamine (0.112 ml, 0.80

mmol). The mixture was stirred at room temperature for 24 h and then filtered. The EtOAc solution was washed with 5% citric acid solution (2 x 25 ml), saturated $NaHCO_3$ solution (2 x 25 ml), 5% citric acid solution (25 ml) and brine (25 ml). The EtOAc solution was dried over $MgSO_4$, filtered and the solvent removed in vacuo. The residue was purified by chromatography over silica using 50% EtOAc : 50% n-hexane to give the desired amide as a white solid and as a mixture of 2 diastereoisomers (222 mg, 64%); mp 86-95°C; IR (film) 1742, 1710 and 1661cm$^{-1}$; NMR ($CDCl_3$) δ 1.49-2.03 (17H, m), 3.22-3.53 (4H, m), 3.68-3.80 (2H, m), 3.94-4.13 (1H, m), 4.80 (1H, m), 5.06-5.40 (3H, m), 5.74-5.78 (1H, m), 6.78-7.39 (10H, m), 7.57 and 7.65 (1H, 2d, J 8Hz), 8.06 and 8.22 (1H, 2s); Anal ($C_{41}H_{46}N_4O_6$. 0.25 $H_2O$), C, H, N.

Step 5

A solution of the benzylester from Step 4 (145 mg, 0.21 mmol) in absolute EtOH (50 ml) was reduced over $Pd(OH)_2/C$ (15 mg) at 40°C under an atmosphere of $H_2$ at 45 psi for 6 h. Filtration of the catalyst and removal of the solvent in vacuo gave a foam. Purification by chromatography over reverse phase silica using 67% MeOH : 33% $H_2O$ then 75% MeOH :25% $H_2O$ gave the product as a white solid and as 2 diastereoisomers (62 mg, 49%); mp 122-131°C; IR (film) 1700 and 1661cm$^{-1}$; NMR ($d^6$-DMSO) δ 1.22-1.97 (17H, m), 3.17-3.67 (6H, m), 3.90 (1H, dd, J 7.5, 15.1Hz), 4.71 (1H, br s), 6.61-6.65 (1H, m), 6.92-7.08 (3H, m), 7.24-7.48 (7H,m), 7.62 and 7.81 (1H, 2br s), 8.29-8.36 (1H, m), 10.88 (1H, s); Anal ($C_{34}H_{40}N_4O_6$. 0.75 $H_2O$), C, H, N.

Example 29

(R)-[[[2-[[3-(1H-indol-3-yl)-1-oxo-2-methyl-2-[[(tricyclo[3.3.1.1$^{3,7}$]dec-2-yloxy) carbonyl]amino]-propyl]aminol-1-phenylethylidene]amino]oxy]acetic acid

**Step 1**

To a stirred suspension of α-aminoacetophenone hydrochloride (6.60 g, 38.5 mmol) in anhydrous THF (100 ml) at 0°C was added 2-(trimethylsilyl) ethyl chloroformate (7.0 g, 38.5 mmol) followed by a solution of triethylamine (7.78 g, 76.9 mmol) in THF (30 ml). The reaction was complete after 10 h as assayed by thin layer chromatography. The reaction mixture was filtered and the solvent removed in vacuo. The residue was purified by chromatography over silica using 25% EtOAc/n-hexane to give the desired urethane (5.62 g, 53%) as a yellow crystalline solid; IR (film) 1692cm$^{-1}$; NMR ($CDCl_3$) δ 0.05 (9H, s), 1.19 (2H, t, J 7Hz), 4.16 (2H, t, J 4Hz), 4.64 (2H, d, J 4Hz), 5.72 (1H, bs), 7.42 (2H, t, J 7Hz), 7.52-7.57 (1H, m), 7.90 (2H, d, J 7Hz).

Step 2

To a stirred solution of the ketone from Step 1 (5.62 g, 20.1 mmol) in absolute EtOH (50 ml) was added a solution of hydroxylamine hydrochloride (2.31 g, 33.2 mmol) and sodium acetate (3.30 g, 40.2 mmol) in water (25 ml). The reaction mixture was refluxed and reaction was complete after 18 h as assayed by thin layer chromatography. The reaction was cooled to room temperature and the solvent removed in vacuo. The organic material was extracted with EtOAc (2 x 100 ml) washed with water (2 x 50 ml) and dried over $MgSO_4$. The solvent was removed in vacuo. The residue was purified by chromatography over silica using 25% EtOAc/n-hexane then 50% EtOAc/n-hexane to give the oxime (3.01 g, 51%) as a pale yellow crystalline solid; mp 61-65°C; IR (film) 1692cm$^{-1}$ ; NMR ($CDCl_3$) δ 0.02 (9H, s), 1.23-1.28 (2H, t, J 7Hz), 4.16 (2H, t, J 8Hz), 4.45 (2H, d, J 6Hz), 5.37 (1H, bs), 7.38 (3H, t, J 3Hz), 7.74 (2H, bs), 8.30 (1H, bs).

Step 3

To a stirred solution of the oxime from Step 2 (1.85 g, 6.3 mmol) in toluene (30 ml) was added tetrabutylammonium bromide (0.37 g, 1.1 mmol) and methyl 2 bromo acetate (1.93 g, 12.6 mmol). To this reaction mixture a NaOH solution (5 ml, 10% w/w) was added dropwise. The reaction was complete after 4 h as assayed by thin layer chromatography. The reaction mixture was diluted with $Et_2O$ (50 ml), the organic layer washed with water, dried with $MgSO_4$ and the solvent removed in vacuo. The residue was purified by chromatography over silica using 25% EtOAc/n-hexane then 50% EtOAc/n-hexane to give the desired oxime ether (1.02 g, 49%) as a pale yellow oil. This was stored under nitrogen in the fridge until required; IR (film) 1751, 1717cm$^{-1}$; NMR ($CDCl_3$) δ 0.03 (9H, s), 0.99-01.02 (2H, m), 3.79 (3H, s), 4.16-4.22 (2H, m), 4.45 (2H, d, J 6Hz), 4.81 (2H, s), 6.05 (1H, bs), 7.36-7.39 (3H, m), 7.75-7.77 (2H, m) .

Step 4

To a stirred solution of the ester from Step 3 (1.00 g, 2.7 mmol) in acetonitrile (50 ml) under a nitrogen atmosphere was added a 1M tetrabutylammonium fluoride solution in THF (2 ml, 6.9 mmol). The reaction was complete after 70 h as assayed by thin layer chromatography. The solvent was removed in vacuo, the residue extracted with EtOAc (2 x 50 ml) washed with saturated $NaHCO_3$ soln, water and dried over $MgSO_4$. The solvent was removed in vacuo and the residue purified by chromatography over silica using 5% $MeOH/CH_2Cl_2$ to give the amine (0.265 g, 44 %) as a yellow oil; IR (film) 1757cm$^{-1}$ ; NMR ($CDCl_3$) δ 1.67 (2H, bs), 3.77 (3H, s), 3.92 (2H, bs), 4.78 (2H, s), 7.37-7.40 (3H, m), 7.61-7.64 (2H,m).

Step 5

To a stirred solution of α-methyl-N-[(tricyclo-[3.3.1.1$^{3,7}$]dec-2-yloxy)carbonyl]-R-tryptophan (446 mg, 1.13 mmol) in EtOAc (20 ml) was added 1-hydroxybenzotriazole hydrate (189 mg, 1.23 mmol) followed by a solution of N, N'-dicyclohexylcarbo-diimide (278 mg, 1.35 mmol) in EtOAc (5 ml). The mixture was stirred for 1 h after which time the amine from Step 4 (250 mg, 1.13 mmol) in EtOAc (10 ml) was added. This mixture was stirred for 24 h, filtered and the solvent removed in vacuo. The residue was purified by chromatography using 25% EtOAc/n-hexane, then 50% EtOAc/n-hexane as eluants. This gave the desired amide (379 mg, 56%), as a white foam; NMR ($CDCl_3$) δ 1.47-1.96 (17H, m), 3.46 (2H, bs), 3.72 (3H, s), 4.53 (2H, d, J 5Hz), 4.75 (2H, s), 4.81 (1H, bs), 6.58 (1H, bs), 6.87-7.72 (12H, m), 7.90 (1H, bs).

Step 6

To a solution of the methyl ester from Step 5 (100 mg, 0.17 mmol) in THF (8 ml) at -15°C was added 0.1M LiOH (1.75 ml, 0.175 mmol) dropwise over a 1 h period. The resulting solution was allowed to slowly warm to room temperature over 10 h. The reaction mixture was acidified with 1M HCl to pH4 and the solvent removed in vacuo. The organic residue was extracted with EtOAc (2 x 20 ml), washed with water, dried over $MgSO_4$ and filtered. The solvent was then removed in vacuo. The crude product was purified by reverse phase chromatography using 2.5 : 1 MeOH : $H_2O$. This gave the desired acid (55 mg, 56%) as a white foam; mp 138-142°C; IR (film) 1726, 1703cm$^{-1}$ ; NMR ($d_6$-DMSO) δ 1.08 (3H, bs), 1.47-1.90 (14H, m), 3.16 (2H, s), 4.43 (2H, d, J 4Hz), 4.64 (1H, bs), 4.70 (2H, bs), 6.56 (1H, bs), 6.87-7.54 (10H, m), 8.04 (1H, bs), 10.8 (1H, bs); Anal ($C_{33}H_{38}N_4O_6$), C, H, N.

Example 30

[R-(R*S*)]-β-[[3-(1H-Indol-3-yl)-2-methyl-1-oxo-2-[[(tricyclo[3.3.1.1$^{3,7}$]dec-2-yloxy)carbonyl]amino]propyl]-amino] benzenebutanoic acid

Step 1

To a stirred solution of N-t-butyloxycarbonyl-S-phenylalanine (7.12 g, 26.8 mmol) and N-methylmorpholine (3.0 ml, 26.8 mmol) in anhydrous THF (50 ml) at -10°C was added dropwise isobutyl-chloroformate (3.4 ml, 26.8 mmol). After 20 min the N-methyl-morpholine hydrochloride was filtered off and a solution of diazomethane (33.4 mmol) in $Et_2O$ (50 ml) was added in one portion to the filtrate at -10°C. The cooled solution was stirred for 30 min and then for 16 h at room temp. The solvents were removed in vacuo and the residue dissolved in EtOAc (50 ml) and washed with water (2 x 25 ml), 5% citric acid solution (2 x 25 ml), 1M $NaHCO_3$ (25 ml) and brine (25 ml). The EtOAc solution was dried over $MgSO_4$, filtered and the solvent removed in vacuo to give the diazoketone as a pale yellow solid (7.04 g, 90%); IR (film) 2109, 1709 and 1641cm$^{-1}$; NMR ($CDCl_3$) δ 1.41 (9H, s), 3.02 (2H, d, J 6.8Hz), 4.40 (1H, br s), 5.08-5.21 (2H, m), 7.17-7.33 (5H, m).

Step 2

To a stirred solution of 2-oxo-3-(t-butyloxycarbonylamino)-3-phenylpropanol (7.04 g, 24.0 mmol) from Step 1 in MeOH (70 ml) was added 7 ml of a solution of silver (I) benzoate (1.37 g, 6.0 mmol) in triethylamine (14 ml) causing evolution of nitrogen. When nitrogen evolution has ceased a further portion of the silver (I) benzoate solution (0.28 ml) was added and the resulting brown coloured solution was stirred for 15 min. After this time the solution was treated with charcoal, filtered and the solvents removed in vacuo giving a residue which was dissolved in EtOAc (50 ml). The yellow EtOAc solution was washed with water (2 x 25 ml), 1M $NaHCO_3$ (2 x 25 ml), 1M HCl (2 x 25 ml), 1M $NaHCO_3$ (25 ml) and brine (25 ml). The EtOAc solution was then dried ($MgSO_4$), filtered and the solvent removed in vacuo giving

the methyl ester as an oil (5.27, 75%); IR (film) 1741 and 1713cm$^{-1}$ ; NMR ($CDCl_3$) δ 1.40 (9H, s), 2.40-2.55 (2H, m), 2.77-2.95 (2H, m), 3.67 (3H, s), 4.08-4.17 (1H, m), 4.97 (1H, br s), 7.11-7.31 (5H, m).

Step 3

To a stirred solution of methyl-3-(t-butyloxycarbonylamino)-4-phenylbutyrate (4.16 g, 14.19 mmol) from Step 2 in $CH_2Cl_2$ (10 ml) was added trifluoroacetic acid (10 ml). After stirring for 1 h at room temperature the solvents were removed in vacuo giving the desired amine as an oil which was used without further purification in the next step.

Step 4

To a stirred solution of α-methyl-N-[(tricyclo-[3.3.1.1$^{3,7}$]dec-2-yloxy)carbonyl]-R-tryptophan (4.5 g, 11.35 mmol) and 1-hydroxybenzotriazole hydrate (1.92 g, 12.54 mmol) in EtOAc (100 ml) at room temperature was added N,N'-dicyclohexylcarbodiimide (2.93 g, 14.19 mmol). After 1 h 4-dimethylaminopyridine (0.14 g, 1.14 mmol) was added followed by dropwise addition of a solution of methyl-3-amino-4-phenylbutyrate trifluoroacetic acid salt (4.36 g, 14.19 mmol) from Step 3 and triethylamine (4.5 ml, 32.00 mmol) in EtOAc (25 ml) and the mixture stirred at room temperature for 72 h. The reaction mixture was then filtered and the EtOAc solution washed with 5% citric acid solution ( 2 x 25 ml), saturated $NaHCO_3$ solution (2 x 25 ml), 5% citric acid solution (50 ml) and brine (50 ml). The EtOAc layer was dried ($MgSO_4$), filtered and the solvent removed in vacuo. The residue was purified by chromatography over silica using 1% MeOH : 99% $CH_2Cl_2$ as eluant which gave the desired amide (3.27 g, 50%) as a white solid; mp 78-84°C; IR (film) 1722 and 1658cm$^{-1}$ ; NMR ($CDCl_3$) δ 1.45 (3H,s), 1.50-2.16 (14H, m), 2.40 (2H, d, J 5.1Hz), 2.71 (1H, dd, J 7.9, 13.7Hz), 2.84 (1H, dd, J 6.6, 13.7Hz), 3.30 (1H, d, J 14.7Hz), 3.47 (1H, d, J 14.7Hz), 3.60 (3H, s), 4.42-4.45 (1H, m), 4.81 (1H, s), 5.14 (1H, s), 6.89-7.28 (9H, m), 7.33 (1H, d, J 8.0Hz), 7.59 (1H, d, J 7.8Hz), 8.20 (1H, s); Anal ($C_{34}H_{41}N_3O_5$. 0.25 $H_2O$ ), C, H, N.

Step 5

To a solution of the methyl ester from Step 4 (2.5 g, 4.37 mmol) in THF (250 ml) at 0°C was added dropwise over 50 min an aqueous solution of 0.1M LiOH (48 ml, 4.80 mmol). The cooled solution was then allowed to warm to room temperature over 2 h and stirred at this temperature for a further 20 h. After this time 1M HCl (5.3 ml, 5.3 mmol) was added and the solution washed with $Et_2O$ (2 x 100 ml), the $Et_2O$ extract dried ($MgSO_4$), filtered and the solvents removed in vacuo which gave the acid as a white solid (2.24 g, 92%; mp 123-137°C; IR (film) 1708 and 1658cm$^{-1}$; NMR ($CDCl_3$) δ 1.51-2.00 (17H, m), 2.27-2.34 (2H, m), 2.70 (1H, dd, J 8.1, 13.5Hz), 2.82 (1H, dd, J 6.3, 13.6Hz), 3.23 (1H, d, J 14.7Hz), 3.43 (1H, d, J 14.7Hz), 4.42 (1H, m), 4.81 (1H, s), 5.41 (1H, br s), 6.87-7.31 (10H, m), 7.55 (1H, d, J 7.8Hz), 8.50 (1H, s); Anal ($C_{33}H_{39}N_3O_5$· 0.1 $H_2O$), C, H, N.

Example 31

[R-(R*,S*)]-N-[3-[[3-(1H-Indol-3-yl)-2-methyl-1-oxo-2-[[(tricyclo-[3.3.1.1$^{3,7}$]dec-2-yloxy)carbony]propyl]amino]-4-phenylbutyl]glycine

Step 1

To a stirred solution of the acid from Step 5 (291 mg, 0.52 mmol) and 1-hydroxybenzotriazole hydrate (88 mg, 0.65 mmol) in EtOAc (30 ml) was added N,N'-dicyclohexylcarbodiimide (129 mg, 0.62 mmol). After 1 h at room temperature 4-dimethylaminopyridine (6 mg, 0.05 mmol) was added followed by triethylamine (0.109 ml, 0.78 mmol) and glycine ethyl ester hydrochloride (109 mg, 0.78 mmol). The mixture was stirred at room temperature for 2 h and then filtered. The EtOAc solution was washed with 5% citric acid solution (2 x 25 ml), saturated $NaHCO_3$ solution (2 x 25 ml), 5% citric acid solution (25 ml) and brine (25 ml). The EtOAc solution was dried over $MgSO_4$, filtered and the solvent removed in vacuo. The residue was purified by chromatography over silica using 2% MeOH : 98% $CH_2Cl_2$ as eluant giving the desired amide as a white solid (212 mg, 64%); mp 82-94°C; IR (film) 1741, 1705 and 1651 cm$^{-1}$ ; NMR ($CDCl_3$) δ 1.27 (3H, t, J 7Hz), 1.37 (3H, s), 1.50-2.01 (14H, m), 2.30 (1H, dd, J 4.4, 14.0Hz), 2.51 (1H, dd, J 3.9, 13.7Hz), 2.70-2.85 (2H, m), 3.31 (2H, s), 3.75 (1H, dd, J 5.2, 17.8Hz), 4.09-4.23 (3H, m), 4.39-4.48 (1H, m), 4.74 (1H, br s), 5.17 (1H, s), 6.73 (1H, m), 6.81 (1H, d, J 2.1Hz), 7.06-7.28 (8H, m), 7.32 (1H, d, J 7.9Hz), 7.57 (1H, d, J 7.8Hz), 8.16 (1H, br s); Anal ($C_{37}H_{46}N_4O_6$), C, H, N.

Step 2

To a stirred solution of the ethyl ester from Step 1 (788 mg, 1.23 mmol) in EtOH (75 ml) at 0°C was added NaOH solution (13.5 ml of a 0.1M soln, 1.35 mmol) over 10 min. The cold solution was stirred with gradual re-warming to room temperature for 5.5 h. The EtOH was removed in vacuo and 5% citric acid solution (25 ml) added to the residue. The aqueous solution was extracted with $Et_2O$ (2 x 25 ml) the $Et_2O$ extract dried over $MgSO_4$, filtered and the solvent removed in vacuo to give the desired acid as a white foam (553 mg, 73%); mp 98-103°C; IR (film) 1700 and 1657cm$^{-1}$; NMR ($CDCl_3$) δ 1.37-1.98 (17H, m), 2.25-2.32 (2H, m), 2.69-2.79 (2H, m), 3.20 (1H, d, J 14.6Hz), 3.29 (1H, d, J 14.5Hz), 3.76 (1H, dd, J 4.7, 18.1Hz), 4.04 (1H, dd J 5.8, 17.7Hz), 4.36-4,40 (1H, m), 4.75 (1H, s), 5.37 (1H, br s), 6.83-7.19 (10H, m), 7.29 (1H, d, J 8.0Hz), 7.53 (1H, d, J 7.8Hz), 8.40-8.65 (1H, m); Anal ($C_{35}H_{42}N_4O_6\cdot$ $1H_2O$ ), C, H, N.

Example 32

2-[[[2-[[3-(1H-indol-3-yl)-2-methyl-1-oxo-2-[[(tricyclo-[3.3.1.1$^{3,7}$]dec-2-yloxy)carbonyl] amino]propyl] amino] -1-phenylethyl]amino]carbonyl]cyclopropanecarboxylic acid (cyclopropane ring is trans-(±) other centres are R).

Step 1

A solution (R)-β-[1-(phenylmethyl)amino]benzeneethanol (6.44 g, 23,8 mmol) in anhydrous $CH_2Cl_2$ (50 ml) was treated with triethylamine (2.88 g, 28.5 mmol), followed by a solution of p-toluene sulphonyl chloride (5.43 g, 28.5 mmol) in $CH_2Cl_2$ (20 ml). After stirring for 18 h at room temperature, the reaction mixture was washed with 1M citric acid solution (2 x 50 ml) and the organic phase dried over $MgSO_4$, filtered and the solvent evaporated in vacuo to give a crude, pale yellow solid (8.49 g) mp 103-105,5°C (EtOAc/n-hexane); IR (film) 3410, 1703, 1361 and 1190cm$^{-1}$; NMR ($CDCl_3$) δ 2.42 (3H, s), 4.25 (2H, m), 4.98 (1H, br s), 5.07 (2H, s), 5.35 (1H, br s), 7.20-7.40 (12H, m), 7.65 (2H, d, J 8Hz); Anal ($C_{16}H_{17}NO_3$) C,H,N. This crude solid (7.57 g) was dissolved in anhydrous DMF (100 ml) and treated with sodium azide (1.21 g, 18.6 mmol) then warmed to 80°C for 3 h, cooled and poured into ice water (200 ml). This mixture was extracted with $Et_2O$ (2 x 200 ml) and the combined organic phases washed with $H_2O$ (200 ml), dried over $MgSO_4$ and evaporated in vacuo to yield a yellow oil (4.95 g); IR (film) 3300, 2130 and 1697cm$^{-1}$; NMR ($CDCl_3$) δ 3.66 (2H, m), 4.95 (1H, m), 5.09 (1H, d, J 11Hz), 5.12 (1H, d, J 11Hz), 5.31 (1H, m), 7.25-7.45 (10H, m). This crude oil (5 g) in EtOAc (100 ml) was treated with Lindlar catalyst (2 g, 40% w/w) and placed under an atmosphere of hydrogen at 45 psi at 30°C for 6 h then filtered through filter aid to give a solution of the desired amine (R)-β-[1-(phenylmethyl)amino]-benzeneethanol which was used immediately assuming a quantitative yield; IR (film) 3300, 1703cm$^{-1}$ .

Step 2

A solution of the acid, α-methyl-N-[(tricyclo-[3.3.1.1$^{3,7}$]dec-2-yloxy)carbonyl]-R-tryptophan (4.60 g, 11.6 mmol) in EtOAc (30 ml) was treated with 1-hydroxybenzotriazole hydrate (1.96 g, 12.8 mmol) and N,N'-dicyclohexyl-carbodiimide (2.87 g, 13.9 mmol) and stirred at room temperature for 2 h before the amine from Step 1 (4.46 g, 16.9 mmol) in EtOAc (10 ml) was added. After stirring a further 18 h the mixture was filtered, concentrated in vacuo and purified by silica gel chromatography to give the desired urethane as a white solid (6.17 g, 56%); mp 69 - 73°C; $[\alpha]^{20}_D$ + 8.9° (c = 1, MeOH); IR (film) 3350, 1700 and 1662cm$^{-1}$; NMR ($CDCl_3$) δ 1.54 (5H, br), 1.60-1.95 (14H, m), 3.23 (1H, d, J 14Hz), 3.35 (1H, m), 3.43 (1H, d, J 14Hz), 3.72 (1H, m) 4.79 (2H, br s), 5.07 (2H, s), 5.13 (1H, s), 5.90 (1H, br s), 6.43 (1H, br s), 6.93 (1H, s), 7.10-7.40 (13H, m), 7.55 (1H, d, J 8Hz), 7.95 (1H, s); Anal ($C_{39}H_{44}N_4O_5.0.5H_2O$) C, H, N.

**Step 3**

A solution of the benzyl urethane from Step 2 (6.17 g, 8.94 mmol) in absolute EtOH (50 ml) was treated with Pearlman's catalyst (620 mg, 10% w/w/). The mixture was put under an atmosphere of hydrogen at 45 psi for 18 h at 25°C, filtered and concentrated in vacuo to yield the amine tricyclo[3.3.1.1$^{3,7}$]dec-2-yl[R-(R*,R*)]-[2-[(2-amino-2-phenylethyl)amino]-1-(1H-indol-3-ylmethyl)-1-methyl-2-oxoethyl]carbamate as a white foam, pure enough to be used directly in the next step (4.44 g, 89%); mp 91-94°C; $[\alpha]^{20}_D$ + 10.3° (c = 1, MeOH); IR (film) 3340, 1701 and 1658cm$^{-1}$ ; NMR ($CDCl_3$) δ 1.54 (5H, br s), 1.70-2.05 (14H, m), 3.15 (1H, ddd, J 6, 8 and 14Hz), 3.31 (1H, d, J 15Hz), 3.54 (1H, d, J 15Hz), 3.55 (1H, m), 3.97 (1H, m) 4.82 (1H, s), 5.15 (1H, s), 6.49 (1H, br s), 6.96 (1H, d, J 2Hz), 7.10-7.40 (8H, m), 7.59 (1H, d, J 8Hz), 8.19 (1H, s); Anal ($C_{31}H_{38}N_4O_3$. 0.75 $H_2O$ ), C, H, N.

Step 4

A solution of RS-mono methyl cyclopropanedicarboxylate (126 mg, 0.88 mmol) in anhydrous EtOAc (10 ml)

was treated with 1-hydroxybenzotriazole hydrate (132 mg, 0.86 mmol) and N,N'-dicyclohexylcarbodiimide (186 mg, 0.90 mmol) and stirred at room temperature for 2 h before the amine from Step 3 (300 mg, 0.58 mmol) was added. After stirring for a further 3 h the reaction mixture was filtered, concentrated in vacuo and purified by silica gel chromatography to give the desired amide as a mixture of 2 diastereoisomers (258 mg, 69%); mp 118-122°C; IR (film) s 3320, 2909, 2855, 1720, 1700, 1659 and 1531cm$^{-1}$ ; NMR ($CDCl_3$) δ 1.25-2.05 (20H, m), 2.15 (2H, m), 3.32 (2H, m), 3.48 (1H, d, J 14Hz), 3.67 and 3.69 (3H, 2s), 3.95 (1H, m), 4.84 (1H, br s), 5.04 (1H, s), 5.11 (1H, br s), 6.40 (1H, br s), 6.95 and 6.97 (1H, 2d, J 3Hz), 7.10-7.35 (9H, m) 7.55 and 7.58 (1H, 2d, J 4Hz), 8.24 (1H, s); Anal ($C_{37}H_{42}N_4O_6$. $0.5H_2O$), C, H, N.

Step 5

The methyl ester from Step 4 (238 mg, 0.37 mmol) as a solution in THF (20 ml) at 0°C was treated dropwise with aqueous LiOH solution (3.72 ml of 0.1M soln, 0.37 mmol). The resulting mixture was stirred at 0°C for 4 h and then allowed to warm to room temperature over 16 h. After this time the reaction was acidified with 1M HCl (0.5 ml), concentrated in vacuo and extracted with EtOAc. The organic phase was dried over $MgSO_4$, filtered and evaporated in vacuo. The residue was purified by reverse phase column chromatography, eluant 2.5 : 1 MeOH : $H_2O$, to give the desired acid as an amorphous white solid and a mixture of two diastereoisomers (45 mg, 20%); mp 138-142°C; NMR ($d^6$-DMSO) δ 1.14 (2H, m), 1.28 (3H s), 1.52 (2H, br s), 1.70-2.15 (14H, m), 3.10-3.50 (4H, m, +$H_2O$ ), 4.71 (1H, s), 5.05 (1H, m), 6.46 (1H, br s), 6.94 (2H, br s), 7.03 (1H, t, J 7Hz), 7.24 (1H, m), 7.31 (5H, br s), 7.46 (1H, d, J 7Hz), 7.68 (1H, m), 8.43 (1H, br s), 10.75 (1H, br s); Anal ($C_{36}H_{42}N_4O_6$ $0.5H_2O$), C, H, N.

Example 33

Tricyclo[3.3.1.1$^{3,7}$]dec-2-yl (R,(R*,S*]-[1-(1H-indol-3-ylmethvl)-1-methvl-2-oxo-2-((2-ff1-oxo-3-(IH-tetrazol-5-yl) proPvl]aminol-2-phenvlethyll-amino]ethyl]carbamic acid ester.

Step 1

To a solution of methyl 3-cyanopropionate (1 g, 8.8 mmol) in anhydrous DMF (15 ml) was added $NaN_3$ (0.77 g, 11.9 mmol) and $NH_4Cl$ (0.65 g, 11.9 mmol). The reaction was then heated to 110°C for 48 h. After this time the reaction mixture was concentrated in vacuo and the residue partitioned between saturated $NaHCO_3$ solution and $Et_2O$. The aqueous phase was separated, acidified to pH3 with 1M HCl and extracted with EtOAc. The organic extract was then dried over $MgSO_4$ and concentrated in vacuo to give the desired tetrazole as a colourless liquid (0.75 g, 69%); IR (film) 2400-3400 br, 1738cm$^{-1}$; NMR ($CDCl_3$) δ 2.89 (2H, t, J 7Hz), 3.30 (2H, t, J 7Hz), 3.70 (3H, s).

Step 2

To a solution of the tetrazole from Step 1 (0.36 g, 2.9 mmol) in anhydrous DMF (7 ml) was added cesium carbonate (1.05 g, 3.2 mmol) and benzyl bromide (0.53 g, 3.1 mmol). The reaction mixture was stirred at room temperature for 72 h. After this time the reaction mixture was filtered and concentrated in vacuo. The residue was partitioned between water and $Et_2O$ and the organic layer was dried, $MgSO_4$, and evaporated to yield a gummy residue (0.4 g). The residue was purified by column chromatography, eluant 50% EtOAc/n-hexane, to give the desired benzyl tetrazole in its two tautomeric forms (0.25 g, 34%); tautomer-I (144 mg, fastest running fraction); IR (film) 3025, 1739cm$^{-1}$ ; NMR ($CDCl_3$) δ 2.83 (2H, t, J 7Hz), 3.20 (2H, t, J 7Hz), 3.65 (3H, s), 5.70 (2H, s), 7.35 (5H, s); tautomer II (104 mg, slowest running fraction) IR (as above); NMR ($CDCl_3$) δ 2.90 (2H, t, J 7Hz), 3.00 (2H, t, J 7Hz), 3.70 (3H, s), 5.60 (2H, s), 7.25 (2H, m), 7.35 (3H, m).

Step 3

To an ice-cooled solution of the combined tautomeric forms of the benzyl tetrazole from Step 2 (248 mg, 1.0 mmol) in THF (15 ml) was added 0.1M LiOH solution (10.6 ml, 1.0 mmol) dropwise over 2 h. The reaction mixture was then slowly allowed to warm to room temperature over 16 h. After this time the reaction was acidified to pH3 with 1M HCl and concentrated in vacuo. The residue was partitioned between water and EtOAc and the organic layer was dried ($MgSO_4$) and concentrated in vacuo to yield the desired acid as a colourless liquid (151 mg, 65%) and as a mixture of two tautomers of the benzyl tetrazole; IR (film) 2600-3600, 1729cm$^{-1}$; NMR ($CDCl_3$) δ 2.90 (α3H, m) and 3.20 (α1H, t, J 7Hz), 5.55 and 5.65 (2H, s), 7.35 (5H, s).

Step 4

To a solution of the acid from Step 3 (135 mg, 0.58 mmol) in anhydrous EtOAc (10 ml) was added pentafluorophenol (108 mg, 0.58 mmol) and N,N'-dicyclohexylcarbodiimide (120 mg, 0.58 mmol). After stirring at room temperature for 1 h the amine, tricyclo[3.3.1.$1^{3,7}$]dec-2-yl[R-(R*,R*)]-[2-[(2-amino-2-phenylethyl)amino]-1-(1H-indol-3-ylmethyl)-1-methyl-2-oxoethyl]carbamate from Step 3, (300 mg, 0.58 mmol) in EtOAc (2 ml) was added. The reaction mixture was stirred for 16 h, filtered and concentrated in vacuo. The residue was purified by column chromatography, eluant 3 : 1 EtOAc/n-hexane, to give the desired amide as two tautomeric forms around the benzyl tetrazole moiety (115 mg, 27%); mp 100-105°C; IR (film) 3300, 2912, 1690 and 1661$cm^{-1}$ ; tautomer I (105 mg, fastest running fraction); NMR ($CDCl_3$) δ 1.47 (3H, s), 1.50-2.00 (14H, m), 2.73 (2H, t, J 7Hz), 3.20 (2H, t, J 7Hz), 3.33 (2H, d, J 15Hz and m), 3.45 (1H, d, J 15Hz), 3.92 (1H, m), 4.81 (1H, br s), 5.10 (1H, m), 5.13 (1H, s), 5.65 (2H, s), 6.39 (1H, m), 6.93 (1H, d, J 7Hz), 6.99 (1H, d, J 2Hz), 7.05-7.20 (7H, m), 7.32 (6H, s), 7.57 (1H, d, J 8Hz), 8.50 (1H, s); tautomer II (110 mg, slowest running fraction); NMR ($CDCl_3$) δ 1.45 (3H, s), 1.50 (2H, m), 1.65-1.95 (12H, m), 2.75-2.95 (3H, m), 3.10 (1H, m), 3.25 (2H, m), 3.45 (1H, d, J 15Hz), 4.00 (1H, m), 4.75 (1H, br s), 5.05 (1H, m), 5.10 (1H, s), 5.45 (2H, s), 6.47 (1H, m), 6.95-7.35 (14H, m), 7.45 (1H, d, J 7Hz), 7.60 (1H, d, J 7Hz), 8.80 (1H, s); Anal ($C_{42}H_{48}N_8O_4$. 0.85 $H_2O$), C, H, N.

Step 5

A solution of the benzyltetrazole tautomer mixture from Step 4 (100 mg, 0.14 mmol) in absolute EtOH (50 ml) was treated with Pearlman's catalyst (20 mg, 20% w/w). The mixture was put under an atmosphere of hydrogen at 45 psi for 18 h at 50°C, filtered and concentrated in vacuo to yield a gum (100 mg). The residue was purified by reverse phase column chromatography - eluant 3:1 MeOH : $H_2O$ - to yield the desired tetrazole as a white solid (30 mg, 34%); mp 169-173°C; IR (film) 3300, 2907, 1704, 1659 and 1535$cm^{-1}$ ; NMR ($d^6$-DMSO) δ 1.28 (3H, s), 1.46 (2H, m), 1.65-1.95 (12H, m), 2.45 (2H, m), 2.89 (2H, t, J 7Hz), 3.20-3.50 (4H, m, and $H_2O$ ), 4.67 (1H, br s), 4.98 (1H, m), 6.80-7.05 (4H, m), 7.25 (6H, m), 7.46 (1H, d, J 8Hz), 8.35 (2H, m), 10.90 (1H, s); Anal ($C_{35}H_{42}N_8O_4$. 1$H_2O$), C, H, N.

Example 34

Carbamic acid, [1-(1H-indol-3-ylmethvl)-1-methyl-2-oxo-2-[[2-[[1-oxo-3-(1H-tetrazol-5-yl)propyl]amino]-2-phenylethyl)-amino]ethyl]-,tricyclo[3.3.1.$1^{3,7}$]dec-2-yl ester,[R,(R*,S*]

Step 1

To a solutionn of methyl 3-cyanopropionate (1 g, 8.8 mmol) in anhydrous DMF (15 mL) was added $NaN_3$ (0.77 g, 11.9 mmol) and $NH_4Cl$ (0.65 g, 11.9 mmol). The reaction was then heated to 110°C for 48 h. After this time the reaction mixture was concentrated in vacuo and the residue partitioned between saturated $NaHCO_3$ solution and $Et_2O$. The aqueous phase was separated, acidified to pH3 with 1M HCl and extracted with EtOAc. The organic extract was then dried over $MgSO_4$ and concentrated in vacuo to give the desired tetrazole as a colourless liquid (0.75 g, 69%); IR (film) 2400-3400 br, 1738$cm^{-1}$; NMR ($CDCl_3$) δ 2.89 (2H, t, J 7Hz), 3.30 (2H, t, J 7Hz), 3.70 (3H, s).

Step 2

To a solution of the tetrazole from Step 1 (0.36 g, 2.9 mmol) in anhydrous DMF (7 mL) was added caesium carbonate (1.05 g, 3.2 mmol) and benzyl bromide (0.53 g, 3.1 mmol). The reaction mixture was stirred at room temperature for 72 h. After this time the reaction mixture was filtered and concentrated in vacuo. The residue was partitioned between water and $Et_2O$ and the organic layer was dried, $MgSO_4$, and evaporated to yield a gummy residue (0.4 g). The residue was purified by column chromatography, eluant 50% EtOAc/n-hexane, to give the desired benzyl tetrazole in its two tautomeric forms (0.25 g, 34%); tautomer-I (144 mg, fastest running fraction); IR (film) 3025, 1739$cm^{-1}$; NMR ($CDCl_3$) δ 2.83 (2H, t, J 7Hz), 3.20 (2H, t, J 7Hz), 3.65 (3H, s), 5.70 (2H, s), 7.35 (5H, s); tautomer II (104 mg, slowest running fraction) IR (as above); NMR ($CDCl_3$) δ 2.90 (2H, t, J 7Hz), 3.00 (2H, t, J 7Hz), 3.70 (3H, s), 5.60 (2H, s), 7.25 (2H, m), 7.35 (3H, m).

Step 3

To an ice-cooled solution of the combined tautomeric forms of the benzyl tetrazole from Step 2 (248 mg, 1.0 mmol) in THF (15 mL) was added 0.1M LiOH solution (10.6 mL, 1.0 mmol) dropwise over 2 h. The reaction mixture was then slowly allowed to warm to room temperature over 16 h. After this time the reaction was acidified to pH3 with 1M HCl and concentrated in vacuo. The residue was partitioned between water and EtOAc and the organic layer was

dried ($MgSO_4$) and concentrated in vacuo to yield the desired acid as a colourless liquid (151 mg, 65%) and as a mixture of two tautomers of the benzyl tetrazole; IR (film) 2600-3600, 1729cm$^{-1}$ ; NMR ($CDCl_3$) δ 2.90 (≈3H, m) and 3.20 (≈1H, t, J 7Hz), 5.55 and 5.65 (2H, s), 7.35 (5H, s).

Step 4

To a solution of the acid from Step 3 (135 mg, 0.58 mmol) in anhydrous EtOAc (10 mL) was added pentafluorophenol (108 mg, 0.58 mmol) and N, N'-dicyclohexylcarbodiimide (120 mg, 0.58 mmol). After stirring at room temperature for 1 h the amine, tricyclo[3.3.1.1$^{3,7}$]dec-2-yl[R-(R*,R*)]-[2-[(2-amino-2-phenylethyl)amino]-1-(1H-indol-3-ylmethyl)-1-methyl-2-oxoethyl]carbamate from Step 3 (300 mg, 0.58 mmol) in EtOAc (2 mL) was added. The reaction mixture was stirred for 16 h, filtered and concentrated in vacuo. The residue was purified by column chromatography, eluant 3 : 1 EtOAc/n-hexane, to give the desired amide as two tautomeric forms around the benzyl tetrazole moiety (115 mg, 27%); mp 100-105°C; IR (film) 3300, 2912, 1690 and 1661cm$^{-1}$ ; tautomer I (105 mg, fastest running fraction); NMR ($CDCl_3$) δ 1.47 (3H, s), 1.50-2.00 (14H, m), 2.73 (2H, t, J 7Hz), 3.20 (2H, t, J 7Hz), 3.33 (2H, d, J 15Hz and m), 3.45 (1H, d, J 15Hz), 3.92 (1H, m), 4.81 (1H, br s), 5.10 (1H, m), 5.13 (1H, s), 5.65 (2H, s), 6.39 (1H, m), 6.93 (1H, d, J 7Hz), 6.99 (1H, d, J 2Hz), 7.05-7.20 (7H, m), 7.32 (6H, s), 7.57 (1H, d, J 8Hz), 8.50 (1H, s); tautomer II (110 mg, slowest running fraction); NMR ($CDCl_3$) δ 1.45 (3H, s), 1.50 (2H, m), 1.65-1.95 (12H, m), 2.75-2.95 (3H, m), 3.10 (1H, m), 3.25 (2H, m), 3.45 (1H, d, J 15Hz), 4.00 (1H, m), 4.75 (1H, br s), 5.05 (1H, m), 5.10 (1H, s), 5.45 (2H, s), 6.47 (1H, m), 6.95-7.35 (14H, m), 7.45 (1H, d, J 7Hz), 7.60 (1H, d, J 7Hz), 8.80 (1H, s); Anal ($C_{42}H_{48}N_8O_4$. 0.85 $H_2O$), C, H, N.

Step 5

A solution of the benzyltetrazole tautomer mixture from Step 4 (100 mg, 0.14 mmol) in absolute EtOH (50 mL) was treated with Pearlman's catalyst (20 mg, 20% w/w). The mixture was put under an atmosphere of hydrogen at 45 psi for 18 h at 50°C, filtered and concentrated in vacuo to yield a gum (100 mg). The residue was purified by reverse phase column chromatography - eluant 3:1 MeOH : $H_2O$ - to yield the desired tetrazole as a white solid (30 mg, 34%); mp 169-173°C; IR (film) 3300, 2907, 1704, 1659 and 1535cm$^{-1}$ ; NMR ($d^6$-DMSO) δ 1.28 (3H, s), 1.46 (2H, m), 1.65-1.95 (12H, m), 2.45 (2H, m), 2.89 (2H, t, J 7Hz), 3.20-3.50 (4H, m, and $H_2O$ ), 4.67 (1H, br s), 4.98 (1H, m), 6.80-7.05 (4H,m), 7.25 (6H, m), 7.46 (1H, d, J 8Hz), 8.35 (2H, m), 10.90 (1H, s); Anal. ($C_{35}H_{42}N_8O_4 \cdot 1H_2O$), C, H, N.

Example 35

Benzeneheptanoic acid, α-[[3-(1H-indol-3-yl)-2-methyl-1-oxo-2-[[(tricyclo[3.3.1.1$^{3,7}$]dec-2-yloxy)carbonyl]amino] propyl]-amino]-,[R-(R*,S*)]

Step 1

To a stirred solution of N-(t-butyloxycarbonyl)phenylalanine (13 g, 49.0 mmol) and N-methylmorpholine (11 ml, 100 mmol) in $CH_2Cl_2$ (125 ml) at -10°C was added isobutyl chloroformate (6.5 ml, 50.0 mmol). After 15 min at -10°C N,O-dimethylhydroxylamine hydrochloride (5.02 g, 51.5 mmol) was added and the cold solution stirred for 1 h then at room temperature for 3 h. The mixture was poured into water (100 ml) and the organic layer separated. The aqueous layer was extracted with $CH_2Cl_2$ (2 x 100 ml), the combined organic layers dried ($MgSO_4$), filtered and the solvents removed in vacuo. The residue was purified by filtering through silica using 2% MeOH : 98% $CH_2Cl_2$ as eluant which gave the product (14.39 g, 95%) as an oil; NMR ($CDCl_3$) δ 1.38 (9H, s), 2.84-3.16 (5H, m), 3.65 (3H, s), 4.94-4.96 (1H, m), 5.22-5.25 (1H, m), 7.16-7.30 (5H, m).

Step 2

To a stirred solution of the hydroxamate from Step 1 (1.38 g, 4.48 mmol) in anhydrous THF (20 ml) at 0°C was added dropwise a solution of 1.0M $LiAH_4$ in THF (11.7 ml, 11.70 mmol). After 30 min wet $Et_2O$ (100 ml) was added followed by an ice-cooled 20% citric acid solution (100 ml). After a further 30 min the $Et_2O$ layer was separated and the aqueous solution was extracted once with $Et_2O$ (100 ml). The combined $Et_2O$ extracts were washed with saturated $NaHCO_3$ solution (50 ml), water (50 ml), 5% citric acid solution (50 ml) and water (50 ml). The $Et_2O$ solution was then dried over $MgSO_4$, filtered and the solvent removed in vacuo to give a white solid (1.09 g, 97%); IR (film) 3367, 1733 and 1689cm$^{-1}$ ; NMR ($CDCl_3$) δ 1.43 (9H, s), 3.11 (2H, d, J 6Hz), 4.38-4.45 (1H, m), 5.10 (1H, m), 7.15-7.35 (5H, m), 9.62 (1H, s).

Step 3

Methyl-4-bromocrotonate (4.48 g, 25 mmol) and triphenylphosphine (6.55 g, 25 mmol) were heated together at 150°C for 25 min. Recrystallization of the brown residue from EtOH/$Et_2O$ gave the phosphonium salt (5.76 g, 52%) as an off white solid; mp 180-181°C.

Step 4

To a stirred solution of the phosphonium salt from Step 3 (1.91 g, 4.33 mmol) in water (100 ml) was added dropwise 1M NaOH (4.5 ml, 4.5 mmol). After 10 min the product was extracted into $CH_2Cl_2$ (50 ml) which was dried over $MgSO_4$, filtered and the solvent removed in vacuo. The residue was dissolved in hot EtOAc and insoluble material filtered off. The volume of the filtrate was reduced and 40 : 60 petrol added causing the ylid to precipitate out (0.86, 55%); mp 132-143°C.

Step 5

To a stirred solution of the ylid from Step 4 (800 mg, 2.22 mmol) in anhydrous THF (20 ml) at room temperature was added a solution of 2-(t-butyloxycarbonylamino)-3-phenylpropanol (553 mg, 2.22 mmol) in THF (10 ml). After 3 h the solvents were removed in vacuo and the residue purified by chromatography on silica using $CH_2Cl_2$ then 1% MeOH : 99% $CH_2Cl_2$ as eluant. Removal of the solvent in vacuo gave the desired product (271 mg, 37%) as a white crystalline solid; IR (film) 3357, 1713 and 1646cm$^{-1}$; NMR ($CDCl_3$) δ 1.40 (9H, s), 2.78-2.92 (2H, m), 3.73 (3H, s), 4.53-4.81 (2H, m), 5.82 (1H, d, J 15.4Hz), 6.03 (1H, dd, J 5.4, 15.3Hz), 6.20 (1H, dd, J 10.8, 15.3Hz),7.14-7.31 (6H, m).

Step 6

To a stirred solution of the ester from Step 5 (335 mg, 1 mmol) in $CH_2Cl_2$ (5 ml) was added trifluoroacetic acid (5 ml). After 1 h at room temperature the solvents were removed in vacuo to give the desired amine as a residue which was used without further purification in the next step.

Step 7

To a stirred solution of α-methyl-N-[(tricyclo-[3.3.1.1$^{3,7}$]dec-2-yloxy)carbonyl]-R-tryptophan (441 mg, 1.11 mmol) and 1-hydroxybenzotriazole hydrate (213 mg, 1.39 mmol) in EtOAc (20 ml) was added N,N'-dicyclohexylcarbodiimide (252 mg, 1.22 mmol). After 1 h at room temperature the amine salt from Step 6 (349 mg, 1.01 mmol) and triethylamine (0.292 ml, 2.10 mmol) were added dropwise in EtOAC (10 ml) over 5 min. After 24 h the solution was filtered and the filtrate washed with 5% citric acid solution (2 x 25 ml), saturated $NaHCO_3$ solution (2 x 25 ml), 5% citric acid solution (25 ml) and brine (25 ml). The EtOAc extract was then dried ($MgSO_4$), filtered and the solvent removed in vacuo. The residue was purified by chromatography over silica using 1% MeOH : 99% $CH_2Cl_2$ as eluant which gave the amide product (286 mg, 46%) as a white solid; mp 111-125°C; IR (film) 1703 and 1646cm$^{-1}$; NMR ($CDCl_3$) δ 1.43 (3H, s), 1.50-1.98 (14H, m), 2.75-2.80 (2H, m), 3.26 (1H, d, J 14.7Hz), 3.52 (1H, d, J 14.7Hz), 3.73 (3H, s), 4.81-4.85 (2H, m), 5.07 (1H, s), 5.78 (1H, d, J 15.4Hz), 5.94 (1H, dd, J 15.4, 5.4Hz), 6.14 (1H, dd, J 10.6, 15.5Hz), 6.37 (1H, d, J 8.1Hz), 6.91 (1H, d, J 2.2Hz), 7.10-7.27 (8H, m), 7.34 (1H, d, J 8.0Hz), 7.58 (1H, d, J 7.9Hz), 8.15 (1H, s); Anal ($C_{37}H_{43}N_3O_5$), C, H, N.

Step 8

A solution of the unsaturated ester from Step 7 (227 mg, 0.37 mmol)in absolute EtOH (30 ml) was hydrogenated over 10% Pd/C (25 mg) at 30°C under an atmosphere of hydrogen at 50 psi for 6.5 h. The catalyst was filtered off and washed with solvent. the combined filtrates were concentrated in vacuo to give the product as a foam (145 mg, 64%); IR (film) 1718 and 1657cm$^{-1}$; NMR ($CDCl_3$) δ 1.22-1.98 (23H, m), 2.24 (2H, t, J 7.4Hz), 2.63 (1H, dd, J 6.9, 13.7Hz), 2.73 (1H, dd, J 6.1, 13.7Hz), 3.26 (1H, d, J 14.7Hz), 3.51 (1H, d, J 14.7Hz), 3.65 (3H, s), 4.12-4.14 (1H, m), 4.80 (1H, s), 5.14 (1H, s), 6.13 (1H, d, J 8.5Hz), 6.91 (1H, d, J 2.3Hz), 7.08-7.29 (7H, m), 7.34 (1H, d, J 7.9Hz), 7.60 (1H, d, J 7.7Hz), 8.34 (1H, s).

Step 9

To a stirred solution of the methyl ester from Step 8 (145 mg, 0.24 mmol) in THF (15 ml) at 0°C was added dropwise an aqueous solution of LiOH (2.6 ml of 0.1M soln, 0.26 mmol). The solution was stirred and slowly allowed

to warm to room temperature over 24 h. A 0.1M HCl (2.9 ml, 0.29 mmol) solution was then added and the reaction mixture extracted with $Et_2O$ (2 x 25 ml). The $Et_2O$ extracts were dried over $MgSO_4$, filtered and the solvent removed in vacuo. The residue was purified by chromatography over reverse phase silica using 75% MeOH : 25% $H_2O$ as eluant. This gave the desired acid (55 mg, 38%) as a white solid; mp 79-90°C; IR (film) 1709 and 1655cm$^{-1}$; NMR ($CDCl_3$) δ 1.20-1.97 (23H, m), 2.22 (2H, t, J 7.2Hz), 2.60 (1H, dd, J 6.8, 13.6Hz), 2.71 (1H, dd, J 6.0, 13.5Hz), 3.24 (1H, d, J 14.7Hz), 3.47 (1H, d, J 14.7Hz), 4.10 (1H, m), 4.80 (1H, s), 5.34 (1H, s), 6.20 (1H, d, J 8.5Hz), 6.93 (1H, d, J 2.0Hz), 7.05-7.24 (7H, m), 7.33 (1H, d, J 7.9Hz), 7.57 (1H, d, J 7.7Hz), 8.67 (1H, s); Anal ($C_{36}H_{45}N_3O_5$. 0.25 $H_2O$), C, H, N.

Example 36

Methyl-(±)-β-[[(2-phenylethyl)amino]carbonyl]-1β-[[(tricyclo-[3.3.1.1$^{3,7}$]dec-2-yloxy)carbonyl]amino]-1H-indole-3-butanoate.

Step 1

(±)-N-formyltryptophan (10.00 g, 43 mmol) was suspended in $H_2O$ (100 ml). Caesium carbonate (7.70 g, 23.5 mmol) was added portion-wise to the soln. The solution was stirred until all (±)-N-formyltryptophan had dissolved completely. The solvent was then evaporated in vacuo, the residue dissolved in anhydrous DMF (50 ml) and benzylbromide (7.50 g, 44 mmol) was added. The solution was left stirring for 4 h, $Et_2O$ (200 ml) added, and the solution washed with $H_2O$ (100 ml). The etheral layer was dried ($MgSO_4$) and concentrated in vacuo to yield the desired benzyl ester (14.32 g, α100%); mp 85-86°C; IR (film) 3294, 1739, 1673cm$^{-1}$; NMR ($CDCl_3$) δ 3.28 (2H, d, J 7Hz), 5.02 (3H, m), 6.66 (1H, d, J 8Hz), 6.77 (1H, s), 7.03-7.33 (8H, m), 7.50 (1H, d, J 7Hz), 7.98 (1H, s), 8.94 (1H, s); Anal ($C_{19}H_{18}N_2O_3$. 0.1 $H_2O$ ), C, H, N.

Step 2

(±)-Benzyl-N-formyltryptophan ester from Step 1 (8.16 g, 24.8 mmol) was suspended in anhydrous DMF (100 ml) under an atmosphere of nitrogen. 4-Dimethylaminopyrridine (Ca. 0.1 g) dissolved in DMF (5 ml) was injected via a syringe. Di-t-butyldicarbonate (5.43, 24.8 mmol) in DMF (10 ml) was added dropwise. The mixture was left stirring at room temperature for 24 h. The solution was concentrated in vacuo and the residue dissolved in $Et_2O$ (100 ml). The etheral solution was washed with 10% citric acid soln, dried ($MgSO_4$), filtered and concentrated to dryness. The desired indole protected product was isolated by column chromatography (75% EtOAc/n-hexane) to give a yellow oil (3.58 g, 34%); IR (film) 3257, 1734, 1687cm$^{-1}$; NMR ($CDCl_3$) δ 1.64 (9H, s), 3.22 (1H, d), 3.24 (1H, d) 5.04 (3H, m), 6.99 (1H, d, J 8Hz), 7.15-7.32 (7H, m), 7.41 (1H, s), 7.49 (1H, d, J 8Hz), 8.09 (1H, d, J 8Hz), 8.14 (1H, s); Anal ($C_{24}H_{26}N_2O_5$. 0.33 $H_2O$), C, H, N.

Step 3

1-[(1,1-dimethylethoxy)carbonyl]-N-formyl-DL-tryptophan benzyl ester from Step 2 (3.04 g, 7.20 mmol) was dissolved in $CH_2Cl_2$ (10 ml) under an atmosphere of nitrogen. The solution was cooled to 0°C in an ice-salt bath. Triethylamine (2.21 g, 21.6 mmol) was added followed by triphosgene (0.80 g, 2.4 mmol) in $CH_2Cl_2$ (15 ml). The solution was allowed to warm to room temperature and was left to stir for 10 h. The solvent was then concentrated in vacuo, and the residue was taken up in $Et_2O$. Triethylamine hydrochloride was filtered off, the filtrate concentrated to dryness and the product was isolated by flash chromatography (75% EtOAc/n-hexane) to give the desired isonitrile as a yellow oil (2.54 g, 87%); IR (film) 2149, 1735cm$^{-1}$ ; NMR ($CDCl_3$) δ 1.67 (9H, s), 3.29 (1H, dd, J 7, 15Hz), 3.41 (1H, dd, J 7, 15Hz), 4.60 (1H, dd, J 7, 7Hz), 5.18 (2H, s), 7.23-7.36 (7H, m), 7.49 (1H, d, J 8Hz), 7.57 (1H, s), 8.15 (1H, d, J 8Hz); Anal ($C_{24}H_{24}N_2O_4$· 0.5 $H_2O$), C, H, N.

Step 4

The isonitrile from Step 3 (2.05 g, 5.1 mmol) was dissolved in anhydrous THF (15 ml) and the solution cooled to - 78°C under an atmosphere of argon. HMPA (0.88 ml, 5.1 mmol) was added followed by a solution of lithium bis (trimethylsilyl) amide (6.0 ml of 1.0M soln). After stirring for 30 min at -78°C methyl iodide (0.31 ml, 5.2 mmol) was added slowly. After a further 3 h the mixture was allowed to warm to room temperature and was stirred for a further 1 h. The solvent was then concentrated in vacuo, the residue dissolved in water and extracted with $Et_2O$ (2 x 25 ml). The combined organic extracts were dried ($MgSO_4$), filtered and concentrated in vacuo. The crude product was purified by flash chromatography (50% $Et_2O$/n-hexane) to yield the desired alkylated product as a white solid (1.94 g, 79%);

mp 29-30°C; IR (film) 2138, 1741cm$^{-1}$; NMR ($CDCl_3$) δ 1.58 (9H, s), 2.72 (1H, d, J 17Hz), 3.13 (1H, d, J 17Hz), 3.20 (1H, d, J 15Hz), 3.29 (1H, d, J 15Hz), 3.54 (3H, s), 4.99 (1H, d, J 12Hz), 5.03 (1H, d, J 12Hz), 7.07-7.28 (7H, m), 7.42 (1H, d, J 8Hz), 7.54 (1H, s), 8.05 (1H, d, J 8Hz); Anal ($C_{27}H_{28}H_2O_6$), C, H, N.

Step 5

1-Methyl-(±)-β-cyano-1-[(1,1-dimethylethoxy)carbonyl]-β-[(phenylmethoxy)carbonyl]-1H-indole-3-butanoate (0.241 g, 0.50 mmol) was dissolved in EtOH (5 ml). The solution cooled to - 5°C in an acetone-ice bath and ethanolic HCL was added dropwise. Water (0.1 ml) was added and the reaction was warmed to room temp. The solution was left to stir for 24 h and the solvent concentrated in vacuo. The oil was dissolved in EtOAc (50 ml) and washed with a 10% $Na_2CO_3$ solution (50 ml). The organic layer was dried ($MgSO_4$), filtered and concentrated in vacuo. The product was isolated by flash chromatography (50% EtOAc/n-hexane) to yield the desired amine (0.120 g, 67%) as a yellow oil; IR (film) 3350, 3245, 1741cm$^{-1}$ ; NMR ($CDCl_3$) δ 2.12 (2H, br s), 3.17 (1H, d, J 18Hz), 3.28 (1H, d, J 18Hz), 3.37 (1H, d, J 15Hz), 3.43 (3H, s), 3.53 (1H, d, J 15Hz), 4.82 (1H, d, J 12Hz), 4.92 (1H, d, J 12Hz), 6.73 (1H, d, J 2Hz), 6.95-7.21 (8H, m), 7.47 (1H, s), 8.42 (1H, s).

Step 6

Methyl-(±)-β-amino-β-[(phenylmethoxy)carbonyl]-1H-indole-3-butanoate (120 mg, 0.33 mmol) from Step 5 was dissolved in anhydrous THF (10 ml) under argon. Triethylamine (55 µl, 0.40 mmol) was injected. The solution was cooled to 0°C in an ice-salt bath and 2-adamantyl chloroformate (77 mg, 0.36 mmol) dissolved in THF (5 ml) was injected. The solution was stirred for 12 h at room temperature before triethylamine hydrochloride was filtered off. Dichloromethane (50 ml) was added and the solution was washed with water (2 x 25 ml). The organic layer was dried ($MgSO_4$), filtered and concentrated in vacuo. The product was isolated by flash chromatography (50% $Et_2O$/n-hexane) to furnish the desired urethane (105 mg, 58 %); mp 61-62°C; IR (film) 3412, 1738cm$^{-1}$ ; NMR ($CDCl_3$) δ 1.49-2.09 (14H, m), 3.12 (1H, d, J 15Hz), 3.30 (1H, d, J 15Hz), 3.38 (3H, s), 3.72 (1H, d, J 15Hz), 3.80 (1H, d, J 15Hz), 4.83 (1H, br s), 4.98 (1H, d, J 12Hz), 5.11 (1H, d, J 12Hz), 6.88 (1H, s), 6.79 (1H, s), 7.03 (1H, t, J 7Hz), 7.14 (1H, t, J 7Hz), 7.17-7.34 (6H, m), 7.48 (1H, d, J 8Hz), 8.30 (1H, s); Anal ($C_{32}H_{36}N_2O_6$), C, H, N.

Step 7

To methyl-(±)-β-[(phenylmethoxy)carbonyl]-β-[[(tricyclo[3.3.1.1$^{3,7}$]dec-2-yloxy)carbonyl]amino]-1H-indole-3-butanoate (105 mg, 0.19 mmol) from Step 6 in a 250 ml vessel was added palladium on charcoal (10%, Ca 20 mg) and EtOH (75 ml). The vessel was sealed in a Parr Hydrogenation Apparatus and charged with $H_2$ gas (45 psi). Shaking was initiated after pressurization and continued for 12 h. Upon completion the palladium on charcoal was filtered off and the filtrate concentrated in vacuo. The product was purified by flash chromatography 2 : 1 MeOH/$H_2O$ to yield the desired acid as a white powder (77 mg, 88%); mp 108-109°C; IR (film) 3413, 1733cm$^{-1}$; NMR ($CDCl_3$) δ 1.47-2.07 (14H, m), 3.14 (1H, d, J 16Hz), 3.26 (1H, d, J 16Hz), 3.64 (3H, s), 3.76 (1H, d, J 15Hz), 3.84 (1H, d, J 15Hz), 4.83 (1H, br s), 5.75 (1H, br s), 5.96 (1H, s), 6.98-7.04 (2H, m), 7.10 (1H, t, J 7Hz), 7.28 (1H, d, J 8Hz), 7.61 (1H, d, J 8Hz), 8.34 (1H, s); Anal ($C_{25}H_{30}N_2O_6$), C, H, N.

Step 8

Methyl-(±)-β-[[(tricyclo-[3.3.1.1$^{3,7}$]dec-2-yloxy)carbonyl]amino]-1H-indole-3-butanoate (200 mg, 0.44 mmol) from Step 7 was dissolved in anhydrous THF (10 ml). Pentafluorophenol (88 mg, 0.48 mmol) was added followed by N,N'-dicyclohexylcarbodiimide (100 mg, 0.48 mmol). The solution was left stirring for 2 h before phenylethylamine (60 mg, 0.50 mmol) was injected into the soln. The mixture was left stirring for 16 h. The solution was concentrated in vacuo, EtOAc added and dicyclohexylurea filtered off. The filtrate was concentrated in vacuo and the product was isolated by flash chromatography (25% EtOAc/n-hexane) to give a white solid (180 mg, 73%; mp 78-79°C; IR (film) 3333, 1730, 1659cm$^{-1}$; NMR ($CDCl_3$) δ 1.51-2.04 (14H, m), 2.61 (2H,m), 2.94 (1H, d, J 16Hz), 3.21 (1H, d, J 16Hz), 3.37 (1H, d, J 7Hz), 3.41 (1H, d, J 7Hz), 3.46 (1H, d, J 15Hz), 3.57 (1H, d, J 15Hz), 3.62 (3H, s), 4.78 (1H, br s), 5.88 (1H, br s), 6.58 (1H, br s), 6.92 (1H, d, J 2Hz), 7.03-7.26 (7H, m), 7.33 (1H, d, J 8Hz), 7.56 (1H, d, J 8Hz); Anal ($C_{33}H_{39}N_3O_5$. 0.75 $H_2O$), C, H, N.

Example 37

Carbamic acid, [1-(1H-indol-3-ylmethyl)-1-[[(2-phenylethyl)-amino]carbonyl]-3-butynyl]-,(tricyclo-[3.3.1.1$^{3,7}$]dec-2-yl ester, (±)

Example 37 is prepared by using propargyl bromide in step 4 of Example 36.

Example 38

Bicyclo[2.2.1]heptane-2-acetic acid, 3-[[[[2-[[1-(hydroxymethyl)-2-phenylethyl]amino]-1-(1H-indol-3-ylmethyl)-1-methyl-2-oxoethyl]amino]carbonyl]oxy]-4,7,7-trimethyl-,[1R-[1α,2β,3α(R*(S*)],4α]]

Step 1

Method exactly as for Example 5 except using (4-nitrophenyl) methyl[1R-(1α,2α,3β)]-2-[(chlorocarbonyl)oxy]-1,7,7-trimethyl-bicyclo[2.2.1]heptane-3-acetate; mp 78-81°C ; $[\alpha]^{20}_D$ + 6.2° (c = 0.62 ; MeOH.) ; IR (film) 1729, 1696 and 1660cm$^{-1}$ ; NMR ($CDCl_3$) δ 0.79 (3H, s), 0.85 (3H, s), 0.96 (3H, s), 1.05-1.20 (1H, m), 1.20-2.00 (7H, m), 2.43 (1H, dd, J 8 and 15Hz), 2.60-2.70 (1H, m), 2.75 - 2.90 (3H, m), 3.00-3.10 (1H, m), 3.29 (1H, d, J 15Hz), 3.35-3.50 (2H, m), 3.40 (1H, d, J, 14Hz), 4.10-4.30 (2H, m), 5.07 (1H, br s), 5.13 (2H, s), 6.23 (1H, br d, J 7Hz), 6.98 (1H, d, J 2Hz), 7.00-7.25 (7H, m), 7.32 (1H, d, J 8Hz), 7.43 (2H, d, J, 8Hz), 8.15 (2H, d, J 8Hz), 8.39 (1H, s) ; Anal. $C_{41}$,$H_{48}O_8N_4$; C, H, N.

Step 2

The ester from Step 1 (430 mg, 0.59 mmol) as a solution in absolute EtOH (100 ml) was treated with 10% Pd/C (43 mg, 10% w/w), and the resulting mixture put under an atmosphere of hydrogen at a pressure of 50psi with agitation for 1 h. After this time the mixture was filtered over filter aid and the solvent removed in vacuo and the residue chromatographed over reverse phase silica gel using 50% MeOH in $H_2O$ as eluant to give the acid as a white solid (130 mg, 37%) ; mp 93.7-97.5°C (MeOH/$H_2O$) ; $[\alpha]^{20}_D$ + 7.7° (c = 0.96, MeOH) ; IR (film) 1708 and 1660 cm$^{-1}$; NMR ($CDCl_3$) δ 0.75 (3H, s), 0.82 (3H, s), 0.93 (3H, s), 1.05-1.40 (2H, m), 1.46 (3H, s), 1.50-1.65 (3H, m), 2.27 (1H, dd, J 8 and 13Hz), 2.35-2.49 (1H, m), 2.50-2.60 (1H, m), 2.67 (1H, dd, J 7 and 14Hz), 2.90 (1H, dd, J 7 and 14Hz), 3.12 (1H, d, J 15Hz), 3.28 (1H, d, J 15Hz), 4.05-4.20 (1H, m), 4.31 (1H, d, J 4Hz), 4.40-4.70 (1H, br), 5.21 (1H, br s), 6.57 (1H, d, J 9Hz), 6.94 (1H, br s), 7.05-7.30 (7H, m), 7.33 (1H, d, J 8Hz), 7.55 (1H, d, J 8Hz), 8.54 (1H, s); Anal. $C_{34}H_{43}N_3O_6 0.5H_2O$ ; C, H, N.

Example 39

[1R-[1α, 2α[R*(S*)]]] and [1S-[1α, 2α(S*(R*)]]][[2-[[[[2-[[1-(hydroxvmethyl)-2-phenylethyl]amino]-1-(1H-indol-3-ylmethyl)-1-methyl-2-oxoethyl]amino]carbonyl]oxy]-1-methylcyclohexyl]-carbonyl]glycine.

Step 1

Method as for Example 5 except using phenylmethyl cis-(±)-[[[2-[(chlorocarbonyl)oxy]1-methyl-1-cyclohexyl] carbonyl]-amino]acetate. mp 78-81°C ; IR (film) 3600-3200, 3000-2800, 1760, 1705 and 1651cm$^{-1}$; NMR ($CDCl_3$) δ 1.16 (1.5H, s), 1.19 (1.5H, s), 1.20-2.20 (11H, m), 2.78 (2H, d, J 8Hz), 3.20-3.75 (4H, m), 3.80-4.00 (1H, m), 4.10-4.30 (2H, m), 4.78 (0.5H, t J 6Hz), 4.90-5.10 (2.5H, m), 5.26 (0.5H, br s), 5.52 (0.5H, br s), 6.38 (0.5H, d, J 8Hz), 6.48 (0.5H, d, J 8Hz), 6.52-6.65 (1H, m), 6.90-7.00 (1H, m), 7.00-7.50 (13H, m), 7.57 (1H, d, J 8Hz), 8.05 (1H. br) ; Anal. $C_{39}H_{46}N_4O_7.0.5H_2O$ ; C, H, N.

Step 2

The ester from step 1 (60 mg, 0.09 mmol) and 10% Pd/C (50 mg), in absolute EtOH (50 ml) was put under an atmosphere of hydrogen at 50 psi and 25°C with agitation for 4 h. After this time the mixture was filtered over filter aid and concentrated in vacuo and the residue chromatographed over reverse phase silica gel using 60% MeOH in $H_2O$ as eluant to give the product as a non-crystalline solid (40 mg, 80%); mp 94-99°C ; IR (film) 1709 and 1694 cm$^{-1}$ ; NMR ($CDCl_3$) δ 1.10-2.00 (13H, m), 2.10-2.30 (1H, m), 2.72 (1H, dd, J 6 and 14Hz), 2.84 (1H, dd, J 7 and 14Hz), 3.15-3.60 (4H, m), 3.75-4.05, (2H, m), 4.15-4.30 (1H, br s), 4.55-4.75 (0.5H, m), 4.80-5.00 (0.5H, m), 6.90-7.10 (3H, m).

Example 40

Butanoic acid, 4-[[2-[[3-(1H-indol-3-yl) -2-methyl-2-[[[(2-methyl-1-cyclohexyl) oxy]carbonyl]amino]-1-oxopropyl] amino]-1-Phenylethyl]amino]-4-oxo-[1R-[1α[R*(R*) ]2β]]-((-) -isomer).

The amine 60K in Scheme IX (100 mg, 0.21 mmol) as a solution in EtOAc (30 ml) was treated with succinic anhydride

[1R-[1α, 2α[R*(S*)]]] and [1S-[1α, 2α[S*(R*)]]] [[2-[[[[2-[[1-(hydroxymethyl)-2-phenylethyl]aminol-1-(1H-indol-3-ylmethyl)-1-methyl-2-oxoethyl]amino]carbonyl]oxy]-1-methylcyclohexyl]-carbonyl]glycine.

Step 1

Method as for Example 5 except using phenylmethyl cis-(±)-[[[2-[(chlorocarbonyl)oxy]1-methyl-1-cyclohexyl] carbonyl] amino]acetate. mp 78-81°C ; IR (film) 3600-3200, 3000-2800, 1760, 1705 and 1651cm$^{-1}$ ; NMR ($CDCl_3$) δ 1.16 (1.5H, s), 1.19 (1.5H, s), 1.20-2.20 (11H, m), 2.78 (2H, d, J 8Hz), 3.20-3.75 (4H, m), 3.80-4.00 (1H, m), 4.10-4.30 (2H, m), 4.78 (0.5H, t J 6Hz), 4.90-5.10 (2.5H, m), 5.26 (0.5H, br s), 5.52 (0.5H, br s), 6.38 (0.5H, d, J 8Hz), 6.48 (0.5H, d, J 8Hz), 6.52-6.65 (1H, m), 6.90-7.00 (1H, m), 7.00-7.50 (13H, m), 7.57 (1H, d, J 8Hz), 8.05 (1H. br) ; Anal. $C_{39}H_{46}N_4O_7.0.5H_2O$ ; C, H, N.

Step 2

The ester from step 1 (60 mg, 0.09 mmol) and 10% Pd/C (50 mg), in absolute EtOH (50 ml) was put under an atmosphere of hydrogen at 50 psi and 25°C with agitation for 4 h. After this time the mixture was filtered over filter aid and concentrated in vacuo and the residue chromatographed over reverse phase silica gel using 60% MeOH in $H_2O$ as eluant to give the product as a non-crystalline solid (40 mg, 80%); mp 94-99°C ; IR (film) 1709 and 1694 cm$^{-1}$; NMR ($CDCl_3$) δ 1.10-2.00 (13H, m), 2.10-2.30 (1H, m), 2.72 (1H, dd, J 6 and 14Hz), 2.84 (1H, dd, J 7 and 14Hz), 3.15-3.60 (4H, m), 3.75-4.05, (2H, m), 4.15-4.30 (1H, br s), 4.55-4.75 (0.5H, m), 4.80-5.00 (0.5H, m), 6.90-7.10 (3H, m).

Example 40

Butanoic acid, 4-[[2-([3-(1H-indol-3-yl) -2-methyl-2-[[[(2-methyl-1-cyclohexyl) oxy]carbonyl]amino]-1-oxopropyl] amino]-1-phenylethyl]amino]-4-oxo-[1R-[1α[R*(R*)]2β]]-((-) -isomer) .

The amine 60K in Scheme IX (100 mg, 0.21 mmol) as a solution in EtOAc (30 ml) was treated with succinic anhydride (30 mg, 0.3 mmol) and left stirring at room temperature for 18 h before the solvent was removed in vacuo and the residue chromatographed over reverse phase silica gel using 60% MeOH in $H_2O$ as eluant to give the product (93 mg, 77%); mp 106-111°C (MeOH/$H_2O$) ; $[\alpha]^{20}_D$ -33.5° (c=0.81, MeOH), IR (film) 3320, 2933, 2860, 1714 and 1661 cm$^{-1}$ ; NMR ($CDCl_3$) δ 0.88 (3H, d, J 6.5Hz), 1.0-1.35 (4H, m), 1.47 (3H, s), 1.40-1.80 (4H, m), 1.95-2.05 (1H, br m), 2.40-2.65 (4H, m), 3.20-3.35 (3H, m), 3.75-3.85 (1H, m), 4.20-4.30 (1H, m), 4.90-5.00 (1H, br s), 5.30-5.40 (1H, br s), 6.40-6.50 (1H, br s), 6.97 (1H, s), 7.05-7.30 (8H, m), 7.33 (1H, d, J 8Hz), 7.54 (1H, d, J 8Hz), 8.60 (1H, s) ; MS(FAB) m/e 577.2 (M+1) and 217.0 (100) ; Anal. $C_{32}H_{40}N_4O_6\cdot 0.5H_2O$; C, H, N.

Example 41

2-Butenoic acid, 4-[[2-[[3-(1H-indol-3-yl)-2-methyl-2-[[[(2-methyl-1-cyclohexyl)oxylcarbonyl)amino]-1-oxopropyl] amino]-1-phenylethyl]amino]-4-oxo-,[1R-[1α [R* (R*) ],2β]]-((-)-isomer).

A stirred solution of mono (2-trimethyl silyl) ethyl fumarate (350 mg, 0.7 mmol) in EtOAc (20 ml) and pentafluorophenol (184 mg, 1.00 mmol) was treated with dicyclohexylcarbodiimide (218 mg, 1.05 mmol) and the amine 6K (Scheme IX) (1 mmol) and left for 18 h at room temp. The reaction mixture was then filtered and the filtrate washed with $H_2O$ (2 x 20 ml) and dried over $MgSO_4$. The solvent was then removed in vacuo and the residue chromatographed over reverse phase silica gel using 75% MeOH in $H_2O$ as eluant to give the slightly impure ester (400 mg) which was dissolved in THF (20 ml) and treated with tetrabutyl ammonium fluoride in THF (3 ml of a 1M soln, 3 mmol) and left stirring at room temperature for 1.5 h. After this time the reaction mixture was concentrated in vacuo and the residue taken up in EtOAc (30 ml) and washed with 1M citric acid solution (30 ml) then $H_2O$ (30 ml). The organic phase was dried over $MgSO_4$ and concentrated in vacuo and the residue chromatographed over reverse phase silica gel using 75% MeOH in $H_2O$ as eluant to give the product as a white solid, (200 mg, 47%); mp 131-135°C (MeOH/$H_2O$); $[\alpha]^{20}_D$

- 36.1° (c = 1, MeOH) ;IR (film) 3307, 2933, 2858, 1707 and 1666cm$^{-1}$; NMR ($CDCl_3$) δ 0.85 (3H, d, J 6.5Hz), 1.00-1.75 (11H, m), 1.95-2.05 (1H, br m), 3.22 (1H, d, J 14.5Hz), 3.33 (1H, d, J 14.5Hz), 3.50-3.80 (2H, m), 3.50-4.20 (1Hz br), 4.20-4.30 (1H, m), 5.10-5.20 (1H, br s), 5.30 (1H, br s), 6.64 (1H, br s), 6.79 (1H, d, J 15Hz), 6.90-7.35 (10H, m), 7.50 (1H, d, J 8Hz), 7.79 (1H, br s), 8.59 (1H, s) ; MS (FAB) m/e 575.1 (M+1) and 288.9 (100) ; Anal. $C_{33}$ $H_{38}$ $N_4$ $O_6$. $0.25H_2O$ ; C, H, N.

Example 42

Butanoic acid, 4-[[2-[[3-(1H-indol-3-yl)-2-methyl-2-[[[(2-methyl-1-cyclohexyl)oxy]carbonyl]amino]-1-oxopropyl]amino]-3-phenylpropyl ]amino]-4-oxo-[1R-[1α [R* (S* ) ], 2β]] -((-) -isomer) .

Methods were employed exactly as for Example 19 except using trans(-)-2-methylcyclohexyloxycarbonyl-α-methyl-R-tryptophan (2K in Scheme I) (216 mg, 61%); mp 97-102°C (MeOH/$H_2O$) ; $[\alpha]^{20}_D$ + 37° (c = 0.22, MeOH) ; IR (film) 3315, 2930, 2859, 1700 and 1660 cm$^{-1}$ ; NMR ($CDCl_3$) δ 0.82 (3H, d, J 6.5Hz), 1.00-1.75 (11H, m), 1.90-2.00 (1H, br s), 2.40-2.70 (6H, m), 2.85-3.00 (1H, br m), 3.23 (1H, d, J 14.5Hz), 3.30 (1H, d, J 14.5Hz), 3.45-3.65 (1H, br s), 4.20-4.30 (2H, br m), 5.26 (1H, s), 5.10-5.80 (1H, br), 6.15-6.25 (1H, br s), 6.90-7.20 (9H, m), 7.33 (1H, d, J 8Hz), 7.53 (1H, d, J 8Hz), 8.72 (1H, s) ; MS (FAB) m/e 591.2(M+1, 100) ; Anal. $C_{33}$ $H_{42}N_4$ $O_6$; C, H, N.

Example 43

2-Butenoic acid, 4-[[2-[(3-)1H-indol-3-yl)-2-methyl-2-[[[(2-methyl-1-cyclohexyl) oxylcarbonyl]amino]-l-oxoprop$^{y}$l] amino]-3-phenylpropyl]amino]-4-oxo-[IR[1α[R*(S*)],2β]]-((-)-isomer).

Methods were employed exactly as for Example 19A except using trans (-)-2-methylcyclohexyloxycarbonyl-α-methyl-R-tryptophan. (170 mg, 7.3%); mp 118-128°C(MeOH/$H_2O$) ; $[\alpha]^{20}_D$ + 74° (c = 0.42, MeOH) ; IR (film) 3500-3200, 2933, 2858, 1695 and 1662 cm$^{-1}$ ; NMR ($CD_3OD$) δ 0.89 (3H, d, J 6.5Hz), 1.00-1.80 (11H, m), 2.00-2.10 (1H, br m), 2.65-2.75 (2H, m), 2.95-3.05 (1H, m), 3.16 (1H, d, J 14.5Hz), 3.36 (1H, d, J 14.5Hz), 3.60-3.70 (1H, m), 4.30-4.40 (2H, m), 6.72 (1H, d, J 15Hz), 6.90-7.30 (9H, m), 7.30 (1H, d, J 8Hz), 7.50 (1H, d, J 8Hz) ; MS (FAB) m/e 589.2 (M+1) 220.2 (100); Anal. $C_{33}$ $H_{40}$ $N_4$ $O_6$. $H_2O$ ; C, H, N.

Example 44

Carbamic acid,(2-[[1-(hydroxymethyl) -2-hydroxy-2-phenylethyl] amino]-1-(1H-indol-3-ylmethyl)-1-methylethyl]-, tricyclo [3.3.1.1$^{3,7}$]dec-2-yl ester.

Method were employed exactly as for Example 19, step 4 except the amine used was L(+)-threo-2-amino-1-phenyl-1,3-propanediol. Yield 2g, 73%; mp 69-73°C ; $[\alpha]^{20}_D$ + 47.3° (c = 0.97, MeOH) ; IR (film) 3396, 1695 and 1663 cm$^{-1}$; NMR ($CDCl_3$) δ 1.48 (3H, s), 1.52-1.97 (14H, m), 3.10 (1H, br s), 3.17 (1H, d, J 15Hz), 3.27 (1H, d, J 15Hz), 3.72-4.10 (4H, m), 4.77 (1H, br s), 5.01 (1H, d, J 3.5Hz), 5.26 (1H, s), 6.69 (1H, d, J 7.5Hz), 6.81 (1H, d, J 2Hz), 7.09-7.40 (8H, m), 7.55 (1H, d, J 8Hz), 8.13 (1H, s) ; Anal. $C_{32}$ $H_{39}$ $N_3$ $0_5$. $0.25H_2$, C, H, N.

Example 45

Carbamic acid,[1-(1H-indol-2-ylmethyl)-1-methyl-2-oxo-2-[(2-phenylethyl)amino]ethyl]-, tricyclo[3.3.1.1$^{3,7}$]dec-2-yl ester, (±)-

Step 1

1-(4-Methylphenyl)sulfonyl-1H-indole-2-carboxylic acid ethyl ester

To a stirred suspension of sodium hydride (3.7g, 120 mmol, 80 % in paraffin oil) in dry THF (75 ml), a solution of indol-2-carboxylic acid ethyl ester (18.9 g, 100 mmol) in dry THF (75 ml) was added in one hour with stirring while the inner temperature was maintained under 30°C. The reaction mixture was stirred for 30 min. and then a solution of p-toluenesulphonyl chloride (22.9 g, 120 mmol) in dry THF (75 ml) was added dropwise to the stirring reactant. After two hours stirring at room temperature and one hour at 45°C the solvent was evaporated in vacuo and the residue partitioned between water and ethyl ether. The organic phase was dried over $MgSO_4$ and the solvent evaporated to leave a solid which was recrystallized from diisopropyl ether (26.8g, 78 %), m.p. 92-95°C.

Step 2

2-Hydroxymethyl-1-(4-methylphenyl)sulfonyl-1H-indole

To stirred solution of Red-Al (sodium dihydro-bis(2-methoxyethoxy)aluminate ≈70% in toluene) (30 ml) in dry THF (100 ml) cooled at 5°C and under nitrogen was added dropwise and at this temperature a solution of compound of step 1 (26,8 g, 78 mmol) in dry THF (75 ml). After stirring one hour at 5°C and then one hour at room temperature the mixture was cooled at 10°C and treated dropwise with 2N NaOH, to effect hydrolysis of the intermediate complex. The organic phase was separated and the solvent in vacuo evaporated. The residue was solved in ethyl ether, the solution washed with water, dried over MgSO4 and evaporated to give the required alcohol (23.3 g, 98 %) as a yellow oil; IR (film) 3500, 1597 $cm^{-1}$.

Step 3

2-Bromomethyl-1-l4-methylphenyl)sulfonyl-1H-indole

To a solution of triphenylphosphine (20.2 g, 77 mmol) in dry $CH_2Cl_2$ (80 ml) was added dropwise a solution of bromine (11.9 g, 77 mmol) in dry $CH_2Cl_2$ (40 ml). The stirring was continued for one hour and then a solution of compound of step 2 (23.2 g, 77 mmol) in dry $CH_2Cl_2$ (40 ml) was added dropwise. The resulting mixture left stirring for 12 hours. After removing the solvent the residue was taken up in ethyl acetate and washed with water. The organic extract was dried over $MgSO_4$ and the solvent evaporated in vacuo. The residue was chromatographed over silica gel using toluene as eluant to give a yellow oil (21.0 g, 75 %); IR (film) 1600 $cm^{-1}$, MS ((70eV): m/z 363 (M+,12.6), 129 (100).

Step 4

Racemic 2-Methyl-3-[[1-(4-methylphenyl)sulfonyl]-1H-indol-2-yl]-N-(phenylmethylene) alanine methyl ester

To a stirred solution of KOt.Bu (5.1 g, 45 mmol) in dry THF (25 ml) cooled at -40°C was added dropwise at this temperature a solution of N-(phenylmethylene)-DL-alanine methyl ester (8.7 g, 45 mmol) in dry THF (40 ml) under nitrogen. The mixture was stirred one hour at -40°C and then was added dropwise maintaining the temperature a solution of compound of step 3 (16.5g, 45 mmol) in dry THF (50 ml). After the addition was completed the mixture was stirred two hours at -20°C, then allowed to warm to room temperature and left overnight. The solvent was evaporated in vacuo given a resin, which on trituration with ethyl ether and water gave the required compound (16.5 g, 75 %) as a white solid, m.p. 151-154°C.

Step 5

Racemic 2-Methyl-3-[[1-(4-methylphenyl)sulfonyl]-1H-indol-2-yl]alanine methyl ester

A suspension of compound of step 4 (16.1 g, 34 mmol) in ethanol (100 ml) and 2N hydrochloric acid (20 ml) was stirred overnight. After removing the solvent in vacuo the residue was suspended in water (400 ml), made basic with $Na_2CO_3$, extracted with ethyl ether and dried over $MgSO_4$. The solvent was evaporated providing an oil. This was subjected to silica gel chromatography using ethyl acetate/toluene 8:92 (v/v) then methanol/toluene 1:99 (v/v) as eluants to give the required compound (9.9 g, 75 %) as an oil; IR (film) 1735 $cm^{-1}$.

Step 6

Racemic N-[(2-Adamantyloxy)carbonyl]-2-methyl-3-[[1-(4-methylphenyl)sulfonyl]-1H-indo1-2-vllalanaine methyl ester

To a stirred solution of compound of step 5 (9.9 g, 25 mmol) in dry THF (100 ml) was added a solution of 2-adamantylchloroformate (6.4 g, 30 mmol) in dry THF (15 ml) dropwise. After one hour stirring, the reaction mixture was filtered and the solvent removed in vacuo. The residue was stirred with a mixture of light petroleum (100 ml) and ethyl ether (20 ml) to give the required compound as a colourless solid, which was removed by filtration (13.9 g, 96 %), m.p. 119-122°C

Step 7

Racemic N-(2-Adamantyloxy)carbonyl]-2-methyl-3-[[1-(4-methylphenyl)sulfonyl]-1H-indol-2-yl]alanine

To a stirred solution of compound of step 6 (0.54 g, 0.95 mmol) in a mixture of 1,4-dioxan (10 ml) and water (2 ml) was added LiOH (11.5 mg, 4.8 mmol) and stirred 5 days. After removing the solvent in vacuo the residue was suspended in water, acidified with 1M citric acid solution to pH 4.5 and extracted with ethyl acetate. The organic phase was dried over $MgSO_4$ and evaporated in vacuo to yield the acid (0.5 g, 96 %) as nearly colourless foam, m.p. (non crystalline) 106°C (sintering).

Step 8

Racemic N-[(2-Adamantyloxy)carbonyl]-2-methyl-3-(1H-indol-2-yl]alanine

A mixture of compound of step 7 (6.8 g, 12 mmol) and KOH (2.7 g, 48 mmol) in ethanol (100 ml) was stirred for 60 hours at 70°C. After removing the solvent in vacuo the residue was partitioned between water (150 ml) and ethyl ether. The clear water phase was separated, acidified to pH 4.5 when an oil precipitated out which slowly solidfied. The solid was collected by filtration, washed successively with water and dried to give the desired carboxylic acid (3.9 g, 81 %) as a white solid, m.p. 210-216°C.

Step 9

A mixture of compound of step 8 (0.53 g, 1.3 mmol) and 1,1'-carbonyldiimidazole (0.22 g, 1.3 mmol) in dry THF (8 ml) was stirred for one hour. To this mixture was then added dropwise a solution of 2-phenethylamine (0.17 g, 1.4 mmol) in dry THF (4 ml). After stirring ovrnight the solvent was evaporated in vacuo. The residue was solved in ethyl ether, washed with water, dried over $MgSO_4$ and the solvent evaporated to leave a colourless foam which was crystallized from diisopropylether to yield the title compound (0.42 g, 64 %), m.p. 168-169°C.

Example 46A + B

Carbamic acid, [2-[1-(hydroxymethyl)-2-phenylethyl]amino-1-(1H-indol-2-ylmethyl)-1-methyl-2-oxo]ethyl-tricyclo [3.3.1.1$^{3,7}$]dec-2-yl ester

Method was as described for example 45 above but instead using (S)-(-)-2-amino-3-phenyl-1-propanol in step 9. The crude residue was chromatographed over silica gel using 1% MeOH/99% $CH_2Cl_2$ as eluant.

Diastereomer 1

Diastereomer 1 (0.26 g, 24 %) was obtained as a foam softens at 87°C, Rf 0.70 ((MeOH/$CH_2Cl_2$ 1:99).

Diastereomer 2

Diastereomer 2 (0.20 g, 18 %) was obtained as a foam softens at 90°C, Rf 0.65 (MeOH/$CH_2Cl_2$ 1:99).

Example 47A + B

4-[[2-[[3-(1H-indol-2-yl)-2-methyl-1-oxo-2-[[(tricyclo-[3.3.1.13,7]dec-2-yloxy)carbonyl]amino]propyl]amino]-1-phenylethyl]amino]-4-oxobutanoic acid benzyl ester

Method was as described for example 45 above but instead using the amine of Step 5 of Example 20. The crude residue was chromatographed over silica gel using 1% MeOH/99% $CH_2Cl_2$ as eluant.

Diastereomer 1

Diastereomer 1 (0.17 g, 13 %) was obtained as amorphous pale beige solid, mp 86-90°C; Rf 0.40 ((MeOH/$CH_2Cl_2$ 1:99).

Diastereomer 2

Diastereomer 2 (0.21 g, 17 %) was obtained as amorphous pale beige solid, mp 88-92°C; Rf 0.35 (MeOH/ $CH_2Cl_2$ 1:99).

Example 48

4-[[2-[[3-(1H-indol-2-yl) -2-methyl-1-oxo-2-[[(tricyclo-[3.3.1.13,7]dec-2-yloxy)carbonyl]amino]propyl]amino]-1-phenylethyl]amino]-4-oxobutanoic acid (Diastereomer 1)

Method was as described for Step 7 of Example 20 above but instead using the compound of Example 47A.

Example 49

4-[[2-[[3-(1H-indol-2-yl)-2-methyl-1-oxo-2-[[(tricyclo-[3.3.1.13,7]dec-2 yloxy) carbonyl]amino]propyl]amino]-1-phenylethyl]amino]-4-oxobutanoic acid (Diastereomer 2)

Method was as described for Step 7 of Example 20 above but instead using the compound of Example 47B.

TABLE I

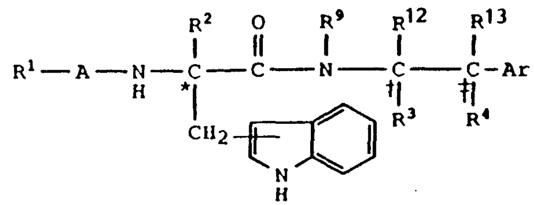 I

| C-Compound Number | $R^1$ | A | $R^2$ | $R^9$ | $R^3$ | $R^{12}$ | $R^4$ | $R^{13}$ | Ar |
|---|---|---|---|---|---|---|---|---|---|
| C 1 | 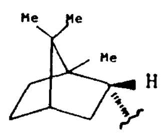 | -O-CO- | Me | H | H | H | $-NHCOCH=CHCOOH$ | H | Ph |
| C 2 | 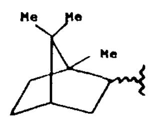 | -O-CO- | Me | Me | H | H | $-NH-CO-(CH_2)_2COOH$ | H | Ph |
| C 3 | Do | -NHCO- | Me | H | H | H | $-NHCO(CH_2)_2COOH$ | H | Ph |
| C 4 | 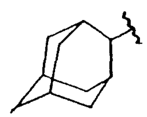 | $-SO_2-$ | Me | H | H | H | $-NHCO(CH_2)_2COOH$ | H | Ph |
| C 5 | Do | $-SO_2-$ | Me | H | $-CH_2NHCO(CH_2)_2COOH$ | H | H | H | Ph |
| | 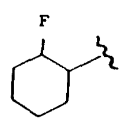 | -O-CO- | Me | H | $-CH_2NHCO(CH_2)_2COOH$ | H | H | H | Ph |

TABLE I CONTINUED

| No. | $R^1$ | A | $R^2$ | $R^9$ | $R^3$ | $R^{12}$ | $R^4$ | $R^{13}$ | Ar |
|---|---|---|---|---|---|---|---|---|---|
| C 7 | Do | -O-CO- | Me | Me | $-CH_2NHCO(CH_2)_2COOH$ | H | H | H | Ph |
| C 8 | 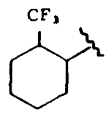 | -O-CO- | Me | H | $-CH_2NHCO(CH_2)_2COOH$ | H | H | H | Ph |
| C 9 | Do | -O-CO- | Me | Me | $-CH_2NHCO(CH_2)_2COOH$ | H | H | H | Ph |
| C 10 | 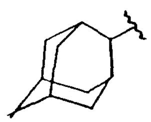 | -O-CO- | Me | Me | $-CH_2NHCO(CH_2)_2COOH$ | H | H | H | Ph |
| C 11 | 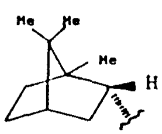 | -O-CO- | Me | H | $-CH_2NHCO(CH_2)_2-$ 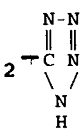 | H | H | H | Ph |

TABLE I CONTINUED

| No. | $R^1$ | A | $R^2$ | $R^9$ | $R^3$ | $R^{12}$ | $R^4$ | $R^{13}$ | Ar |
|---|---|---|---|---|---|---|---|---|---|
| C12 | Do | -O-CO- | Me | H | H | H | $-NHCO(CH_2)_2$ | H | Ph |
| C13 | 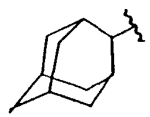 | -O-CO- | Me | H | $-CONHCH_2CO_2H$ | H | H | H | Ph |
| C14 | Do | -O-CO- | Me | H | $-CONHCH_2CH_2COOH$ | H | H | H | Ph |
| C15 | Do | -O-CO- | Me | Me | H | H | H | H | Ph |
| C16 | 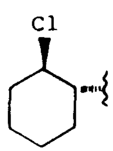 | -O-CO- | Me | H | H | H | H | H | Ph |

TABLE I CONTINUED

| No. | $R^1$ | A | $R^2$ | $R^9$ | $R^3$ | $R^{12}$ | $R^4$ | $R^{13}$ | Ar |
|---|---|---|---|---|---|---|---|---|---|
| C 17 | 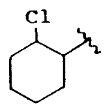 | -O-CO- | Me | H | $-CH_2OH$ | H | H | H | Ph |
| C 18 | Do | -O-CO- | Me | H | $-CH_2OCOCH_2CH_2COOH$ | H | H | H | Ph |
| C 19 | 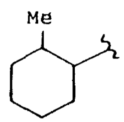 | -O-CO- | Me | H | $-CH_2OCOCH_2CH_2COOH$ | H | H | H | Ph |
| C 20 | 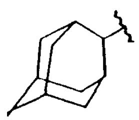 | -O-CO- | Me | H | H | H | H | H | Ph |
| C 21 | 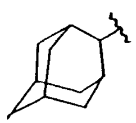 | -O-CO- | $-CH_2O$ | H | H | H | H | H | Ph |
| C 22 | Do | -O-CO- | Me | H | $-CH_2OCO(CH_2)_2COOH$ | H | H | H | Ph |

TABLE I CONTINUED

| No. | $R^1$ | A | $R^2$ | $R^9$ | $R^3$ | $R^{12}$ | $R^4$ | $R^{13}$ | Ar |
|---|---|---|---|---|---|---|---|---|---|
| C 23 | Do | -O-CO- | Me | H | H | H | $-CH_2OCO(CH_2)_2COOH$ | H | Ph |
| C 24 | Do | -O-CO- | Me | H | H | H | $-NHCO(CH_2)_2COOH$ | H | Ph |
| C 25 | 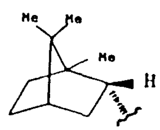 | -O-CO- | Me | H | H | H | $-NHCO(CH_2)_2COOH$ | H | Ph |
| C 26 | 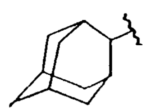 | -O-CO- | Me | H | $-CH_2NHCOCH=CHCOOH$ | H | H | H | Ph |
| C27 | Do | -O-CO- | Me | H | $-CH_2NHCO(CH_2)_2COOH$ | H | H | H | Ph |

TABLE I CONTINUED

| No. | $R^1$ | A | $R^2$ | $R^9$ | $R^3$ | $R^{12}$ | $R^4$ | $R^{13}$ | Ar |
|---|---|---|---|---|---|---|---|---|---|
| C 28 | Do | -O-CO- | Me | Me | H | H | H | H | Ph |
| C 29 | Do | -O-CO- | Me | H | $-CH_2-S(=O)-CH_2COOH$ | H | H | H | Ph |
| C 30 | Do | -O-CO- | Me | H | $-CH_2-SO_2-CH_2COOEt$ | H | H | H | Ph |
| C 31 | Do | -O-CO- | Me | H | $-CH_2-S(=O)-CH_2COOEt$ | H | H | H | Ph |
| C 32 | Do | -O-CO- | Me | H | H | H | -NHCOCH=CHCOOH | H | Ph |

TABLE I CONTINUED

| No. | $R^1$ | A | $R^2$ | $R^9$ | $R^3$ | $R^{12}$ | $R^4$ | $R^{13}$ | Ar |
|---|---|---|---|---|---|---|---|---|---|
| C 33 | Do | -O-CO- | Me | H | $-CH_2SCH_2COOH$ | H | H | H | Ph |
| C 34 | 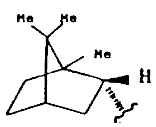 | -O-CO- | Me | H | H | H | -NHCOCH=CH-COOMe | H | Ph |
| C 35 | Do | -O-CO- | Me | H | H | H | -NHCOCH=CHCOOH | H | Ph |
| C 36 | 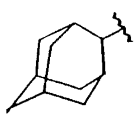 | -O-CO- | Me | H | H | H | $-NHCOCH_2COOH$ | H | Ph |
| C 37 | Do | -O-CO- | Me | H | $-CH_2SOCH_2COOEt$ | H | H | H | Ph |

TABLE I CONTINUED

| No. | $R^1$ | A | $R^2$ | $R^9$ | $R^3$ | $R^{12}$ | $R^4$ | $R^{13}$ | Ar |
|---|---|---|---|---|---|---|---|---|---|
| C 38 | Do | -O-CO- | Me | H | $-CH_2COOH$ | H | H | H | Ph |
| C 39 | Do | -O-CO- | Me | H | $-CH_2CH_2CH(NH_2)COOH$ | H | H | H | Ph |
| C 40 | 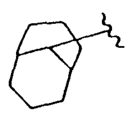 | -O-CO- | Me | H | $-CH_2NHCOCH=CHCOOH$ | H | H | H | Ph |
| C 41 | 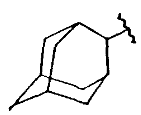 | -O-CO- | Me | H | H | H | $-O-CO-(CH_2)COOH$ | H | Ph |
| C 42 | Do | -O-CO- | Me | H | H | H | $-CONH-(CH_2)_2COOH$ | H | Ph |

TABLE I CONTINUED

| No. | $R^1$ | A | $R^2$ | $R^9$ | $R^3$ | $R^{12}$ | $R^4$ | $R^{13}$ | Ar |
|---|---|---|---|---|---|---|---|---|---|
| C 43 | 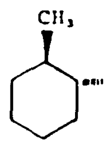 | -O-CO- | Me | H | $-CH_2NH-CO-CH=CHCOOH$ | H | H | H | Ph |
| C 44 | Do | -O-CO- | Me | H | $-CH_2NH-CO-(CH_2)_2COOH$ | H | H | H | Ph |
| C 45 | Do | -O-CO- | Me | H | H | H | -NHCO-CH=CHCOOH | H | Ph |
| C 46 | Do | -O-CO- | Me | H | H | H | $-NHCO-(CH_2)_2COOH$ | H | Ph |
| C47 | Do | -O-CO- | Me | H | $CH_2OH$ | H | H | H | Ph |

TABLE I CONTINUED

| No. | $R^1$ | A | $R^2$ | $R^9$ | $R^3$ | $R^{12}$ | $R^4$ | $R^{13}$ | Ar |
|---|---|---|---|---|---|---|---|---|---|
| C 48 | 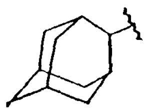 | -O-CO- | Me | H | $-(CH=CH)_2COOH$ | H | H | H | Ph |
| C49 | Do | -O-CO- | Me | H | H | H | $-NH-CO-(CH_2)_2CO-NH-$ 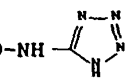 | H | Ph |
| C 50 | Do | -O-CO- | Me | H | $-CH_2NHCOCH=CHCOOMe$ | H | H | H | Ph |
| C 51 | Do | -O-CO- | Me | H | $-CH_2NHCOCH_2COOH$ | H | H | H | Ph |

TABLE II

C69.

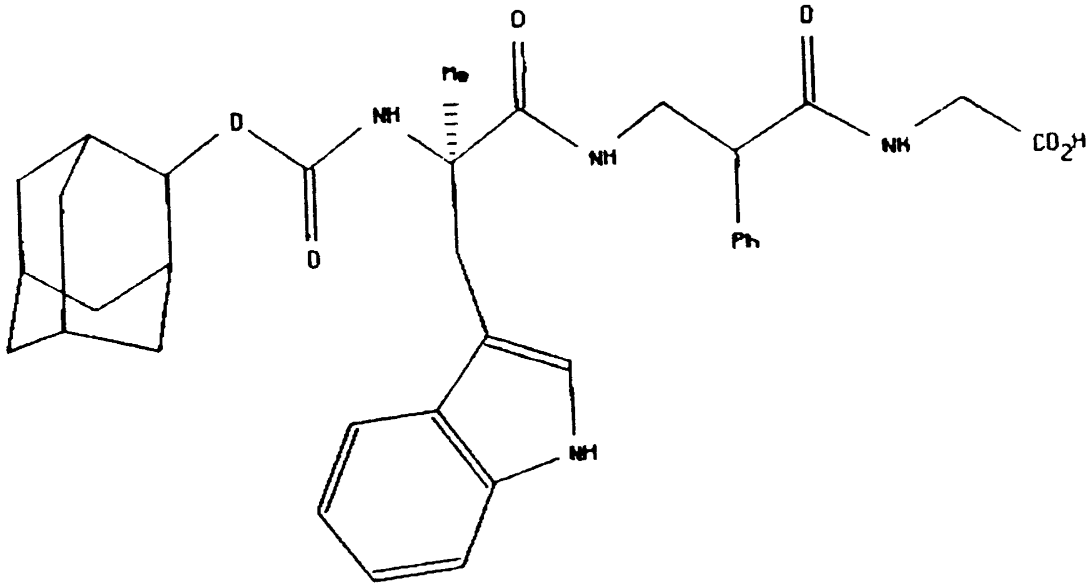

C70.

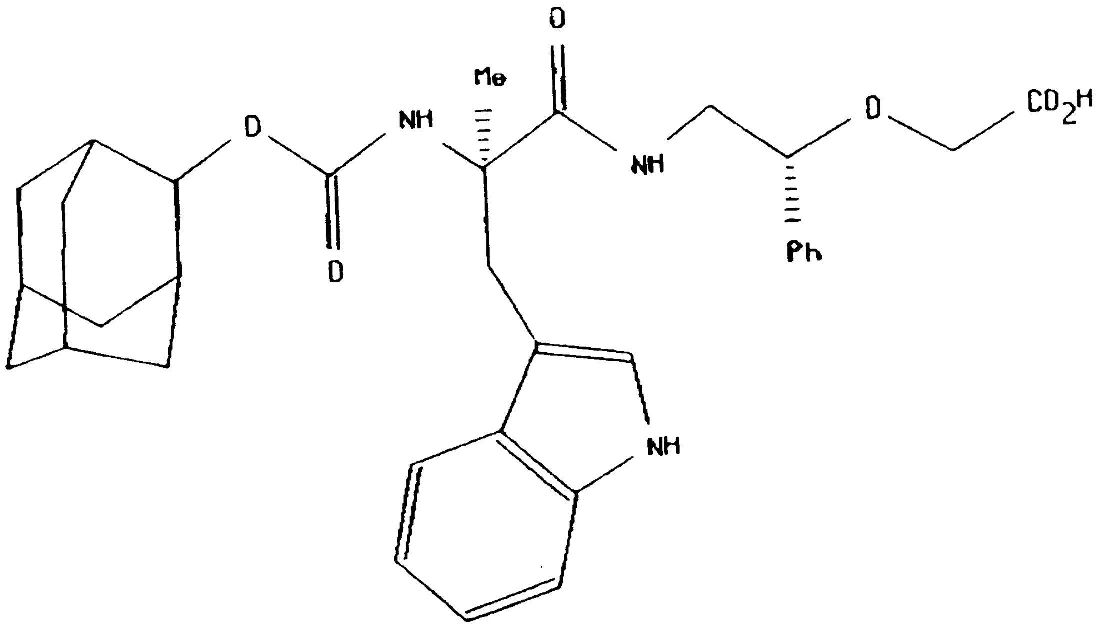

TABLE II CONTINUED

C71.

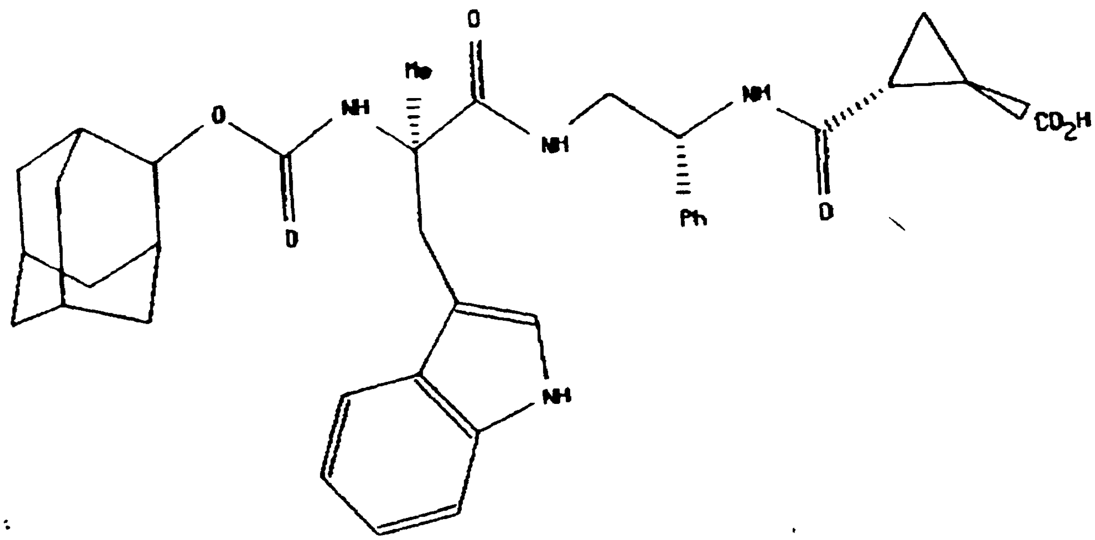

C72.

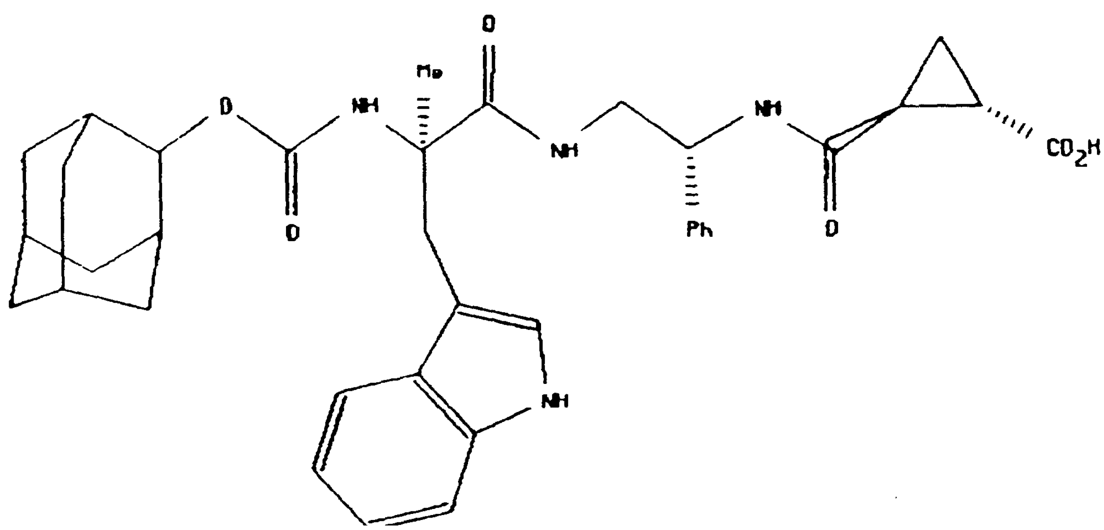

TABLE II CONTINUED

C 73.

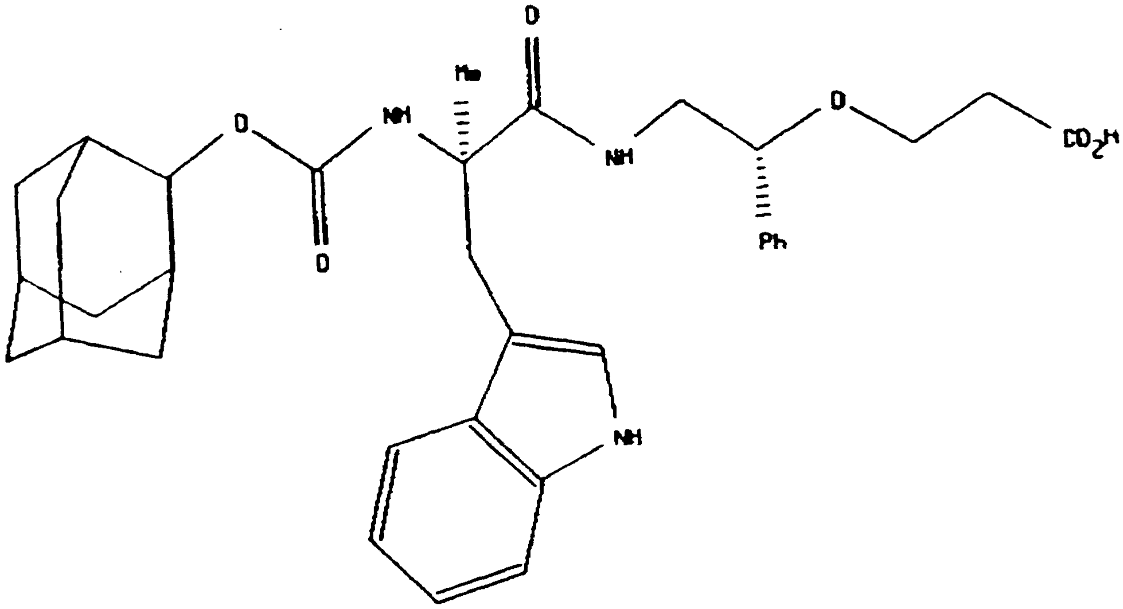

C74.

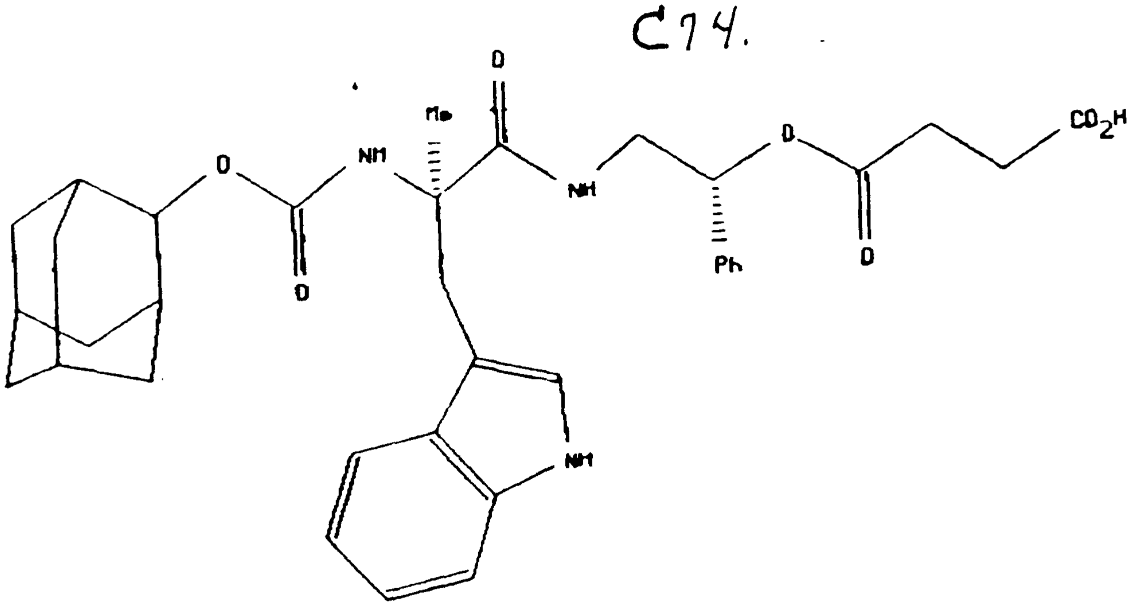

TABLE II CONTINUED

C75.

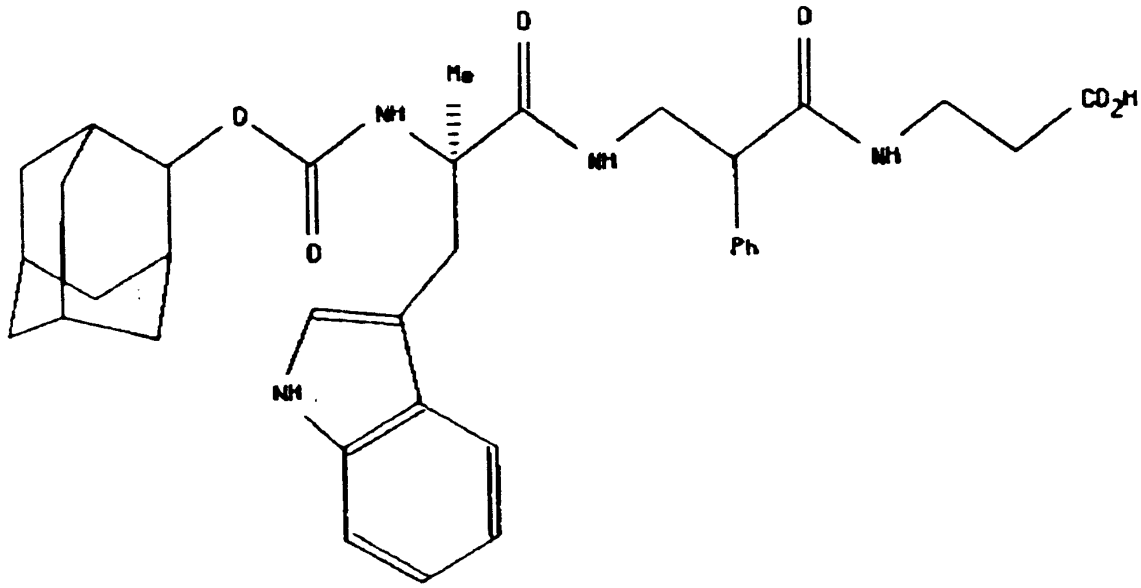

C76.

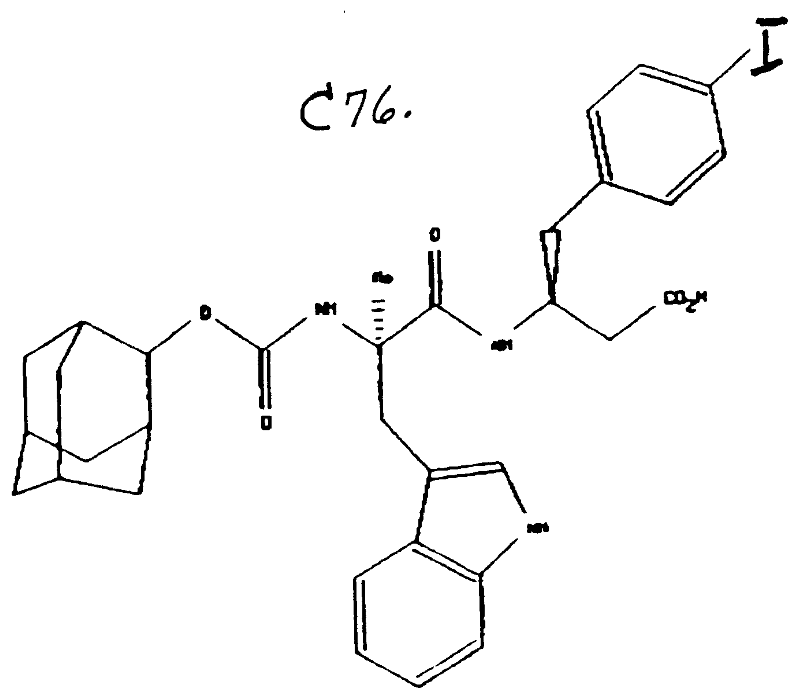

TABLE II CONTINUED

C77.

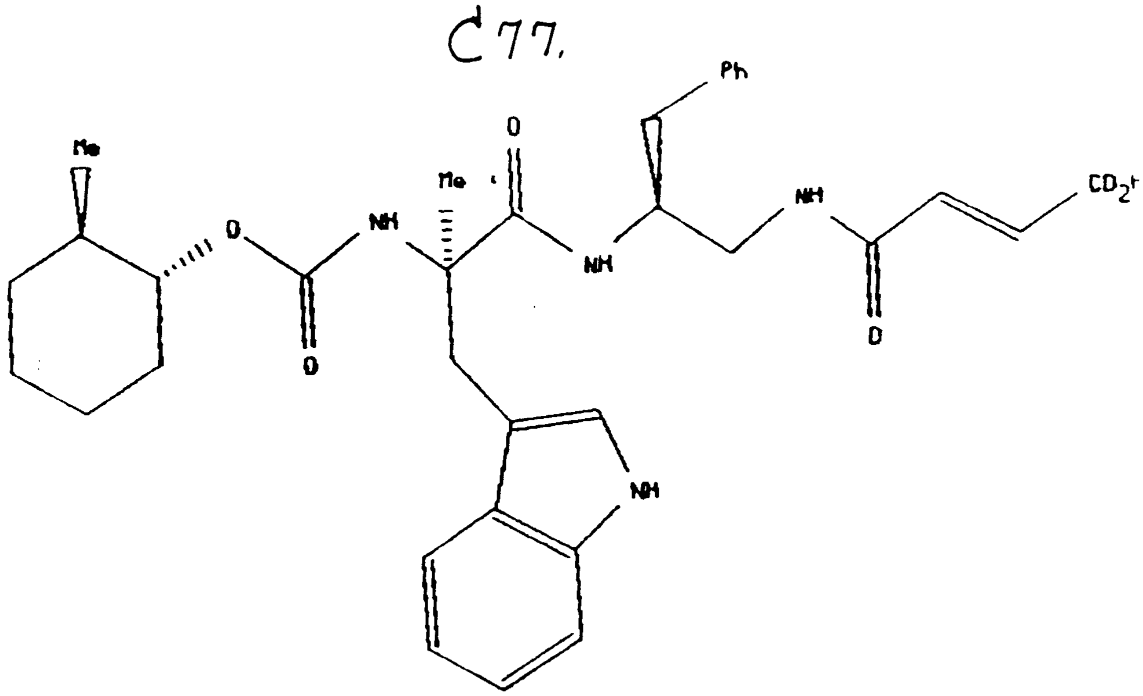

C78.

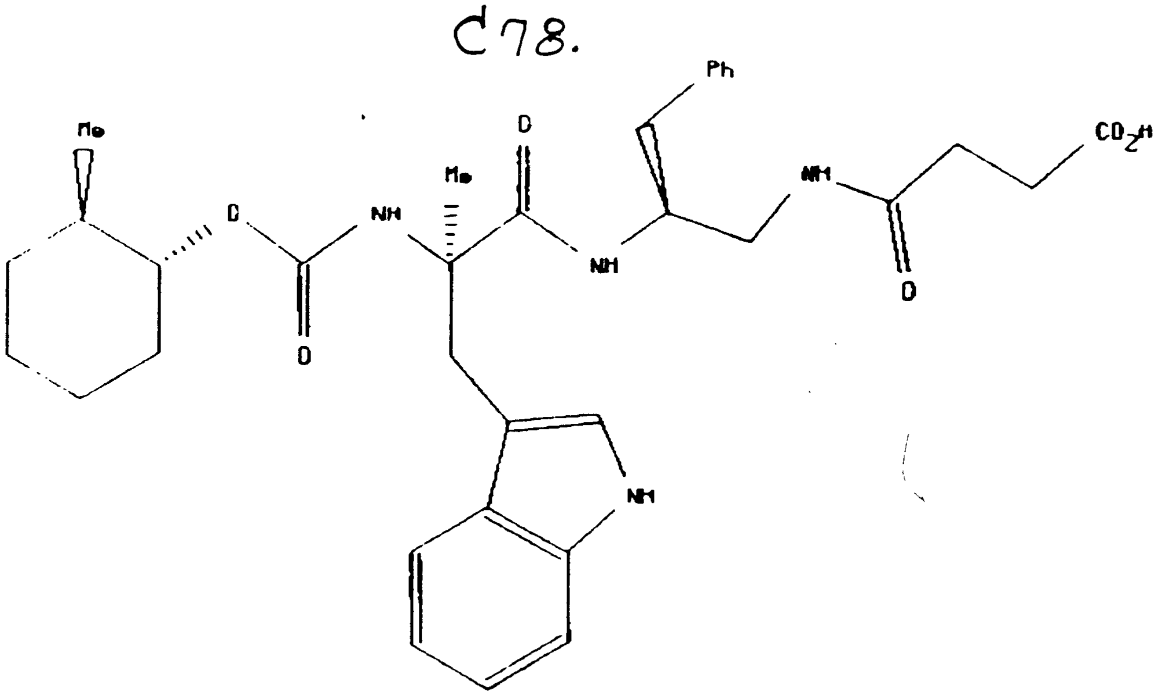

TABLE II CONTINUED

C79.

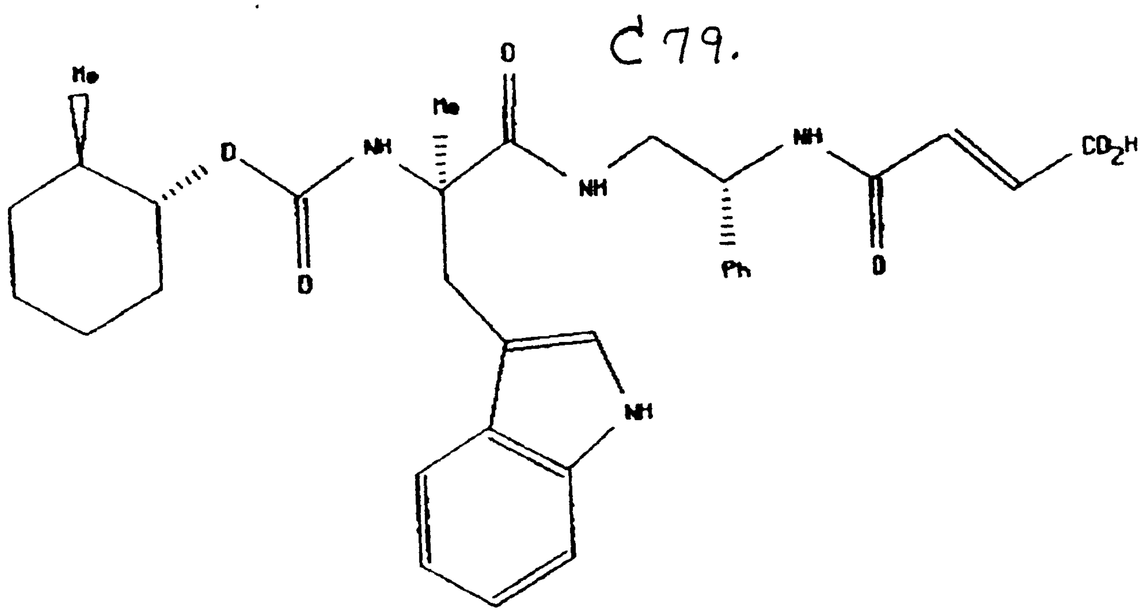

C80.

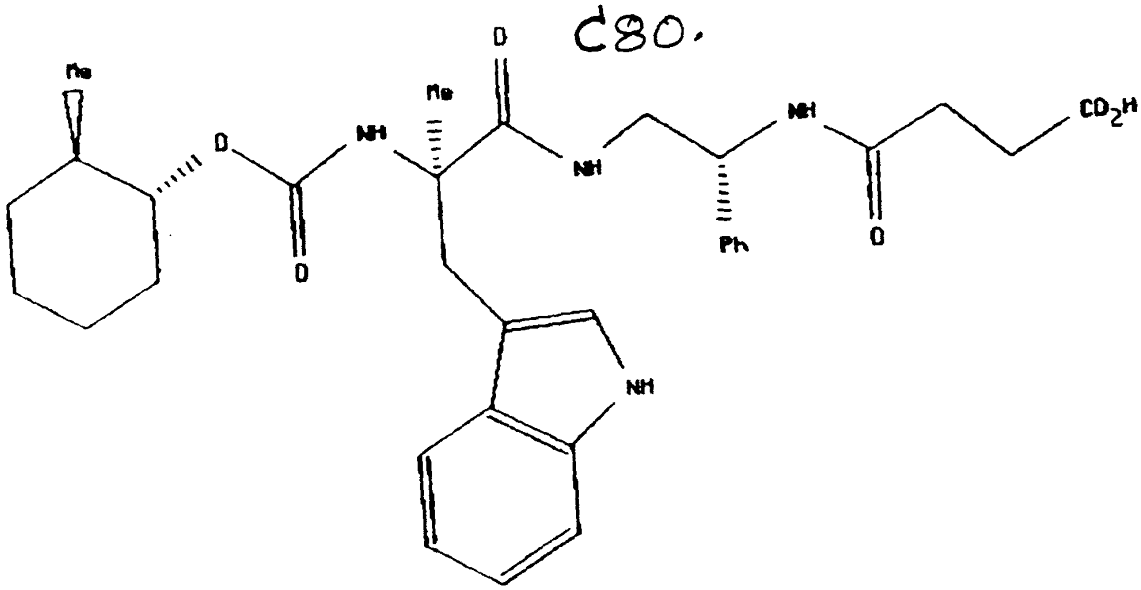

TABLE II CONTINUED

C81.

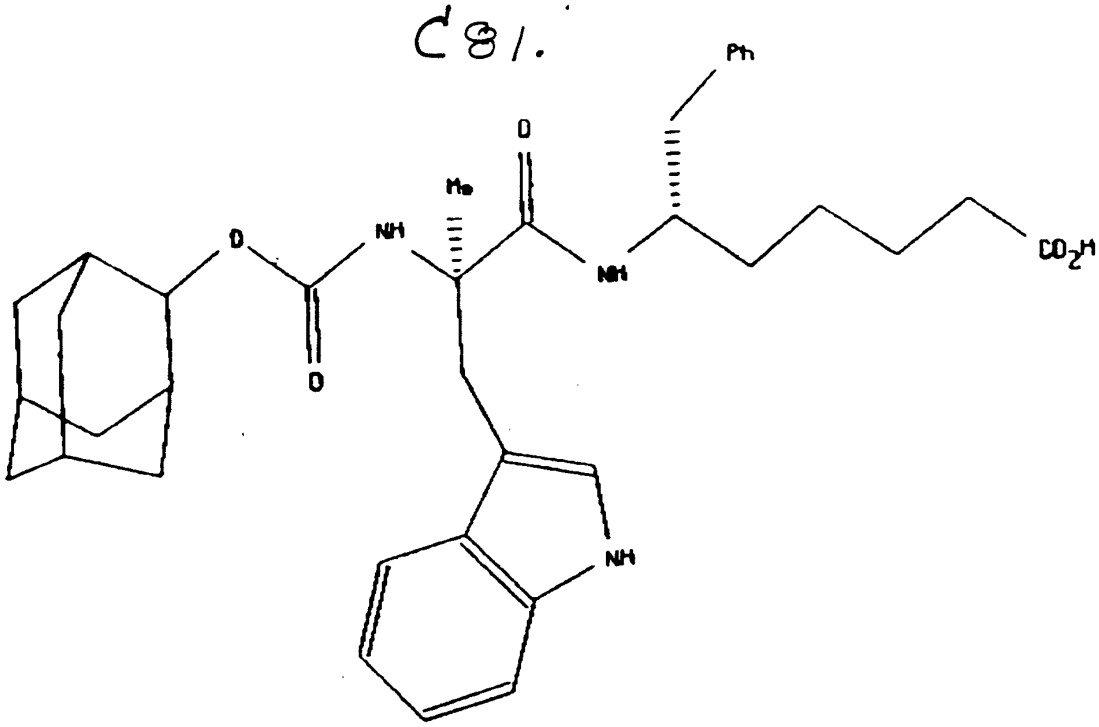

C82.

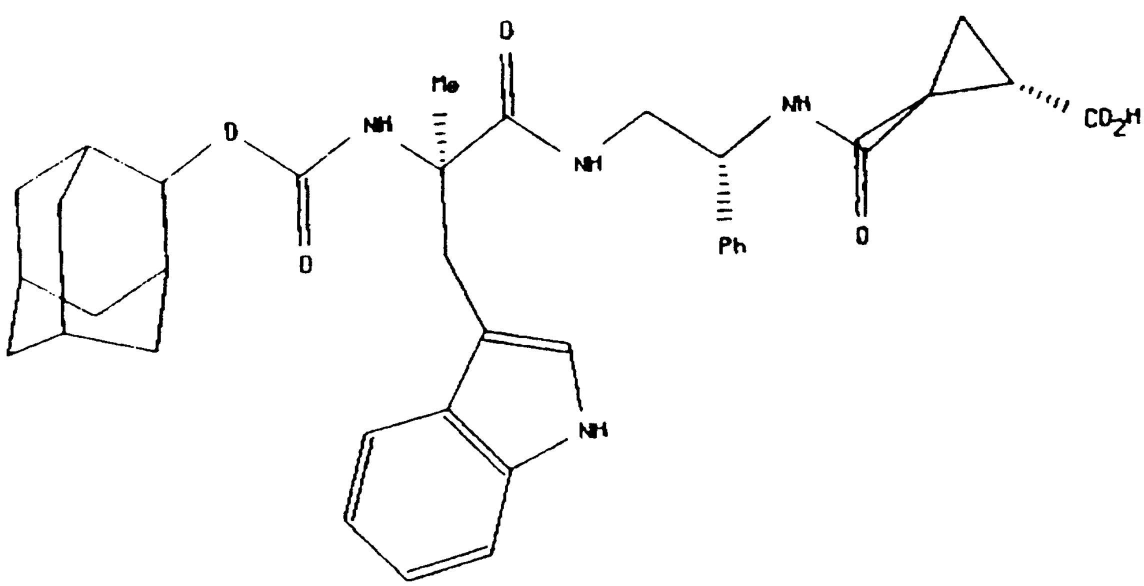

TABLE II CONTINUED

C83.

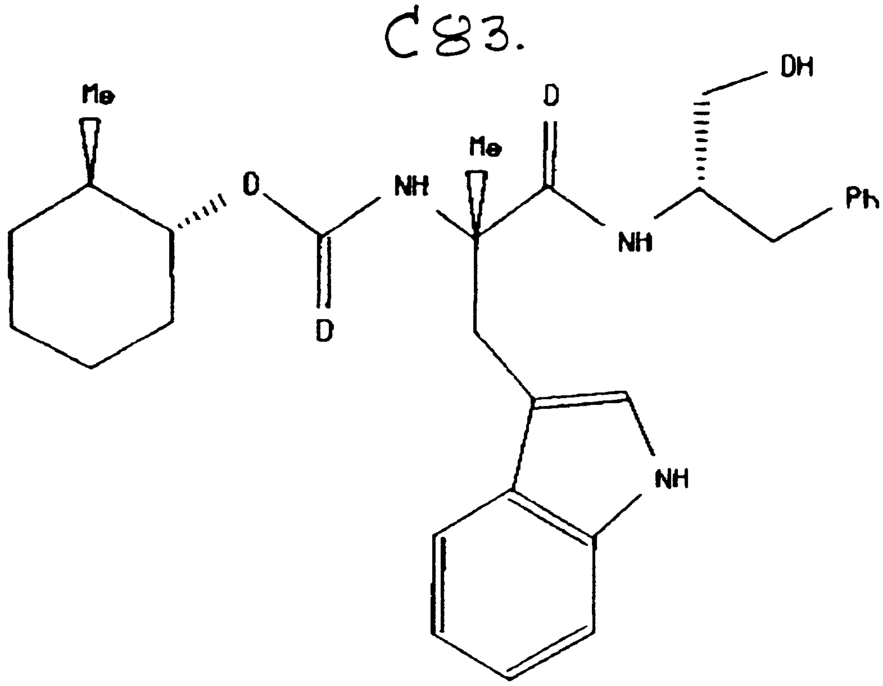

C84.

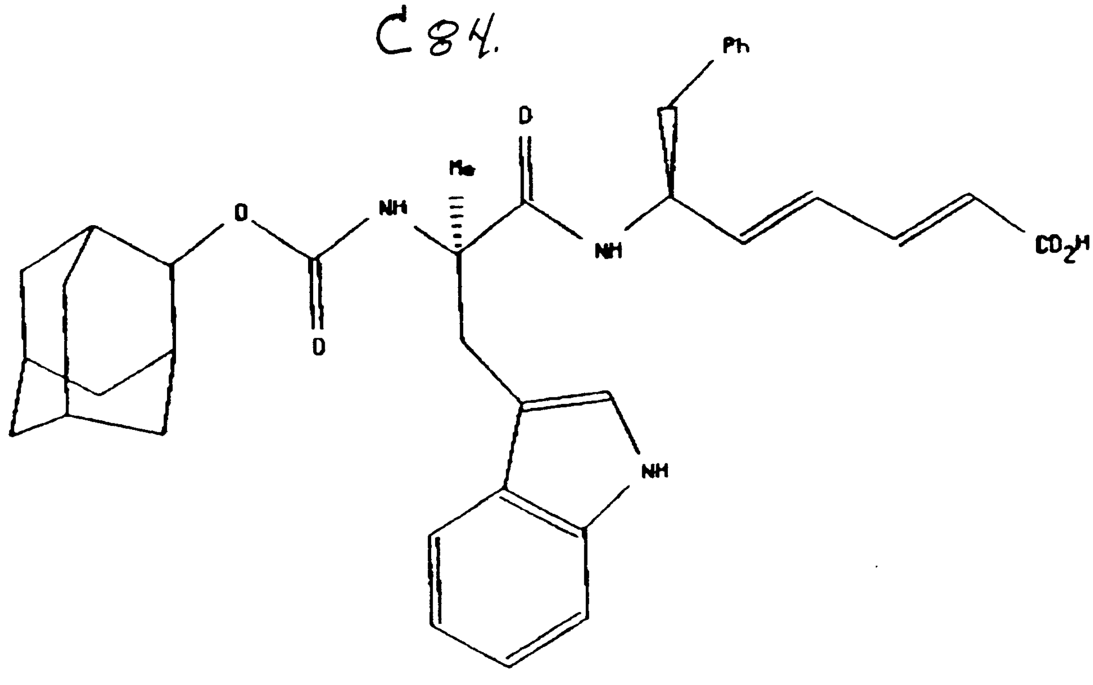

TABLE II CONTINUED

C85.

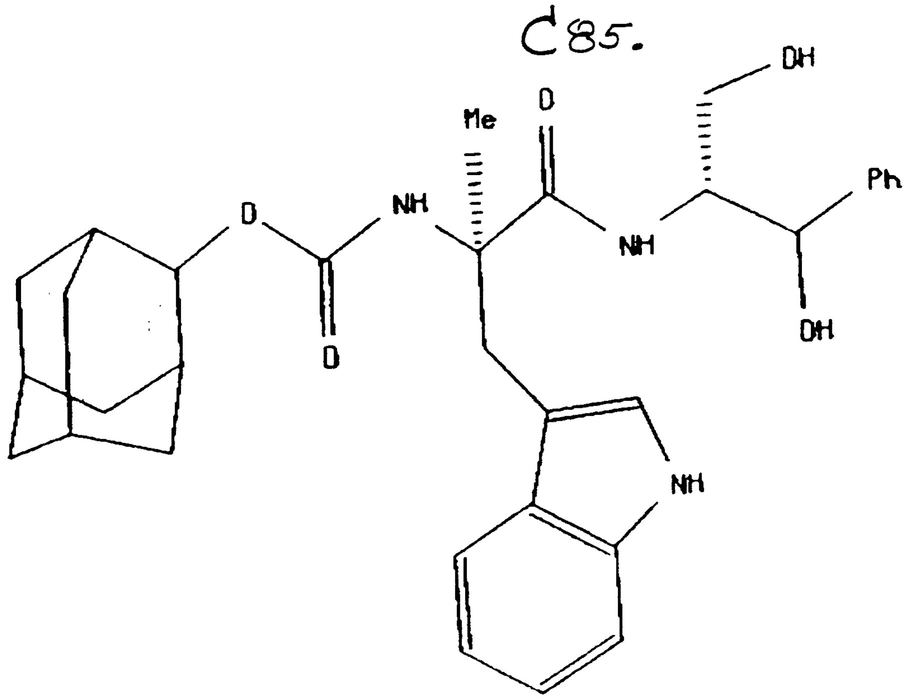

C86.

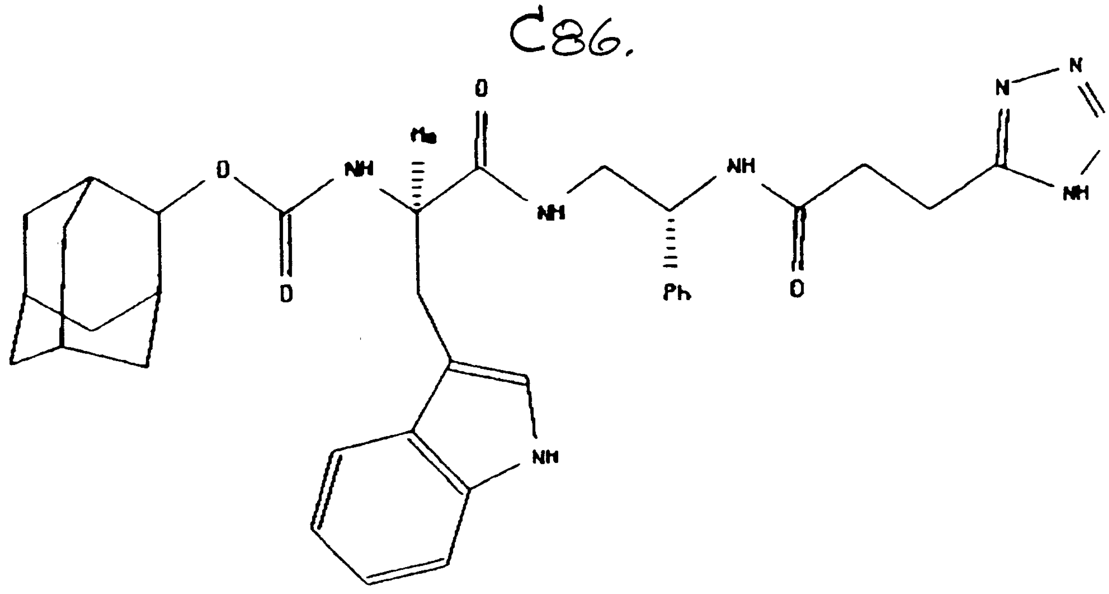

TABLE II CONTINUED

C87.

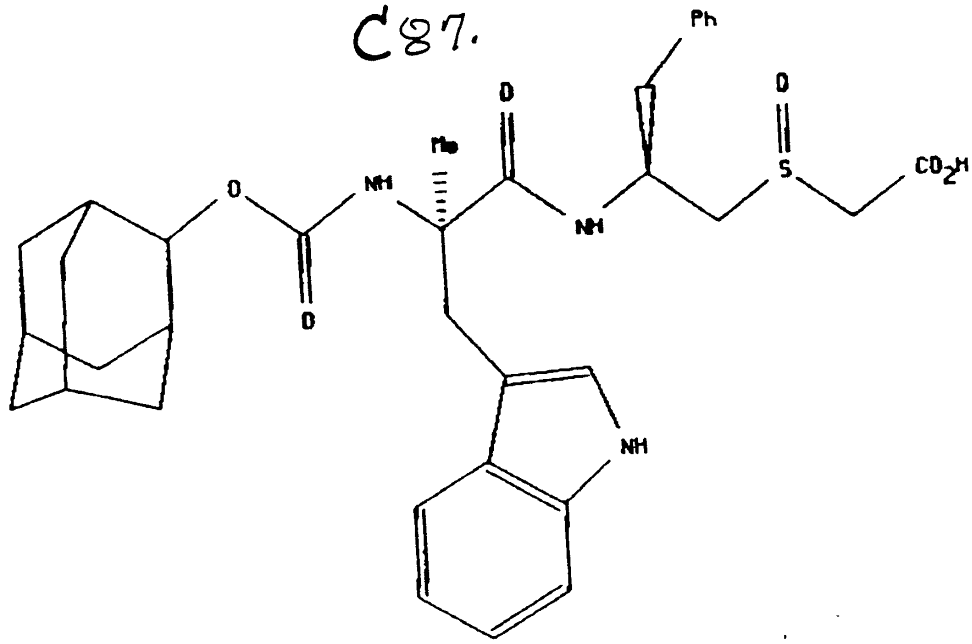

C88.

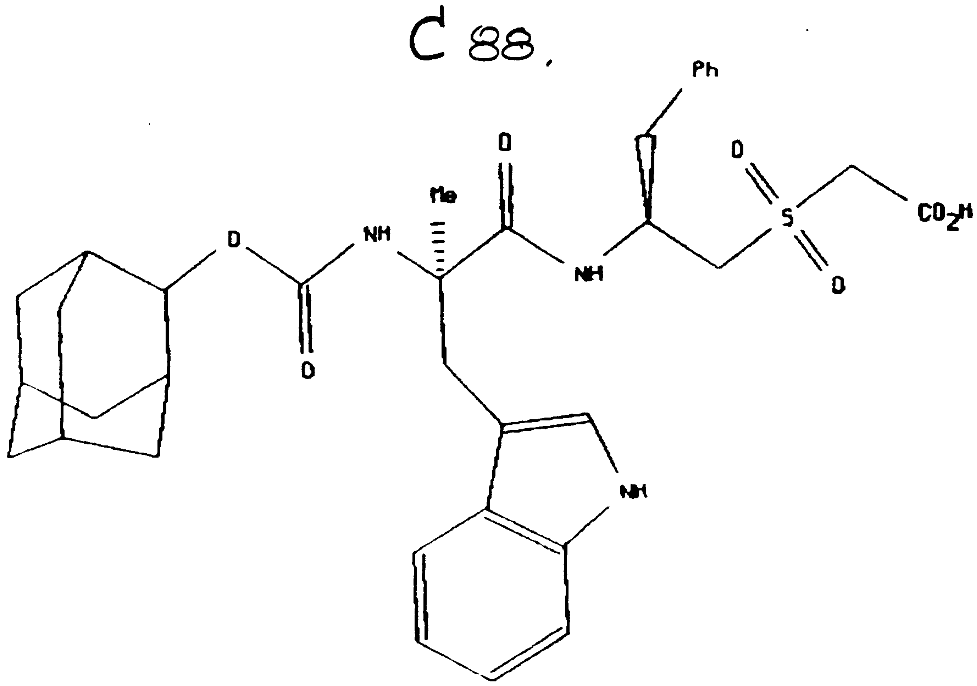

TABLE II CONTINUED

C89.

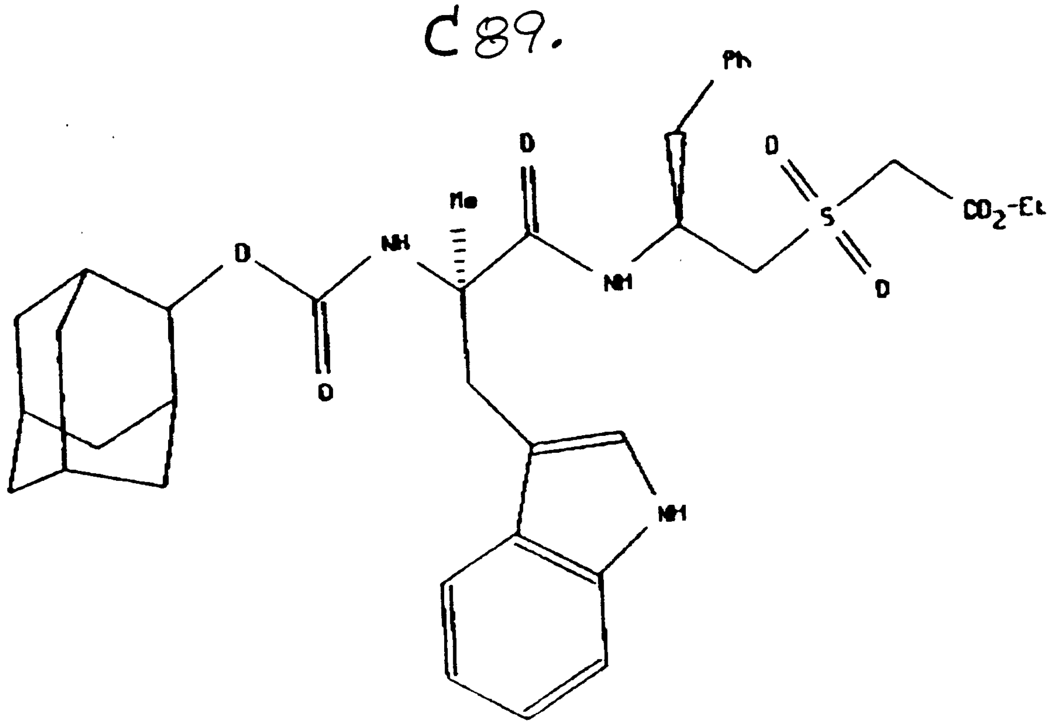

C100.

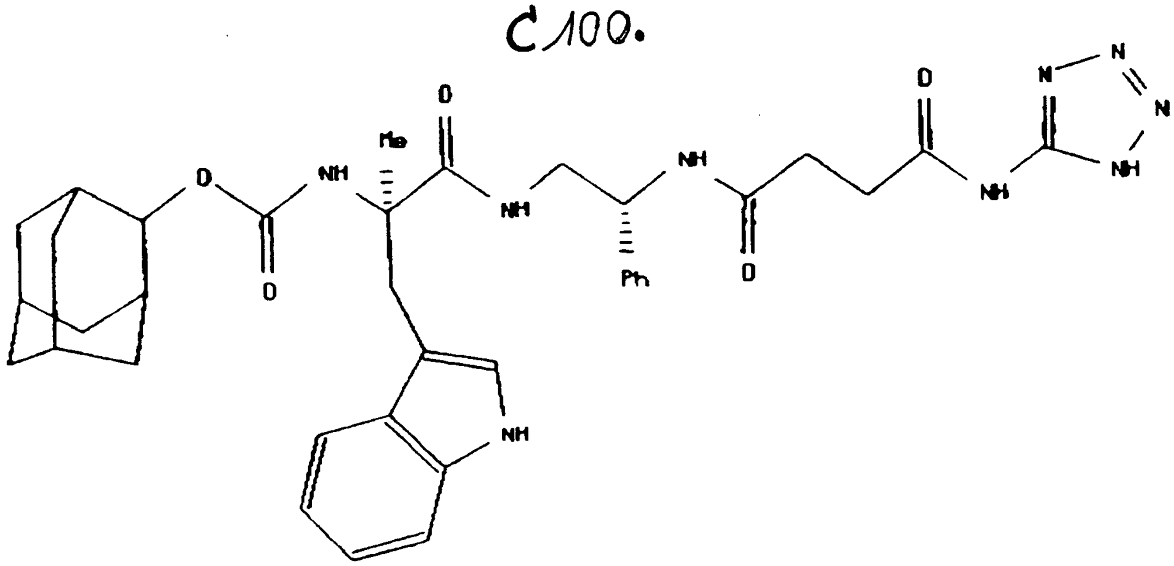

TABLE II CONTINUED

C101.

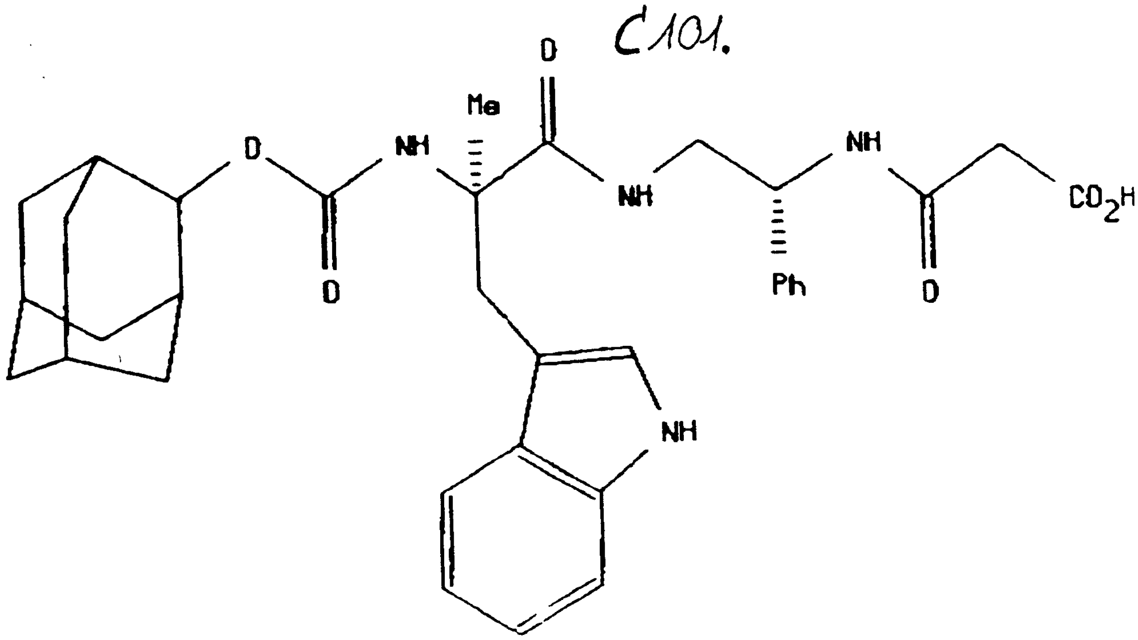

C102.

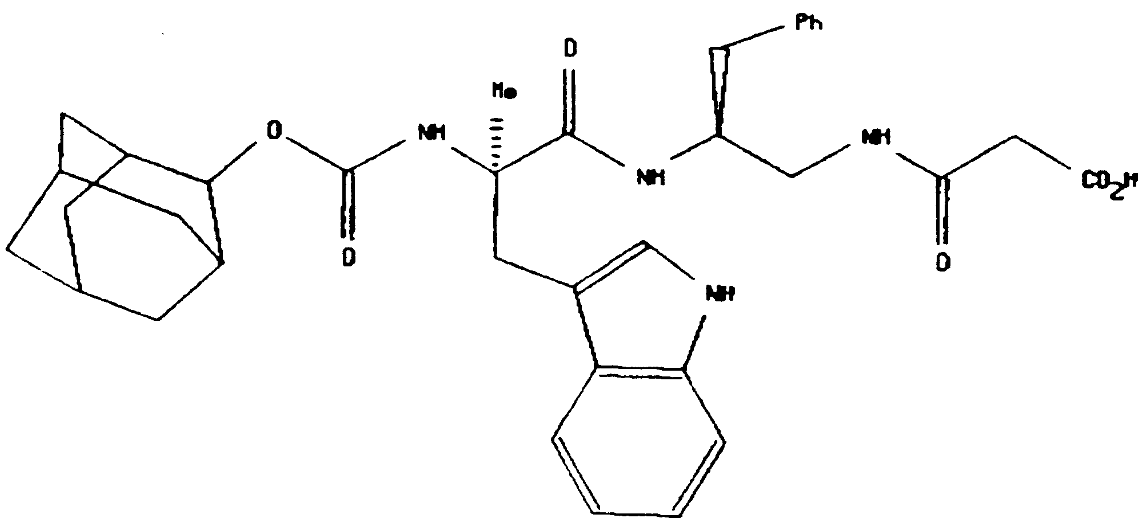

TABLE II CONTINUED

C103.

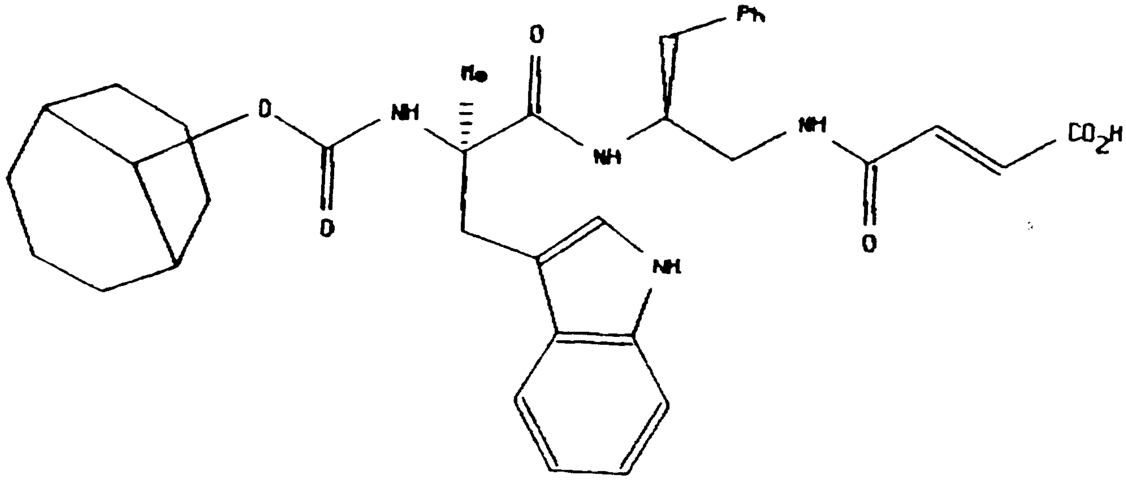

C104.

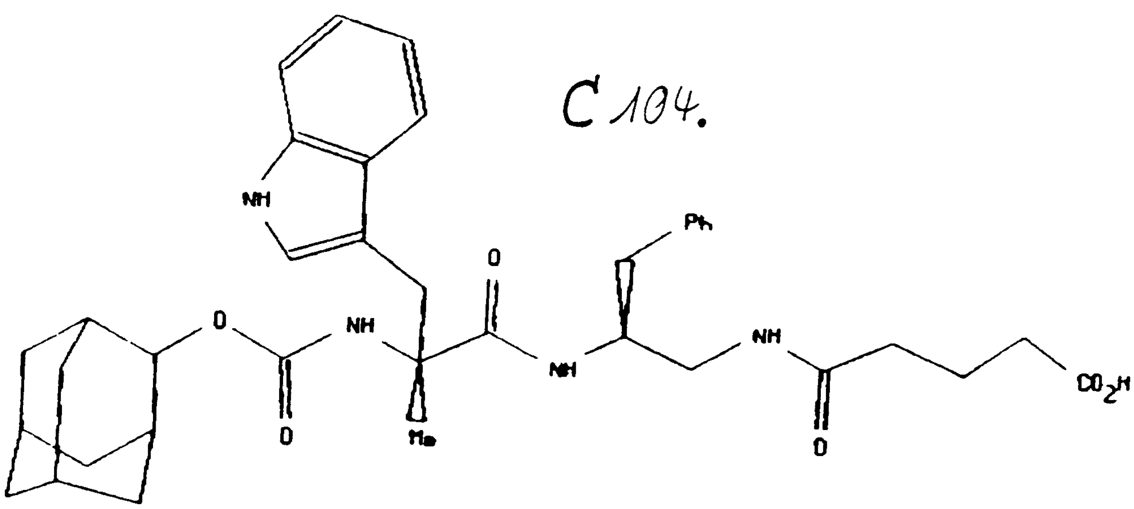

TABLE II CONTINUED

C 105.

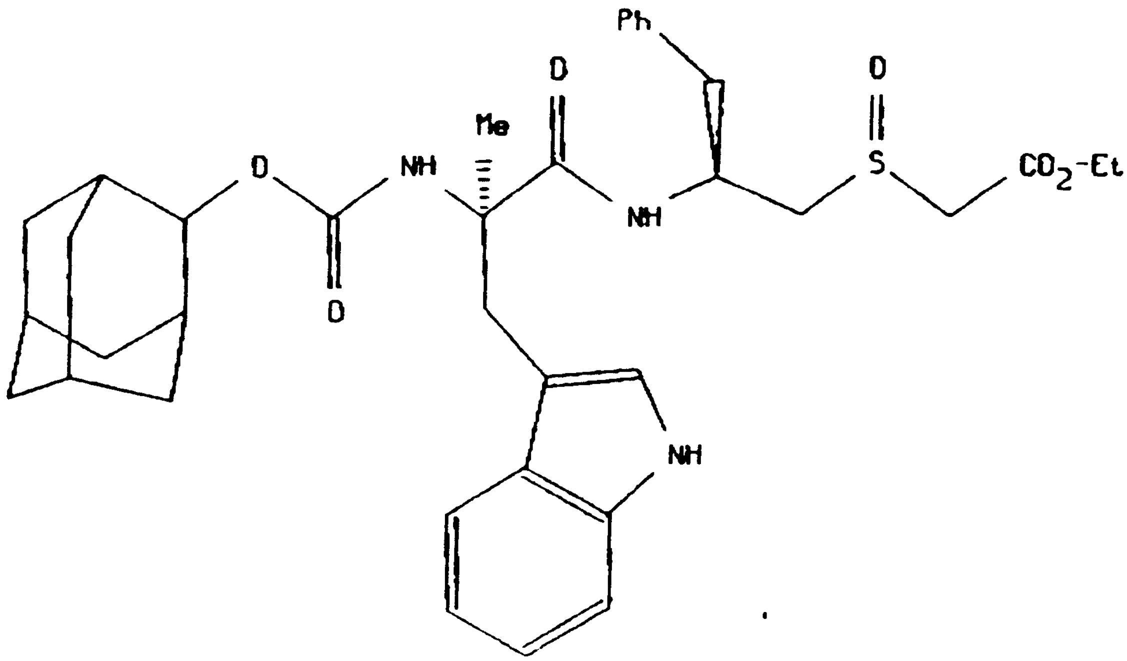

C106.

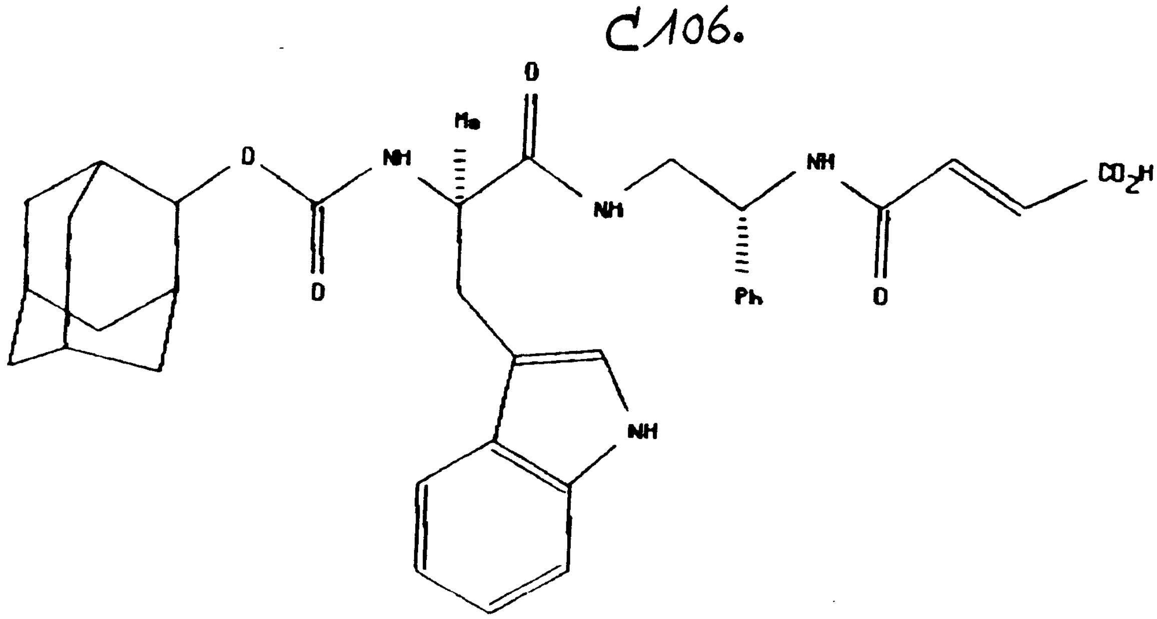

TABLE II CONTINUED

C107.

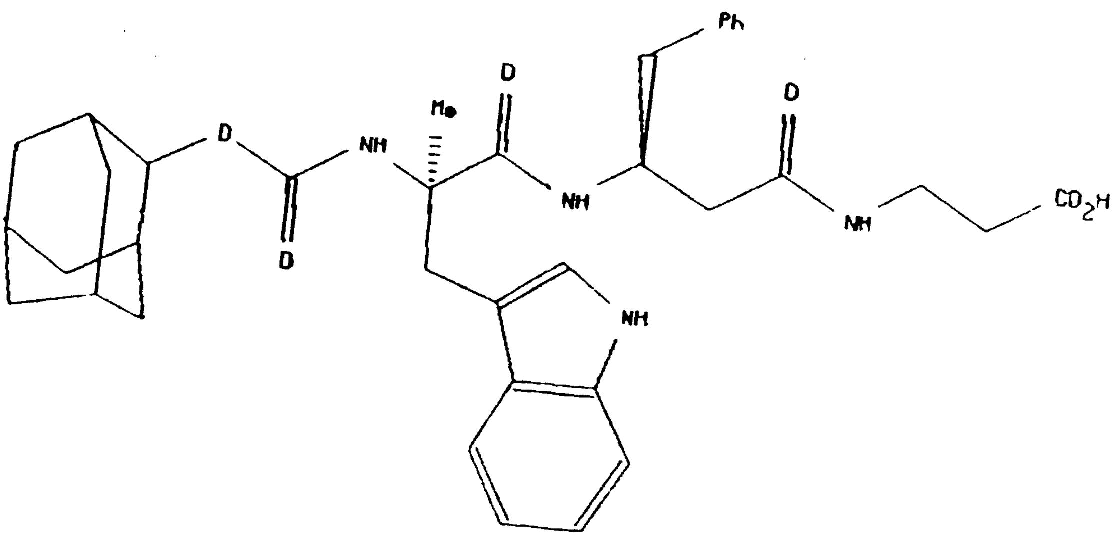

C108.

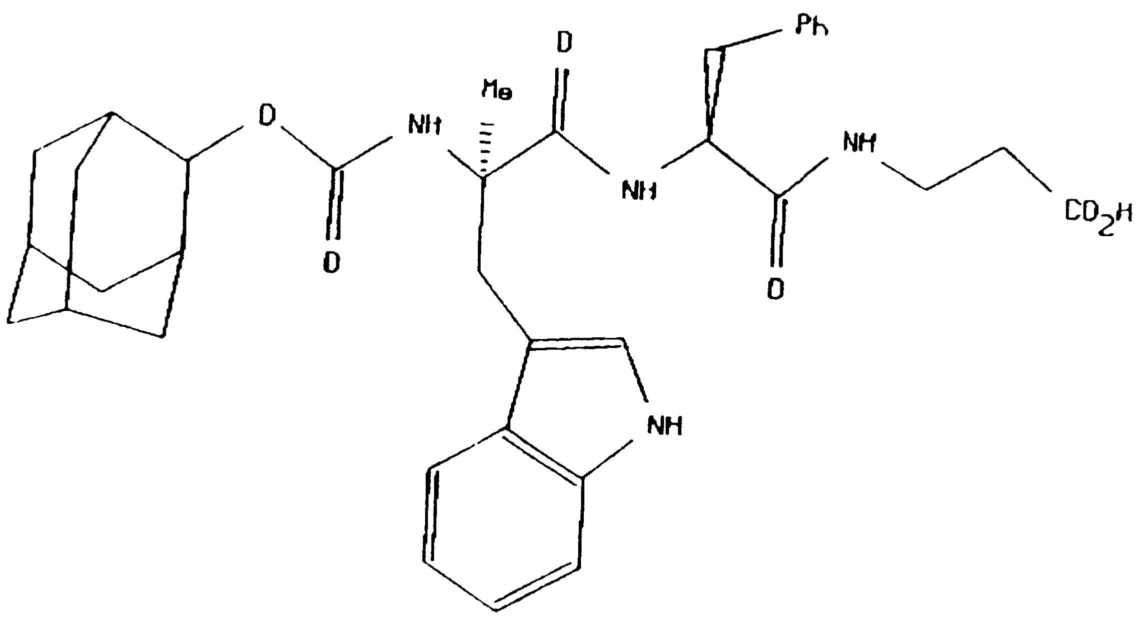

TABLE II CONTINUED

C109.

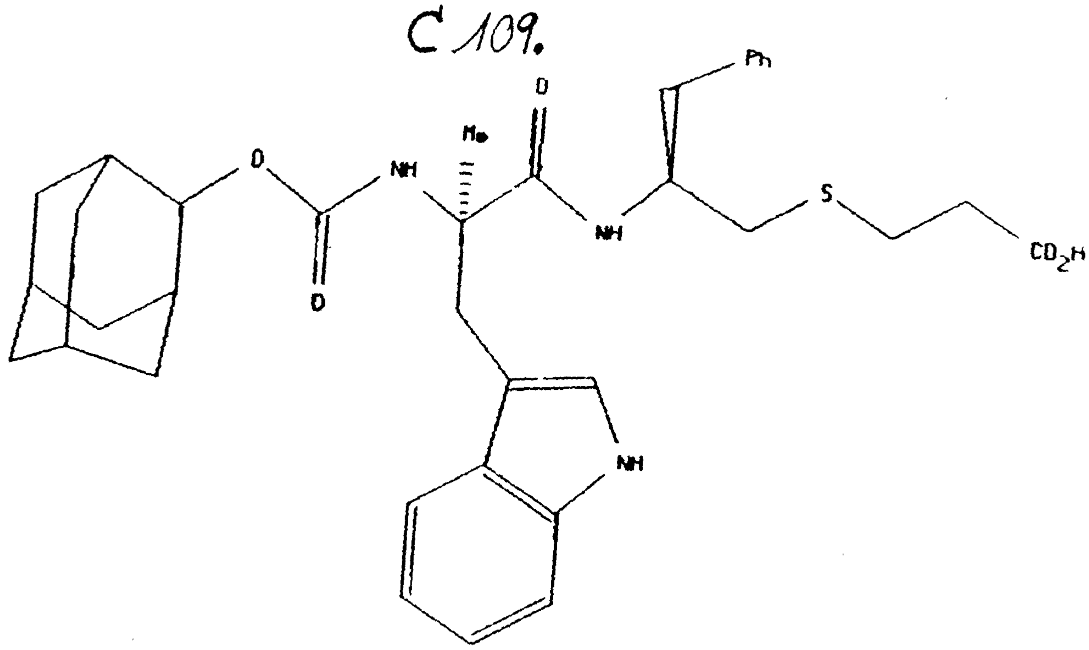

C110.

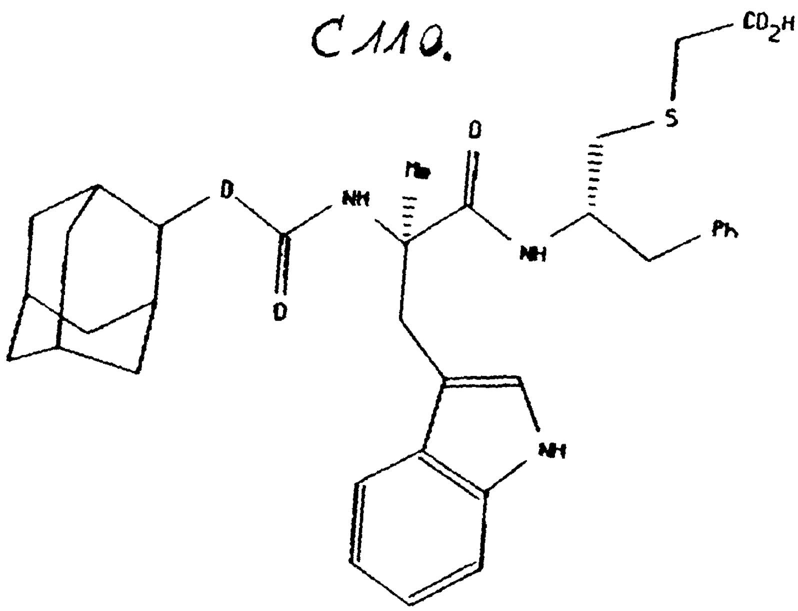

TABLE II CONTINUED

C111.

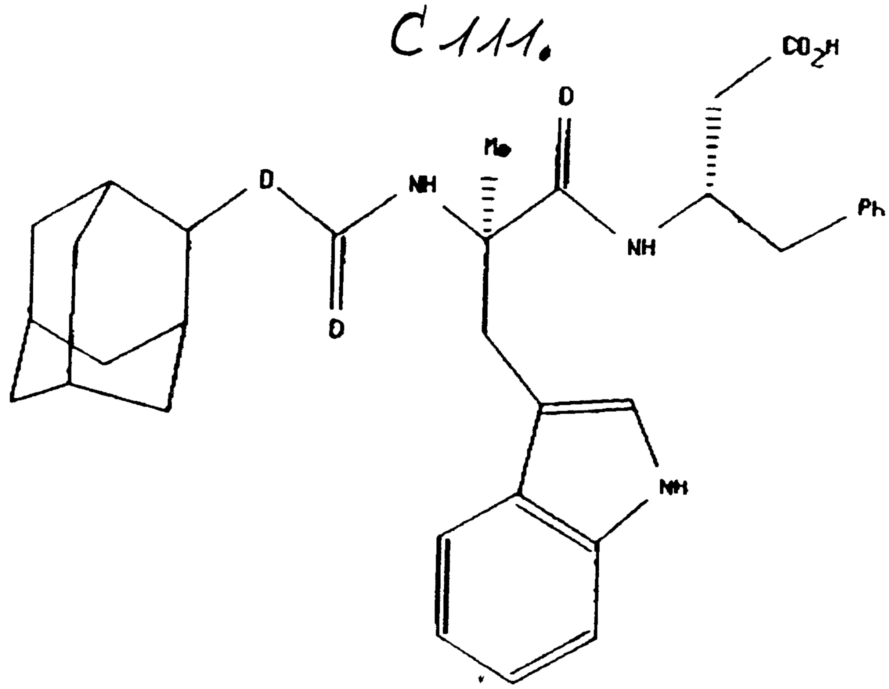

C112.

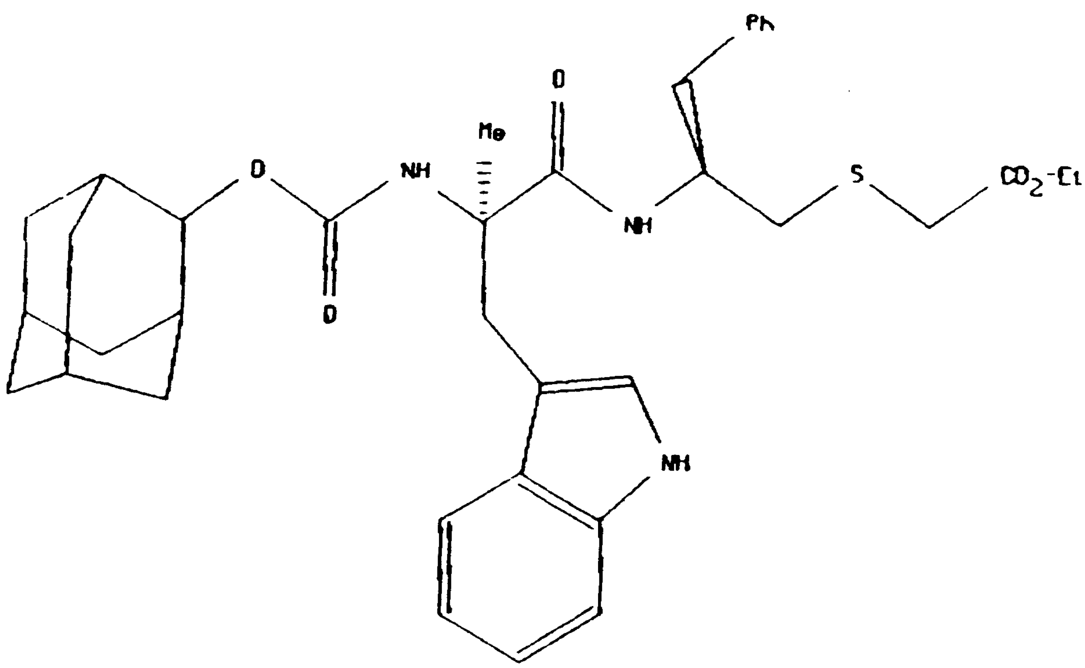

TABLE II CONTINUED

C 95 [S*[S*(E)]]
C 96 [S*(S*)]

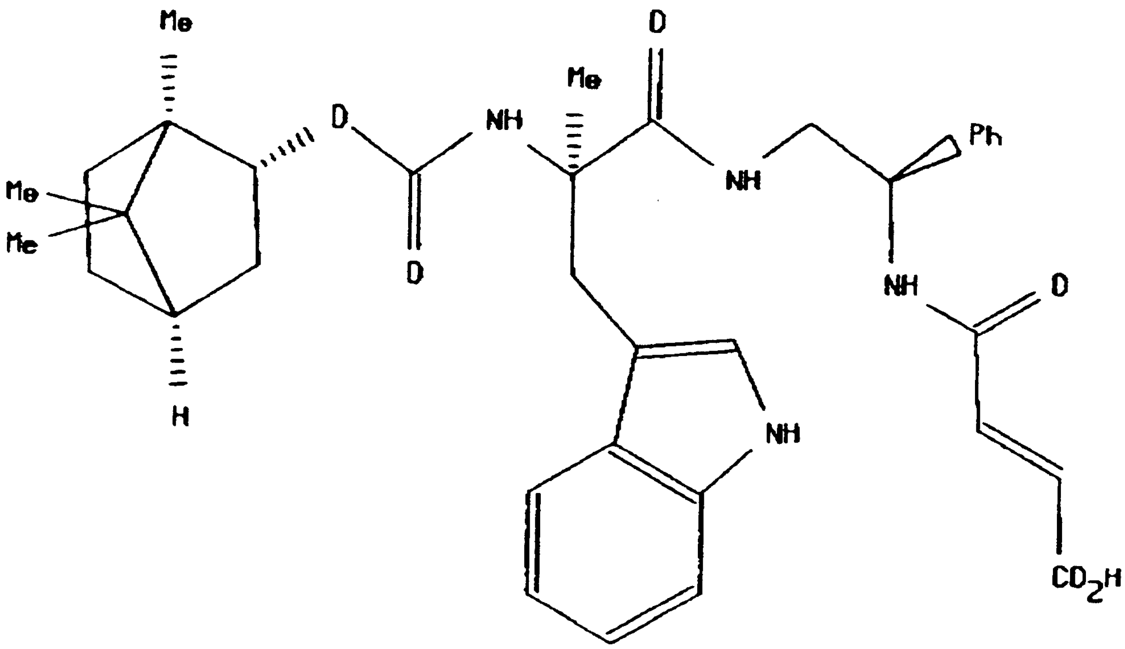

C96A

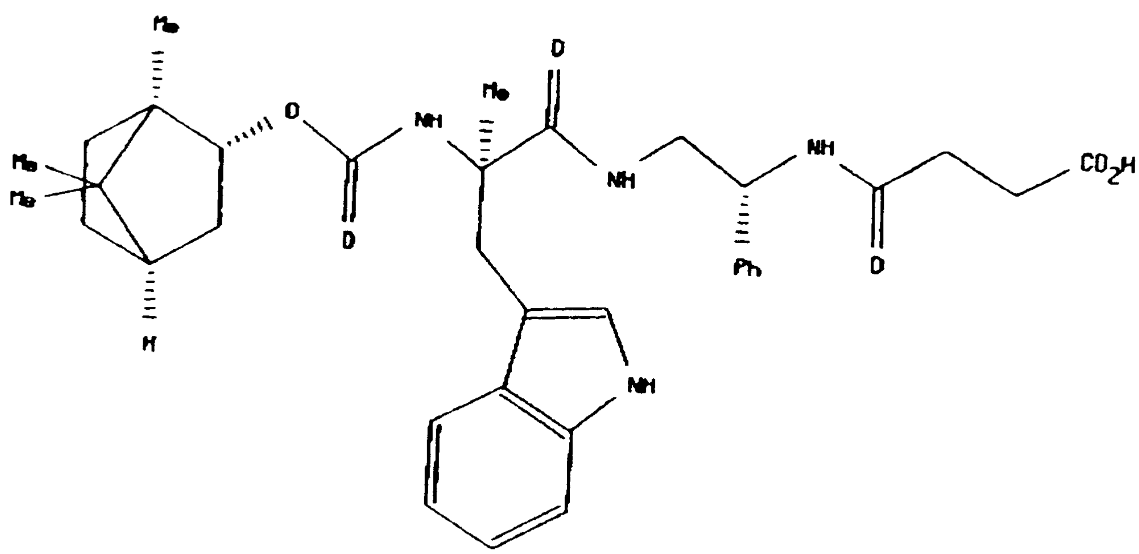

TABLE II CONTINUED

C97.

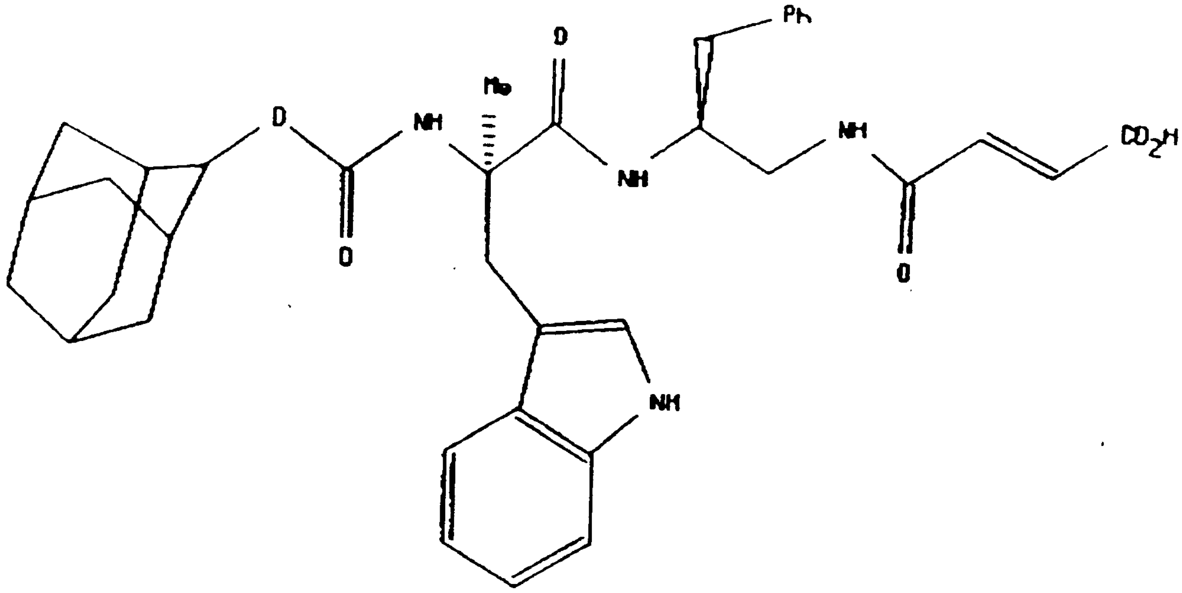

C98.

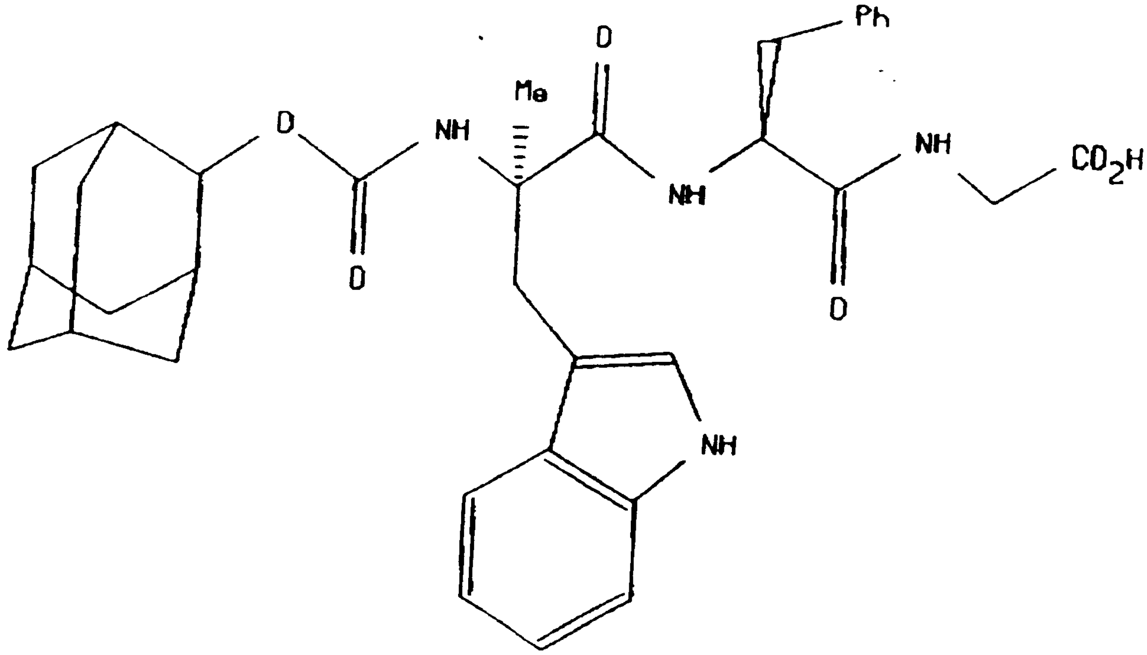

TABLE II CONTINUED

C 99.

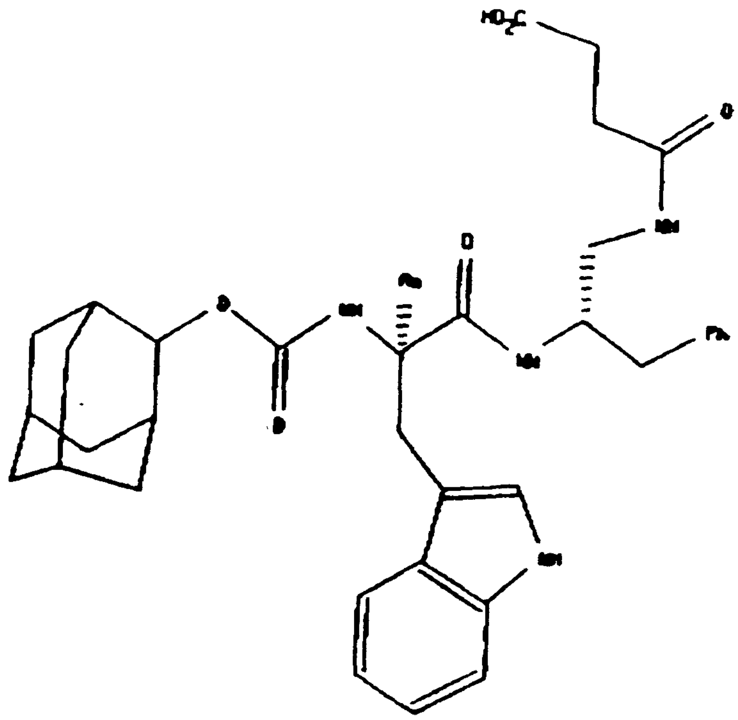

TABLE II CONTINUED

C91.

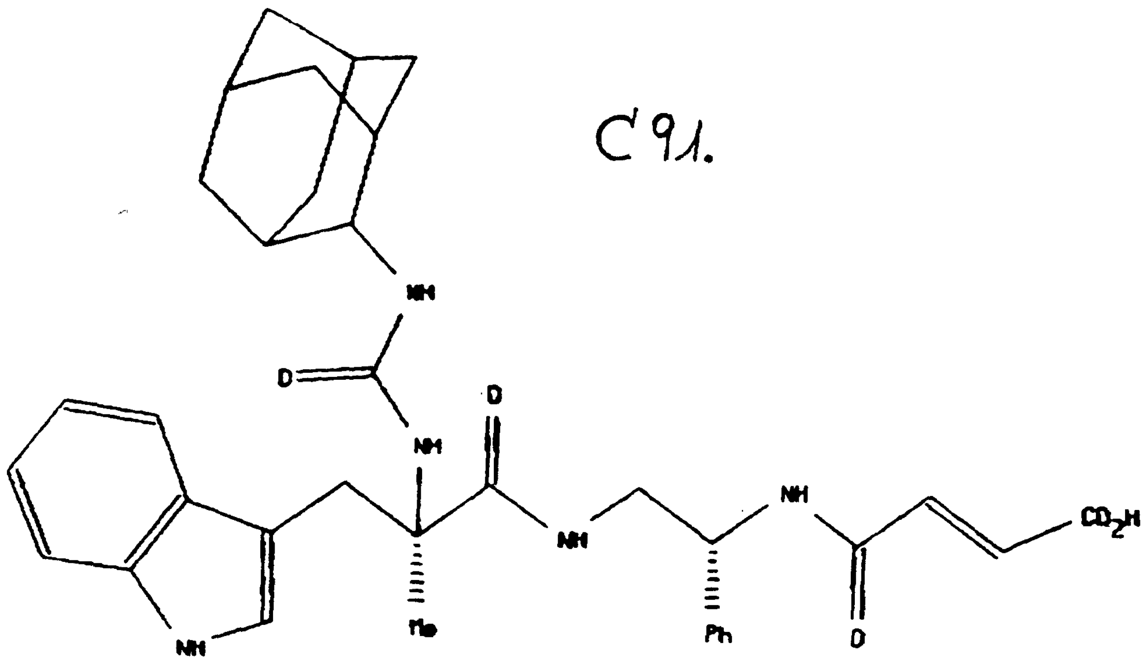

TABLE II CONTINUED

c 92.

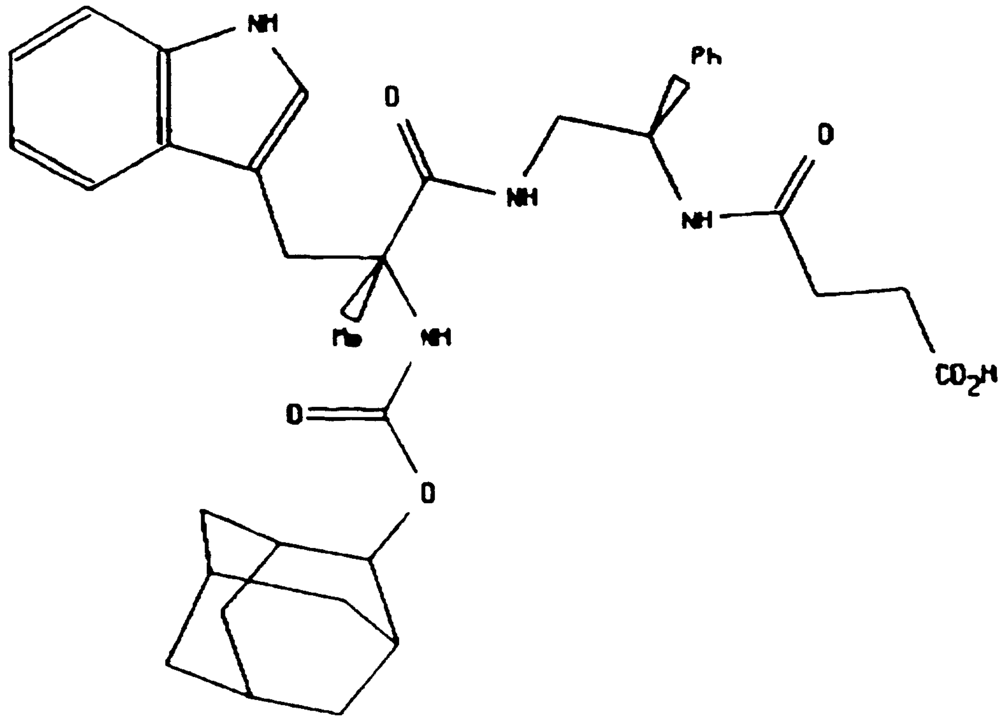

TABLE II CONTINUED

C93.

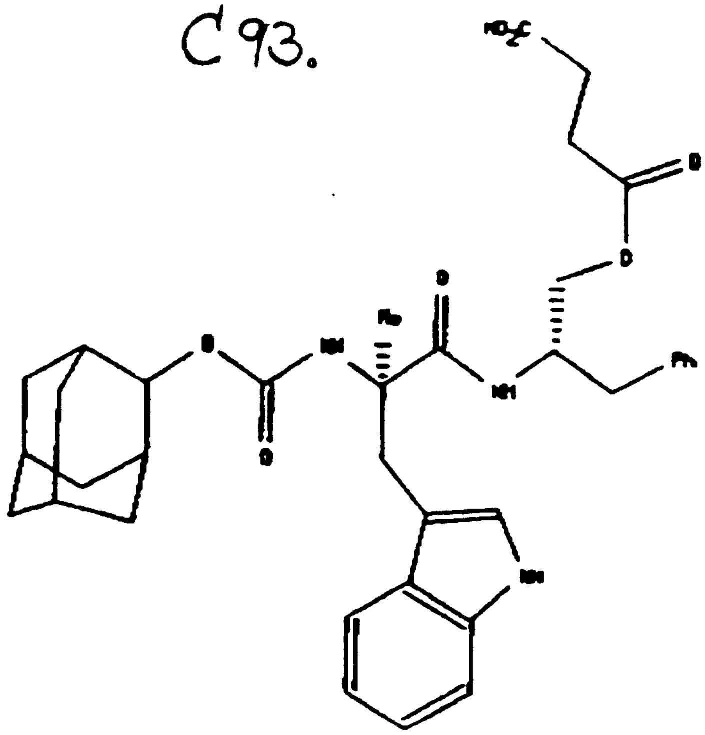

TABLE II CONTINUED

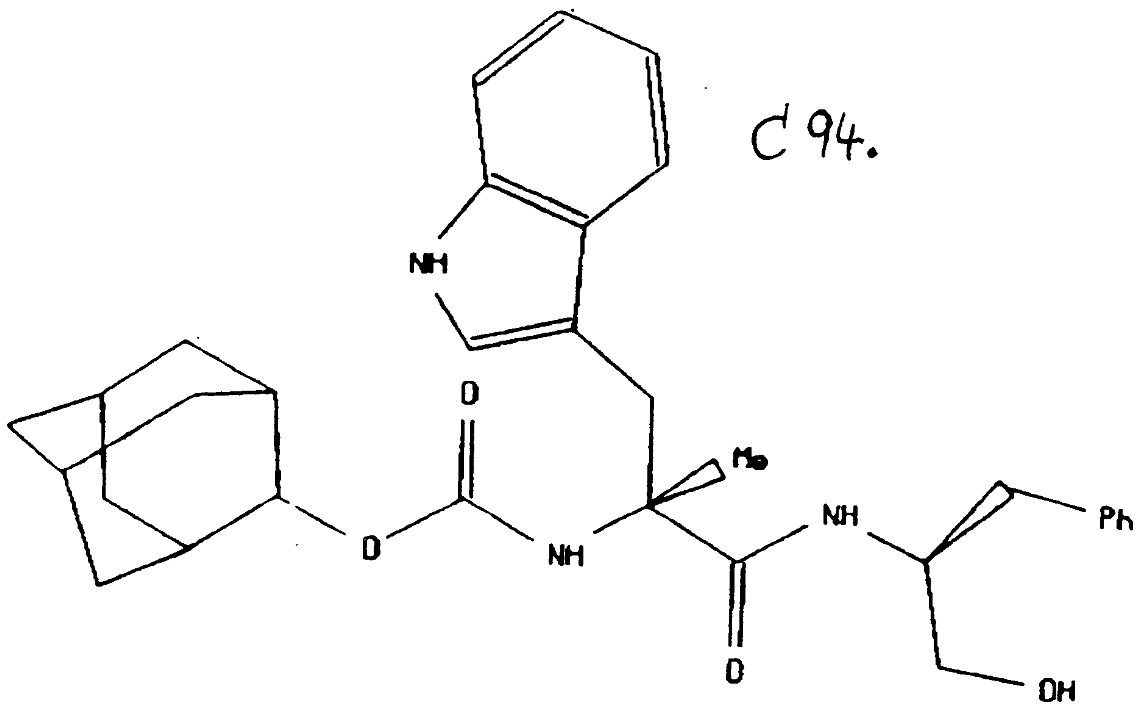

C 94.

## Claims

**1.** A compound of the formula

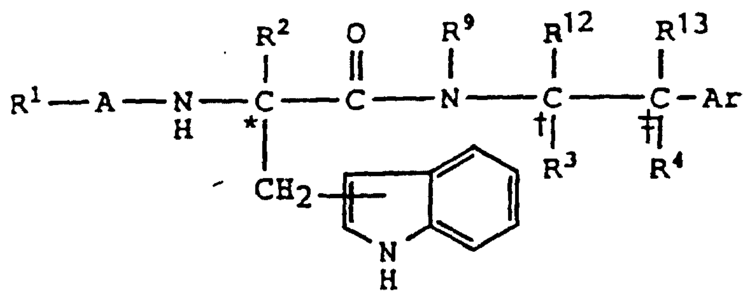

I

or a pharmaceutically acceptable salt thereof wherein:

$R^1$ is a cycloalkyl or polycycloalkyl hydrocarbon of from three to twelve carbon atoms with from zero to four substituents each independently selected from the group consisting of a straight or branched alkyl of from one

to six carbon atoms, halogen, CN, $OR^*$, $SR^*$, $CO_2R^*$,$CF_3$, $NR^5R^6$, and $-(CH_2)_nOR^5$ wherein $R^*$ is hydrogen or a straight or branched alkyl of from one to six carbon atoms, $R^5$ and $R^6$ are each independently hydrogen or alkyl of from one to six carbon atoms and n is an integer from zero to six;
A is $-(CH_2)_nCO-$,$-SO_2-$, -S(=O)-, -NHCO-,

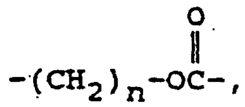

-SCO-, $-O-(CH_2)_nCO-$ or -HC=CHCO- wherein n is an integer from zero to six;
$R^2$ is a straight or branched alkyl of from one to six carbon atoms, $-HC=CH_2$, $-C\equiv CH$, $-CH_2-CH=CH_2$, $-CH_2C\equiv CH$, $-CH_2Ar$, $-CH_2OR^*$, $-CH_2OAr$, $-(CH_2)_nCO_2R^*$, or $-(CH_2)_nNR^5R^6$ wherein n, $R^*$, $R^5$ and $R^6$ are as defined above and Ar is as defined below;
$R^3$ and $R^4$ are each independently selected from hydrogen, $R^2$ and $-(CH_2)_{n'}-B-D$ wherein:
n' is an integer of from zero to three;
B is a bond,

$-OCO(CH_2)_n-$,

$-O(CH_2)_n-$,

$-NHCO(CH_2)_n-$,

$-CONH(CH_2)_n$

-NHCOCH=CH-,

$-COO(CH_2)_n-$,

$-CO(CH_2)_n-$,

$-S-(CH_2)_n-$,

$-S(=O)-(CH_2)_n-$,

$-SO_2-(CH_2)_n-$,

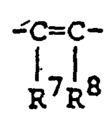

or

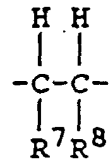

wherein $R^7$ and $R^8$ are independently selected from hydrogen and $R^2$ or together form a ring $(CH_2)_m$ wherein m is an integer of from 1 to 5 and n is as defined above;
D is

$-COOR^*$,

$-CONR^5R^6$,

-CN,

$-NR^5R^6$,

-OH,

-H and acid replacements selected from

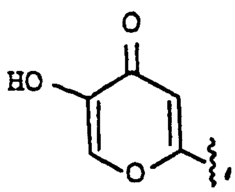,

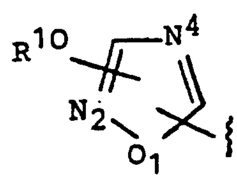

1,2,4oxadiazole

$R^{10}$ is OH, $NH_2$, $CH_3$ or Cl,

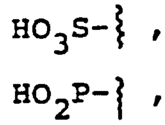

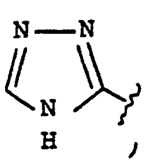,

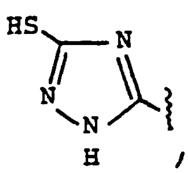,

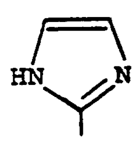,

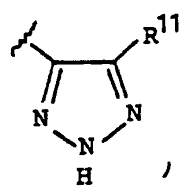,

$R^{11}$ is CN, $CO_2H$, $CF_3$,

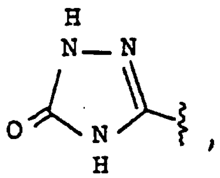 ,

$PhSO_2NHCO$- ,
$CF_3CONHCO$- ,
$CF_3SO_2NHCO$- ,
$H_2NSO$- ,

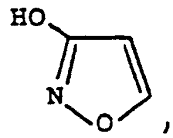 ,

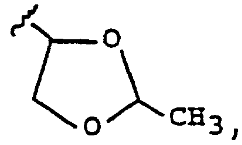 ,

$-CH_2OR^*$,

$-CHR^2OR^*$,

$-CH_2SR^*$,

$-CHR^2SR^*$,

wherein $R^*$, $R^2$, $R^5$, and $R^6$ are as defined above;
$R^9$ is hydrogen or a straight or branched alkyl of from one to about six carbon atoms, $-(CH_2)_nCO_2R^*$, $-(CH_2)_nOAr'$, $-(CH_2)_nAr'$or $(CH_2)_nNR^5R^6$,wherein n, $R^*$, $R^5$, and $R^6$ are as defined above or taken from $R^3$ and Ar' is taken from Ar as defined below;
$R^{12}$ and $R^{13}$ are each independently hydrogen or are each independently taken with $R^3$ and $R^4$ respectively to form a moiety doubly bonded to the carbon atom; and
Ar is 2- or 3-thienyl, 2- or 3-furanyl, 2-, 3- or 4-pyridinyl or an unsubstituted or substituted phenyl whose substituents if any are each independently hydrogen, fluorine, chlorine, bromine, iodine, methyl, methoxy trifluoromethyl or nitro.

**2.** A compound according to Claim 1 wherein the cycloalkyl or polycycloalkyl has from six to ten carbon atoms.

**3.** A compound according to Claim 1 wherein each substituent on the cycloalkyl or polycycloalkyl is independently methyl, F, Cl or Br.

**4.** A compound according to Claim 1 wherein the Polycycloalkyl is selected from the group consisting of

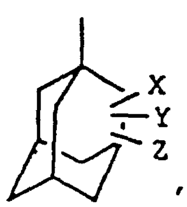 ,
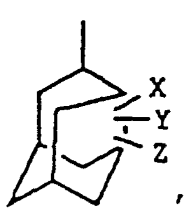 ,
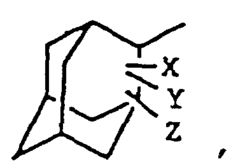 ,
and
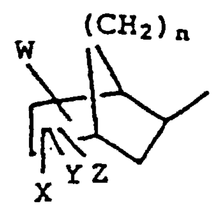

wherein W, X, Y, and Z are each independently hydrogen, a straight or branched alkyl of from one to six carbon atoms, $CF_3$, $NR^5R^6$, $-(CH_2)_nCO_2R^*$, CN, F, Cl, Br, $OR^*$, $SR^*$, wherein $R^*$, $R^5$ and $R^6$ are as defined in Claim 1 and n is an integer of from 1 to 3.

**5.** A compound according to Claim 1 wherein A is -NHCO-, OC(=O)-, $-SO_2$-, -S(=O)-, -SCO- or $-CH_2CO$-.

**6.** A compound according to Claim 1 wherein:

$R^1$ is 2-adamantyl or 1-(S)-2-endobornyl;
A is -NHCO-, -OCO-, $-SO_2-$, -S(=O)- or $-CH_2CO-$;
$R^2$ is $-CH_3$, $-CH_2CO_2H$ or $-CH_2C{\equiv}CH$;
$R^3$ is $-CH_2$-B-D or H;
$R^4$ is $-(CH_2)_n$-B-D or H;
$R^9$ is hydrogen or methyl.

**7.** A compound according to Claim 1 wherein:

$R^1$ is 2-adamantyl or 1-(S)-2-endobornyl;
A is -OC(=O)-;
$R^2$ is $-CH_3$;
$R^3$ is H, $CH_2OH$, $CH_2OCOCH_2CH_2CO_2H$, $CH_2OCOCH{=}CHCO_2H$, $CH_2NHCOCH_2CH_2CO_2H$, or $CH_2NH$-$COCH{=}CHCO_2H$
$R^4$ is H, $-NHCOCH_2CH_2CO_2H$ ([D] configuration) or NHCOCH=CHCO2H ([D] configuration).

**8.** A compound according to Claim 1 named
(±)-trans-2-chlorocyclohexyl[1-(1H-indol-3-ylmethyl)-1-methyl-2-oxo-2-[(2-phenylethyl)amino]ethyl]carbamate.

**9.** A compound according to Claim 1 named
2-chlorocyclohexyl[2-[[1-(hydroxymethyl)-2-phenylethyl]-amino]-1-(1H-indol-3-ylmethyl)-1-methyl-2-oxoethyl]-carbamate.

**10.** A compound according to Claim 1 named
2-[[2-[[[(2-chlorocyclohexyl)oxy]carbonyl]amino]-3-(1H-indol-3-yl)-2-methyl-1-oxopropyl]amino]-3-phenylpropyl butanedioate.

**11.** A compound according to Claim 1 named
2-[[2-[[[(2-methylcyclohexyl)oxy]carbonyl]amino]-3-(1H-indol-3-yl)-2-methyl-1-oxopropyl]amino]-3-phenylpropyl butanedioate.

**12.** A compound according to Claim 1 named
(±)-tricyclo[3.3.1.1$^{3,7}$]dec-2-yl [1-(1H-indol-3-ylmethyl)-1-methyl-2-oxo-2-[(2-phenylethyl)amino]-ethyl]carbamate.

**13.** A compound according to Claim 1 named
(+) or (-)-2-chlorocyclohexyl[1-(1H-indol-3-ylmethyl)-1-methyl-2-oxo-2-[(2-phenylethyl)amino]ethyl] carbamate.

**14.** A compound according to Claim 1 named
tricyclo[3.3.1.1$^{3,7}$]dec-2-yl [2-[[1-(hydroxymethyl)-2-phenylethyl]amino]-1-(1H-indol-3-ylmethyl)-1-methyl-2-oxoethyl] carbamate.

**15.** A compound according to Claim 1 named
2-[[3-(1H-indol-3-yl)-2-methyl-1-oxo-2-[[(tricyclo-[3.3.1.1$^{3,7}$]dec-2-yloxy)carbonyl]amino]propyl]amino]-3-phenylpropyl butanedioate.

**16.** A compound according to Claim 1 named
2-[[3-(1H-indol-3-yl)-2-methyl-1-oxo-2-[[(tricyclo-[3.3.1.1$^{3,7}$]dec-2-yloxy)carbonyl]amino]propyl]amino]-1-phenylethyl butanedioate.

**17.** A compound according to Claim 1 named
[R-(R*,R*)]-4-[[2-[[3-(1H-indol-3-yl)-2-methyl-1-oxo-2-[[(tricyclo[3.3.1.1$^{3,7}$]dec-2-yloxy)-carbonyl]amino]propyl]-amino]-1-phenylethyl]-amino]-4-oxobutanoic acid.

**18.** A compound according to Claim 1 named
[1S-[1α,2β[S*(S*)],4α]]-4-[[2-[[3-(1H-indol-3-yl)-2-methyl-1-oxo-2-[[[(1,7,7-trimethylbicyclo-2.2.1]hept-2-yl)amino] carbonyl]amino]propyl]amino]-1-phenylethyl]amino]-4-oxobutanoic acid.

**19.** A compound according to Claim 1 named

[R-(R*,S*)]-4-[[2-[[(1H-indol-3-yl)-2-methyl-1-oxo-2-[[(tricyclo[3.3.1.$1^{3,7}$]dec-2-yloxy)carbonyl]amino]-propyl]amino]-3-phenylpropyl]amino]-4-oxo-2-butenoic acid.

**20.** A compound according to Claim 1 named [R-(R*,S*)]-4-[[2-[[3-(1H-indol-3-yl)-2-methyl-1-oxo-2-[[(tricyclo[3.3.1.$1^{3,7}$]dec-2-yloxy)carbonyl]amino]-propyl] amino]-3-phenylpropyl]amino]-4-oxo-butanoic acid.

**21.** A compound according to Claim 1 named (R)-tricycle [3.3.1.$1^{3,7}$] dec-2-yl-[1-(1H-indol-3-ylmethyl)-1-methyl-2-[methyl-(2-phenylethyl)amino]-2-oxoethylcarbamate.

**22.** A compound according to Claim 1 named [R-(R*,S*)]-2-[[2-[[3-(1H-indol-3-yl)-2-methyl-1-oxo-2-[[(tricyclo[3.3.1.$1^{3,7}$]dec-2-yloxy)carbonyl]amino]-propyl] amino]-3-phenylpropyl]sulfinyl]acetic acid.

**23.** A compound according to Claim 1 named [R-(R*,S*)]-[[2-[[3-(1H-indol-3-yl)-2-methyl-1-oxo-2-[[(tricyclo[3.3.1.$1^{3,7}$]dec-2-yloxy)]carbonyl]amino]-propyl]amino]-3-phenylpropyl]sulfonyl]acetic acid.

**24.** A compound according to Claim 1 named [R-(R*,S*)]-[[2-[[3-(1H-indol-3-yl)-2-methyl-1-oxo-2-[[(tricyclo[3.3.1.$1^{3,7}$]dec-2-yloxy)carbonyl]amino]-propyl]amino]-3-phenylpropyl]sulfinyl]acetic acid or the ethyl ester thereof.

**25.** A compound according to Claim 1 named [R-(R*,S*)]-[[2-[[3-(1H-indol-3-yl)-2-methyl-1-oxo-2-[[(tricyclo[3.3.1.$1^{3,7}$]dec-2-yloxy),carbonyl]amino]-propyl]amino]-3-phenylpropyl]sulfonyl]acetic acid.

**26.** A compound according to Claim 1 named [R-[R*,R*-(E)]]-4-[[2-[[3-(1H-indol-3-yl)-2-methyl-1-oxo-2-[[(tricyclo[3.3.1.$1^{3,7}$]dec-2-yl oxy)carbonyl]amino]-propyl]amino]-1-phenylethyl]amino]-4-oxo-2-butenoic acid.

**27.** A compound according to Claim 1 named [R-(R*,S*)]-[[2-[2-[3-(1H-indol-3-yl)-2-methyl-1-oxo-2-[[(tricyclo(3.3.1.$1^{3,7}$]dec-2-yloxy)-carbonyl]amino]-propyl] amino]-3-phenylpropyl]thio]acetic acid.

**28.** A compound according to Claim 1 named [1S-[1α,2β[S*[S*(E)]],4α]]-4-[[2-[[3-(1H-indol-3-yl)-2-methyl-1-oxo-2-[[[(1,7,7-trimethylbicyclo[2.2.1]hept-2-yl)oxy] carbonyl]amino]propyl]amino]-1-phenylethyl]amino]-4-oxo-2-butenoic acid, methyl ester, (bicyclo system is 1S-endo).

**29.** A compound according to Claim 1 named [1S-[1α,2β[S*[S*(E)]],4α]]-4-[[2-[[3-(1H-indol-3-yl)-2-methyl-1-oxo-2-[[[(1,7,7-trimethylbicyclo[2.2.1]hept-2-yl)oxy) carbonyl]amino]propyl)amino)-l-phenylethyl]amino)-4-oxo-2-butenoic acid (bicyclo system is 1S-endo).

**30.** A compound according to Claim 1 named [R-(R*,R*)]-3-[[2-[[3-(1H-indol-3-yl)-2-methyl-1-oxo-2-[[(tricyclo[3.3.1.$1^{3,7}$]dec-2-yloxy)carbonyl]amino]-propyl] amino]-1-phenylethyl]amino]-3-oxopropanoic acid.

**31.** , A compound according to Claim 1 named [R-(R*,S*) ]-3-(1H-indol-3-ylmethyl)-3-methyl-4,10-dioxo-6-(phenylmethyl)-11-oxo-8-thia-2,5-diazatridecanoic acid, tricyclo[3.3.1.$1^{3,7}$]dec-2-yl or ester.

**32.** A compound according to Claim 1 named (R-(R*,S*)]-β-[[3-(1H-indol-3-yl)-2-methyl-1-oxo-2-[[(tricyclo[3.3.1.$1^{3,7}$]dec-2-yloxy)carbonyl]-amino]-propyl]amino]benzenebutanoic acid.

**33.** A compound according to Claim 1 named [R-(R*,S*)]-β-[[3-(1H-indol-3-yl)-2-methyl-1-oxo-2-[ [(tricyclo[3.3.1.$1^{3,7}$]dec-2-yloxy) carbonyl] -amino]-propyl]amino]-4-iodo-benzenebutanoic acid, where the iodo group may be I-125 or I-127.

**34.** A compound according to Claim 1 named [R-(R*,S*)]-N-[3-[[3-(1H-indol-3-yl)-2-methyl-1-oxo-2-[[(tricyclo[3.3.1.1$^{3,7}$]dec-2-yloxy)carbonyl]amino]-propyl] amino]-4-phenylbutyl]glycine.

**35.** A compound according to Claim 1 named [R-[R*,S*-(E)]]-4-[[2-[[3-(1H-indol-3-yl)-2-methyl-2-[[(bicyclo[3.3.1]non-9-yloxy)carbonyl]amino]-1-oxopropyl]amino]-3-phenylpropyl]amino]-4-oxo-2-butenoic acid.

**36.** A pharmaceutical composition comprising a compound according to Claims 1 to 5 and a pharmaceutically acceptable carrier.

**37.** Use of a compound according to Claims 1 to 5 for preparing a pharmaceutical composition useful in suppressing appetite in a mammal.

**38.** Use of a compound according to Claims 1 to 5 for preparing a pharmaceutical composition useful in reducing gastric secretion in a mammal.

**39.** Use of a compound according to Claims 1 to 5 for preparing a pharmaceutical composition useful in reducing anxiety in a mammal.

**40.** A compound of formula:

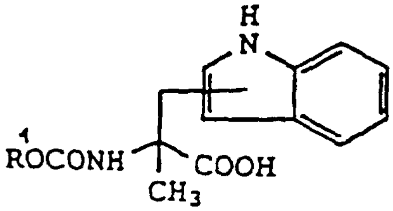

wherein R$^1$ is as defined in Claim 1.

**41.** A compound of formula:

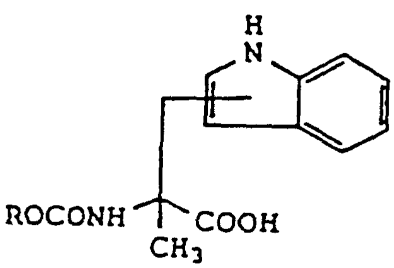

(II)

wherein R is 1-adamantyl, 2-adamantyl, 4-protoadamantyl, 9-fluorenylmethyl, exo-bornyl, endo-bornyl, exo-norbornyl, endo-norbornyl, 2-chlorocyclohexyl, 2-methylcyclohexyl, or camphoryl.

**42.** A process for preparing a compound according to Claim 1 , comprising reacting a compound of formula

ROH (III)

with a phosgene or phosgene substitute to produce a compound of formula

ROCOCl (IV)

and reacting a compound of formula IV with [D]-α-methyltryptophan to produce a compound of Claim 41.

**43.** A process for preparing a compound of formula

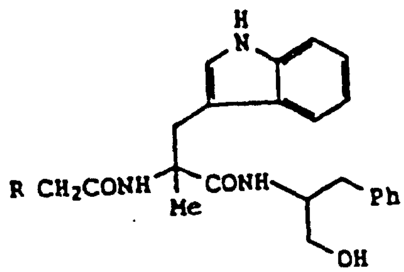

wherein R is 1-adamantyl, 2-adamantyl, 4-protoadamantyl, 9-fluorenylmethyl, exo-bornyl, endo-bornyl, exo-norbornyl, endo-norbornyl, 2-chlorocyclohexyl, 2-methylcyclohexyl, or camphoryl, comprising reacting a free amine of

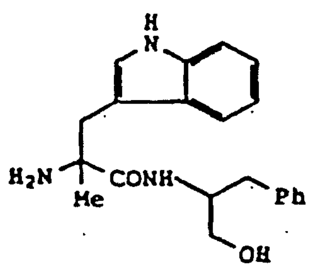

with a substituted acetylchloride

$RCH_2COCl$

to form a compound of formula I and converting it, if desired, to a pharmaceutically acceptable salt.

**44.** A process for preparing a sulphonamide

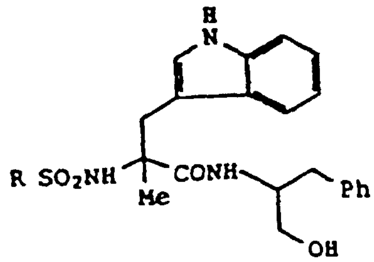

wherein R is 1-adamantyl, 2-adamantyl, 4-protoadamantyl, 9-fluorenylmethyl, exo-bornyl, endo-bornyl, exo-norbornyl, endo-norbornyl, 2-chlorocyclohexyl, 2-methylcyclohexyl, or camphoryl, comprising reacting a free amine

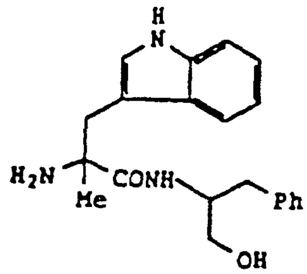

with a substituted sulphonylchloride

$$RSO_2Cl$$

to form a compound of formula I and converting it, if desired, to a pharmaceutically acceptable salt.

**45.** A process for preparing a compound of formula

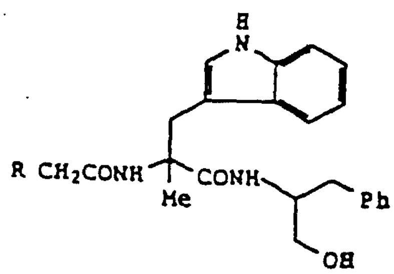

wherein R is 1-adamantyl, 2-adamantyl, 4-protoadamantyl, 9-fluorenylmethyl, exo-bornyl, endo-bornyl, exo-norbornyl, endo-norbornyl, 2-chlorocyclohexyl, 2-methylcyclohexyl, or camphoryl, comprising reacting a free amine

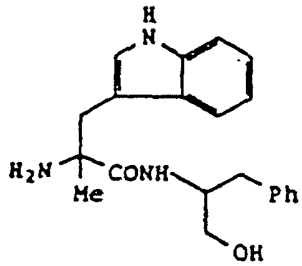

with a substituted isocyanate

R-N=C=O

to form a compound of formula I and converting it, if desired, to a pharmaceutically acceptable salt.

**46.** Use of a compound according to Claims 1 to 35 for preparing a pharmaceutical composition useful for treating grastrointestinal ulcer in a mammal.

**47.** Use of a compound according to Claims 1 to 35 for preparing a pharmaceutical composition useful for treating psychotic behaviour in a mammal.

**48.** Use of a compound according to Claims 1 to 35 for preparing a pharmaceutical composition useful for treating psychosis in a mammal.

**49.** Use of a compound according to Claims 1 to 35 for preparing a pharmaceutical composition useful to block the reaction caused by withdrawal from drug or alcohol use in a mammal.

**50.** Use of a compound according to Claims 1 to 35 for preparing a pharmaceutical composition useful for blocking or treating drug or alcohol withdrawal reaction in a mammal.

**51.** Use of a compound according to Claims 1 to 35 for preparing a pharmaceutical composition useful for treating reaction from cocaine withdrawal in a mammal,

**52.** Use of a compound according to Claims 1 to 35 for preparing a pharmaceutical composition useful for treating reaction from benzodiazepine withdrawal in a mammal.

**53.** Use of a compound according to Claims 1 to 35 for preparing a pharmaceutical composition useful for treating reaction from diazepam withdrawal in a mammal.

**54.** Use of a compound according to Claims 1 to 35 for preparing a pharmaceutical composition useful for treating reaction from nicotine withdrawal in a mammal.

**55.** Use of a compound according to Claims 1 to 35 for preparing a pharmaceutical composition useful to potentiate the effects of morphine and other opioids in treating pain.

**56.** Use of a compound according to Claims 1 to 35 for preparing a pharmaceutical composition useful for trating pain in a mammal.

**57.** A process for the preparation of a compound of formula I according to claims 1 to 36 which comprises condensing a compound of formula

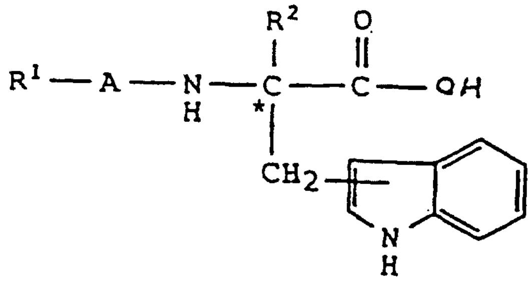

with an appropriate amine of formula

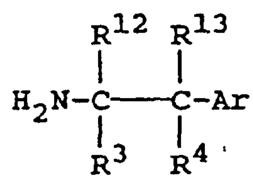

using a suitable condensing agent and a suitable solvent at a temperature of from about 20°C to about 80°C.

**58.** Method of use of a radioactive iodo compound of formula I according to claims 1 to 36 to prepare a pharmaceutical or diagnostic composition for the treatment or diagnosis of gastrin dependent tumors.

**59.** A compound named Tricyclo [3.3.1.1$^{3,7}$]dec-2-yl[R,(R*,S*]-[1-(1H-indol-3-ylmethyl)-1-methyl-2-axo-2-[[2-[[1-oxo-3-(1H-tetrazol-5-yl) propyl]amino]-2-phenylethyl]-amino]ethyl]carbamic acid ester.

**60.** A compound named carbamic acid, [1-(1H-indol-3-ylmethyl)-1-methyl-2-oxo-2-[[2-[[2-oxo-3-(1H-tetrazol-5-yl)propyl]amino]-2-phenylethyl]-amino]ethyl]-,tricyclo[3.3.1.$^{3,7}$]dec-2-yl ester, [R,(R*,S*]-

**61.** A compound of the formula

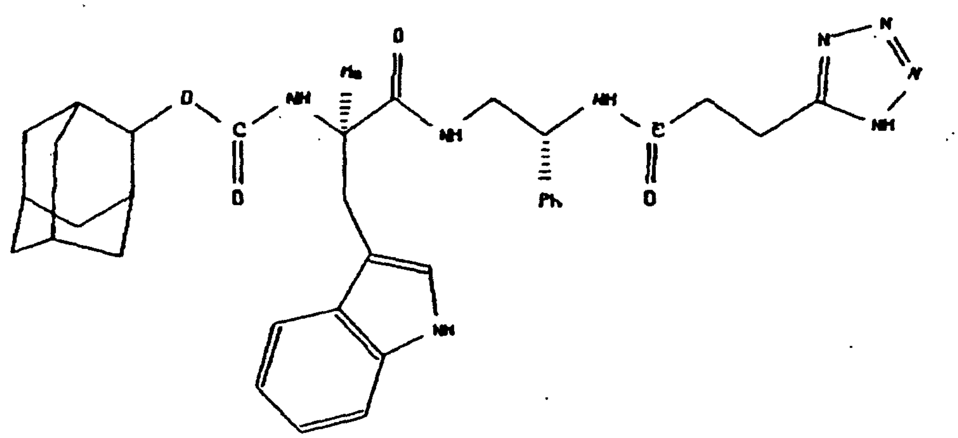

**62.** A compound of the formula

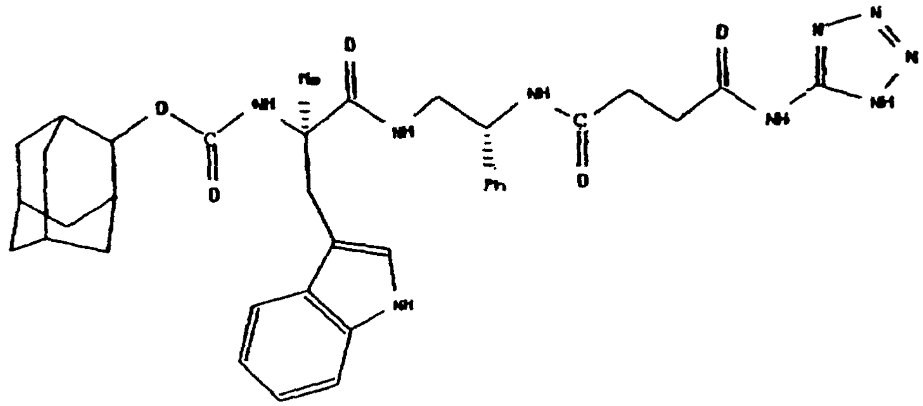

**Patentansprüche**

1. Verbindung der Formel

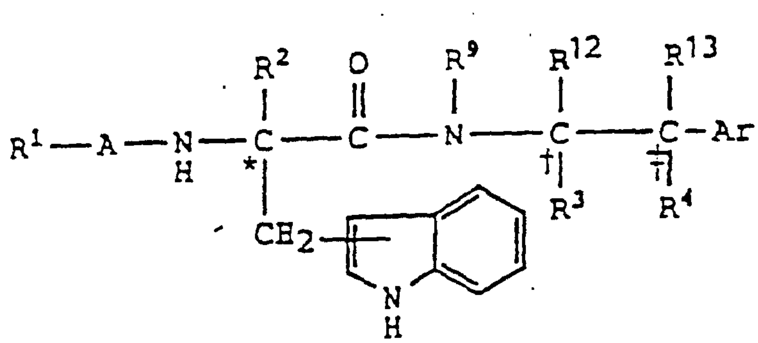

I

oder ein pharmazeutisch akzeptables Salz derselben, worin:

$R^1$ einen Cycloalkyl- oder Polycycloalkylkohlenwasserstoff mit 3 bis 12 Kohlenstoffatomen mit 0 bis 4 Substituenten, die jeweils unabhängig voneinander aus der Gruppe von einem geradkettigen oder verzweigten Alkyl mit 1 bis 6 Kohlenstoffatomen, einem Halogen, CN, OR*, SR*, $CO_2R^*$, $CF_3$, $NR^5R^6$ und $-(CH_2)_nOR^5$, wobei R* Wasserstoff oder ein geradkettiges oder verzweigtes Alkyl mit 1 bis 6 Kohlenstoffatomen ist, $R^5$ und $R^6$ jeweils unabhängig voneinander Wasserstoff oder Alkyl mit 1 bis 6 Kohlenstoffatomen sind und n eine ganze Zahl von 0 bis 6 ist, ausgewählt sind, bedeutet;

A-$(CH_2)_nCO$-, $-SO_2$-, -S (=O) -, -NHCO-,

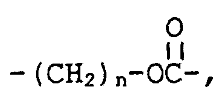

-SCO-, $-O-(CH_2)_nCO$- oder -HC=CHCO-, wobei n eine ganze Zahl von 0 bis 6 ist, bedeutet;

$R^2$ ein geradkettiges oder verzweigtes Alkyl mit 1 bis 6 Kohlenstoffatomen, $-HC=CH_2$, -C=CH, $-CH_2-CH=CH_2$, $-CH_2C\equiv CH$, $-CH_2Ar$, $-CH_2OR^*$, $-CH_2OAr$, $-(CH_2)_nCO_2R^*$ oder $-(CH_2)_nNR^5R^6$, wobei n, R*, $R^5$ und $R^6$ wie im Vorhergehenden definiert sind und Ar wie im Folgenden definiert ist, bedeutet;

$R^3$ und $R^4$ jeweils unabhängig voneinander aus Wasserstoff, $R^2$ und $-(CH_2)_n$,-B-D ausgewählt sind, wobei

n' eine ganze Zahl von 0 bis 3 ist,

B für eine Bindung,

$$-OCO(CH_2)_n.$$

$$-O(CH_2)_n-,$$

$$-NHCO(CH_2)_n-,$$

$$-CONH(CH_2)_n-,$$

$$-NHCOCH=CH-,$$

$$-COO(CH_2)_n-,$$

$$-CO(CH_2)_n-,$$

$$-S-(CH_2)_n-,$$

$$-S(=O)-(CH_2)_n-,$$

$$-SO_2-(CH_2)_n-,$$

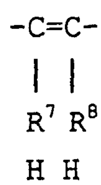

oder

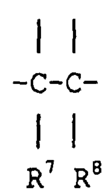

steht,
wobei $R^7$ und $R^8$ unabhängig voneinander aus Wasserstoff und $R^2$ ausgewählt sind oder zusammen einen Ring $(CH_2)_m$,
wobei m eine ganze Zahl von 1 bis 5 ist, bilden und n wie im Vorhergehenden definiert ist;

D für

$-COOR^*$,

$-CONR^5R^6$,

-CN,

$-NR^5R^6$,

-OH,

-H und Säuresubstitutionen, die ausgewählt sind aus

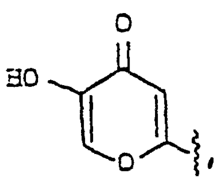,
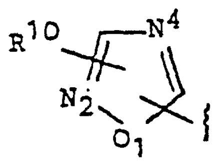

1,2,4-Oxadiazol

$R^{10}$ ist OH, $NH_2$, $CH_3$ oder Cl

$HO_3S-$ ,

$HO_2P-$ ,

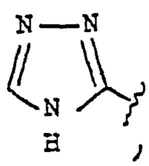, 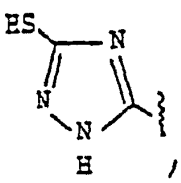, 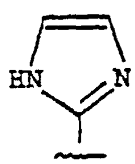, 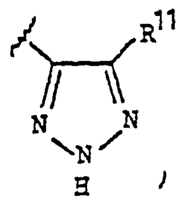, $R^{11}$ ist CN, $CO_2H$, $CF_3$

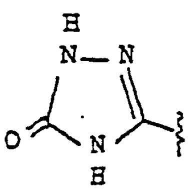,

$PhSO_2NHCO-$,

$CF_3CONHCO-$,

$CF_3SO_2NHCO-$,

$H_2NSO-$,

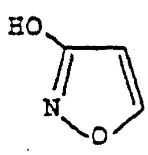, 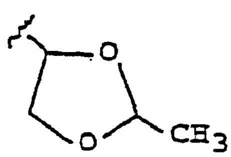,

$-CH_2OR^*$ ,

$-CHR^2OR^*$,

$-CH_2SR^*$,

$-CHR^2SR^*$

steht,
wobei $R^*$, $R^2$, $R^5$ und $R^6$ wie im Vorhergehenden definiert sind;

$R^9$ Wasserstoff oder ein geradkettiges oder verzweigtes Alkyl mit 1 bis etwa 6 Kohlenstoffatomen, $-(CH_2)_nCO_2R^*$, $-(CH_2)_nOAr'$ , $-(CH_2)_nAr'$ oder $(CH_2)_nNR^5R^6$, wobei n, $R^*$, $R^5$ und $R^6$ wie im Vorhergehenden definiert sind oder von $R^3$ übernommen sind und Ar' von dem im Folgenden definierten Ar übernommen ist, bedeutet;

$R^{12}$ und $R^{13}$ jeweils unabhängig voneinander Wasserstoff bedeuten oder jeweils unabhängig voneinander zusammengenommen mit $R^3$ bzw. $R^4$ eine über eine Doppelbindung an das Kohlenstoffatom gebundene Einheit bilden; und

Ar 2- oder 3-Thienyl, 2- oder 3-Furanyl, 2-, 3- oder 4-Pyridinyl oder ein unsubstituiertes oder substituiertes Phenyl, dessen Substituenten gegebenenfalls jeweils unabhängig voneinander Wasserstoff, Fluor, Chlor, Brom, Iod, Methyl, Methoxy, Trifluormethyl oder Nitro sind, bedeutet.

**2.** Verbindung gemäß Anspruch 1, wobei das Cycloalkyl oder Polycycloalkyl 6 bis 10 Kohlenstoffatome aufweist.

**3.** Verbindung gemäß Anspruch 1, wobei jeder Substituent an dem Cycloalkyl oder Polycycloalkyl unabhängig voneinander Methyl, F, Cl oder Br ist.

**4.** Verbindung gemäß Anspruch 1, wobei das Polycycloalkyl ausgewählt ist aus der Gruppe von

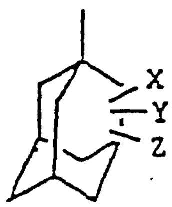 , 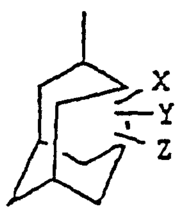 , 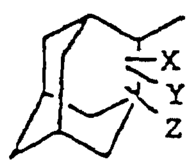 , und 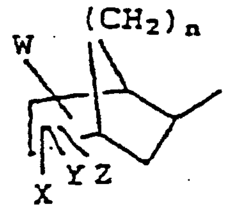

worin W, X, Y und Z jeweils unabhängig voneinander Wasserstoff, ein geradkettiges oder verzweigtes Alkyl mit 1 bis 6 Kohlenstoffatomen, $CF_3$, $NR^5R^6$, $-(CH_2)_nCO_2R^*$, CN, F, Cl, Br, $OR^*$, $SR^*$, wobei $R^*$, $R^5$ und $R^6$ wie in Anspruch 1 definiert sind und n eine ganze Zahl von 1 bis 3 ist, bedeuten.

**5.** Verbindung gemäß Anspruch 1, worin A -NHCO-, OC(=O)-, $-SO_2-$, -S (=O-) -, -SCO- oder $-CH_2CO-$ bedeutet.

**6.** Verbindung gemäß Anspruch 1, worin:

$R^1$ 2-Adamantyl oder 1-(S)-2-endo-Bornyl bedeutet;
A -NHCO-, -OCO-, $-SO_2-$, -S (=O) - oder $-CH_2CO-$ bedeutet;
$R^2$ $-CH_3$, $-CH_2CO_2H$ oder $-CH_2C{=}CH$ bedeutet;
$R^3$ $-CH_2$-B-D oder H bedeutet;
$R^4$ $-(CH_2)_n$-B-D oder H bedeutet;
$R^9$ Wasserstoff oder Methyl bedeutet.

**7.** Verbindung gemäß Anspruch 1, worin:

$R^1$ 2-Adamantyl oder 1-(S)-2-endo-Bornyl bedeutet;
A -OC(=O)- bedeutet;
$R^2$ -CH3 bedeutet;
$R^3$ H, $CH_2OH$ $CH_2OCOCH_2CH_2CO_2H$, $CH_2OCOCH{=}CHCO_2H$, $CH_2NHCOCH_2CH_2CO_2H$ oder $CH_2NHCOCH{=}CHCO_2H$ bedeutet;
$R^4$ H, $-NHCOCH_2CH_2CO_2H$ (D-Konfiguration) oder $NHCOCH{=}CHCO_2H$ (D-Konfiguration) bedeutet.

**8.** Verbindung gemäß Anspruch 1 mit dem Namen (±)-trans-2-Chlorcyclohexyl-[1-(1H-indol-3-ylmethyl)-1-methyl-2-oxo-2-[(2-phenylethyl)amino]ethyl]carbamat.

**9.** Verbindung gemäß Anspruch 1 mit dem Namen 2-Chlorcyclohexyl-[2-[[1-(hydroxymethyl)-2-phenylethyl]-amino]-1-(1H-indol-3-ylmethyl)-1-methyl-2-oxoethyl]-carbamat.

**10.** Verbindung gemäß Anspruch 1 mit dem Namen 2-[[2-[[[(2-Chlorcyclohexyl)oxy)carbonyl]amino]-3-(1Hindol-3-yl)-2-methyl-1-oxopropyl)amino]-3-phenylpropylbutandioat.

**11.** Verbindung gemäß Anspruch 1 mit dem Namen 2-[[2-[[[(2-Methylcyclohexyl)oxy]carbonyl]amino]-3-(1Hindol-3-yl)-2-methyl-1-oxopropyl)amino]-3-phenylpropyl-butandioat.

**12.** Verbindung gemäß Anspruch 1 mit dem Namen (±)-Tricyclo[3.3.1.1$^{3,7}$]dec-2-yl-[1-(1H-indol-3-ylmethyl)-1-methyl-2-oxo-2-[(2-phenylethyl)amino]ethyl]carbamat.

**13.** Verbindung gemäß Anspruch 1 mit dem Namen (+)- oder (-)-2-Chlorcyclohexyl-[1-(1H-indol-3-ylmethyl)-1-methyl-2-oxo-2-[(2-phenylethyl)amino]ethyl]carbamat.

**14.** Verbindung gemäß Anspruch 1 mit dem Namen Tricyclo(3.3.1.1$^{3,7}$]dec-2-yl-[2-[[1-(hydroxymethyl)-2-phenylethyl]amino]-1-(1H-indol-3-ylmethyl)-1-methyl-2-oxoethylcarbamat

**15.** Verbindung gemäß Anspruch 1 mit dem Namen 2-[[3-(1H-Indol-3-yl)-2-methyl-1-oxo-2-[[(tricyclo-[3.3.1.1$^{3,7}$]dec-2-yloxy)carbonyl]amino]propyl]amino]-3-phenylpropyl-butandioat.

**16.** Verbindung gemäß Anspruch 1 mit dem Namen 2-[[3-(1H-Indol-3-yl)-2-methyl-1-oxo-2-[[(tricyclo-[3.3. 1.1$^{3,7}$]dec-2-yloxy)carbonyl]amino]propyl]amino]-1-phenylethyl-butandioat.

**17.** Verbindung gemäß Anspruch 1 mit dem Namen [R-(R*,R*)]-4-[[2-[[3-(1H-Indol-3-yl)-2-methyl-1-oxo-2-[[(tricyclo[3.3. 1.1$^{3,7}$]dec-2-yloxy)carbonyl]amino]propyl]-amino]-1-phenylethyl]-amino]-4-oxobutansäure.

**18.** Verbindung gemäß Anspruch 1 mit dem Namen [1S-[1α,2β[S*(S*)],4α]]-4-[[2-([3-(1H-Indol-3-yl)-2-methyl-1-oxo-2-[[[(1,7,7-trimethylbicyclo-2.2.1]hept-2-yl)amino]carbonyl]amino]propyl]amino]-1-phenylethyl]-amino]-4-oxobutansäure.

**19.** Verbindung gemäß Anspruch 1 mit dem Namen (R-(R*,S*)]-4-[[2-[[(1H-Indol-3-yl)-2-methyl-1-oxo-2-[[(tricyclo[3.3.1.1$^{3,7}$]dec-2-yloxy)carbonyl]amino]propyl]-amino]-3-phenylpropyl]amino]-4-oxo-2-butensäure.

**20.** Verbindung gemäß Anspruch 1 mit dem Namen [R-(R*,S*)]-4-[[2-[[3-(1H-Indol-3-yl)-2-methyl-1-oxo-2-[[(tricyclo[3.3.1.1$^{3,7}$]dec-2-yloxy)carbonyl]amino]propyl]-amino]-3-phenylpropyl]amino]-4-oxo-butansäure.

**21.** Verbindung gemäß Anspruch 1 mit dem Namen (R)-Tricyclo[3.3.1 .1$^{3,7}$]dec-2-yl-[1-(1H-indol-3-ylmethyl)-1-methyl-2-[methyl-(2-phenylethyl)amino]-2-oxoethyl-carbamat.

**22.** Verbindung gemäß Anspruch 1 mit dem Namen [R-(R*,S*)]-2-([2-[[3-(1H-Indol-3-yl)-2-methyl-1-oxo-2-[[(tricyclo(3.3.1.1$^{3,7}$]dec-2-yloxy)carbonyl)amino]propyl]-amino]-3-phenylpropyl]sulfinyl]essigsäure.

**23.** Verbindung gemäß Anspruch 1 mit dem Namen

[R-(R*,S*)]-[[2-[[3-(1H-Indol-3-yl)-2-methyl-1-oxo-2-[[(tricyclo[3.3.1.1$^{3,7}$]dec-2-yloxy)carbonyl]aminolpropyl]-amino]-3-phenylpropyl]sulfonyl]essigsäure.

**24.** Verbindung gemäß Anspruch 1 mit dem Namen [R-(R*,S*)]-[[2-[[3-(1H-Indol-3-yl)-2-methyl-1-oxo-2-[[(tricyclo[3.3.1.1$^{3,7}$]dec-2-yloxy)carbonyl]amino]propyl]-amino]-3-phenylpropyl]sulfinyl]essigsäure oder der Ethylester derselben.

**25.** Verbindung gemäß Anspruch 1 mit dem Namen [R-(R*,S*)]-[[2-[[3-(1H-Indol-3-yl)-2-methyl-1-oxo-2-[[(tricyclo[3.3.1.1$^{3,7}$]dec-2-yloxy)carbonyl]amino]propyl]-amino]-3-phenylpropyl]sulfonyl]essigsäure.

**26.** Verbindung gemäß Anspruch 1 mit dem Namen [R-[R*,R*-(E)]]-4-[[2-[[3-(1H-Indol-3-yl)-2-methyl-1-oxo-2-[[(tricyclo[3.3.1.1$^{3,7}$]dec-2-yloxy)carbonyl]amino]-propyl]amino]-1-phenylethyl]amino]-4-oxo-2-butensäure.

**27.** Verbindung gemäß Anspruch 1 mit dem Namen [R-(R*,S*)]-[[2-[2-[3-(1H-Indol-3-yl)-2-methyl-1-oxo-2-[[(tricyclo[3.3.1.1$^{3,7}$]dec-2-yloxy)carbonyl]amino]propyl]-amino]-3-phenylpropyl]thio]essigsäure.

**28.** Verbindung gemäß Anspruch 1 mit dem Namen [1S-[1α,2β[S* (E)]],4α]]-4-[ [2-[[3-(1H-Indol-3-yl)-2-methyl-1-oxo-2-[[[(1,7,7-trimethylbicyclo(2.2.1]hept-2-yl)oxy] carbonyl]amino]propyl]amino]-1-phenylethyl]-amino]-4-oxo-2-butensäure-methylester (Das Bicyclosystem ist 1S-endo).

**29.** Verbindung gemäß Anspruch 1 mit dem Namen [1S-[1α, 2β [S* [S* (E) ]], 4α]]-4-[[2-[[3-(1H-Indol-3-yl) -2-methyl-1-oxo-2-[[[(2,7,7-trimethylbicyclo[2.2.1]hept-2-yl) oxy]carbonyl]amino]propyl]amino]-1-phenylethyl]-amino]-4-oxo-2-butensäure (Das Bicyclosystem ist 1S-endo).

**30.** Verbindung gemäß Anspruch 1 mit dem Namen [R-(R*,R*)]-3-[[2-[[3-(1H-Indol-3-yl)-2-methyl-1-oxo-2-[[(tricyclo[3.3.1.1$^{3,7}$]dec-2-yloxy)carbonyl]amino]propyl]-amino]-1-phenylethyl]amino]-3-oxopropansäure.

**31.** Verbindung gemäß Anspruch 1 mit dem Namen [R-(R*,S*)]-3-(1H-Indol-3-ylmethyl)-3-methyl-4,10-dioxo-6-(phenylmethyl)-11-oxo-8-thia-2,5-diazatridecansäure, Tricyclo[3.3.1.1$^{3,7}$]dec-2-yl- oder Ester

**32.** Verbindung gemäß Anspruch 1 mit dem Namen [R-(R*,S*)]-β-[[3-(1H-Indol-3-yl)-2-methyl-1-oxo-2-[[ (tricyclo[3.3.1.1$^{3,7}$]dec-2-yloxy)carbonyl]amino]propyl]-amino]benzolbutansäure.

**33.** Verbindung gemäß Anspruch 1 mit dem Namen [R-(R*,S*)]-β-[[3-(1H-Indol-3-yl)-2-methyl-1-oxo-2-[[(tricyclo[3.3.1.1$^{3,7}$]dec-2-yloxy) carbonyl]amino]propyl]-amino]-4-iod-benzolbutansäure, wobei die Iodgruppe I-125 oder I-127 sein kann.

**34.** Verbindung gemäß Anspruch 1 mit dem Namen [R-(R*,S*)]-N-[3-[[3-(1H-Indol-3-yl)-2-methyl-1-oxo-2-[[(tricyclo[3.3.1.1$^{3,7}$]dec-2-yloxy)carbonyl]amino]propyl]-amino]-4-phenylbutyl]glycin.

**35.** Verbindung gemäß Anspruch 1 mit dem Namen [R-[R*,S*-(E)]]-4-[[2-[[3-(1H-Indol-3-yl)-2-methyl-2-[[(bicyclo[3.3.1]non-9-yloxy)carbonyl]amino]-1-oxopropyl]amino]-3-phenylpropyl]amino]-4-oxo-2-butensäure.

**36.** Pharmazeutische Zusammensetzung, die eine Verbindung gemäß den Ansprüchen 1 bis 5 und einen pharmazeutisch akzeptablen Träger umfasst.

**37.** Verwendung einer Verbindung gemäß den Ansprüchen 1 bis 5 zur Herstellung einer pharmazeutischen Zusammensetzung, die zur Appetitzügelung bei einem Säuger verwendbar ist.

**38.** Verwendung einer Verbindung gemäß den Ansprüchen 1 bis 5 zur Herstellung einer pharmazeutischen Zusammensetzung, die zur Verringerung der Magensaftsekretion bei einem Säuger verwendbar ist.

**39.** Verwendung einer Verbindung gemäß den Ansprüchen 1 bis 5 zur Herstellung einer pharmazeutischen Zusammensetzung, die zur Verringerung von Angst bei einem Säuger verwendbar ist.

**40.** Verbindung der Formel

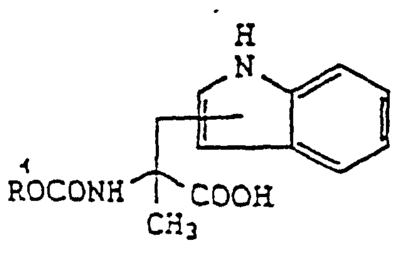

worin $R^1$ wie in Anspruch 1 definiert ist.

**41.** Verbindung der Formel

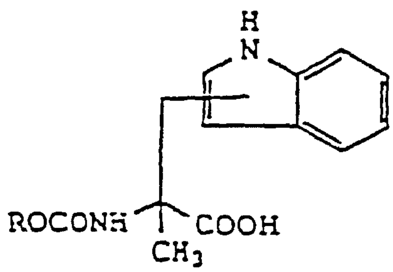

(II)

worin R 1-Adamantyl, 2-Adamantyl, 4-Protoadamantyl, 9-Fluorenylmethyl, exo-Bornyl, endo-Bornyl, exo-Norbornyl, endo-Norbornyl, 2-Chlorcyclohexyl, 2-Methylcyclohexyl oder Campheryl bedeutet.

**42.** Verfahren zur Herstellung einer Verbindung gemäß Anspruch 1, das die Reaktion einer Verbindung der Formel

ROH (III)

mit einem Phosgen oder Phosgenersatzstoff unter Bildung einer Verbindung der Formel

ROCOCl (IV)

und die Reaktion einer Verbindung der Formel IV mit [D]-α-Methyltryptophan unter Bildung einer Verbindung nach Anspruch 41 umfasst.

**43.** Verfahren zur Herstellung einer Verbindung der Formel

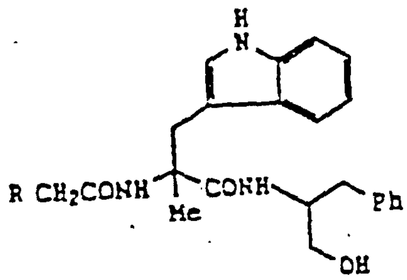

worin R 1-Adamantyl, 2-Adamantyl, 4-Protoadamantyl, 9-Fluorenylmethyl, exo-Bornyl, endo-Bornyl, exo-Norbornyl, endo-Norbornyl, 2-Chlorcyclohexyl, 2-Methylcyclohexyl oder Campheryl bedeutet, das die Reaktion eines freien Amins der Formel

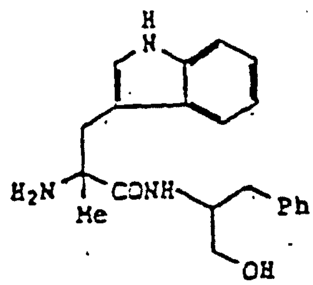

mit einem substituierten Acetylchlorid

$$RCH_2COCl$$

unter Bildung einer Verbindung der Formel I und die Umwandlung derselben, falls gewünscht, in ein pharmazeutisch akzeptables Salz umfasst.

**44.** Verfahren zur Herstellung eines Sulfonamids der Formel

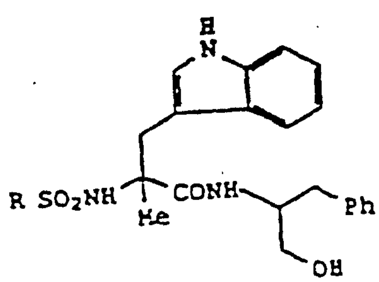

worin R 1-Adamantyl, 2-Adamantyl, 4-Protoadamantyl, 9-Fluorenylmethyl, exo-Bornyl, endo-Bornyl, exo-Norbornyl, endo-Norbornyl, 2-Chlorcyclohexyl, 2-Methylcyclohexyl oder Campheryl bedeutet, das die Reaktion eines freien Amins der Formel

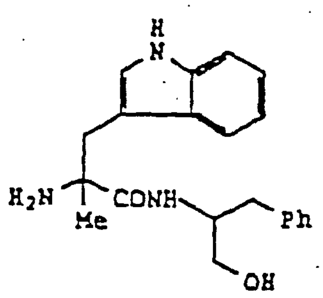

mit einem substituierten Sulfonylchlorid

$$RSO_2Cl$$

unter Bildung einer Verbindung der Formel I und die Umwandlung derselben, falls gewünscht, in ein pharmazeutisch akzeptables Salz umfasst.

**45.** Verfahren zur Herstellung einer Verbindung der Formel

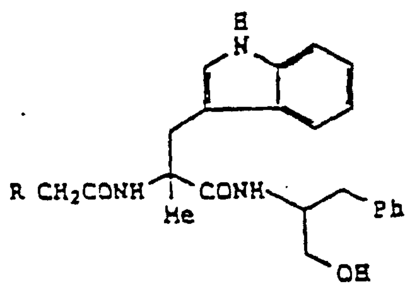

worin R 1-Adamantyl, 2-Adamantyl, 4-Protoadamantyl, 9-Fluorenylmethyl, exo-Bornyl, endo-Bornyl, exo-Norbornyl, endo-Norbornyl, 2-Chlorcyclohexyl, 2-Methylcyclohexyl oder Campheryl bedeutet, das die Reaktion eines freien Amins der Formel

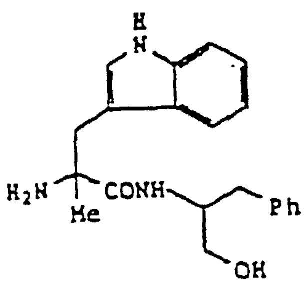

mit einem substituierten Isocyanat

$$R\text{-}N{=}C{=}O$$

unter Bildung einer Verbindung der Formel I und die Umwandlung derselben, falls gewünscht, in ein pharmazeutisch akzeptables Salz umfasst.

**46.** Verwendung einer Verbindung gemäß den Ansprüchen 1 bis 35 zur Herstellung einer pharmazeutischen Zusammensetzung, die zur Behandlung von Magen-Darm-Geschwüren bei einem Säuger verwendbar ist.

**47.** Verwendung einer Verbindung gemäß den Ansprüchen 1 bis 35 zur Herstellung einer pharmazeutischen Zusammensetzung, die zur Behandlung von psychotischem Verhalten bei einem Säuger verwendbar ist.

**48.** Verwendung einer Verbindung gemäß den Ansprüchen 1 bis 35 zur Herstellung einer pharmazeutischen Zusammensetzung, die zur Behandlung einer Psychose bei einem Säuger verwendbar ist.

**49.** Verwendung einer Verbindung gemäß den Ansprüchen 1 bis 35 zur Herstellung einer pharmazeutischen Zusammensetzung, die zur Blockierung der durch Drogenentzug oder einen Entzug nach Alkoholkonsum verursachten Reaktion bei einem Säuger verwendbar ist.

**50.** Verwendung einer Verbindung gemäß den Ansprüchen 1 bis 35 zur Herstellung einer pharmazeutischen Zusammensetzung, die zur Blockierung oder Behandlung einer Drogen- oder Alkoholentzugsreaktion bei einem Säuger verwendbar ist.

**51.** Verwendung einer Verbindung gemäß den Ansprüchen 1 bis 35 zur Herstellung einer pharmazeutischen Zusammensetzung, die zur Behandlung einer Reaktion aufgrund eines Kokainentzugs bei einem Säuger verwendbar ist.

**52.** Verwendung einer Verbindung gemäß den Ansprüchen 1 bis 35 zur Herstellung einer pharmazeutischen Zusammensetzung, die zur Behandlung einer Reaktion aufgrund eines Benzodiazepinentzugs bei einem Säuger verwendbar ist.

**53.** Verwendung einer Verbindung gemäß den Ansprüchen 1 bis 35 zur Herstellung einer pharmazeutischen Zusammensetzung, die zur Behandlung einer Reaktion aufgrund eines Diazepamentzugs bei einem Säuger verwendbar ist.

**54.** Verwendung einer Verbindung gemäß den Ansprüchen 1 bis 35 zur Herstellung einer pharmazeutischen Zusammensetzung, die zur Behandlung einer Reaktion aufgrund eines Nikotinentzugs bei einem Säuger verwendbar ist.

**55.** Verwendung einer Verbindung gemäß den Ansprüchen 1 bis 35 zur Herstellung einer pharmazeutischen Zusammensetzung, die zur Verstärkung der Wirkungen von Morphin und anderen Opioiden bei einer Schmerzbehandlung verwendbar ist.

**56.** Verwendung einer Verbindung gemäß den Ansprüchen 1 bis 35 zur Herstellung einer pharmazeutischen Zusammensetzung, die zur Schmerzbehandlung bei einem Säuger verwendbar ist.

**57.** Verfahren zur Herstellung einer Verbindung der Formel I gemäß den Ansprüchen 1 bis 36, das die Kondensation einer Verbindung der Formel

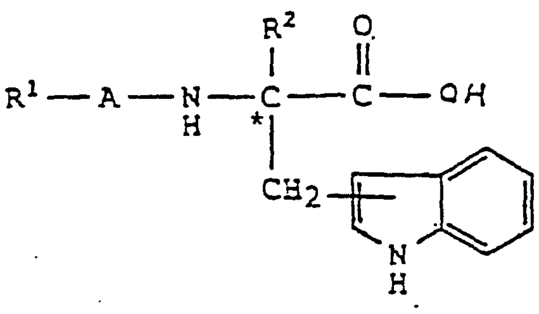

mit einem geeigneten Amin der Formel

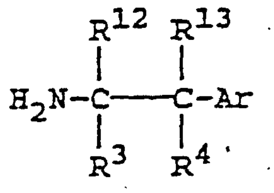

unter Verwendung eines geeigneten Kondensationsmittels und eines geeigneten Lösemittels bei einer Temperatur von etwa 20 °C bis etwa 80 °C umfasst.

**58.** Verfahren der Verwendung einer radioaktiven Iodverbindung der Formel I gemäß den Ansprüchen 1 bis 36 zur Herstellung einer pharmazeutischen oder diagnostischen Zusammensetzung zur Behandlung oder Diagnose von gastrinabhängigen Tumoren.

**59.** Verbindung mit dem Namen
Tricyclo [3.3.1.1$^{3,7}$]dec-2-yl-[R, (R*, S*)]-[1-(1H-indol-3-yl-methyl)-1-methyl-2-oxo-2-[[2-[[1-oxo-3-(1H-tetrazol-5-yl)propyl]amino]-2-phenylethyl]-amino]ethyl]carbaminsäureester.

**60.** Verbindung mit dem Namen
[R, (R*, S*)]-[1-(1H-indol-3-yl-methyl)-1-methyl-2-oxo-2-[[2-[[1-oxo-3-(1H-tetrazol-5-yl)propyl]amino]-2-phenylethyl]-amino]ethyl]carbaminsäure-tricyclo-[3.3.1.1$^{3,7}$]dec-2-yl-ester

**61.** Verbindung der Formel

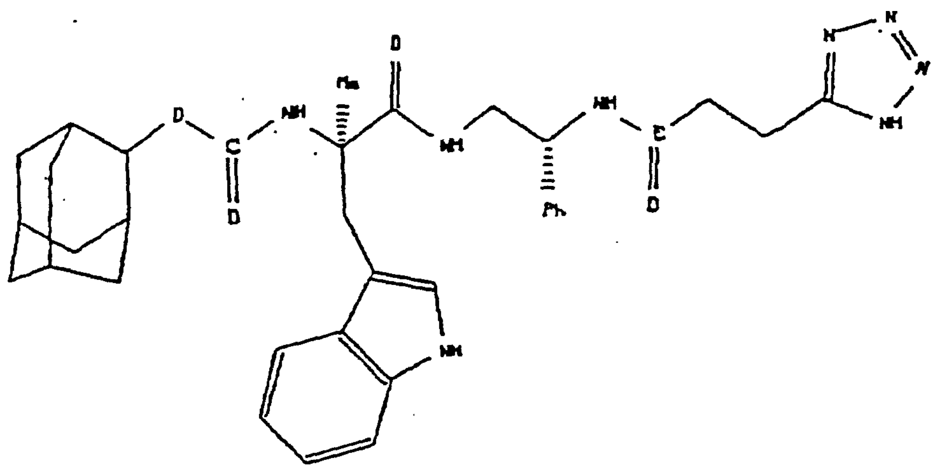

**62.** Verbindung der Formel

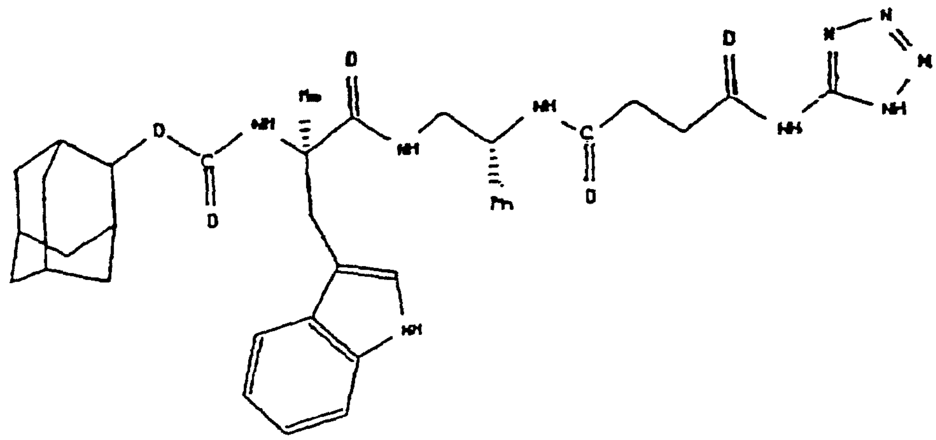

## Revendications

**1.** Composé de formule

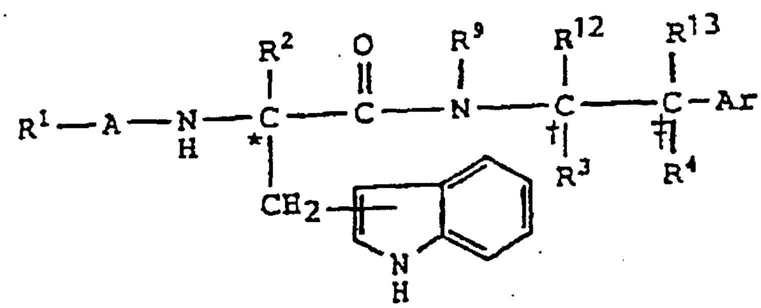

I

ou sel pharmaceutiquement acceptable d'un tel composé, formule dans laquelle

$R^1$ est un groupe hydrocarboné cycloalkyle ou polycycloalkyle ayant de 3 à 12 atomes de carbone et portant de zéro à 4 substituants choisis chacun indépendamment dans l'ensemble constitué par un groupe alkyle à chaîne droite ou ramifiée ayant de 1 à 6 atomes de carbone, un atome d'halogène, les groupes CN, OR*, SR*, $CO_2R^*$ $CF_3$, $NR^5R^6$ et $-(CH_2)_nOR^5$, où R* est un atome d'hydrogène ou un groupe alkyle à chaîne droite ou ramifiée ayant de 1 à 6 atomes de carbone, R5 et R6 représentent chacun indépendamment un atome d'hydrogène ou un groupe alkyle avant de 1 à 6 atomes de carbone et n est un nombre entier allant de 0 à 6 ;

A est un groupe $-(CH_2)_nCO-$, $-SO_2-$, -S(=O)-, -NHCO-,

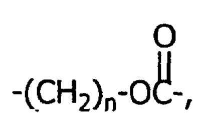

-SCO-, $-O-(CH_2)_nCO-$ ou -HC=CHCO-, n étant un nombre entier allant de zéro à 6 ;

$R^2$ est un groupe alkyle à chaîne droite ou ramifiée ayant de 1 à 6 atomes de carbone, $-HC=CH_2$, -C≡CH, $-CH_2-CH=CH_2$, $-CH_2C≡CH$, $-CH_2Ar$, $-CH_2OR^*$, $-CH_2OAr$, $-(CH_2)_nCO_2R^*$ ou $-(CH_2)_nNR^5R^6$, n, R*, $R^5$ et $R^6$ étant tels que définis ci-dessus et Ar étant tel que défini ci-dessous ;

R3 et R4 sont choisis chacun indépendamment parmi un atome d'hydrogène, $R^2$ et $-(CH_2)_{n'}-B-D$, où :

n' est un nombre entier allant de zéro à trois ;

B est une liaison,

$-OCO(CH_2)_n-$,

$-O(CH_2)_n-$,

$-NHCO(CH_2)_n-$,

$-CONH(CH_2)_n-$,

$-NHCOCH=CH-$,

$-COO(CH_2)_n-$,

$-CO(CH_2)_n-$,

$-S-(CH_2)_n-$,

$-S(=O)-(CH_2)_n-$

$-SO_2-(CH_2)_n-$

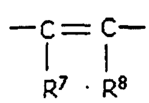

ou

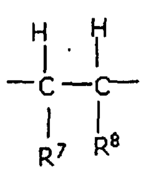

$R^7$ et $R^8$ étant choisis indépendamment parmi un atome d'hydrogène et $R^2$ ou formant ensemble un cycle $(CH_2)_m$ dans lequel m est un nombre entier allant de 1 à 5, et n est tel que défini plus haut ;
D est

$-COOR^*$,

$-CONR^5R^6$,

-CN,

$-NR^5R^6$,

-OH,

H et des remplacements d'acide choisis parmi

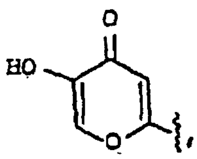,

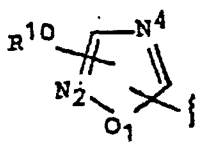

1,2,4oxadiazole

$R^{10}$ is OH, $NH_2$, $CH_3$ ou Cl,

$HO_3S$-{ ,

$HO_2P$-{ ,

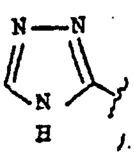,

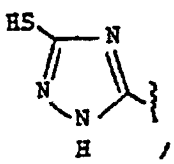,

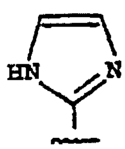,

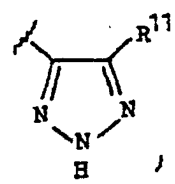,

$R^{11}$ est CN, $CO_2H$, $CF_3$,

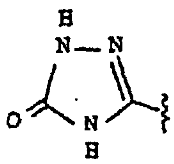,

$PhSO_2NHCO$- {,

$CF_3CONHCO$- {,

$CF_3SO_2NHCO$-{,

$H_2NSO$-{,

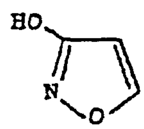,

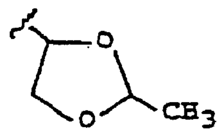,

$-CH_2OR$

$-CHR^2OR^*$,

$-CH_2SR^*$,

$-CHR^2SR^*$,

$R^*$, $R^2$, $R^5$ et R6 étant tels que définis plus haut ;

$R^9$ est un atome d'hydrogène ou un groupe alkyle à chaîne droite ou ramifiée ayant de 1 à environ 6 atomes de carbone, $-(CH_2)_nCO_2R^*$, $-(CH_2)_nOAr'$, $-(CH_2)_nAr'$ ou $(CH_2)_nNR^5R^6$, n, $R^*$, $R^5$ et $R^6$ étant tels que définis plus haut ou étant choisis parmi les groupes représentés par $R^3$ et Ar' étant choisi parmi les groupes représentés par Ar tels que définis ci-dessous ;

$R^{12}$ et $R^{13}$ représentent chacun indépendamment un atome d'hydrogène ou sont pris chacun indépendamment avec $R^3$ et $R^4$, respectivement, pour former un groupe lié par une double liaison à l'atome de carbone ; et Ar est un groupe 2- ou 3-thiényle, 2- ou 3-furanyle, 2-, 3- ou 4-pyridinyle ou un groupe phényle substitué ou non substitué dont les substituants s'ils existent représentent chacun indépendamment un atome d'hydrogène, de fluor, de chlore, de brome ou d'iode ou le groupe méthyle, méthoxy, trifluorométhyle ou nitro.

**2.** Composé selon la revendication 1, dans lequel le groupe cycloalkyle ou polycycloalkyle a de 6 à 10 atomes de carbone.

**3.** Composé selon la revendication 1, dans lequel chaque substituant sur le groupe cycloalkyle ou polycycloalkyle est indépendamment le groupe méthyle, F, Cl ou Br.

**4.** Composé selon la revendication 1, dans lequel le groupe polycycloalkyle est choisi dans l'ensemble constitué par

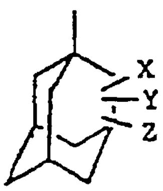 , 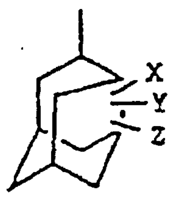 , 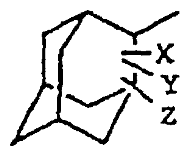 , et 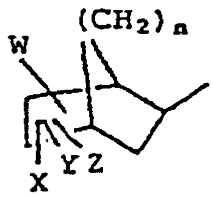

où W, X, Y et Z représentent chacun indépendamment un atome d'hydrogène, un groupe alkyle à chaîne droite ou ramifiée ayant de 1 à 6 atomes de carbone, $CF_3$, $NR^5R^6$, $-(CH_2)_nCO_2R^*$, CN, F, Cl, Br, OR*, SR*, où R*, $R^5$ et $R^6$ sont tels que définis dans la revendication 1 et n est un nombre entier allant de 1 à 3.

**5.** Composé selon la revendication 1, dans lequel A est -NHCO-, -OC(=O)-, $-SO_2-$, -S(=O)-, -SCO- ou $-CH_2CO-$.

**6.** Composé selon la revendication 1, dans lequel :

$R^1$ est le groupe 2-adamantyle ou 1-(S)-2-endobornyle ;
A est -NHCO-, -OCO-, $-SO_2-$, -S(=O)- ou $-CH_2CO-$ ;
$R^2$ est $-CH_3$, $-CH_2CO_2H$ ou $-CH_2{\equiv}CH$ ;
R3 est $-CH_2$-B-D ou H ;
$R^4$ est $-(CH_2)_n$-B-D ou H ;
$R^9$ est un atome d'hydrogène ou le groupe méthyle.

**7.** Composé selon la revendication 1, dans lequel :

$R^1$ est le groupe 2-adamantyle ou 1-(S)-2-endobornyle ;
A est -OC(=0)- ;
R2 est $-CH_3$ ;
R3 est H, $-CH_2OH$, $CH_2OCOCH_2CH_2CO_2H$, $CH_2OCOCH{=}CHCO_2H$, $CH_2NHCOCH_2CH_2CO_2H$ ou $CH_2NHCOCH{=}CHCO_2H$ ;
$R^4$ est H, $-NHCOCH_2CH_2CO_2H$ (configuration [D]) ou $NHCOCH{=}CHCO_2H$ (configuration [D]).

**8.** Composé selon la revendication 1, dénommé
carbamate de (±)-*trans*-2-chlorocyclohexyl-[1-(1H-indol-3-ylméthyl)-1-méthyl-2-oxo-2-[(2-phényléthyl)amino] éthyle].

**9.** Composé selon la revendication 1, dénommé
carbamate de 2-chlorocyclohexyl-[2-[[1-(hydroxyméthyl)-2-phényléthyl]amino]-1-(1H-indol-3-ylméthyl)-1-méthyl-2-oxoéthyle].

**10.** Composé selon la revendication 1, dénommé
butanedioate de 2-[[2-[[[(2-chlorocyclohexyl)oxy]carbonyl]amino]-3-(1H-indol-3-yl)-2-méthyl-1-oxopropyl]amino]-3-phénylpropyle.

**11.** Composé selon la revendication 1, dénommé
butanedioate de 2-[[2-[[[(2-méthylcyclohexyl)oxy]carbonyl]amino]-3-(1H-indol-3-yl)-2-méthyl-1-oxopropyl]amino]-3-phénylpropyle.

**12.** Composé selon la revendication 1, dénommé
carbamate de [1-(1H-indol-3-ylméthyl)-1-méthyl-2-oxo-2-[(2-phényléthyl)amino]éthyl] (±)-tricyclo[3.3.1.1$^{3,7}$]déc-2-yle.

**13.** Composé selon la revendication 1, dénommé
carbamate de (+)- ou (-)-2-chlorocyclohexyl-[1-(1H-indol-3-ylméthyl)-1-méthyl-2-oxo-2-[(2-phényléthyl)amino] éthyle].

**14.** Composé selon la revendication 1, dénommé
carbamate de [2-[[1-(hydroxyméthyl)-2-phényléthyl]amino]-1-(1H-indol-3-ylméthyl)-1-méthyl-2-oxoéthyl]tricyclo [3.3.1.1$^{3,7}$]déc-2-yle.

**15.** Composé selon la revendication 1, dénommé
butanedioate de 2-[[3-(1H-indol-3-yl)-2-méthyl-1-oxo-2-[[(tricyclo[3.3.1.1$^{3,7}$]déc-2-yloxy)carbonyl]amino]propyl] amino]-3-phénylpropyle.

**16.** Composé selon la revendication 1, dénommé
butanedioate de 2-[[3-(1H-indol-3-yl)-2-méthyl-1-oxo-2-[[(tricyclo[3.3.1.1$^{3,7}$]déc-2-yloxy)carbonyl]amino]propyl] amino]-1-phényléthyle.

**17.** Composé selon la revendication 1, dénommé
acide [R-(R*,R*)]-4-[[2-[[3-(1H-indol-3-yl)-2-méthyl-1-oxo-2-[[(tricyclo-[3.3.1.1$^{3,7}$]déc-2-yloxy)carbonyl]amino]propyl]amino]-1-phényléthyl]amino]-4-oxobutanoïque.

**18.** Composé selon la revendication 1, dénommé
acide [1S-[1α,2β[S*(S*)],4α]]-4-[[2-[[3-(1H-indol-3-yl)-2-méthyl-1-oxo-2-[[[(1,7,7-triméthylbicyclo[2.2.1]hept-2-yl) amino]carbonyl]amino]propyl]amino]-1-phényléthyl]amino]-4-oxobutanoïque.

**19.** Composé selon la revendication 1, dénommé
acide [R-(R*,S*)]-4-[[2-[[(1H-indol-3-yl)-2-méthyl-1-oxo-2-[[(tricyclo[3.3.1.1$^{3,7}$]déc-2-yloxy)carbonyl]amino]propyl] amino]-3-phénylpropyl]amino]-4-oxo-2-buténoïque.

**20.** Composé selon la revendication 1, dénommé
acide [R-(R*,S*)]-4-[[2-[[3-(1H-indol-3-yl)-2-méthyl-1-oxo-2-[[(tricyclo-[3.3.1.1$^{3,7}$]déc-2-yloxy)carbonyl]amino]propyl]amino]-3-phénylpropyl]amino]-4-oxobutanoïque.

**21.** Composé selon la revendication 1, dénommé
carbamate de (R)-tricyclo[3.3.1.1$^{3,7}$]déc-2-yl-[1-(1H-indol-3-ylméthy1)-1-méthyl-2-[méthyl-(2-phényléthyl)amino]-2-oxoéthyle.

**22.** Composé selon la revendication 1, dénommé
acide [R-(R*,S*)]-2-[[2-[[3-(1H-indol-3-yl)-2-méthyl-1-oxo-2-[[(tricyclo-[3.3.1.1$^{3,7}$]déc-2-yloxy)carbonyl]amino]propyl]amino]-3-phénylpropyl]sulfinyl]-acétique.

**23.** Composé selon la revendication 1, dénommé
acide [R-(R*,S*)]-[[2-[[3-(1H-indol-3-yl)-2-méthyl-1-oxo-2-[[(tricyclo[3.3.1.1$^{3,7}$]déc-2-yloxy)carbonyl]amino]propyl] amino]-3-phénylpropyl]sulfonyl]acétique.

**24.** Composé selon la revendication 1, dénommé
acide [R-(R*,S*)]-[[2-[[3-(1H-indol-3-yl)-2-méthyl-1-oxo-2-[[(tricyclo[3.3.1.1$^{3,7}$]déc-2-yloxy)carbonyl]amino]propyl] amino]-3-phénylpropyl]suffinyl]acétique ou ester éthylique de celui-ci.

**25.** Composé selon la revendication 1, dénommé
acide [R-(R*,S*)]-[[2-[[3-(1H-indol-3-yl)-2-méthyl-1-oxo-2-[[(tricyclo[3.3.1.1$^{3,7}$]déc-2-yloxy)carbonyl]amino]propyl]amino]-3-phénylpropyl]sulfonyl]acétique.

**26.** Composé selon la revendication 1, dénommé acide [R-[R*,R*-(E)]]-4-[[2-[[3-(1H-indol-3-yl)-2-méthyl-1-oxo-2-[[(tricyclo-[3.3.1.1$^{3,7}$]déc-2-yloxy)carbonyl]amino]propyl]amino]-1-phényléthyl]amino]-4-oxo-2-buténoïque.

**27.** Composé selon la revendication 1, dénommé acide [R-(R*,S*)]-[[2-[2-[3-(1H-indol-3-yl)-2-méthyl-1-oxo-2-[[(tricyclo-[3.3.1.1$^{3,7}$]déc-2-yloxy)carbonyl]amino]propyl]amino]-3-phénylpropyl]thio]acétique.

**28.** Composé selon la revendication 1, dénommé ester méthylique d'acide [1S-[1α,2β[S*[S*(E)]],4α]]-4-[[2-[[3-(1H-indol-3-yl)-2-méthyl-1-oxo-2-[[[(1,7,7-triméthylbicyclo[2.2.1]hept-2-yl)oxy]carbonyl]-amino]propyl]amino]-1-phényléthyl]amino]-4-oxo-2-buténoïque (le système bicyclo est 1S-endo).

**29.** Composé selon la revendication 1, dénommé acide [1S-[1α,2β[S*[S*(E)]],4α]]-4-[[2-[[3-(1H-indol-3-yl)-2-méthyl-1-oxo-2-[[[(1,7,7-triméthylbicyclo[2.2.1]hept-2-yl)oxy]carbonyl]amino]propyl]amino]-1-phényléthyl]amino]-4-oxo-2-buténoïque (le système bicyclo est 1S-endo).

**30.** Composé selon la revendication 1, dénommé acide [R-(R*,R*)]-3-[[2-[[3-(1H-indol-3-yl)-2-méthyl-1-oxo-2-[[(tricyclo-[3.3.1.1$^{3,7}$]déc-2-yloxy)carbonyl]amino]propyl]amino]-1-phényléthyl]amino]-3-oxopropionique.

**31.** Composé selon la revendication 1, dénommé acide [R-(R*,S*)]-3-(1H-indol-3-ylméthyl)-3-méthyl-4,10-dioxo-6-(phénylméthyl)-11-oxo-8-thia-2,5-diazatridécanoïque ou ester tricyclo[3.3.1.1$^{3,7}$]déc-2-ylique de celui-ci.

**32.** Composé selon la revendication 1, dénommé acide [R-(R*,S*)]-β-[[3-(1H-indol-3-yl)-2-méthyl-1-oxo-2-[[(tricyclo[3.3.1.1$^{3,7}$]déc-2-yloxy)carbonyl]amino]propyl]amino]benzènebutanoïque.

**33.** Composé selon la revendication 1, dénommé acide [R-(R*,S*)]-β-[[3-(1H-indol-3-yl)-2-méthyl-1-oxo-2-[[(tricyclo[3.3.1.1$^{3,7}$]déc-2-yloxy)carbonyl]amino]propyl]amino]-4-iodobenzènebutanoïque, dans lequel le groupe iodo peut être I-125 ou I-127.

**34.** Composé selon la revendication 1, dénommé [R-(R*,S*)]-N-[3-[[3-(1H-indol-3-yl)-2-méthyl-1-oxo-2-[[(tricyclo[3.3.1.1$^{3,7}$]déc-2-yloxy)carbonyl]amino]propyl]amino]-4-phénylbutyl]glycine.

**35.** Composé selon la revendication 1, dénommé acide [R-(R*,S*-(E)]]-4-[[2-[[3-(1H-indol-3-yl)-2-méthyl-2-[[(bicyclo[3.3.1]non-9-yloxy)carbonyl]amino]-1-oxopropyl]amino]-3-phénylpropyl]amino-4-oxo-2-buténoïque.

**36.** Composition pharmaceutique comprenant un composé selon les revendications 1 à 5 et un véhicule pharmaceutiquement acceptable.

**37.** Utilisation d'un composé selon les revendications 1 à 5, pour la préparation d'une composition pharmaceutique utile pour supprimer l'appétit chez un mammifère.

**38.** Utilisation d'un composé selon les revendications 1 à 5, pour la préparation d'une composition pharmaceutique utile pour réduire la sécrétion gastrique chez un mammifère.

**39.** Utilisation d'un composé selon les revendications 1 à 5, pour la préparation d'une composition pharmaceutique utile pour réduire l'anxiété chez un mammifère.

**40.** Composé de formule :

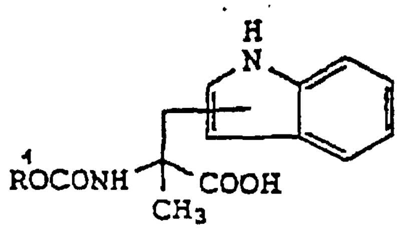

dans laquelle $R^1$ est tel que défini dans la revendication 1.

**41.** Composé de formule :

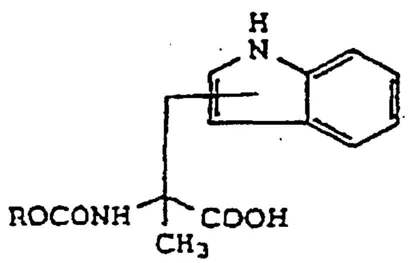

(II)

dans laquelle R est le groupe 1-adamantyle, 2-adamantyle, 4-protoadamantyle, 9-fluorénylméthyle, exobornyle, endobornyle, exonorbornyle, endonorbornyle, 2-chlorocyclohexyle, 2-méthylcyclohexyle ou camphoryle.

**42.** Procédé pour la préparation d'un composé selon la revendication 1, comprenant la mise en réaction d'un composé de formule

ROH (III)

avec du phosgène ou un composé de remplacement du phosgène, pour l'obtention d'un composé de formule

ROCOCl (IV)

et la mise en réaction d'un composé de formule IV avec du [D]-$\alpha$-méthyltryptophane, pour l'obtention d'un composé de la revendication 41.

**43.** Procédé pour la préparation d'un composé de formule

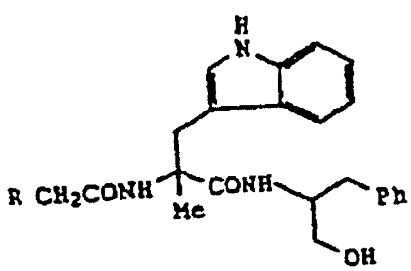

dans laquelle R est le groupe 1-adamantyle, 2-adamantyle, 4-protoadamantyle, 9-fluorénylméthyle, exobornyle, endobornyle, exonorbornyle, endonorbornyle, 2-chlorocyclohexyle, 2-méthylcyclohexyle ou camphoryle, comprenant la mise en réaction d'une amine libre de formule

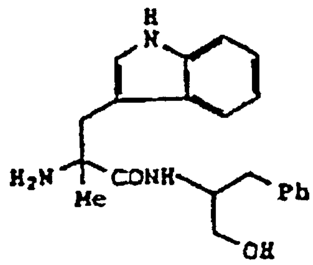

avec un chlorure d'acétyle substitué

$RCH_2COCl$,

pour l'obtention d'un composé de formule I, et la conversion de ce dernier, si on le désire, en un sel pharmaceutiquement acceptable.

**44.** Procédé pour la préparation d'un sulfonamide

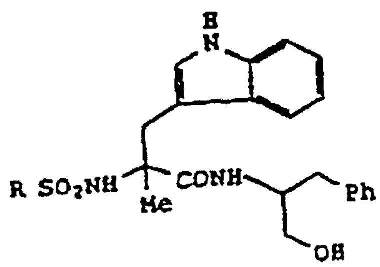

dans lequel R est le groupe 1-adamantyle, 2-adamantyle, 4-protoadamantyle, 9-fluorénylméthyle, exobornyle, endobornyle, exonorbornyle, endonorbornyle, 2-chlorocyclohexyle, 2-méthylcyclohexyle ou camphoryle, comprenant la mise en réaction d'une amine libre

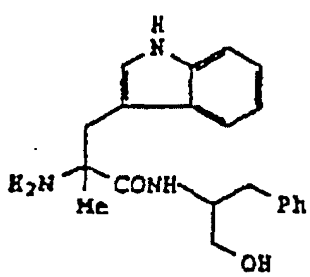

avec un chlorure de sulfonyle substitué

$RSO_2Cl$,

pour l'obtention d'un composé de formule I, et la conversion de ce dernier, si on le désire, en un sel pharmaceutiquement acceptable.

**45.** Procédé pour la préparation d'un composé de formule

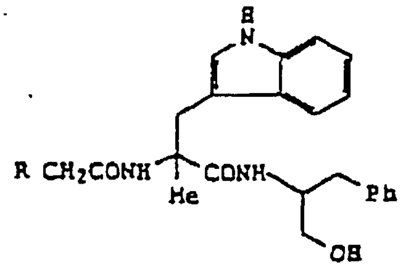

dans laquelle R est le groupe 1-adamantyle, 2-adamantyle, 4-protoadamantyle, 9-fluorénylméthyle, exobornyle, endobornyle, exonorbornyle, endonorbornyle, 2-chlorocyclohexyle, 2-méthylcyclohexyle ou camphoryle, comprenant la mise en réaction d'une amine libre

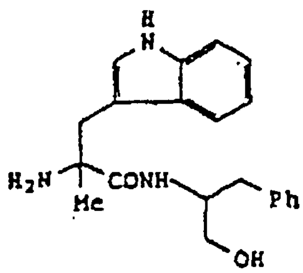

avec un isocyanate substitué

R-N=C=O,

pour l'obtention d'un composé de formule I, et la conversion de ce dernier, si on le désire, en un sel pharmaceutiquement acceptable.

**46.** Utilisation d'un composé selon les revendications 1 à 35, pour la préparation d'une composition pharmaceutique utilisable pour le traitement d'un ulcère gastro-intestinal chez un mammifère.

**47.** Utilisation d'un composé selon les revendications 1 à 35, pour la préparation d'une composition pharmaceutique utilisable pour le traitement d'un comportement psychotique chez un mammifère.

**48.** Utilisation d'un composé selon les revendications 1 à 35, pour la préparation d'une composition pharmaceutique utilisable pour le traitement d'une psychose chez un mammifère.

**49.** Utilisation d'un composé selon les revendications 1 à 35, pour la préparation d'une composition pharmaceutique utilisable pour bloquer la réaction provoquée par le sevrage de l'usage de drogue ou d'alcool chez un mammifère.

**50.** Utilisation d'un composé selon les revendications 1 à 35, pour la préparation d'une composition pharmaceutique utilisable pour le blocage ou le traitement de la réaction de sevrage de drogue ou d'alcool chez un mammifère.

**51.** Utilisation d'un composé selon les revendications 1 à 35, pour la préparation d'une composition pharmaceutique utilisable pour le traitement de la réaction due au sevrage de la cocaïne chez un mammifère.

**52.** Utilisation d'un composé selon les revendications 1 à 35, pour la préparation d'une composition pharmaceutique utilisable pour le traitement de la réaction due au sevrage d'une benzodiazépine chez un mammifère.

**53.** Utilisation d'un composé selon les revendications 1 à 35, pour la préparation d'une composition pharmaceutique

utilisable pour le traitement d'une réaction due au sevrage d'un diazépam chez un mammifère.

**54.** Utilisation d'un composé selon les revendications 1 à 35, pour la préparation d'une composition pharmaceutique utilisable pour le traitement de la réaction due au sevrage de la nicotine chez un mammifère.

**55.** Utilisation d'un composé selon les revendications 1 à 35, pour la préparation d'une composition pharmaceutique utilisable pour potentialiser les effets de la morphine et d'autres opioïdes dans le traitement de la douleur.

**56.** Utilisation d'un composé selon les revendications 1 à 35, pour la préparation d'une composition pharmaceutique utilisable pour le traitement de la douleur chez un mammifère.

**57.** Procédé pour la préparation d'un composé de formule I selon les revendications 1 à 36, comprenant la condensation d'un composé de formule

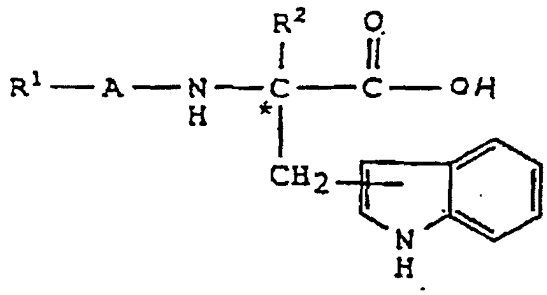

avec une amine appropriée de formule

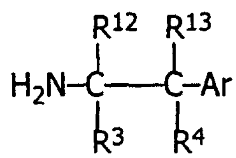

au moyen d'un agent de condensation approprié et d'un solvant convenable, à une température dans la plage allant d'environ 20°C à environ 80°C.

**58.** Procédé d'utilisation d'un composé iodé radioactif de formule I selon les revendications 1 à 36, pour la préparation d'une composition pharmaceutique ou de diagnostic destinée au traitement ou au diagnostic de tumeurs dépendant de la gastrine.

**59.** Composé dénommé
ester d'acide tricyclo[3.3.1.1$^{3,7}$]déc-2-yl-[R,(R*,S*)]-[1-(1H-indol-3-ylméthyl)-1-méthyl-2-oxo-2-[[2-[[1-oxo-3-(1H-tétrazol-5-yl)propyl]amino]-2-phényléthyl]-amino]éthyl]carbamique.

**60.** Composé dénommé
carbamate de [R,(R*,S*)]-[1-(1H-indol-3-ylméthyl)-1-méthyl-2-oxo-2-[[2-[[1-oxo-3-(1H-tétrazol-5-yl)propyl]amino]-2-phényléthyl]amino]éthyl]tricyclo[3.3.1.1$^{3,7}$]déc-2-yle.

**61.** Composé de formule

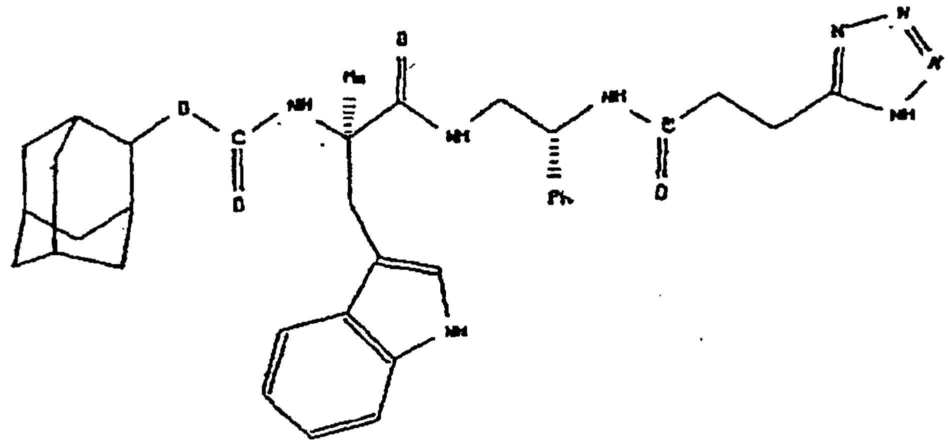

**62.** Composé de formule

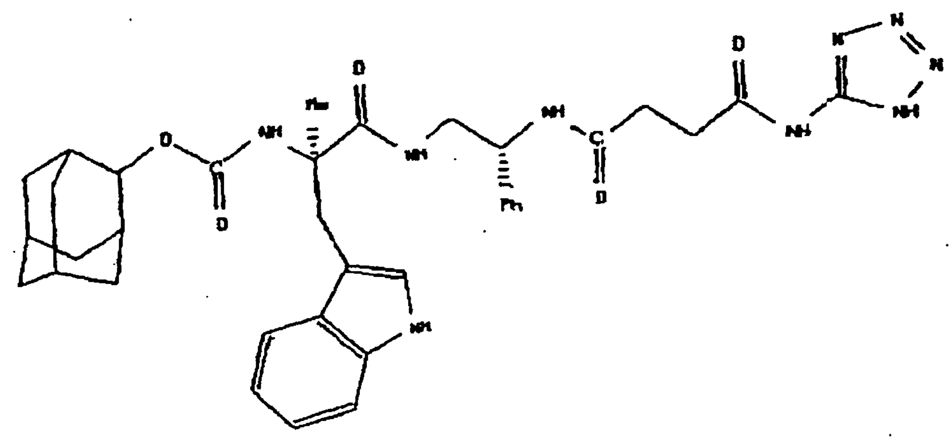

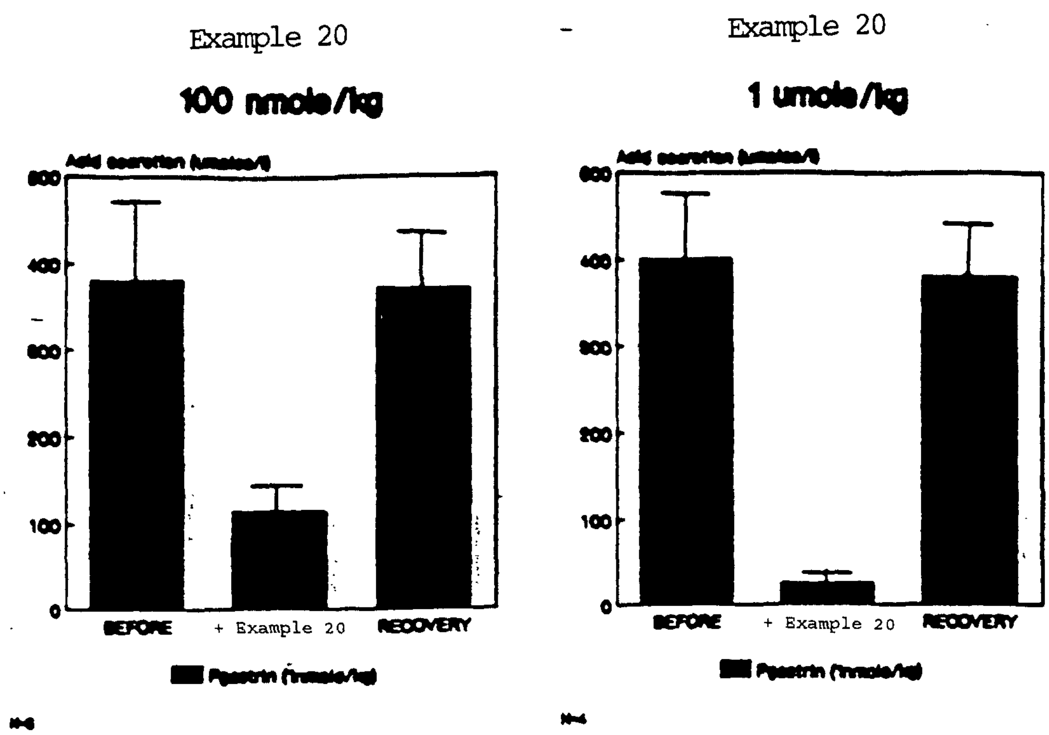
Example 20
100 nmole/kg
Example 20
1 umole/kg
BEFORE
+ Example 20
RECOVERY
BEFORE
+ Example 20
RECOVERY

FIGURE I

Example 20

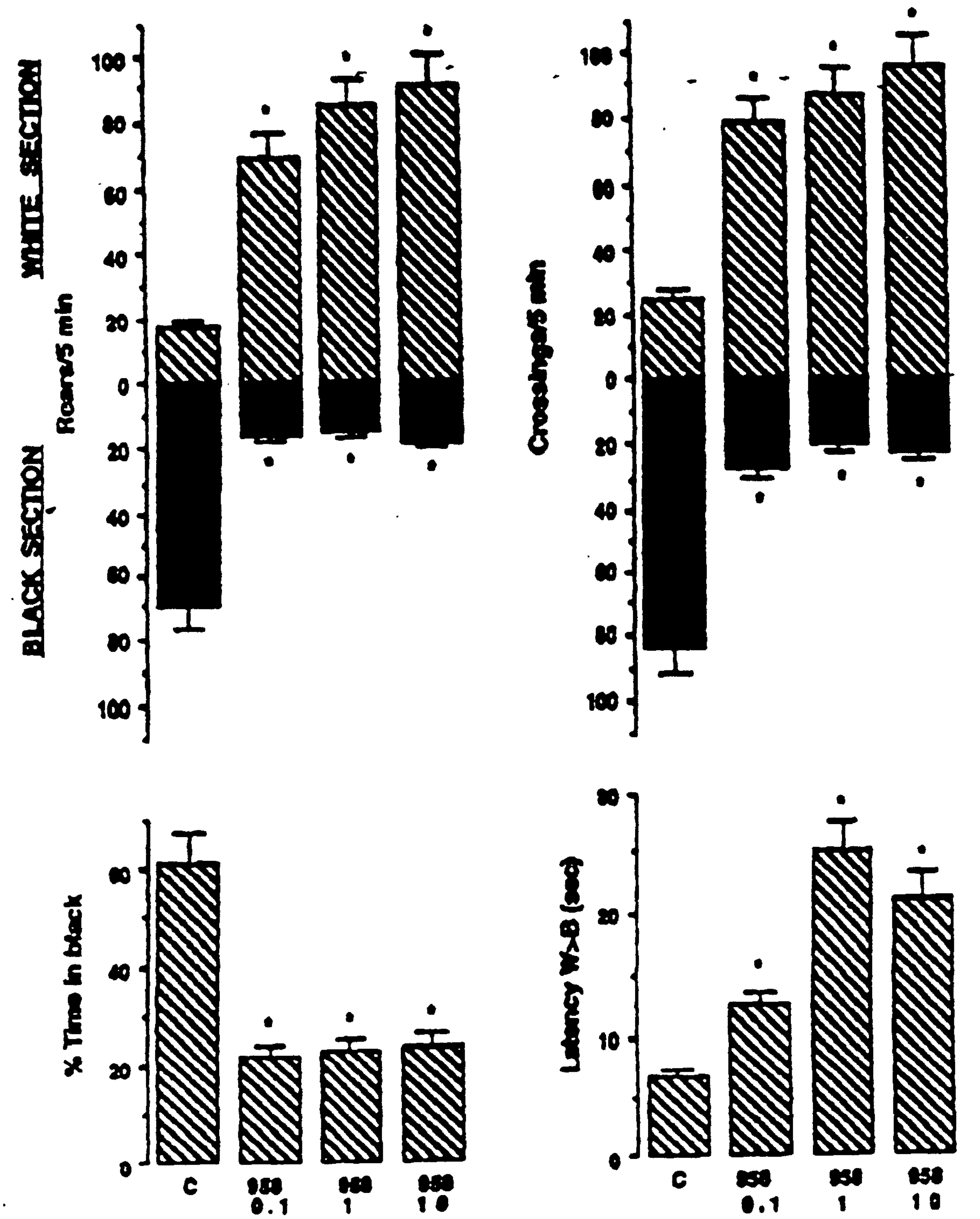


FIGURE 2

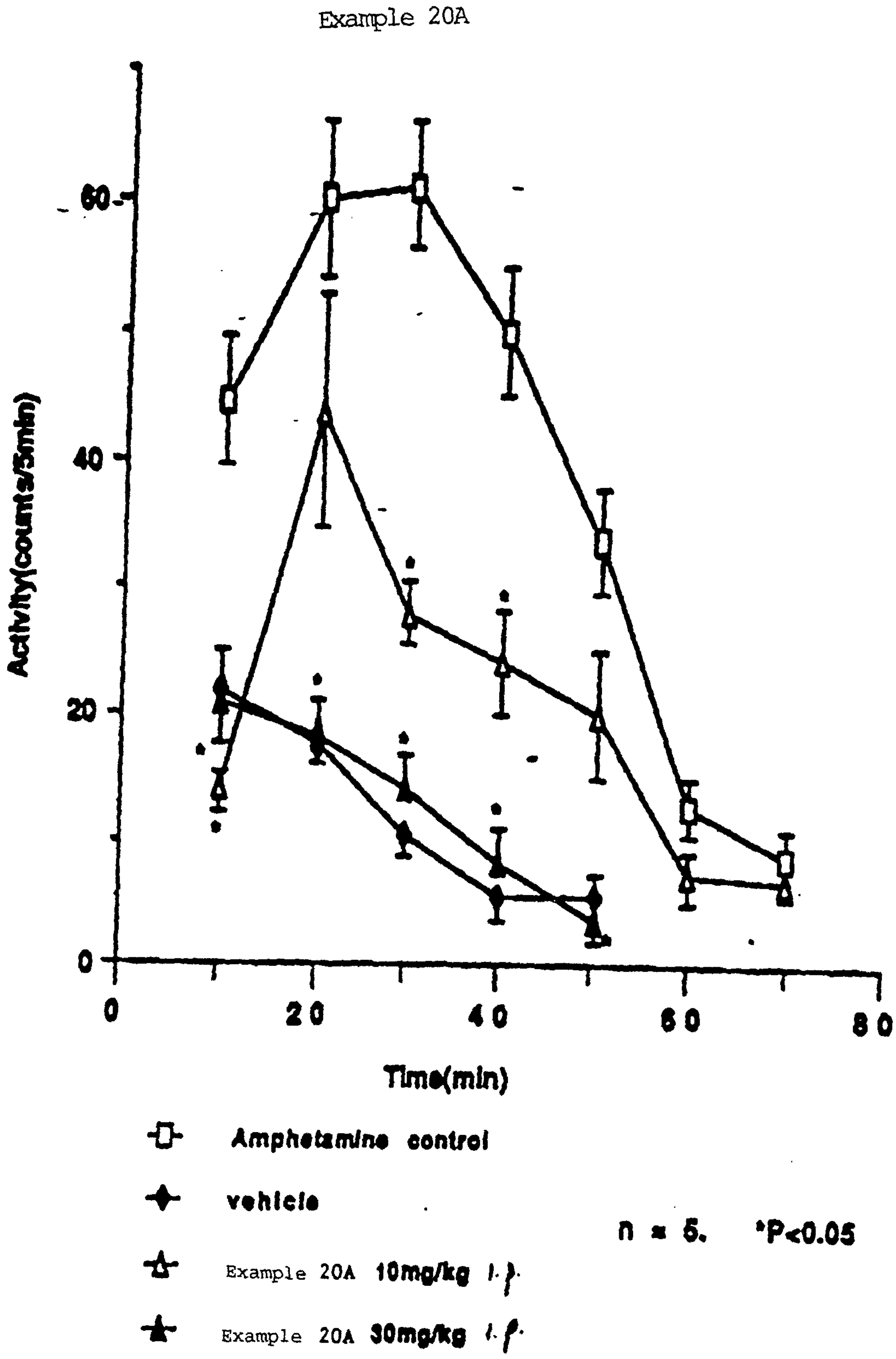
Example 20A
Activity(counts/5min)
0
20
40
60
0
20
40
60
80
Time(min)
Amphetamine control
vehicle
Example 20A 10mg/kg i.p.
Example 20A 30mg/kg i.p.
n = 5. *P<0.05

FIGURE 3

**Antagonism of Intra-accumbens amphetamine (20µg) by Example 20**

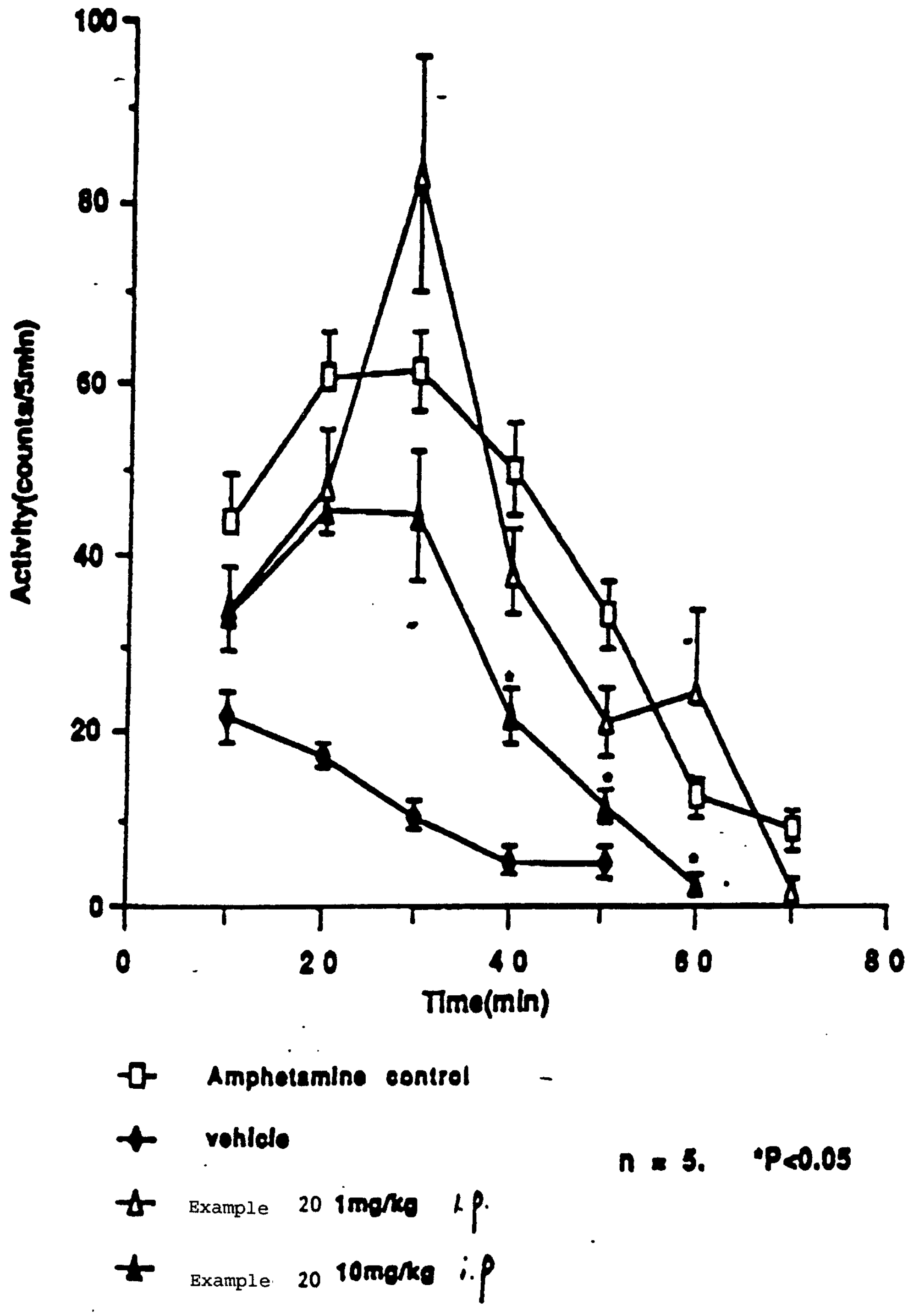


FIGURE 4

Effect of long-term treatment and withdrawal from nicotine: Intervention with Example 20

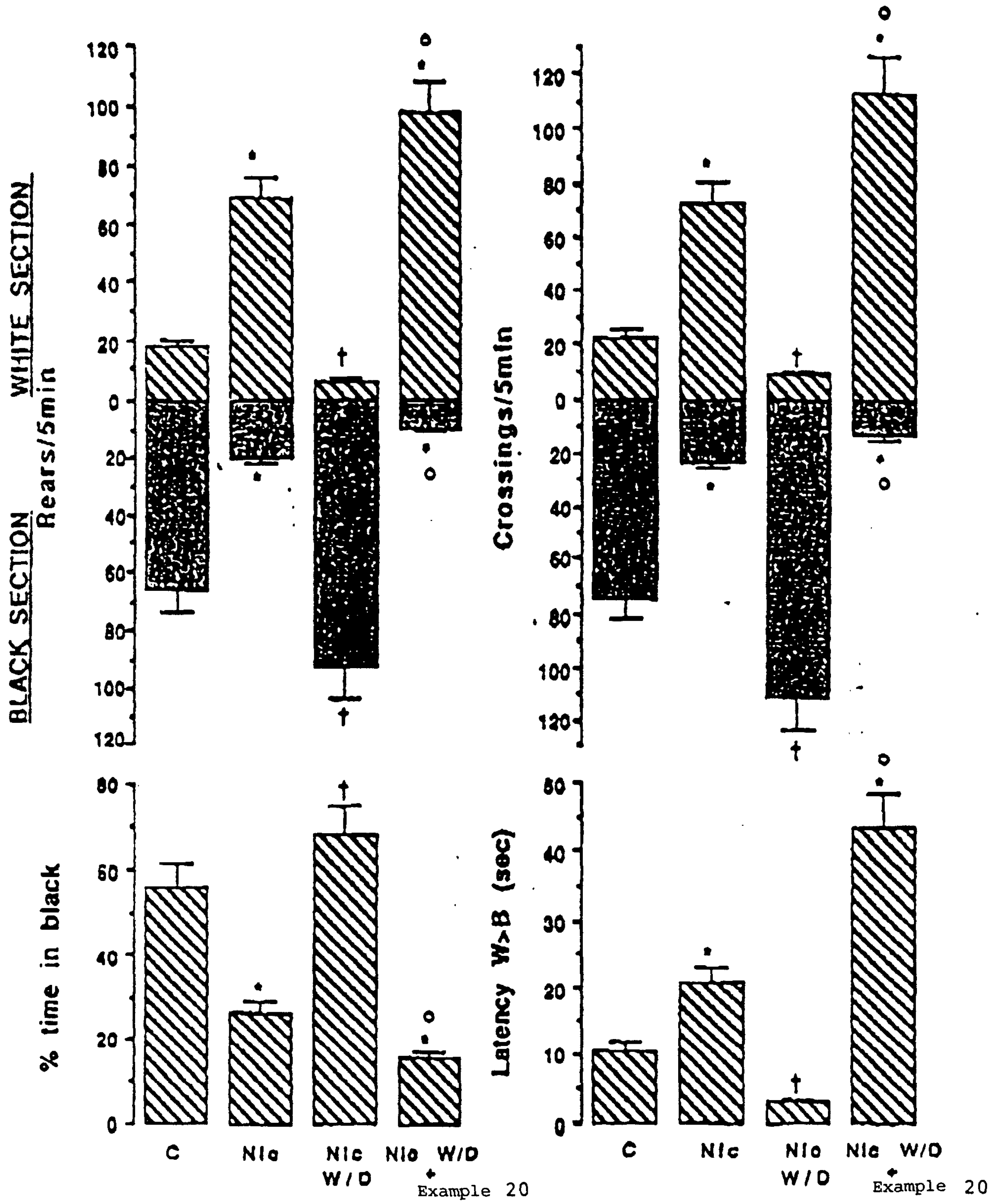

* P<0.001(anxiolysis). † P<0.001 (anxiogenesis)

Figure 5

**Effect of long-term treatment and withdrawal from nicotine: Intervention with Example 20A**

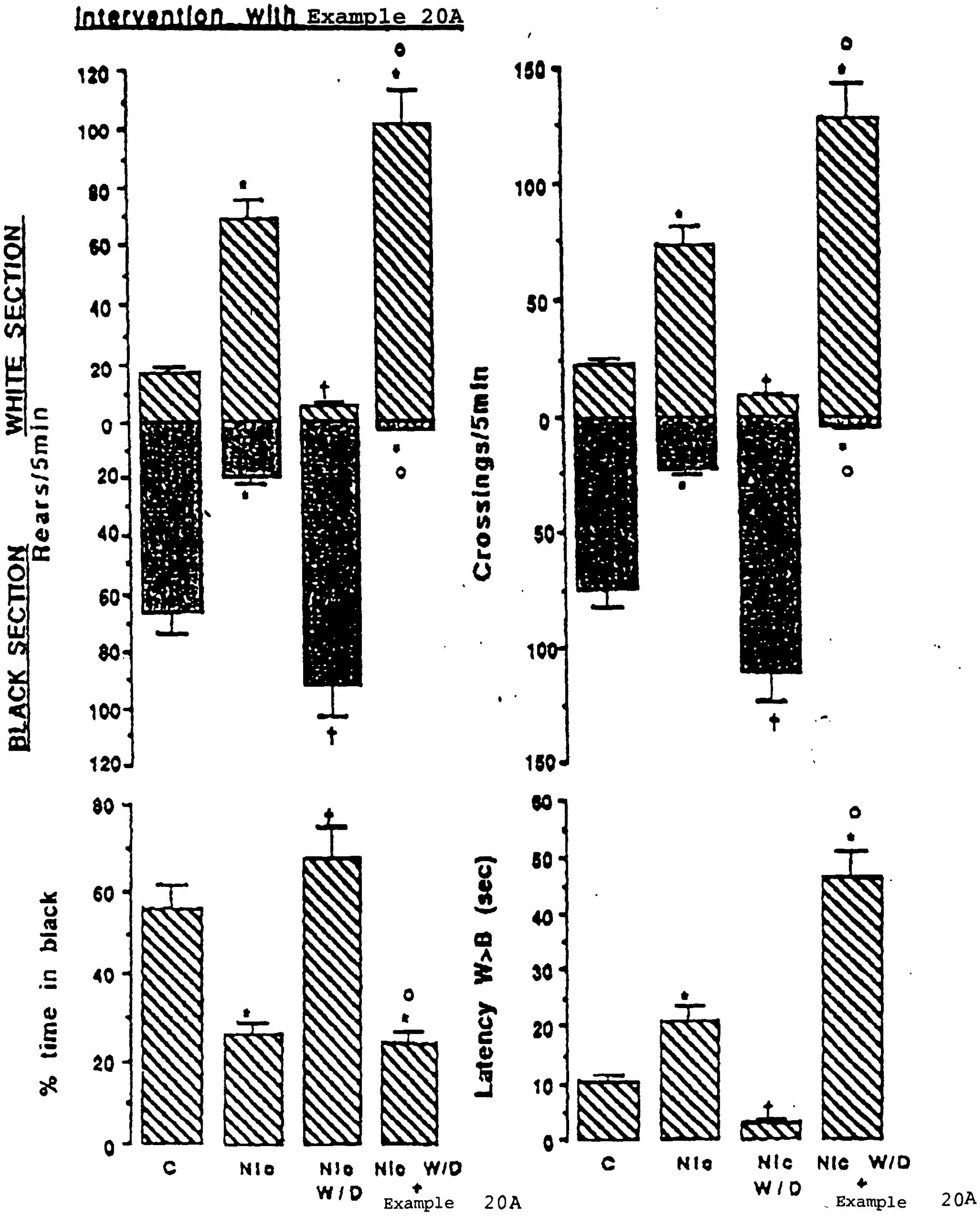


* P<0.001(anxiolysis). †P<0.001 (anxiogenesis)
(reversal anxiogenesis).

Figure 6

**Effect of long-term treatment and withdrawal from diazepam: Intervention with Example 20**

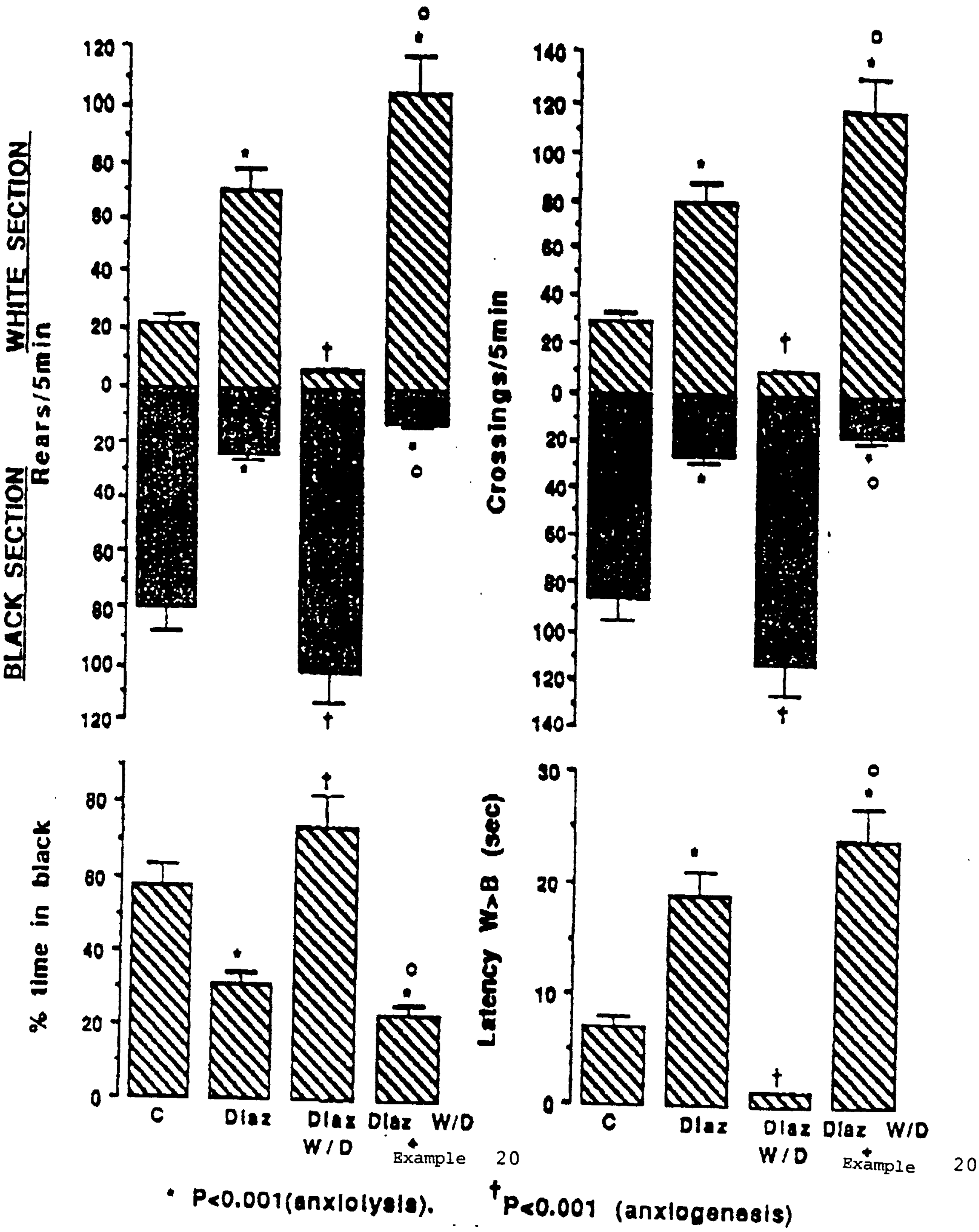


Figure 7

**Effect of long-term treatment and withdrawal from diazepam: Intervention with Example 20A**

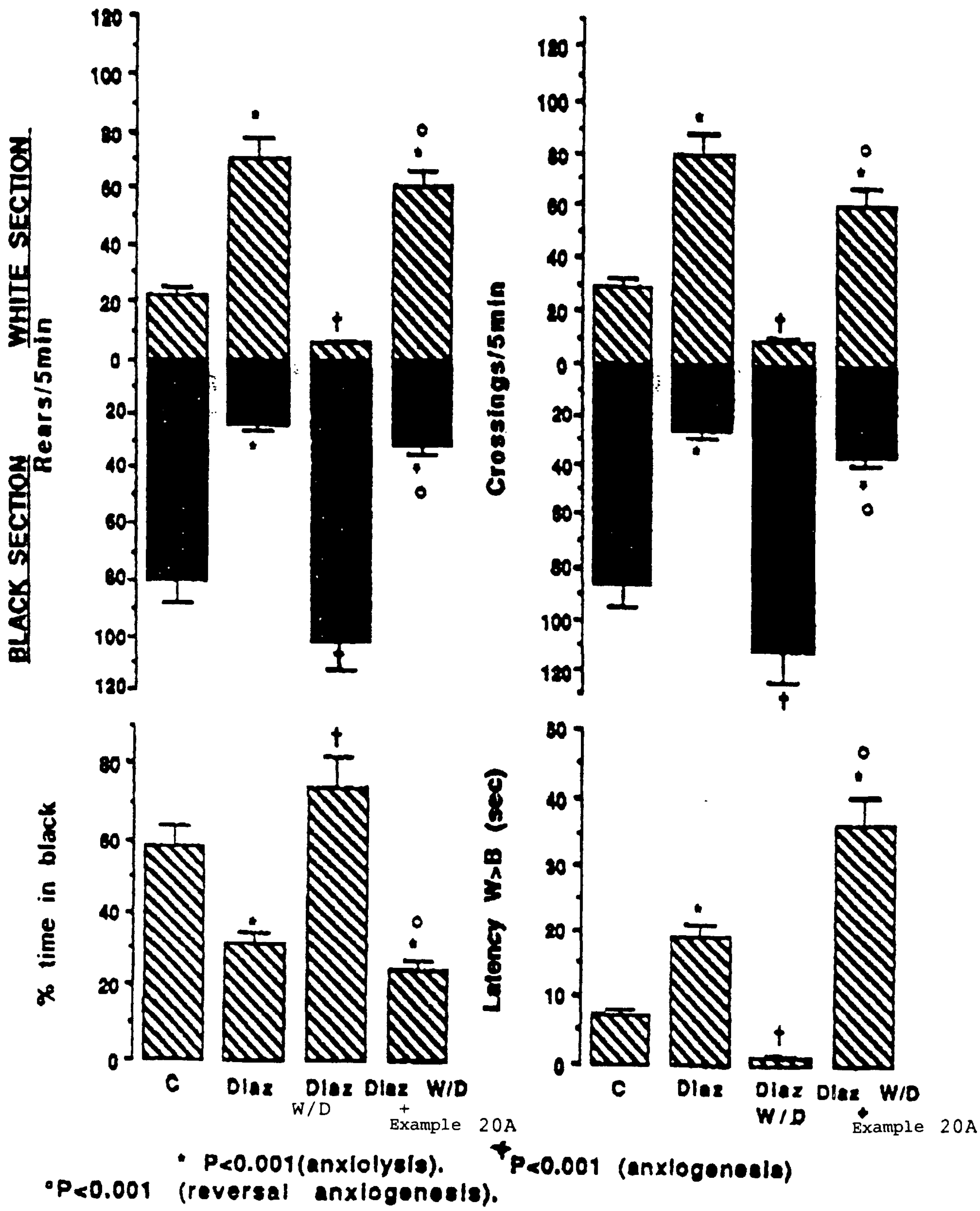


Figure 8

Effect of long-term treatment and withdrawal from alcohol: intervention with Example 20

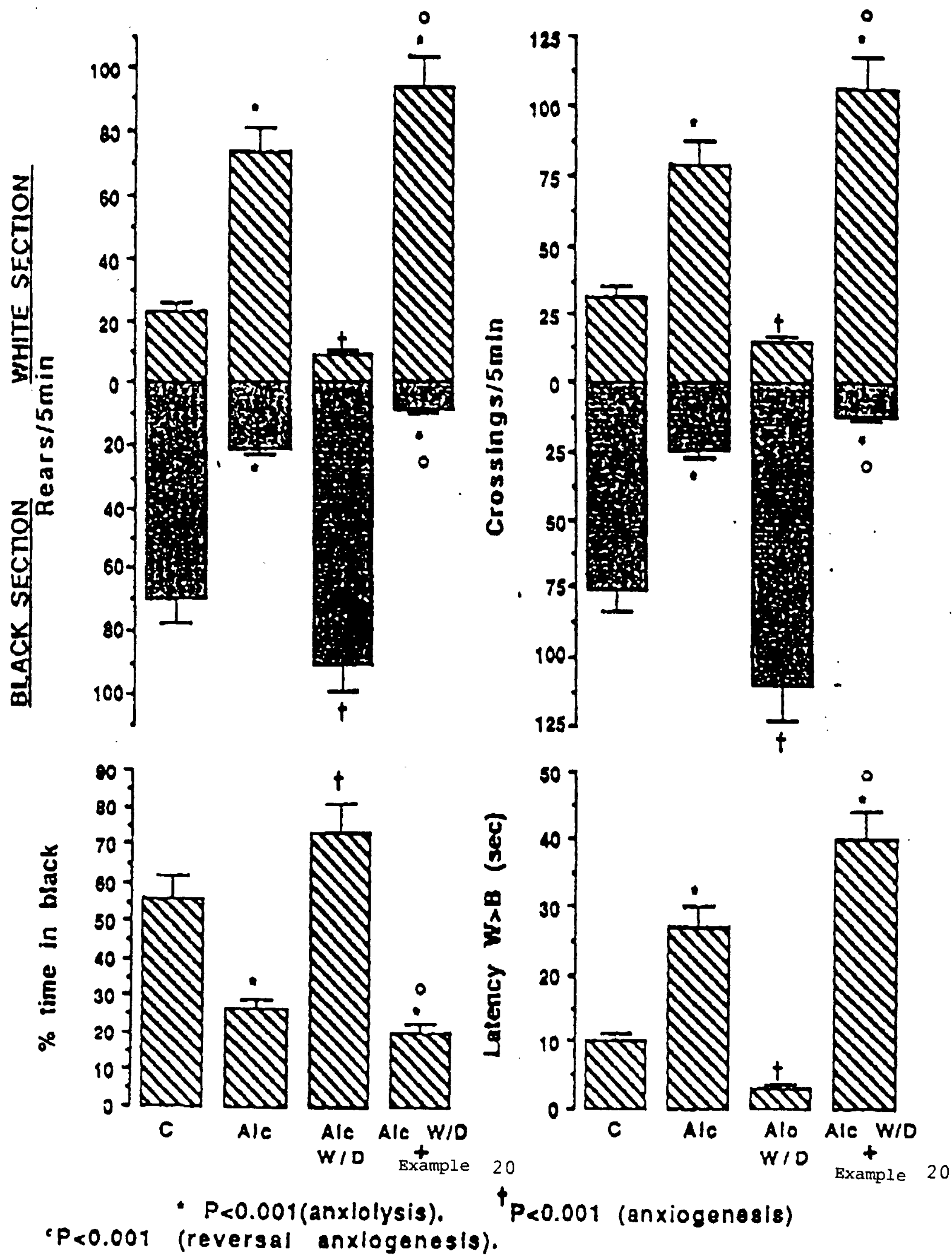


* P<0.001(anxiolysis). †P<0.001 (anxiogenesis)
°P<0.001 (reversal anxiogenesis).

Figure 9

**Effect of long-term treatment and withdrawal from alcohol: Intervention with Example 20A**

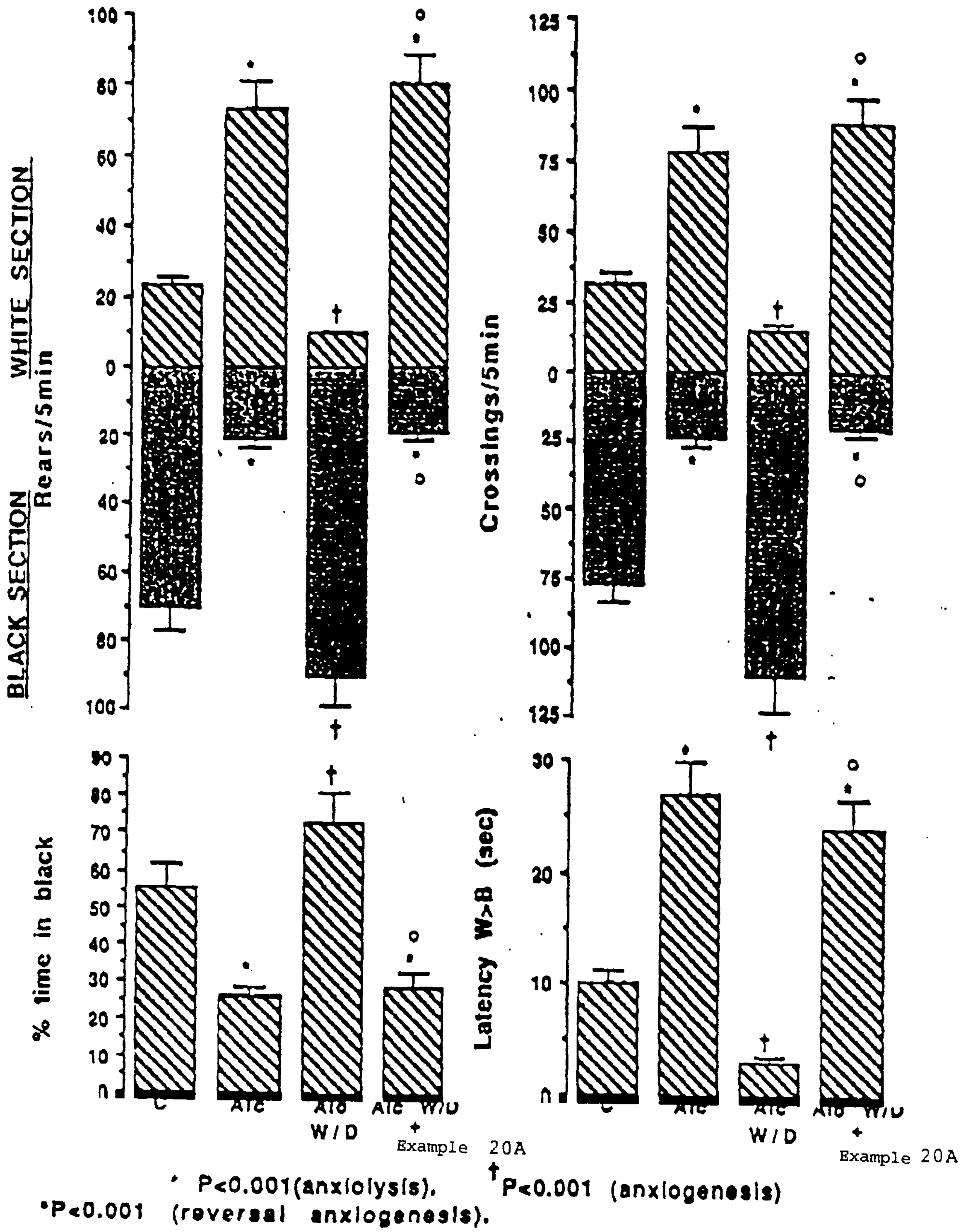


Figure 10

Effect of long-term treatment and withdrawal from cocaine: Intervention with Example 20

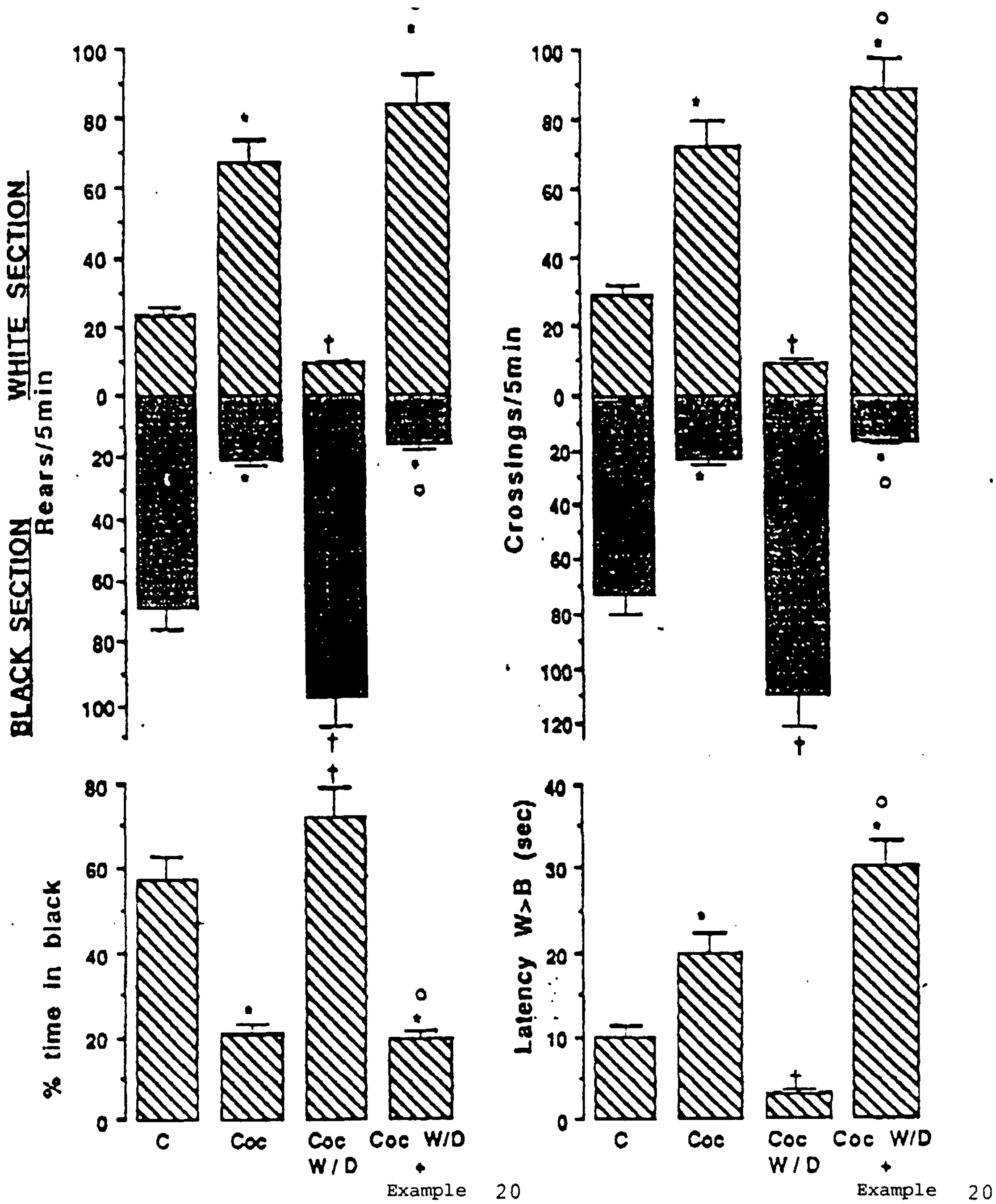


* P<0.001(anxiolysis). † P<0.001 (anxiogenesis)
°P<0.001 (reversal anxiogenesis).

Figure 11

**Effect of long-term treatment and withdrawal from cocaine: Intervention with Example 20A**

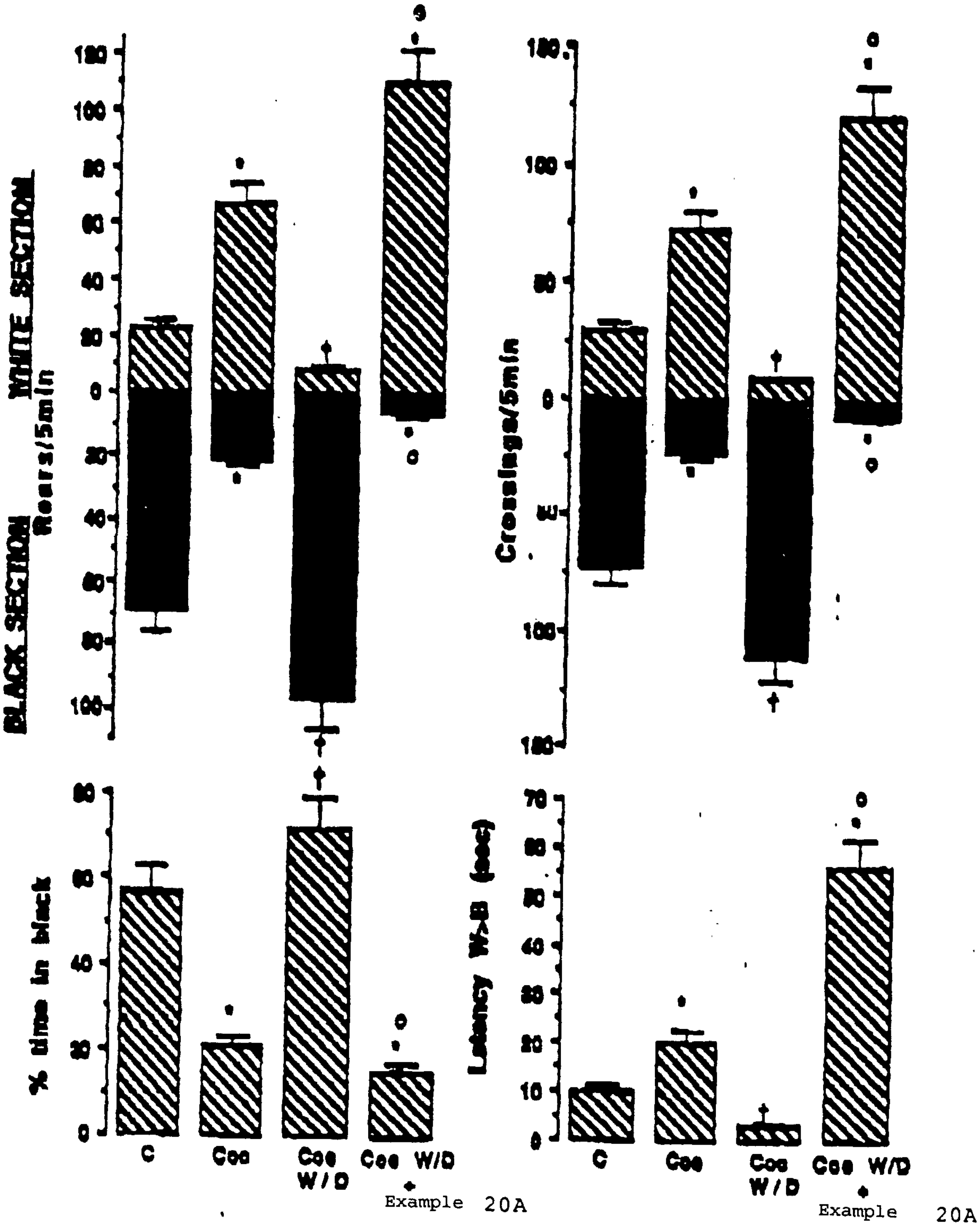


* P<0.001(anxiolysis). † P<0.001 (anxiogenesis)
°P<0.001 (reversal anxiogenesis).

Figure 12

# Fig. 13

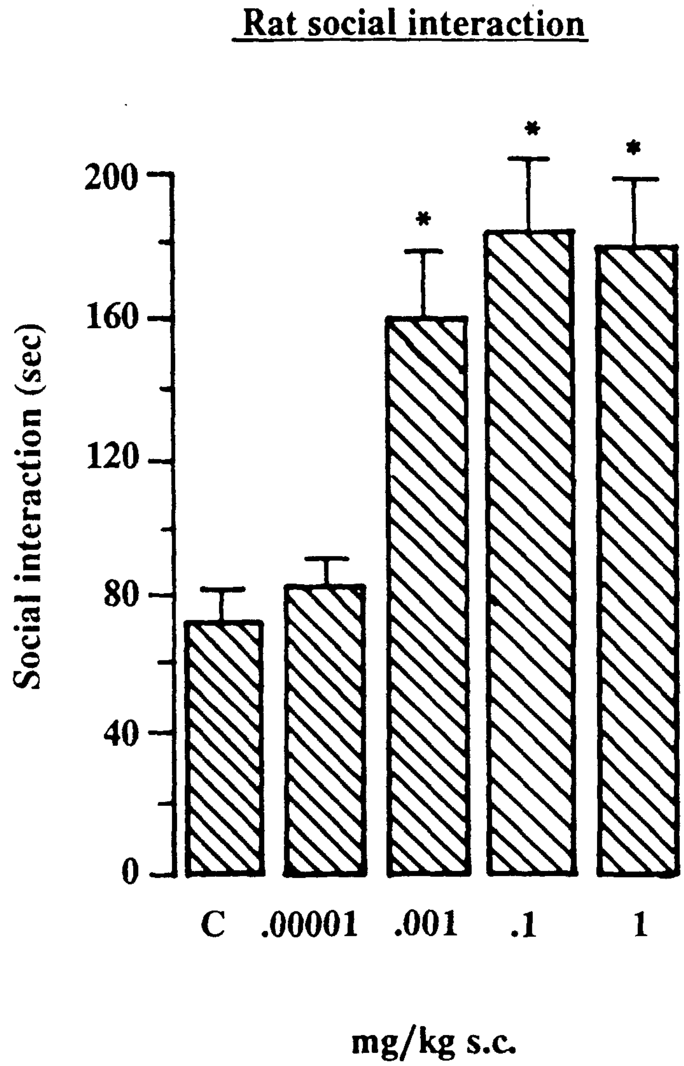


Example 20

C is control value

# Fig. 14

Rat elevated x-maze

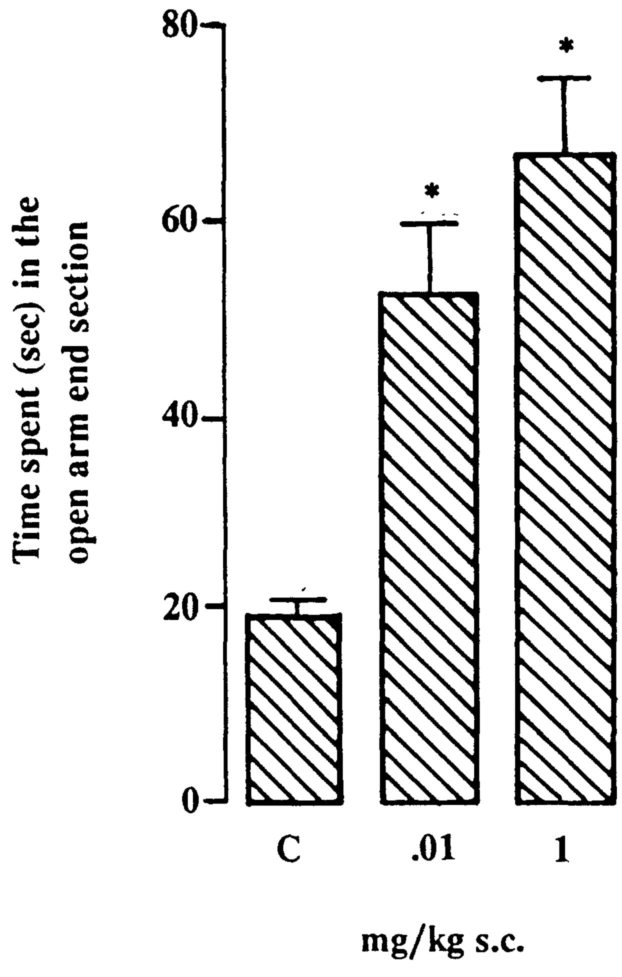


Example 20

C is control value

**Fig. 15**

**Effect of CCK-B receptor antagonists in the rat elevated X-maze**

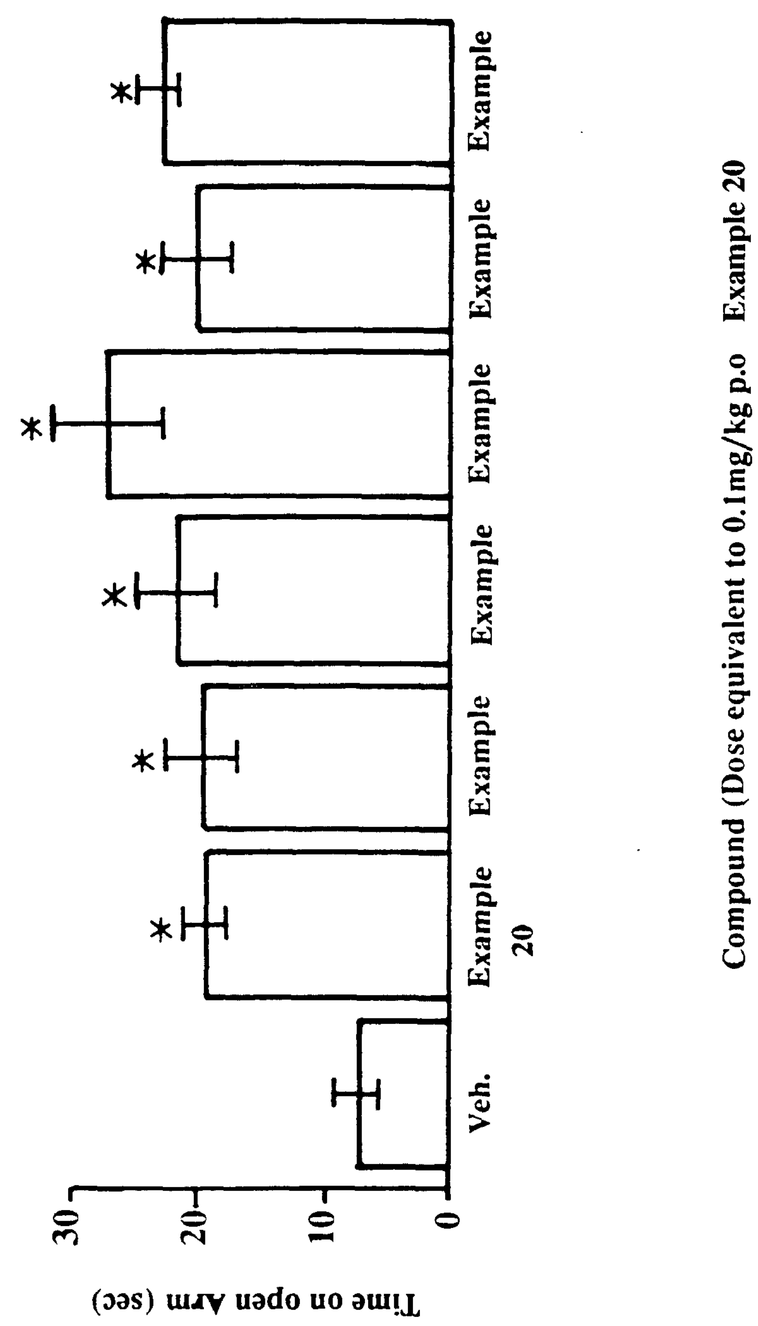

# Fig. 16

Depression of Flexor Reflex Response
to Compound 20 and Morphine